# EUROPEAN PATENT APPLICATION

(11) **EP 1 736 467 A1**
(43) Date of publication of application: **27.12.2006**
(21) Application number: 05728832.6
(22) Date of filing: 04.04.2005
(51) Int. Cl.: C07C 307/02, A61K 31/255, A61K 31/44, A61K 31/454, A61K 31/506, A61K 31/55, A61K 31/664, A61P 3/06, A61P 9/10, A61P 43/00, C07C 307/06, C07C 311/05, C07C 311/18, C07C 317/32, C07D 211/64, C07D 213/06, C07D 223/06, C07D 401/04, C07D 413/04, C07D 417/04, C07F 9/59

(54) **NOVEL SULFONAMIDE DERIVATIVE**

(30) Priority: 06.04.2004 JP 2004112503
(71) Applicant: Dainippon Sumitomo Pharma Co., Ltd., Osaka 541-8524 (JP)
(72) Inventor: BAN, Hitoshi, 33-94 Enoki-cho Suita-shi Osaka 564-0053 (JP); ASANO, Shigehiro, 33-94,Enoki-cho Suita-shi Osaka 564-0053 (JP)
(74) Representative: Vossius & Partner
(86) International application number: PCT/JP2005/006977
(87) International publication number: WO 2005/097738

(57) **Abstract**

A compound of the formula (1):

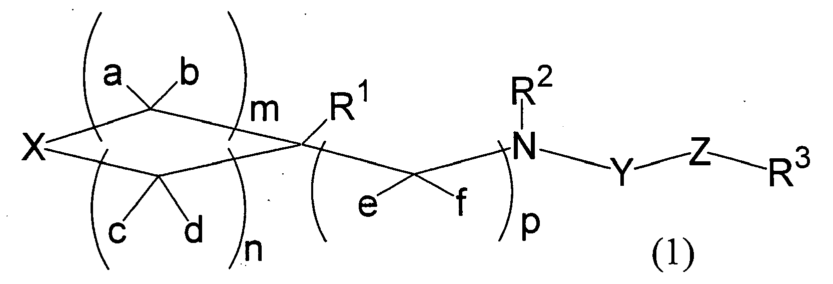

wherein
m, n and p is independently an integer of 0 to 4 with the proviso that 3 ≦ m + n ≦ 8; X is the formula: NR⁴, etc.; R¹, R³ and R⁴ are a substituted or unsubstituted aryl group, etc.; R² is a hydrogen atom, etc.; a, b, c, d, e and f are a hydrogen atom or a substituted or unsubstituted alkyl group, etc.; Y is the formula: -SO₂-, etc.; and Z is an oxygen atom, etc.;
or a prodrug thereof or a pharmaceutically acceptable salt of the same has an activity of potentiating an expression of a low density lipoprotein receptor and thus is useful as an agent for treating hyperlipidemia or arteriosclerosis.

## Description

### TECHNICAL FIELD

This invention relates to an agent for potentiating an expression of a low density lipoprotein (hereinafter, referred to as to LDL) receptor.

### PRIOR ART

A LDL receptor in a hepatic cell plays an important role in a regulation of a cholesterol level in blood. That is, it is well known that inhibition of cholesterol synthesis in a hepatic cell by 3-hydroxy-3-methylglutaryl Coenzyme A (HMG-CoA) reductase inhibitor leads to increase of expression of a LDL receptor indirectly, and as the result, increases an uptake of LDL from blood by a LDL receptor and thus results in lowering of a blood cholesterol, in particular a blood LDL cholesterol.

The HMG-CoA reductase inhibitor has been clinically highly appreciated as an agent being capable of lowering blood cholesterol level. However, this inhibitor does not have a sufficient efficacy to achieve a targeted low blood cholesterol level in a patient with a familial hypercholesterolemia exhibiting a high level of blood cholesterol or a patient with a history of a coronary artery disease, thus it has been desired to develop an agent for treating a hyperlipidemia having a more sharp effect on lowering a blood LDL cholesterol level, and is also useful in these patients.

HMG-CoA reductase inhibitor promotes a production of a LDL receptor indirectly via an inhibition of a cholesterol synthesis. On the other hand, the agent for potentiating an expression of a LDL receptor more directly is expected to exhibit a more sharp effect on lowering a blood cholesterol level.

It has been elucidated recently that a LDL receptor is expressed after undergoing a transcription and an addition of sugar chain, as a receptor protein having a function of an uptake of a LDL, thus exhibit a function of an uptake of a LDL in blood into cell (for example, see: Nature, 1990, 343 volume, page 425; Science, 1986, 232 volume, page 34). Thus, the agent having a new mechanism of action for lowering blood cholesterol can be developed by potentiating an expression of a LDL receptor protein.

4-cyano-4-(3-methoxyphenyl)-1-phenylpiperidine has been well-known as a piperidine derivative, but it is not found any specific description on the compound with respect to usefulness of lowering a level of a LDL cholesterol etc. (for example, see: The Journal of Heterocyclic Chemistry, 1983, vol.20, page 771).

PCT International Publication No. 03/03397 pamphlet discloses sulfonamide derivatives as a steroidsulfatase. PCT International Publication No. 03/063797 pamphlet and PCT International Publication No. 03/088908 pamphlet discloses sulfonamide derivatives as a potassium channel blocker.

### DISCLOSURE OF INVENTION

An object of the present invention is to provide a compound having an activity of potentiating an expression of a LDL receptor which can promote an expression of a LDL receptor protein, and is useful for a treatment of a hyperlipidemia, more specifically a hypercholesterolemia. An object of the present invention is also to provide a compound which is useful for controlling production of a LDL receptor, lowering of a level of blood LDL cholesterol, and prevention and/or treatment of arteriosclerosis.

The present inventors have intensively studied in order to achieve the above-mentioned objects, and have found that a compound of the following formula (1) of a sulfonamide derivative, a prodrug thereof, and a pharmaceutically acceptable salt of the same (hereinafter, referred to as "the present compound") exhibit a potent activity for potentiating an expression of a LDL receptor, and have accomplished the present invention. That is, the present invention relates to the following embodiments:
[1] An agent for potentiating an expression of a low density lipoprotein receptor comprising a compound of the formula (1): 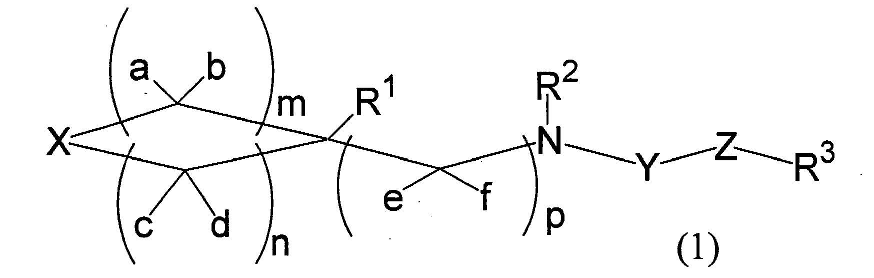 wherein
   m, n and p are independently an integer of 0 to 4 with the proviso that 3≦m + n≦8;
   X is
   (i) an oxygen atom,
   (ii) a sulfur atom,
   (iii) a group of the formula: NR⁴
      (R⁴ is a hydrogen atom; a substituted or unsubstituted alkyl group; a substituted or unsubstituted alkenyl group; a substituted or unsubstituted alkynyl group; a substituted or unsubstituted cycloalkyl group; a substituted or unsubstituted aryl group; a substituted or unsubstituted heteroaryl group; a substituted or unsubstituted arylalkyl group; a substituted or unsubstituted heteroarylalkyl group; a saturated heterocyclic group having 3 to 8 carbon atoms and one - NR¹⁰⁰- or one oxygen atom as ring-forming members (wherein a substituent may optionally present on the carbon atom of said saturated heterocyclic group); a group of the formula: -C(=O)R⁷ (R⁷ is a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, or a substituted or unsubstituted heteroarylalkyl group); a group of the formula: -C(=O)NR⁸R⁹ (R⁸ and R⁹ are independently a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, or a substituted or unsubstituted heteroarylalkyl group); a group of the formula: -C(=O)OR¹⁰ (R¹⁰ is a substituted or unsubstituted alkyl group, a substituted or unsubstituted alkenyl group, a substituted or unsubstituted alkynyl group, a substituted or unsubstituted cycloalkyl group, a substituted or unsubstituted aryl group, or a substituted or unsubstituted heteroaryl group); or a group of the formula: -SO₂R^{10a} (R^{10a} is a substituted or unsubstituted alkyl group, a substituted or unsubstituted alkenyl group, a substituted or unsubstituted alkynyl group, a substituted or unsubstituted cycloalkyl group, a substituted or unsubstituted aryl group, or a substituted or unsubstituted heteroaryl group)),
   (iv) a group of the formula: CR⁵R⁶
      (R⁵ and R⁶ are independently a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, or a substituted or unsubstituted heteroarylalkyl group, or alternatively R⁵ and R⁶ may combine to form a benzylidene group; or
   when R⁵ is a hydrogen atom, R⁶ is a group of the following:
   (1) the formula: -NR¹¹R¹² (R¹¹ and R¹² are independently a hydrogen atom; a substituted or unsubstituted alkyl group; a substituted or unsubstituted alkenyl group; a substituted or unsubstituted alkynyl group; a substituted or unsubstituted cycloalkyl group; a substituted or unsubstituted aryl group; a substituted or unsubstituted heteroaryl group; a substituted or unsubstituted arylalkyl group; a substituted or unsubstituted heteroarylalkyl group; a group of the formula: -C(=O)R¹⁰¹; a group of the formula: -SO₂-R¹⁰²; or a saturated heterocyclic group having 3 to 8 carbon atoms and one - NR¹⁰⁰- or one oxygen atom as ring-forming members (wherein a substituent may optionally present on the carbon atom of said saturated heterocyclic group); or alternatively R¹¹ and R¹² may combine together with the adjacent nitrogen atom to form a saturated heterocyclic group having 3 to 8 carbon atoms and optionally further one -NR¹⁰⁰- or one oxygen atom as ring-forming members (wherein a substituent may optionally present on the carbon atom of said saturated heterocyclic group));
   (2) a group of the formula: -OR¹³ (R¹³ is a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, or a substituted or unsubstituted heteroarylalkyl group); or
   (3) a group of the formula: -SR¹⁴ (R¹⁴ is a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, or a substituted or unsubstituted heteroarylalkyl group);
      R¹, R² and R³ are independently a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylcarbonyl group, a substituted or unsubstituted heteroarylcarbonyl group, a substituted or unsubstituted arylsulfonyl group, a substituted or unsubstituted heteroarylsulfonyl group, a substituted or unsubstituted arylalkyl group, or a substituted or unsubstituted heteroarylalkyl group;
      Y is a group of the formula: -SO₂-, a group of the formula: - P(O)OR¹⁵- (R¹⁵ is a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, or a substituted or unsubstituted heteroarylalkyl group), or a group of the formula: -C(=O)-;
   Z is
   (i) an oxygen atom,
   (ii) a sulfur atom,
   (iii) a group of the formula: -NR¹⁶ (R¹⁶ is a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, a substituted or unsubstituted heteroarylalkyl group, a group of the formula: -C(=O)R¹⁰¹, or a group of the formula: -C(=O)OR¹⁰³), or
   (iv) a group of the formula: -(CH₂)_{q}- (q is an integer of 1 to 4),
   with the proviso that when Z is a group of the formula: -NR¹⁶, then R³ and R¹⁶ may combine together with the adjacent nitrogen atom to form a saturated heterocyclic group having 2 to 8 carbon atoms and optionally further one oxygen atom as ring-forming members (wherein a substituent may optionally present on the carbon atom of said saturated heterocyclic group);
   a, b, c, d, e and f are
   (i) the same or different or alternatively independently when two or more exist, a hydrogen atom, a hydroxy group, a substituted or unsubstituted alkyl group, a substituted or unsubstituted alkoxy group, a substituted or unsubstituted alkoxycarbonyl group, a substituted or unsubstituted aryl group, a substituted or unsubstituted heteroaryl group, a substituted or unsubstituted arylcarbonyl group, a substituted or unsubstituted heteroarylcarbonyl group, a substituted or unsubstituted arylalkyl group, a substituted or unsubstituted heteroarylalkyl group, a substituted or unsubstituted arylalkyloxy group, or a substituted or unsubstituted heteroarylalkyloxy group;
   (ii) one or more of the combination selected from the group consisting of the combinations of a and b, c and d, and e and f may be independently an oxo group;
   (iii) the combination of e and f may be a thioxo group;
   (iv) a and c may combine to form an alkylene group;
   (v) when any two of a, b, c, d, e or f exist on the adjacent carbon atom, they may form a double bond, or alternatively
      when R¹, and any one of a, b, c, d, e or f exist on the adjacent carbon atom, they may form a double bond;
      each R¹⁰⁰, when two or more exist, is independently a hydrogen atom, a substituted or unsubstituted alkyl group, a substituted or unsubstituted aryl group, a substituted or unsubstituted heteroaryl group, a substituted or unsubstituted alkoxycarbonyl group, a substituted or unsubstituted arylalkyl group, or a substituted or unsubstituted heteroarylalkyl group;
      R¹⁰¹, R¹⁰² and R¹⁰³ may be the same or different or alternatively independently when two or more exist, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, or a substituted or unsubstituted arylalkyl group;
      with the proviso that when Y is a group of the formula: -C(=O), then -Z-R³ is not a substituted or unsubstituted alkoxy group] or a prodrug thereof or a pharmaceutically acceptable salt of the same as an active ingredient.
[2] The agent for potentiating an expression of a low density lipoprotein receptor as set forth in [1], wherein the agent is an agent for treating hyperlipidemia or arteriosclerosis.
[3] A compound of the formula (1): 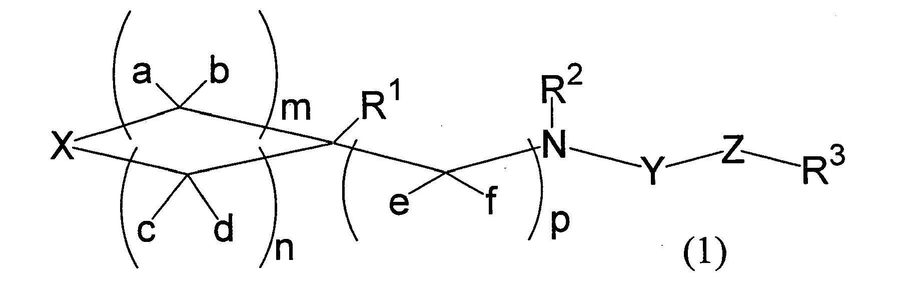 wherein
   m, n and p are independently an integer of 0 to 4 with the proviso that 3≦m + n≦8;
   X is
   (i) an oxygen atom,
   (ii) a sulfur atom,
   (iii) a group of the formula: NR⁴
      (R⁴ is a hydrogen atom; a substituted or unsubstituted alkyl group; a substituted or unsubstituted alkenyl group; a substituted or unsubstituted alkynyl group; a substituted or unsubstituted cycloalkyl group; a substituted or unsubstituted aryl group; a substituted or unsubstituted heteroaryl group; a substituted or unsubstituted arylalkyl group; a substituted or unsubstituted heteroarylalkyl group; a saturated heterocyclic group having 3 to 8 carbon atoms and one - NR¹⁰⁰- or one oxygen atom as ring-forming members (wherein a substituent may optionally present on the carbon atom of said saturated heterocyclic group); a group of the formula: -C(=O)R⁷ (R⁷ is a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, or a substituted or unsubstituted heteroarylalkyl group); a group of the formula: -C(=O)NR⁸R⁹ (R⁸ and R⁹ are independently a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, or a substituted or unsubstituted heteroarylalkyl group); a group of the formula: -C(=O)OR¹⁰ (R¹⁰ is a substituted or unsubstituted alkyl group, a substituted or unsubstituted alkenyl group, a substituted or unsubstituted alkynyl group, a substituted or unsubstituted cycloalkyl group, a substituted or unsubstituted aryl group, or a substituted or unsubstituted heteroaryl group); or a group of the formula: -SO₂R^{10a} (R^{10a} is a substituted or unsubstituted alkyl group, a substituted or unsubstituted alkenyl group, a substituted or unsubstituted alkynyl group, a substituted or unsubstituted cycloalkyl group, a substituted or unsubstituted aryl group, or a substituted or unsubstituted heteroaryl group)),
   (iv) a group of the formula: CR⁵R⁶
      (R⁵ and R⁶ are independently a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, or a substituted or unsubstituted heteroarylalkyl group, or alternatively R⁵ and R⁶ may combine to form a benzylidene group; or
      when R⁵ is a hydrogen atom, R⁶ is a group of the following:
      (1) a group of the formula: -NR¹¹R¹² (R¹¹ and R¹² are independently a hydrogen atom; a substituted or unsubstituted alkyl group; a substituted or unsubstituted alkenyl group; a substituted or unsubstituted alkynyl group; a substituted or unsubstituted cycloalkyl group; a substituted or unsubstituted aryl group; a substituted or unsubstituted heteroaryl group; a substituted or unsubstituted arylalkyl group; a substituted or unsubstituted heteroarylalkyl group; a group of the formula: -C(=O)R¹⁰¹; a group of the formula: -SO₂-R¹⁰²; or a saturated heterocyclic group having 3 to 8 carbon atoms and one - NR¹⁰⁰- or one oxygen atom as ring-forming members (wherein a substituent may optionally present on the carbon atom of said saturated heterocyclic group); or alternatively R¹¹ and R¹² may combine together with the adjacent nitrogen atom to form a saturated heterocyclic group having 3 to 8 carbon atoms and optionally further one -NR¹⁰⁰- or one oxygen atom as ring-forming members (wherein a substituent may optionally present on the carbon atom of said saturated heterocyclic group));
      (2) a group of the formula: -OR¹³ (R¹³ is a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, or a substituted or unsubstituted heteroarylalkyl group); or
      (3) a group of the formula: -SR¹⁴ (R¹⁴ is a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, or a substituted or unsubstituted heteroarylalkyl group);
         R¹, R² and R³ are independently a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylcarbonyl group, a substituted or unsubstituted heteroarylcarbonyl group, a substituted or unsubstituted arylsulfonyl group, a substituted or unsubstituted heteroarylsulfonyl group, a substituted or unsubstituted arylalkyl group, or a substituted or unsubstituted heteroarylalkyl group;
         Y is a group of the formula: -SO₂-, a group of the formula: - P(O)OR¹⁵- (R¹⁵ is a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, or a substituted or unsubstituted heteroarylalkyl group), or a group of the formula: -C(=O)-;
   Z is
   (i) an oxygen atom,
   (ii) a sulfur atom,
   (iii) a group of the formula: -NR¹⁶ (R¹⁶ is a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, a substituted or unsubstituted heteroarylalkyl group, a group of the formula: -C(=O)R¹⁰¹, or a group of the formula: -C(=O)OR¹⁰³), or
   (iv) a group of the formula: -(CH₂)_{q}- (q is an integer of 1 to 4),
      with the proviso that when Z is a group of the formula: -NR¹⁶, then R³ and R¹⁶ may combine together with the adjacent nitrogen atom to form a saturated heterocyclic group having 2 to 8 carbon atoms and optionally further one oxygen atom as ring-forming members (wherein a substituent may optionally present on the carbon atom of said saturated heterocyclic group);
   a, b, c, d, e and f are
   (i) the same or different or alternatively independently when two or more exist, a hydrogen atom, a hydroxy group, a substituted or unsubstituted alkyl group, a substituted or unsubstituted alkoxy group, a substituted or unsubstituted alkoxycarbonyl group, a substituted or unsubstituted aryl group, a substituted or unsubstituted heteroaryl group, a substituted or unsubstituted arylcarbonyl group, a substituted or unsubstituted heteroarylcarbonyl group, a substituted or unsubstituted arylalkyl group, a substituted or unsubstituted heteroarylalkyl group, a substituted or unsubstituted arylalkyloxy group, or a substituted or unsubstituted heteroarylalkyloxy group;
   (ii) one or more of the combination selected from the group consisting of the combinations of a and b, c and d, and e and f may be independently an oxo group;
   (iii) the combination of e and f may be a thioxo group;
   (iv) a and c may combine to form an alkylene group;
   (v) when any two of a, b, c, d, e or f exist on the adjacent carbon atom, they may form a double bond, or alternatively
      when R¹, and any of a, b, c, d, e or f exist on the adjacent carbon atom, they may form a double bond;
      each R¹⁰⁰, when two or more exist, is independently a hydrogen atom, a substituted or unsubstituted alkyl group, a substituted or unsubstituted aryl group, a substituted or unsubstituted heteroaryl group, a substituted or unsubstituted alkoxycarbonyl group, a substituted or unsubstituted arylalkyl group, or a substituted or unsubstituted heteroarylalkyl group;
      R¹⁰¹, R¹⁰² and R¹⁰³ may be the same or different or alternatively independently when two or more exist, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, or a substituted or unsubstituted arylalkyl group;
      with the proviso that when Y is a group of the formula: -C(=O), then -Z-R³ is not a substituted or unsubstituted alkoxy group] or a prodrug thereof or a pharmaceutically acceptable salt of the same.
[4] The compound as set forth in [3], wherein R¹ is a substituted or unsubstituted aryl group, a substituted or unsubstituted heteroaryl group, a substituted or unsubstituted arylcarbonyl group, a substituted or unsubstituted heteroarylcarbonyl group, a substituted or unsubstituted arylsulfonyl group, a substituted or unsubstituted heteroarylsulfonyl group, a substituted or unsubstituted arylalkyl group, or a substituted or unsubstituted heteroarylalkyl group, or a prodrug thereof or a pharmaceutically acceptable salt of the same.
[5] The compound as set forth in [3], wherein R¹ is a substituted or unsubstituted phenyl group, a substituted or unsubstituted pyridyl group, a substituted or unsubstituted benzyl group, a substituted or unsubstituted benzoyl group, a substituted or unsubstituted pyridylmethyl group, a substituted or unsubstituted pyridylcarbonyl group, or a substituted or unsubstituted benzenesulfonyl group, or a prodrug thereof or a pharmaceutically acceptable salt of the same.
[6] The compound as set forth in [3], wherein R¹ is a substituted or unsubstituted phenyl group or a substituted or unsubstituted benzenesulfonyl group, or a prodrug thereof or a pharmaceutically acceptable salt of the same.
[7] The compound as set forth in [3], wherein R¹ is a substituted or unsubstituted phenyl group, or a prodrug thereof or a pharmaceutically acceptable salt of the same.
[8] The compound as set forth in [3], wherein R¹ is a hydrogen atom, and at least one of a, b, c, d, e and f are a substituted or unsubstituted aryl group, a substituted or unsubstituted heteroaryl group, a substituted or unsubstituted arylcarbonyl group, a substituted or unsubstituted heteroarylcarbonyl group, a substituted or unsubstituted arylalkyl group, or a substituted or unsubstituted heteroarylalkyl group, or a prodrug thereof or a pharmaceutically acceptable salt of the same.
[9] The compound as set forth in any one of [3] to [8], wherein X is a group of the formula: NR⁴, or a prodrug thereof or a pharmaceutically acceptable salt of the same.
[10] The compound as set forth in [9], wherein R⁴ is a substituted or unsubstituted aryl group or a substituted or unsubstituted heteroaryl group, or a prodrug thereof or a pharmaceutically acceptable salt of the same.
[11] The compound as set forth in [9], wherein R⁴ is a phenyl group, a pyridyl group, or a pyrimidinyl group, and these group may be optionally substituted, or a prodrug thereof or a pharmaceutically acceptable salt of the same.
[12] The compound as set forth in any one of [3] to [8], wherein X is a group of the formula: CR⁵R⁶, or a prodrug thereof or a pharmaceutically acceptable salt of the same.
[13] The compound as set forth in [12], wherein R⁵ is a hydrogen atom and R⁶ is a group of the formula: -NR¹¹R¹², or a prodrug thereof or a pharmaceutically acceptable salt of the same.
[14] The compound as set forth in [13], wherein R¹¹ and R¹² are independently a hydrogen atom; a substituted or unsubstituted alkyl group; a substituted or unsubstituted aryl group; a substituted or unsubstituted heteroaryl group; a substituted or unsubstituted arylalkyl group; a substituted or unsubstituted heteroarylalkyl group; or a saturated heterocyclic group having 3 to 8 carbon atoms and one - NR¹⁰⁰- as ring-forming members (R¹⁰⁰ is a hydrogen atom, a substituted or unsubstituted alkyl group, a substituted or unsubstituted aryl group, a substituted or unsubstituted heteroaryl group, a substituted or unsubstituted alkoxycarbonyl group, a substituted or unsubstituted arylalkyl group, or a substituted or unsubstituted heteroarylalkyl group) or one oxygen atom (wherein a substituent may optionally present on the carbon atom of said saturated heterocyclic group), or a prodrug thereof or a pharmaceutically acceptable salt of the same.
[15] The compound as set forth in [14], wherein R¹¹ is a saturated heterocyclic group having as ring-forming members 3 to 8 carbon atoms and one -NR¹⁰⁰- (R¹⁰⁰ is a hydrogen atom, a substituted or unsubstituted alkyl group, a substituted or unsubstituted aryl group, or a substituted or unsubstituted arylalkyl group) or one oxygen atom (wherein a substituent may optionally present on the carbon atom of said saturated heterocyclic group), or a prodrug thereof or a pharmaceutically acceptable salt of the same.
[16] The compound as set forth in [14], wherein R¹¹ is a substituted or unsubstituted arylalkyl group, or a prodrug thereof or a pharmaceutically acceptable salt of the same.
[17] The compound as set forth in any one of [3] to [16], wherein m is 2 or 3 and n is 2, or a prodrug thereof or a pharmaceutically acceptable salt of the same.
[18] The compound as set forth in [17] wherein m is 2, or a prodrug thereof or a pharmaceutically acceptable salt of the same.
[19] The compound as set forth in any one of [3] to [16], wherein R¹ is a hydrogen atom, and one of a, b, c and d is a substituted or unsubstituted aryl group, a substituted or unsubstituted heteroaryl group, a substituted or unsubstituted arylcarbonyl group, a substituted or unsubstituted heteroarylcarbonyl group, a substituted or unsubstituted arylalkyl group, or a substituted or unsubstituted heteroarylalkyl group, or a prodrug thereof or a pharmaceutically acceptable salt of the same.
[20] The compound as set forth in [19], wherein m is 3 and n is 1, or a prodrug thereof or a pharmaceutically acceptable salt of the same.
[21] The compound as set forth in any one of [3] to [16], wherein R¹ is a hydrogen atom, and a partial structural formula (2): 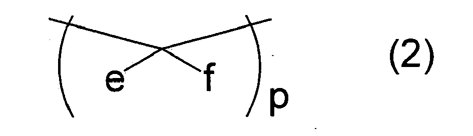 of formula (1) is the group of any the following formula: 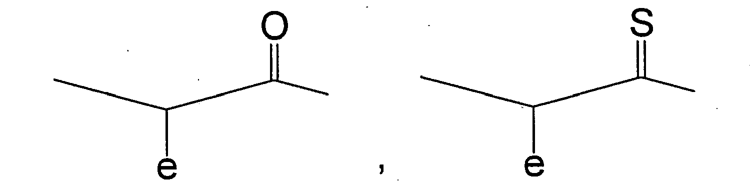 wherein e is a substituted or unsubstituted aryl group, a substituted or unsubstituted heteroaryl group, a substituted or unsubstituted arylcarbonyl group, a substituted or unsubstituted heteroarylcarbonyl group, a substituted or unsubstituted arylalkyl group, or a substituted or unsubstituted heteroarylalkyl group, or a prodrug thereof or a pharmaceutically acceptable salt of the same.
[22] The compound as set forth in any one of [3] to [16], wherein a and c may combine to form an alkylene group, or a prodrug thereof or a pharmaceutically acceptable salt of the same.
[23] The compound as set forth in any one of [3] to [16], wherein p is 1, and e and f combine to form a thioxo group, or a prodrug thereof or a pharmaceutically acceptable salt of the same.
[24] The compound as set forth in any one of [3] to [16], wherein p is 0, 1, or 2, and e and f are independently a hydrogen atom or a substituted or unsubstituted alkyl group, or a prodrug thereof or a pharmaceutically acceptable salt of the same.
[25] The compound as set forth in [24], wherein p is 1, or a prodrug thereof or a pharmaceutically acceptable salt of the same.
[26] The compound as set forth in any one of [3] to [25], wherein R² is a hydrogen atom, or a prodrug thereof or a pharmaceutically acceptable salt of the same.
[27] The compound as set forth in any one of [3] to [26], wherein Y is a group of the formula: -SO₂- group, or a prodrug thereof or a pharmaceutically acceptable salt of the same.
[28] The compound as set forth in any one of [3] to [26], wherein Y is a group of the formula: -P(O)OR¹⁵-, or a prodrug thereof or a pharmaceutically acceptable salt of the same.
[29] The compound as set forth in [28], wherein R¹⁵ is an alkyl group, or a prodrug thereof or a pharmaceutically acceptable salt of the same.
[30] The compound as set forth in [28], wherein R¹⁵ is a substituted or unsubstituted aryl group or a substituted or unsubstituted heteroaryl group, or a prodrug thereof or a pharmaceutically acceptable salt of the same.
[31] The compound as set forth in [28], wherein R¹⁵ is a substituted or unsubstituted phenyl group, or a prodrug thereof or a pharmaceutically acceptable salt of the same.
[32] The compound as set forth in any one of [3] to [31], wherein Z is an oxygen atom, or a prodrug thereof or a pharmaceutically acceptable salt of the same.
[33] The compound as set forth in any one of [3] to [31], wherein Z is a group of the formula: -NR¹⁶, or a prodrug thereof or a pharmaceutically acceptable salt of the same.
[34] The compound as set forth in [33], wherein R¹⁶ is a hydrogen atom, a substituted or unsubstituted alkyl group, a substituted or unsubstituted cycloalkyl group, a substituted or unsubstituted aryl group, a substituted or unsubstituted heteroaryl group, a substituted or unsubstituted arylalkyl group, a substituted or unsubstituted heteroarylalkyl group, a group of the formula: -C(=O)R¹⁰¹ or a group of the formula: -C(=O)OR¹⁰² (R¹⁰¹ and R¹⁰² are independently a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, or a substituted or unsubstituted arylalkyl group); or alternatively R³ and R¹⁶ combine together with the adjacent nitrogen atom to form a saturated heterocyclic group having 2 to 8 carbon atoms and optionally one oxygen atom as ring-forming members (wherein a substituent may optionally present on the carbon atom of said saturated heterocyclic group), or a prodrug thereof or a pharmaceutically acceptable salt of the same.
[35] The compound as set forth in [34], wherein R¹⁶ is a group of the formula: -C(=O)R¹⁰¹ and R¹⁰¹ is a substituted or unsubstituted alkyl group, a substituted or unsubstituted cycloalkyl group, a substituted or unsubstituted aryl group, a substituted or unsubstituted heteroaryl group, or a substituted or unsubstituted arylalkyl group, or a prodrug thereof or a pharmaceutically acceptable salt of the same.
[36] The compound as set forth in [34], wherein R¹⁶ is a group of the formula: -C(=O)OR¹⁰² and the R¹⁰² is a substituted or unsubstituted alkyl group, a substituted or unsubstituted cycloalkyl group, a substituted or unsubstituted aryl group, a substituted or unsubstituted heteroaryl group, or a substituted or unsubstituted arylalkyl group, or a prodrug thereof or a pharmaceutically acceptable salt of the same.
[37] The compound as set forth in any one of [3] to [36], wherein Z is a group of the formula: -(CH₂)_{q}-, or a prodrug thereof or a pharmaceutically acceptable salt of the same.
[38] The compound as set forth in [37], wherein q is 0 or 1, or a prodrug thereof or a pharmaceutically acceptable salt of the same.
[39] The compound as set forth in any one of [3] to [38], wherein R³ is a substituted or unsubstituted alkyl group, a substituted or unsubstituted aryl group, a substituted or unsubstituted heteroaryl group, a substituted or unsubstituted arylalkyl group, or a substituted or unsubstituted heteroarylalkyl group, or a prodrug thereof or a pharmaceutically acceptable salt of the same.
[40] The compound as set forth in any one of [3] to [38], wherein R³ is a hydrogen atom, a substituted or unsubstituted aryl group, or a substituted or unsubstituted heteroaryl group, or a prodrug thereof or a pharmaceutically acceptable salt of the same.
[41] The compound as set forth in any one of [3] to [38], wherein R³ is a hydrogen atom or a substituted or unsubstituted phenyl group, or a prodrug thereof or a pharmaceutically acceptable salt of the same.
[42] A pharmaceutical composition comprising a compound as set forth in any one of [3] to [41] or a prodrug thereof or a pharmaceutically acceptable salt of the same as an active ingredient.
[43] A method for treating hyperlipidemia or arteriosclerosis, which comprises administering a therapeutically effective amount of a compound as set forth in any one of [3] to [41], or a prodrug thereof or a pharmaceutically acceptable salt of the same to a patient in need thereof.
[44] A use of a compound as set forth in any one of [3] to [41], or a prodrug thereof or a pharmaceutically acceptable salt of the same in preparation of an agent for treating hyperlipidemia or arteriosclerosis.
[45] An agent for potentiating a low density lipoprotein receptor expression comprising a compound as set forth in any one of [3] to [41], or a prodrug thereof or a pharmaceutically acceptable salt of the same as an active ingredient.
[46] An agent for treating hyperlipidemia or arteriosclerosis comprising a compound as set forth in any one of [3] to [41], or a prodrug thereof or a pharmaceutically acceptable salt of the same as an active ingredient.
[47] A compound of the formula (1'): 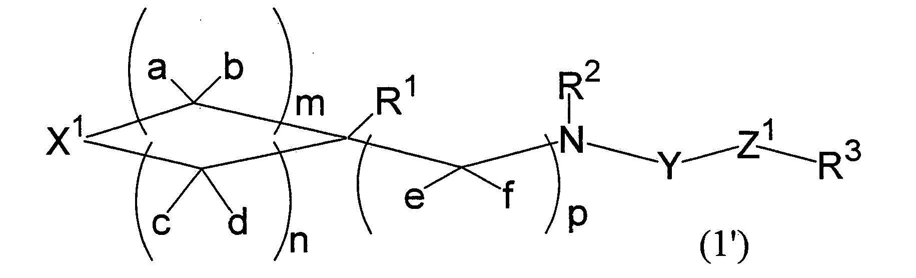 wherein
   m, n and p are independently an integer of 0 to 4 with the proviso that 3≦m + n≦8;
   X¹ is
   (1) an oxygen atom,
   (2) a sulfur atom,
   (3) a group of the formula: NR⁴
      (R⁴ is a hydrogen atom; a substituted or unsubstituted alkyl group; a substituted or unsubstituted alkenyl group; a substituted or unsubstituted alkynyl group; a substituted or unsubstituted cycloalkyl group; a substituted or unsubstituted aryl group; a substituted or unsubstituted heteroaryl group; a substituted or unsubstituted arylalkyl group; a substituted or unsubstituted heteroarylalkyl group; a group of the formula: -C(=O)R⁷ (R⁷ is a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, or a substituted or unsubstituted heteroarylalkyl group); a group of the formula: -C(=O)NR⁸R⁹ (R⁸ and R⁹ are independently a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, or a substituted or unsubstituted heteroarylalkyl group); a group of the formula: -C(=O)OR¹⁰ (R¹⁰ is a substituted or unsubstituted alkyl group, a substituted or unsubstituted alkenyl group, a substituted or unsubstituted alkynyl group, a substituted or unsubstituted cycloalkyl group, a substituted or unsubstituted aryl group, or a substituted or unsubstituted heteroaryl group); or a group of the formula: -SO₂R^{10a} (R^{10a} is a substituted or unsubstituted alkyl group, a substituted or unsubstituted alkenyl group, a substituted or unsubstituted alkynyl group, a substituted or unsubstituted cycloalkyl group, a substituted or unsubstituted aryl group, or a substituted or unsubstituted heteroaryl group)),
   (4) a group of the formula: CR⁵R⁶
      (R⁵ and R⁶ are independently a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, or a substituted or unsubstituted heteroarylalkyl group; or
      when R⁵ is a hydrogen atom, R⁶ is a group of the formula: -NR¹¹R¹² (R¹¹ and R¹² are independently a hydrogen atom; a substituted or unsubstituted alkyl group; a substituted or unsubstituted alkenyl group; a substituted or unsubstituted alkynyl group; a substituted or unsubstituted cycloalkyl group; a substituted or unsubstituted aryl group; a substituted or unsubstituted heteroaryl group; a substituted or unsubstituted arylalkyl group; a substituted or unsubstituted heteroarylalkyl group); a group of the formula: -OR¹³ (R¹³ is a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, or a substituted or unsubstituted heteroarylalkyl group); or a group of the formula: - SR¹⁴ (R¹⁴ is a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, or a substituted or unsubstituted heteroarylalkyl group) ;
      R¹, R² and R³ are independently a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylcarbonyl group, a substituted or unsubstituted heteroarylcarbonyl group, a substituted or unsubstituted arylsulfonyl group, a substituted or unsubstituted heteroarylsulfonyl group, a substituted or unsubstituted arylalkyl group, or a substituted or unsubstituted heteroarylalkyl group;
      Y is a group of the formula: -SO₂-, a group of the formula: -
      P(O)OR¹⁵- (R¹⁵ is a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, or a substituted or unsubstituted heteroarylalkyl group), or a group of the formula: -C(=O)-;
      Z¹ is an oxygen atom, a sulfur atom, or a group of the formula: -
      NR¹⁶ (R¹⁶ is a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, or a substituted or unsubstituted heteroarylalkyl group), or a group of the formula: -(CH₂)_{q}- (q is an integer of 1 to 4),
      a, b, c, d, e and f are
      (a) the same or different or alternatively independently when two or more exist, a hydrogen atom, a hydroxy group, a substituted or unsubstituted alkyl group, a substituted or unsubstituted alkoxy group, a substituted or unsubstituted alkoxycarbonyl group, a substituted or unsubstituted aryl group, a substituted or unsubstituted heteroaryl group, a substituted or unsubstituted arylcarbonyl group, a substituted or unsubstituted heteroarylcarbonyl group, a substituted or unsubstituted arylalkyl group, a substituted or unsubstituted heteroarylalkyl group, a substituted or unsubstituted arylalkyloxy group, or a substituted or unsubstituted heteroarylalkyloxy group;
      (b) one or more of the combination selected from the group consisting of the combinations of a and b, c and d, and e and f may be independently an oxo group;
      (c) the combination of e and f may be a thioxo group;
      (d) a and c may combine to form an alkylene group;
      (e) when any two of a, b, c, d, e or f exist on the adjacent carbon atom, they may form a double bond, or alternatively
         when R¹, and any of a, b, c, d, e or f exist on the adjacent carbon atom, they may form a double bond;
   with the proviso that
   (I) when Y is a group of the formula: -C(=O), then -Z-R³ is not a substituted or unsubstituted alkoxy group;
   (II) when R² is a hydrogen atom, Y is -SO₂-, Z is -(CH₂)_{q}-, and a partial structural formula (2): 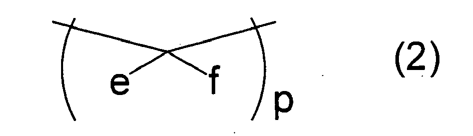 in the formula (1) is the formula (3): 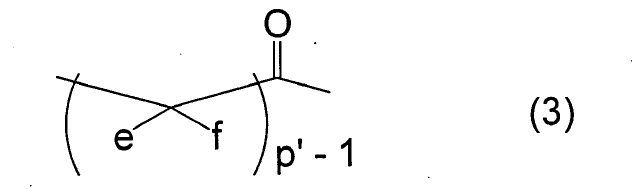 (in the formula (3), p' is an integer of 1 to 4),
      and further X is
      1) a group of the formula: NR⁴, or
      2) a group of the formula: CR⁵R⁶ (R⁶ is a substituted or unsubstituted alkyl group, a substituted or unsubstituted aryl group, a substituted or unsubstituted heteroaryl group, or a group of the formula: -NR¹¹R¹²), then R¹ is not a hydrogen atom, an alkyl group, a phenyl group;
      with the proviso that
   (III) when R¹ is a substituted or unsubstituted alkyl group, a substituted or unsubstituted alkenyl group, a substituted or unsubstituted cycloalkyl group, a substituted or unsubstituted aryl group, a substituted or unsubstituted heteroaryl group, a substituted or unsubstituted arylalkyl group, or a substituted or unsubstituted heteroarylalkyl group, then
      1) the combination of e and f is a thioxo group;
      2) a and c combine to form an alkylene group; or
      3) Y is a group of the formula: -P(O)OR¹⁵-] or a prodrug thereof or a pharmaceutically acceptable salt of the same.
[48] A compound as set forth in [47] wherein R¹ is a substituted phenyl group or a substituted or unsubstituted benzenesulfonyl group, or a prodrug thereof or a pharmaceutically acceptable salt of the same.
[49] A compound as set forth in [47] wherein R¹¹ is a hydrogen atom, and R¹² is a substituted or unsubstituted alkyl group, a substituted or unsubstituted aryl group, a substituted or unsubstituted heteroaryl group, a substituted or unsubstituted arylalkyl group, or a substituted or unsubstituted heteroarylalkyl group, or a prodrug thereof or a pharmaceutically acceptable salt of the same.
[50] A compound as set forth in [47] wherein R¹¹ is a hydrogen atom, and R¹² is a substituted or unsubstituted aryl group, a substituted or unsubstituted heteroaryl group, a substituted or unsubstituted arylalkyl group, or a substituted or unsubstituted heteroarylalkyl group, or a prodrug thereof or a pharmaceutically acceptable salt of the same.
[51] A compound as set forth in [47] wherein R¹¹ is a hydrogen atom, and R¹² is a substituted or unsubstituted phenyl group, or a prodrug thereof or a pharmaceutically acceptable salt of the same.
[52] A compound as set forth in [47] wherein R³ is a substituted or unsubstituted aryl group or a substituted or unsubstituted heteroaryl group, or a prodrug thereof or a pharmaceutically acceptable salt of the same.
[53] A compound as set forth in [47] wherein R³ is a substituted or unsubstituted phenyl group, or a prodrug thereof or a pharmaceutically acceptable salt of the same.

The compound of this invention has an activity of potentiating an expression of a LDL receptor and an activity for controlling production of a LDL receptor, and thus is useful for lowering of a blood LDL cholesterol level and prevention and treatment of arteriosclerosis.

### BRIEF DECRIPTION OF THE DRAWINGS

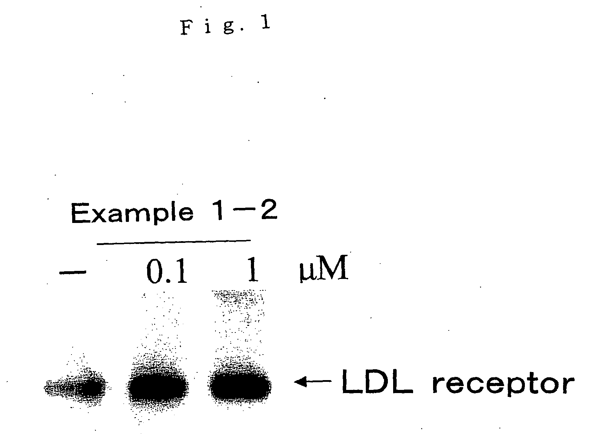

Figure 1 shows a result of an immunoblotting analysis in an experiment for an effect of potentiating an expression of a LDL receptor by the compound in Example 1-2. The each lane represents the result of a control, and test compound at 0.1 µM and 1 µM respectively in order from left. The arrow represents the positions of LDL receptor proteins.

### BEST MODE FOR CARRING OUT AN INVENTION

The term "potentiating an expression of a low density lipoprotein receptor " means an increase of an amount of expression of a LDL receptor protein and also is called as a rise of an expression of a LDL receptor or an upregulation of an expression of a LDL receptor.

Each group in the present invention is explained below. Unless defined otherwise, the definition for each group shall also be applied to where said group is a part of another group.

The alkyl group includes, for example, a straight chain or branched chain alkyl group having 1 to 10 carbon atoms, specifically such as methyl, ethyl, propyl, 2-propyl, butyl, 2-butyl, 2-methylpropyl, 1,1-dimethylethyl, pentyl, 3-pentyl, 3-methylbutyl, hexyl, 3-hexyl, 4-methylpentyl, 4-heptyl, octyl, 4-octyl, or decyl, etc. Preferred alkyl group is a straight chain or branched chain alkyl group having 1 to 6 carbon atoms.

The alkenyl group includes, for example, a straight chain or branched chain alkenyl group having 2 to 10 carbon atoms, specifically such as vinyl, aryl, 2-propenyl, 2-methyl-2-propenyl, 2-butenyl, 3-butenyl, 3-methyl-2-butenyl, 4-pentenyl, 3-hexenyl, 3-ethyl-2-pentenyl, or 4-ethyl-3-hexenyl, etc. Preferred alkenyl group is a straight chain or branched chain alkyl group having 2 to 6 carbon atoms.

The alkynyl group includes, for example, a straight chain or branched chain alkenyl group having 3 to 10 carbon atoms, specifically such as 2-propynyl, 3-butynyl, 4-pentynyl, 3-hexynyl, 5-methyl-2-hexynyl, or 6-methyl-4-heptynyl, etc. Preferred alkynyl group is a straight chain or branched chain alkyl group having 3 to 6 carbon atoms.

The alkoxy group includes, for example, the group that an oxygen atom binds to the above alkyl group. Preferred alkoxy group is an alkoxy group having 1 to 6 carbon atoms.

The alkoxycarbonyl group includes, for example, the group that the above alkyl group binds to the oxygen atom moiety in the group of the formula: -C(=O)O-. Preferred alkoxycarbonyl group is an alkoxycarbonyl group having 2 to 7 carbon atoms.

The substituents of a substituted alkyl group, a substituted alkenyl group, and a substituted alkynyl group may be one or more ones which are the same or different, and the substituents are, for example, a substituted or unsubstituted aryl group, a substituted or unsubstituted heteroaryl group, a substituted or unsubstituted cycloalkyl group, a halogen atom, a cyano group, a hydroxy group, an alkoxy group, an alkanoyl group, an alkanoyloxy group, an amino group, a monoalkylamino group, a dialkylamino group, a carbamoyl group, an alkylaminocarbonyl group, a dialkylaminocarbonyl group, an alkoxycarbonylamino group, a carboxy group, an alkoxycarbonyl group, an alkylthio group, an alkylsulfinyl group, an alkylsulfonyl group, an alkanoylamino group, or an alkylsulfonylamide group, etc.

More specifically, a substituted alkyl group includes, for example, trifluoromethyl group, 2,2,2-trifluoroethyl group, or methoxymethyl group, etc.

The halogen atom includes, for example, fluorine atom, chlorine atom, bromine atom, or iodine atom.

The alkanoyl group includes, for example, an alkanoyl group having 1 to 6 carbon atoms, such as formyl, acetyl, or propanoyl.

The cycloalkyl group includes, for example, a cycloalkyl group having 3 to 8 carbon atoms, specifically such as cyclopropyl, cyclobutyl, cyclopentyl, cyclohexyl, cycloheptyl, or cyclooctyl, etc. Preferred cycloalkyl group is a cycloalkyl group having 3 to 6 carbon atoms.

The substituent of a substituted cycloalkyl group may be one or more ones which are the same or different, and the substituents are, for example, an alkyl group, a halogen atom, a cyano group, a hydroxy group, an alkoxy group, an alkanoyl group, an alkanoyloxy group, an amino group, a monoalkylamino group, a dialkylamino group, a carbamoyl group, an alkylaminocarbonyl group, a dialkylaminocarbonyl group, an alkoxycarbonylamino group, a carboxy group, an alkoxycarbonyl group, an alkylthio group, an alkylsulfinyl group, an alkylsulfonyl group, an alkanoylamino group, or an alkyl sulfonamide group, etc. A preferred halogen atom as a substituent of the substituted cycloalkyl group is fluorine atom.

The aryl group includes, for example, an aryl group having less than 10 carbon atoms, such as phenyl group, and naphthyl group, etc.

The heteroaryl group includes, for example, a 5- to 6-membered monocyclic group having 1 to 2 nitrogen atoms, a 5- to 6-membered monocyclic group having 1 to 2 nitrogen atoms and one oxygen atom or one sulfur atom, a 5-membered monocyclic group having one oxygen atom or one sulfur atom, or a bicyclic group having 1 to 4 nitrogen atoms, formed by fusing a 6-membered ring and a 5- or 6-membered ring, specifically such as 2-pyridyl, 3-pyridyl, 4-pyridyl, 2-pyrimidinyl, 4-pyrimidinyl, 5-pyrimidinyl (collectively referred as to pyridyl or pyrimidinyl solely), 2-thienyl, 3-thienyl, 3-oxadiazolyl, 4-thiadiazolyl, 1-imidazolyl, 2-imidazolyl, 2-thiazolyl, 3-isothiazolyl, 2-oxazolyl, 3-isoxazolyl, 2-furyl, 3-furyl, 3-pyrrolyl, 2-quinolyl, 3-quinolyl, 8-quinolyl, 2-quinazolinyl, 8-purinyl, 1,3-benzoxazol-2-yl, 2-benzofuranyl, 3-indolyl, 1-triazole, 2-triazole, or 4-triazole, etc. A preferred heteroaryl group is, for example, pyridyl or pyrimidinyl, etc.

The substituents of a substituted aryl group, a substituted phenyl group, a substituted heteroaryl group, a substituted pyridyl group, a substituted pyridylmethyl group, a substituted pyridylcarbonyl group, a substituted pyrimidinyl group, a substituted benzyl group, a substituted benzoyl group, and a substituted benzenesulfonyl group may be one or more ones which are the same or different, and the substituents are, for example, a halogen atom, a cyano group, a nitro group, a hydroxy group, a methylenedioxy group, an ethylenedioxy group, an alkyl group, an alkyl group substituted with one or more halogen atom (for example, trifluoromethyl group, etc.), an alkoxy group, an alkoxy group substituted with a hydroxy group, an alkoxy group substituted with an alkoxy group, an alkoxy group substituted with one or more halogen atom (for example, trifluoromethoxy group, etc.), a benzyloxy group, an alkanoyl group, an alkanoyloxy group, an amino group, a monoalkylamino group, a dialkylamino group, an alkoxycarbonylamino group, a carbamoyl group, an alkylaminocarbonyl group, a dialkylaminocarbonyl group, a carboxy group, an alkoxycarbonyl group, an alkylthio group, an alkylsulfinyl group, an alkylsulfonyl group, an alkanoylamino group, an alkylsulfonylamide group, or a group of the formula: -E¹-E²-A {E¹ is an oxygen atom or a single bond; E² is a divalent hydrocarbon group having 1 to 8 carbon atoms and optionally containing an unsaturated bond, or a single bond; A is a hydrogen atom, a hydroxy group, a carboxy group, an alkoxycarbonyl group, a benzyloxycarbonyl group, a halogen atom, a cyano group, a benzyloxy group, an alkoxy group, an alkanoyloxy group, an alkylthio group, an alkylsulfinyl group, an alkylsulfonyl group, an alkyl-substituted or unsubstituted benzenesulfonyloxy group, an alkanoylamino group, an alkoxycarbonylamino group, an alkylsulfonylamide group, a phtalimido group, a cycloalkyl group, an aryl group (which may be optionally substituted with a halogen atom, an alkyl group, or an alkoxy group), a heteroaryl group (whch may be optionally substituted with a halogen atom, an alkyl group or an alkoxy group), or the group of the formula: -NR¹⁷R¹⁸ (R¹⁷ and R¹⁸ are independently a hydrogen atom, an alkyl group, an alkoxyalkyl group, a cycloalkyl group, an alkoxycarbonyl group, an arylalkyl group or a heteroarylalkyl group, or alternatively R¹⁷ and R¹⁸ may combine together with the adjacent nitrogen atom to form a saturated cyclic amino group having as ring-forming members 3 to 8 carbon atoms and optionally further one -NR¹⁹- (R¹⁹ is a hydrogen atom, an alkyl group, a phenyl group, an alkoxycarbonyl group, or a benzyl group) or one oxygen atom (wherein a substituent may optionally present on the carbon atom of said saturated heterocyclic group)), or a group of the formula: -C(=O)NR¹⁷R¹⁸ (R¹⁷ and R¹⁸ are defmed above)}, etc.

The substituents of an aryl moiety or a heteroaryl moiety in a substituted arylalkyl group, a substituted heteroarylalkyl group, a substituted arylalkyloxy group, a substituted heteroarylalkyloxy group, a substituted arylcarbonyl group, a substituted heteroarylcarbonyl group, a substituted arylsulfonyl group, and a substituted heteroaryl sulfonyl group includes the same one as defined above.

A preferred arylalkyl group is, for example, benzyl group, etc. A preferred heteroarylalkyl group includes, for example, pyridylmethyl group, etc. A preferred arylcarbonyl group is, for example, phenylcarbonyl group, etc. A preferred heteroarylcarbonyl group includes, for example, pyridylcarbonyl group, etc. A preferred arylsulfonyl group is, for example, benzenesulfonyl group, etc. A preferred heteroarylsulfonyl group is, for example, pyridylsulfonyl group, etc.

The alkylene group includes, for example, an alkylene group having 1 to 8 carbon atoms, specifically such as methylene, ethylene, trimethylene, or tetramethylene, etc. Preferred alkylene group is, for example, an alkylene group having 1 to 3 carbon atoms, such as methylene, ethylene, or trimethylene, etc.

The phrase "a and c combine to form an alkylene group" means specifically, for example, that the partial formula (4): 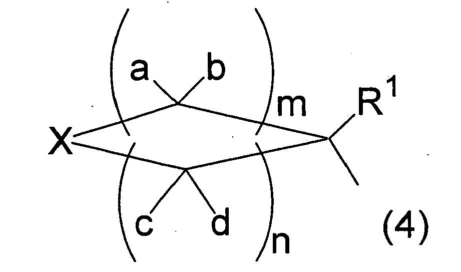 in the formula (1) is the following structural formulae: 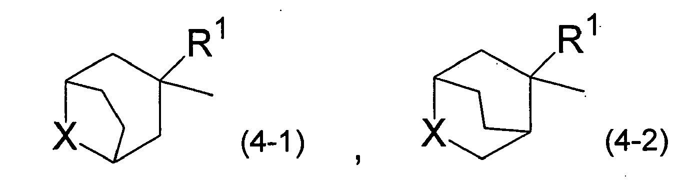 ,etc.

The divalent hydrocarbon group having 1 to 8 carbon atoms and optionally containing an unsaturated bond includes, for example, an alkylene chain such as methylene, ethylene, trimethylene, tetramethylene, pentamethylene, or hexamethylene, etc., an alkenylene chain such as propenylene or butenylene, etc., or an alkynylene chain such as ethynylene, propynylene or butynylene, etc. These groups may be a straight chain or a branched chain.

The saturated cyclic amino group having 3 to 8 carbon atoms as ring-forming members, which is formed by combining R¹¹ and R¹², or R¹⁷ and R¹⁸ together with the adjacent nitrogen atom, includes specifically, for example, 1-pyrolidinyl, 1-pipelidino, 1-pyperadinyl, morphorino, or 1-(4-methyl)pyperadinyl, etc.

In the above case of R¹¹ and R¹², the saturated heterocyclic group having 3 to 8 carbon atoms and one -NR¹⁰⁰- or one oxygen atom as ring-forming members, includes specifically, for example, piperidinyl, pyrolidinyl, tetrahydrofuranyl or tetrahydropyranyl, etc.

The saturated heterocyclic group having 2 to 8 carbon atoms and optionally one oxygen atom as ring-forming members, which is formed by combing R¹³ and R¹⁶ together with the adjacent nitrogen atom, includes specifically, for example, piperidinyl, pyrolidinyl, or morphorino, etc.

The substitutent on the carbon atom in the above saturated cyclic amino group or the saturated heterocyclic group includes, for example, a halogen atom, a substituted or unsubstituted alkyl group, a substituted or unsubstituted alkoxy group, a substituted or unsubstituted aryl group, a substituted or unsubstituted heteroaryl group, a substituted or unsubstituted arylalkyl group, a substituted or unsubstituted heteroarylalkyl group, etc.

When any two of R¹, a, b, c, d, e and f exist on the adjacent carbon atoms, one or more double bonds may form in the ring of the compound of the formula (1). Preferred compounds contain one double bond.

The "prodrug" includes a compound which can easily be hydrolyzed in the living body, and can reproduce the compound of the formula (1), and, for example, when the compound has a carboxyl group, it is a compound wherein the carboxyl group is replaced by an alkoxycarbonyl group, an alkylthiocarbonyl group, or an alkylaminocarbonyl group. When the compound has an amino group, the prodrug is, for example, a compound wherein said amino group is substituted by an alkanoyl group to form an alkanoylamino group, or substituted by an alkoxycarbonyl group to form an alkoxycarbonylamino group, or converted into an acyloxymethylamino group or a hydroxyamine, when said compound has an amino group. When said compound has a hydroxy group, the prodrug includes, for example, a compound wherein said hydroxy group is substituted by an alkanoyl group as described above to form an alkanoyloxy group, or converted into a phosphate ester or an acyloxymethyloxy group. The alkyl moiety of groups composing the prodrug may include the above-described alkyl groups, which may optionally be substituted with, for example, an alkoxy group having 1 to 6 carbon atoms, etc. The preferable examples are, for example, the compounds wherein a carboxyl group is converted into an alkoxycarbonyl group such as methoxycarbonyl, or a carboxyl group is substituted with an alkoxy group to convert into an alkoxycarbonyl group such as methoxymethoxycarbonyl, ethoxymethoxycarbonyl, 2-methoxyethoxycarbonyl, 2-methoxyethoxymethoxycarbonyl, or pivaloylmethoxycarbonyl.

The pharmaceutically acceptable salts includes, for example, a salt with an inorganic acid such as hydrochloric acid, hydrobromic acid, sulfuric acid, or phosphoric acid, etc., with an organic carboxylic acid such as formic acid, acetic acid, fumaric acid, maleic acid, oxalic acid, citric acid, malic acid, tartaric acid, aspartic acid, or glutamic acid, etc., with a sulfonic acid such as methanesulfonic acid, benzenesulfonic acid, p-toluenesulfonic acid, hydroxybenzenesulfonic acid, a dihydroxybenzenesulfonic acid, etc.; and with an alkali metal such as sodium or potassium, an alkaline earth metal such as calcium or magnesium, or ammonium, triethylamine, pyridine, picoline, ethanolamine, dicyclohexylamine, or N,N'-dibenzylethylenediamine, etc.

The compound of the present invention includes the asymmetric carbon and thus may optionally present as a stereoisomer. This invention also includes each of the stereisomer and mixture thereof.

The compound of the present invention may optionally present in a tautomer form. This invention also includes a tautomer thereof and the mixture of the same.

The compound of the present invention also includes the solvate thereof such as a hydrate and a solvate with ethanol, etc.

The compound of the above described formula (1), or a prodrug thereof or a pharmaceutically acceptable salt of the same can be administered either parenterally or orally. That is, these compounds can be formulated in liquid preparations such as solutions, emulsions, suspensions, etc., which can be administered by injection, and if necessary, buffering agents, solubilizers and isotonic agents may be added thereto. The present compounds can also be administered rectally in the form of a suppository. The present compounds can also be administered orally in the form of a conventional dosage form such as tablets (including sugar coated tablet), papers, granules, capsules (including soft gelatin capsules), syrups, and suspensions. These dosage forms can be formulated by mixing an active ingredient with conventional carriers, excipients, binding agents, lubricant, disintegrator or stabilizers in a conventional manner. Also if necessary, additive such as antiseptic, antioxidant, coloring agent or sweetening agent may be used.

The dosage and the frequency of administration of the present compounds may vary according to the conditions, ages, weights of the patients and the dosage form, etc., but when be administered by injection, the present compound can usually be administered in a dose of 0.1 to 100 mg per day in adult, once a day, or divided into several dosage units (for example, 2 to 4 dosage units). When administered orally, the present compound can be administered in a dose of 0.1 to 1000 mg (preferably 1 to 400 mg) per day, once a day, or divided into several dosage units (for example, 2 to 4 dosage units).

Examples of the compounds of the present invention are shown below, but are not limited thereto.

In the following formulae, the term "Me" means methyl group.

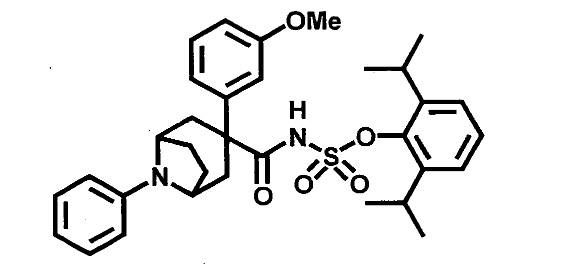

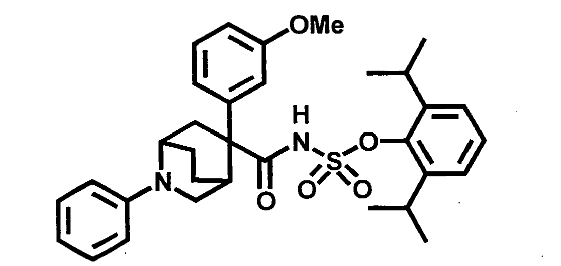

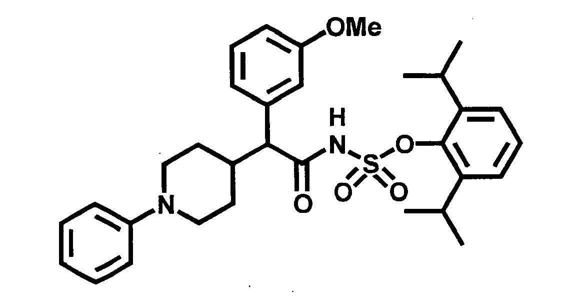

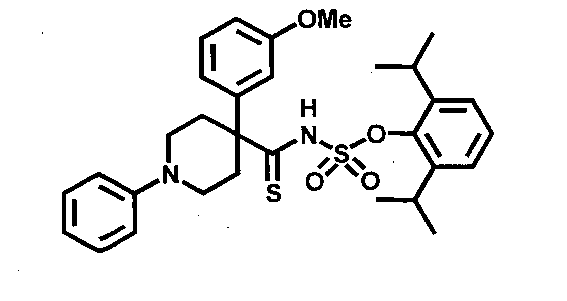

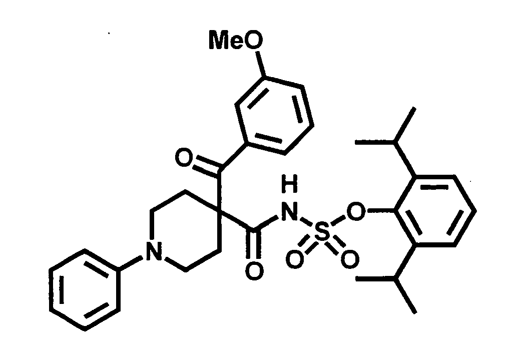

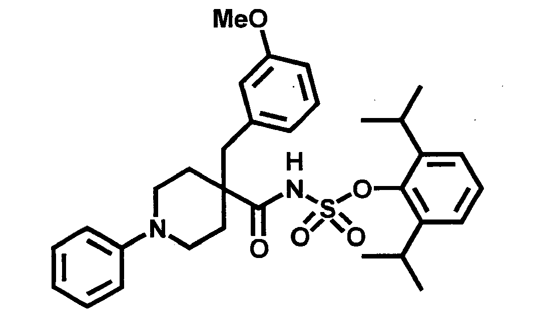

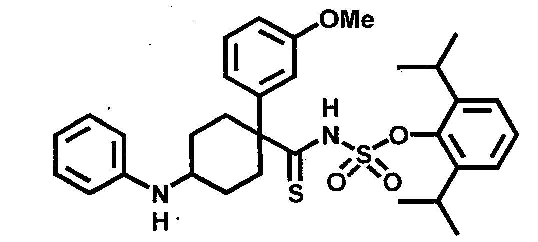

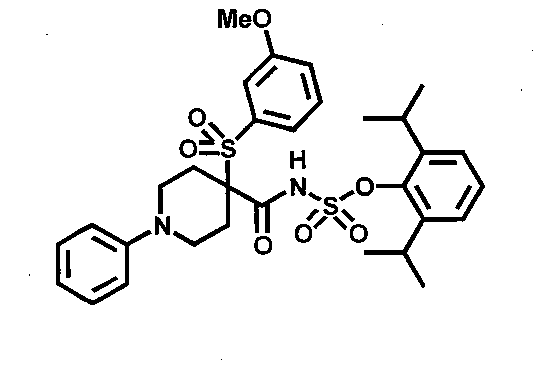

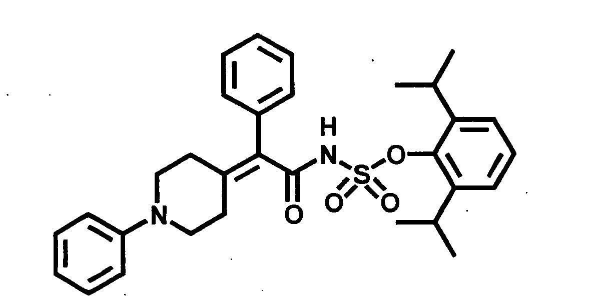

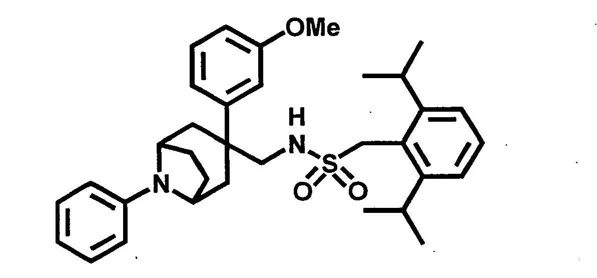

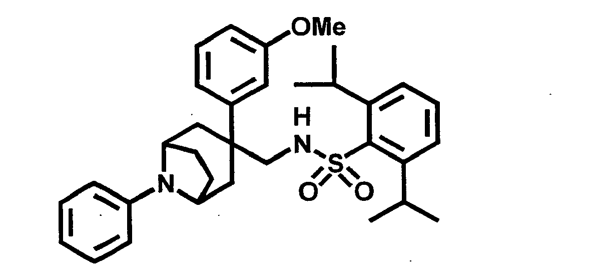

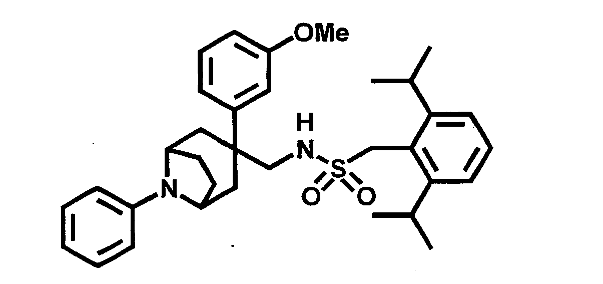

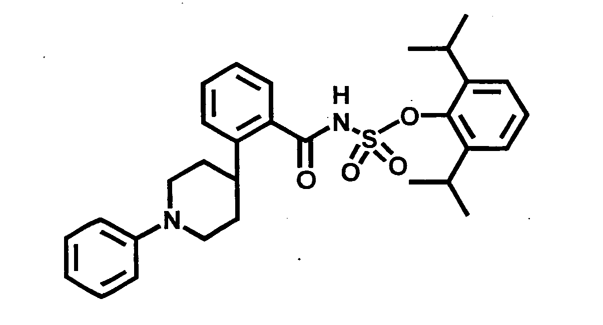

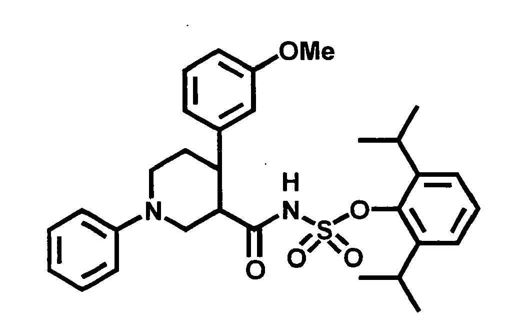

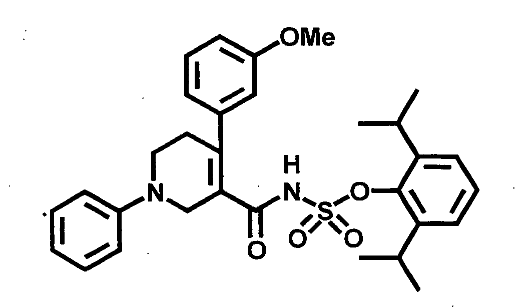

The compounds of the present invention can be prepared, for example, by the following method. When a starting compound in the following reaction has a reactive group such as an amino group, etc. as a substituent, the reaction can preferably be performed by protecting optionally these reactive group, and subjecting to a deprotection after completion of each reaction or a series of reactions, besides the case that describe specifically a use of a protecting group. The procedure for a protection and a deprotection may be performed by the methods described in literatures (for example, PROTECTIVE GROUPS IN ORGANIC SYNTHESIS, 3rd ed., JOHN WILEY & SONS, INC.: New York (1999), etc.).

### Procedure (A)

Among the present compounds, the compounds of the formulae (15), (16), (17), or (120) can be prepared, for example as follows: 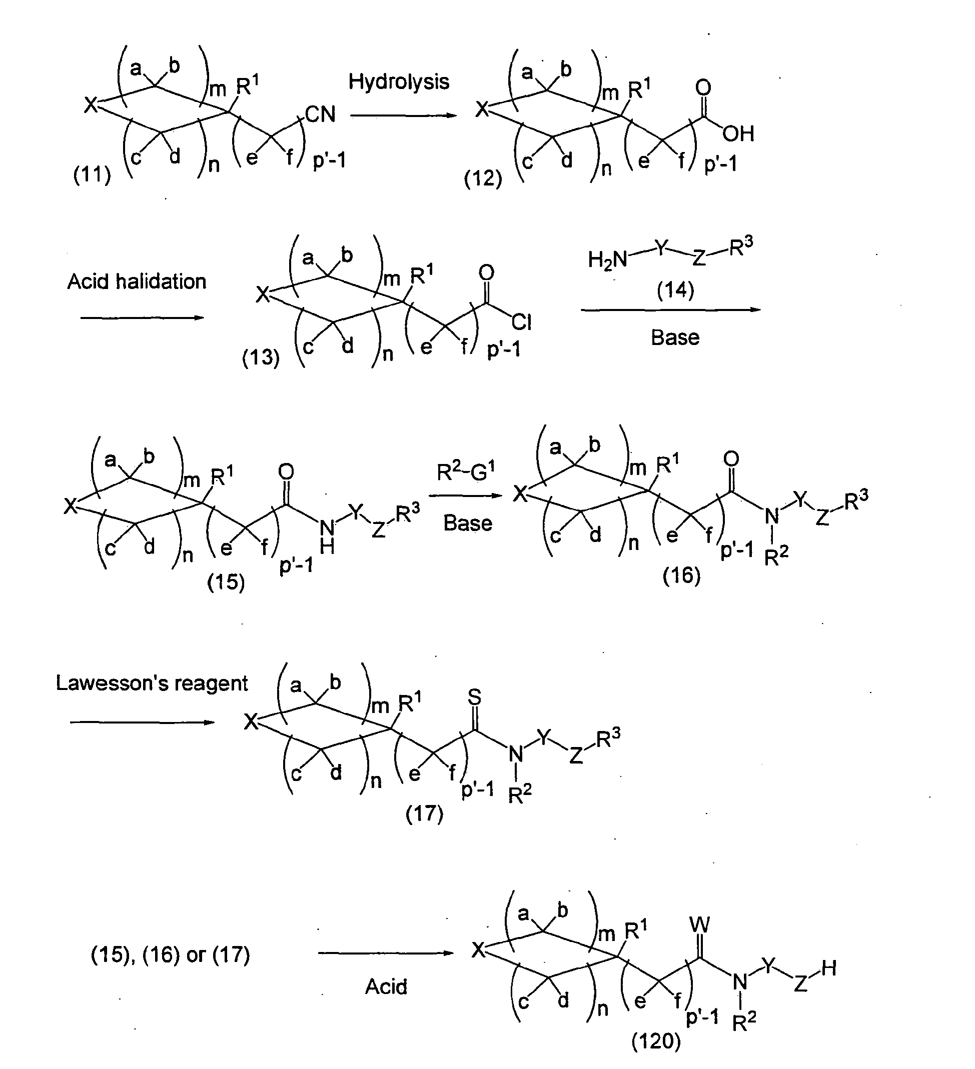 (wherein, G¹ is a leaving group such as an iodine atom, a bromine atom, a chlorine atom, or p-toluenesulfonyl group, etc.; p' is an integer of 1 to 4; a, b, c, d, e, f, X, Y, Z, m, n, R¹, R² and R³ are as defined above; W is an oxygen atom or a sulfur atom)

The compound of the formula (11) can be hydrolyzed, for example, with the acid such as 48% aqueous hydrobromic acid solution, etc. to convert into the compound of the formula (12).

Then the carboxy group in the compound of the formula (12) can be reacted with, for example, 1.0 to 3.0 equivalents or more of oxalyl chloride, etc. in a halogenated hydrocarbon solvent such as dichloromethane at a temperature of from 0°C to room temperature to give the acid chloride thereof (13) and then the resultant is reacted with, for example, 1.0 to 3.0 equivalents or more of the compound of the formula (14) in a halogenated hydrocarbon solvent such as dichloromethane at a temperature of from 0°C to room temperature in the presence of a base such as triethylamine or sodium hydride, etc. to convert into the compound of the formula (15) of the present invention.

Also the compound of the formula (15) can be reacted with, for example, 1.0 to 3.0 equivalents or more of an alkylating agent of the formula: R²-G¹ and a base such as potassium carbonate, etc. in a polar solvent such as N,N-dimethylformamide, etc. at a temperature of from room temperature to 150°C to convert into the compound of the formula (16) of the present invention.

Also the compound of the formula (16) can be reacted with, for example, 1.0 to 3.0 equivalents or more of a sulfuring agent such as Lawesson's reagent, etc. in an aromatic hydrocarbon solvent such as toluene, etc. at a temperature of from room temperature to a boiling point of the solvent to be used to convert into the compound of the formula (17) of the present invention.

Among the compounds of the formula (17), the compound wherein R² is a hydrogen atom can be directly prepared in the same manner as described above from the compound of the formula (15).

Among the compounds of the formula (15), formula (16) and formula (17), these compounds wherein R³ is tert-butyl group or tert-butoxycarbonyl group can be reacted in an acidic solution such as trifluoroacetic acid or 4N hydrogen chloride/dioxane, etc. at a temperature of from room temperature to a boiling point of the solvent to be used to convert into the compound of the formula (120) of the present invention.

### Procedure (B)

Among the present compounds, the compounds of the formula (20), formula (21), formula (20'), formula (21'), formula (121) or formula (121') can be prepared, for example, as follows: 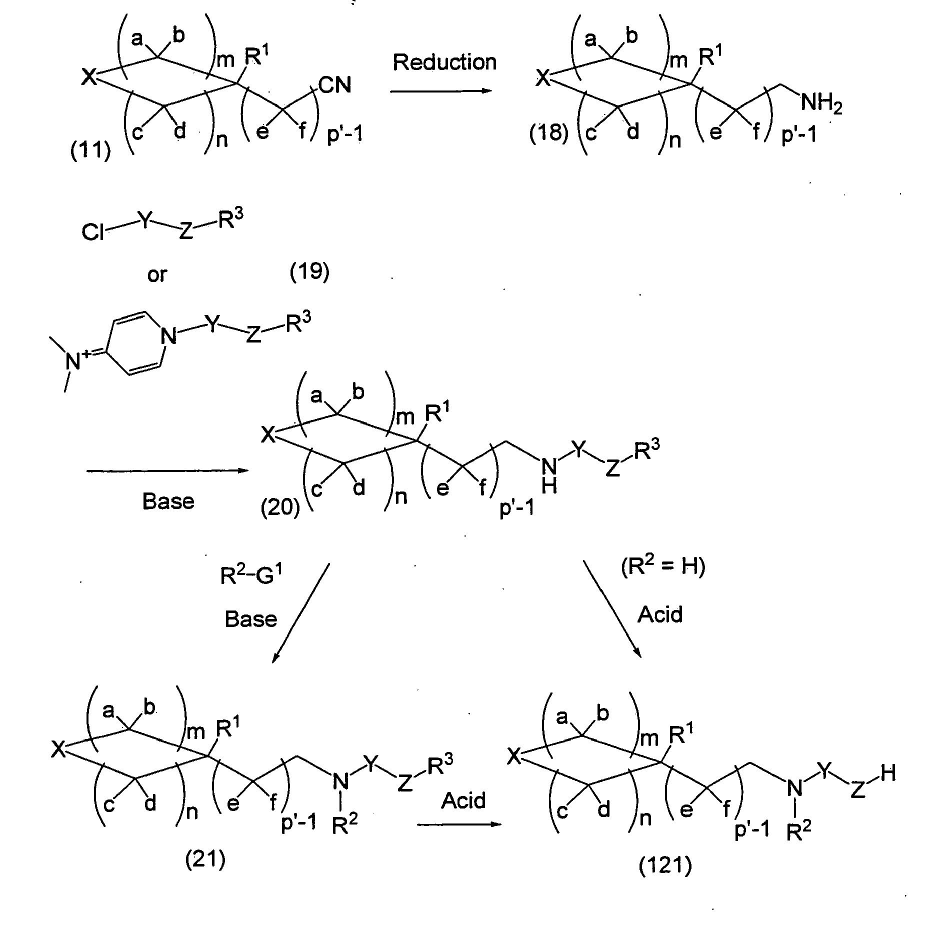 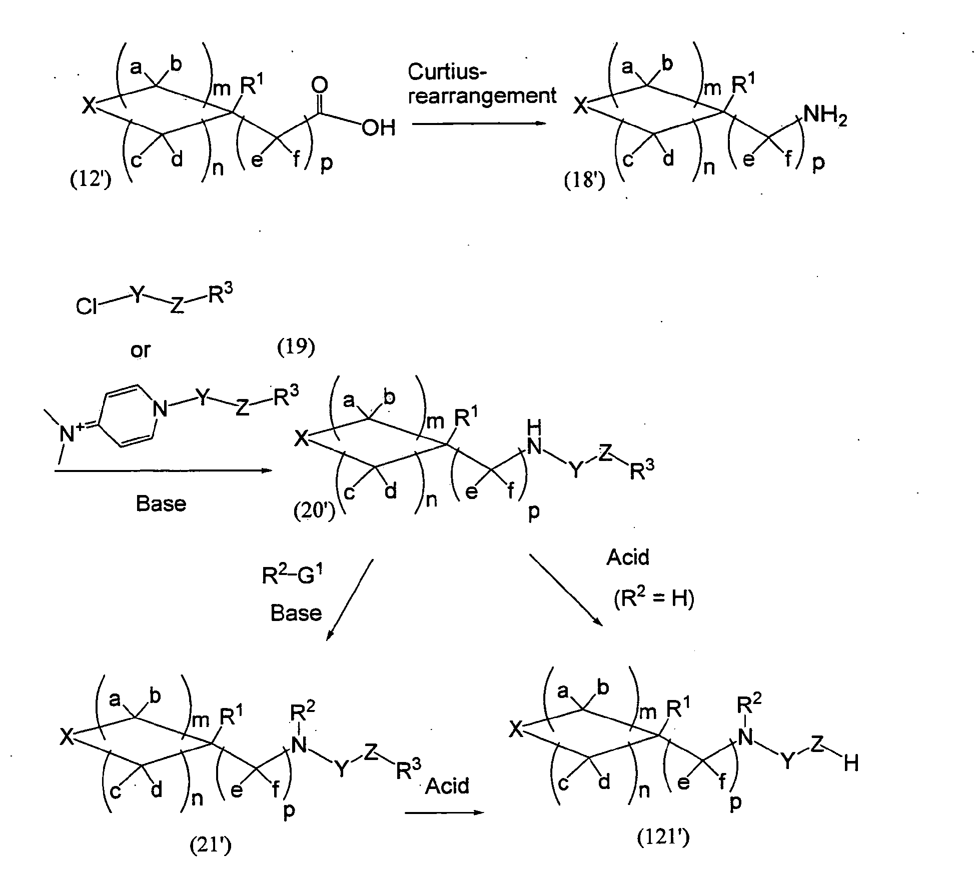 (wherein, a, b, c, d, e, f, G¹, X, Y, Z, m, n, p, p', R¹, R² and R³ are as defined above)

The compound of the formula (11) can be reduced with, for example, lithium aluminum hydride, etc. by using a conventional method to convert into the compound of the formula (18).

The resulting compound can be reacted with, for example, 1.0 to 3.0 equivalents or more of the compound of formula (19) in a halogenated hydrocarbon solvent such as dichloromethane at a temperature of from 0°C to room temperature in the presence of a base such as triethylamine or sodium hydride, etc. to convert into the present compound of the formula (20).

The compound of the formula (20) can be reacted with, for example, 1.0 to 3.0 equivalents or more of an alkylating agent of the formula: R²-G¹ and a base such as potassium carbonate, etc. in a polar solvent such as N,N-dimethylformamide, etc. at a temperature of from room temperature to 150°C to convert into the present compound of the formula (21).

Among the compound of the formula (20) or the formula (21), these compounds wherein R³ is tert-butyl group or tert-butoxycarbonyl group, can be reacted in an acidic solution such as trifluoroacetic acid or 4N hydrogen chloride/dioxane, etc. at a temperature of from room temperature to a boiling point of the solvent to be used to convert into the present compound of the formula (21).

The compound of the formula (12') can undergo a curtius rearrangement by using a conventional method to convert into the compound of the formula (18').

The compounds of the formula (20'), the formula (21') and the formula (121') can be prepared in a similar manner as described above.

The compound of the formula (14) or the formula (19) used in the above procedure (A) or (B) is a well-known compound or can be prepared from a well-known compound by using a well-known technique. For example, these compounds can be prepared in the following manner.

### Procedure (C)

The compound of the formula (14) wherein Z is an oxygen atom, a group of the formula: -NR¹⁶- or a sulfur atom and Y is a group of the formula: -SO₂- can be prepared, for example, by the following method. 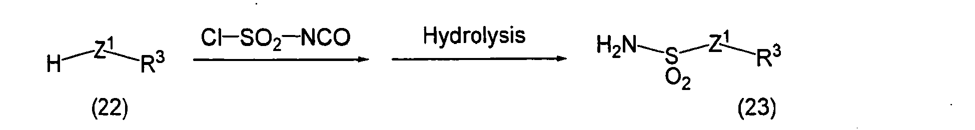 (wherein, Z¹ is an oxygen atom, a group of the formula: -NR¹⁶ or a sulfur atom, and R³ and R¹⁶ are as defined above).

The compound of the formula (22) can be reacted with, for example, 1.0 to 3.0 equivalents or more chlorosulfonyl isocyanate, etc. in a hydrocarbon solvent such as heptane at a temperature of from 0°C to the boiling point of the solvent to be used, followed by, for example, thereto adding 1.0 to 3.0 equivalents or more of a base such as sodium hydroxide, etc. and then hydrolyzing at a temperature of from 0°C to room temperature to convert into the present compound of the formula (23).

### Procedure (C')

The compound of the formula (14) or the formula (19) wherein Z is a group of the formula: -NR¹⁶-, and R¹⁶ is a group of the formulae: - C(=O)R¹⁰¹, -C(=O)OR¹⁰³ or a hydrogen atom, and Y is a group of the formula: -SO₂- can be prepared, for example, by the following method. 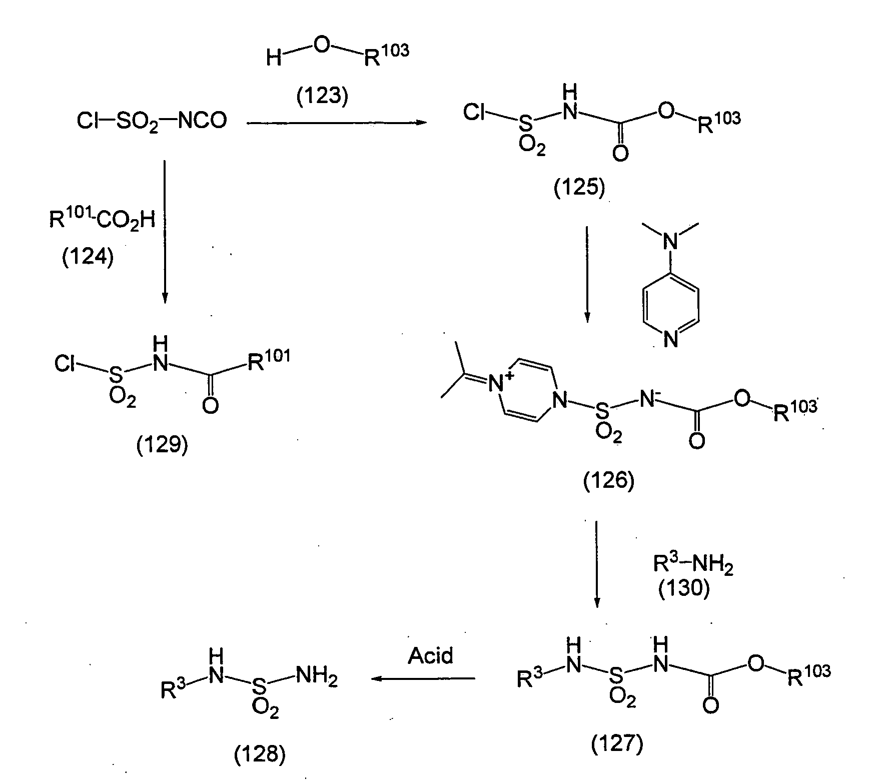 (wherein, R³, R¹⁰¹ and R¹⁰³ are as defined above)

The compound of the formula (123) can be reacted with, for example, 1.0 to 3.0 equivalents or more of chlorosulfonyl isocyanate, etc. in an aromatic hydrocarbon solvent such as toluene, etc. at a temperature of from 0°C to room temperature to convert into the compound of the formula (125).

The compound of the formula (125) can be reacted with, for example, 1.0 to 3.0 equivalents or more of dimethylaminopyridine, etc. in a halogenated hydrocarbon solvent such as dichloromethane, etc. at a temperature of from 0°C to a boiling point of the solvent to be used to convert into the compound of the formula (126).

The compound of the formula (126) can be reacted with, for example, 1.0 to 3.0 equivalents or more of the compound of the formula (130) in a halogenated hydrocarbon solvent such as dichloromethane at a temperature of from 0°C to a boiling point of the solvent to be used to convert into the compound of the formula (127).

The compound of the formula (127) can be reacted in an acidic solution such as trifluoroacetic acid or 4N hydrogen chloride/dioxane, etc. at a temperature of from 0°C to room temperature to convert into the present compound of the formula (128).

The compound of the formula (124) can be reacted with, for example, 1.0 to 3.0 equivalents or more of chlorosulfonyl isocyanate, etc. in a halogenated hydrocarbon solvent such as dichloromethane, etc. at a temperature of from 0°C to a boiling point of the solvent to be used to convert into the compound of the formula (129).

### Procedure (D)

The compound of the formula (14) or the formula (19) wherein Z is a group of the formula: -(CH₂)_{q}- and Y is a group of the formula: -SO₂ can be prepared, for example, by the following method. 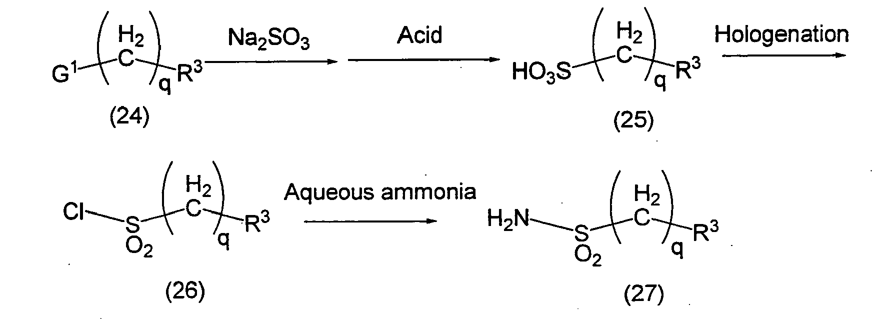 (wherein, G¹, q and R³ are as defined above)

The compound of the formula (24) can be reacted with, for example, 1 to 5 equivalents of sodium sulfite in a mixed solvent of an ether solvent such as 1,4-dioxane, etc. and water at a temperature of from room temperature to a boiling point of the solvent to be used to convert into the compound of the formula (25).

Then the compound of the formula (25) can be reacted with 1.0 to 3.0 equivalents or more of oxalyl chloride, etc. in a polar solvent such as N,N-dimethylformamide, etc. at a temperature of from 0°C to room temperature to give the acid chloride thereof (26), then which is reacted with an excess amount of aqueous ammonia to convert into the compound of the formula (27).

### Procedure (E)

The compound of the formula (14) or the formula (19) wherein Z is an oxygen atom, a group of the formula: -NR¹⁶- or a sulfur atom and Y is a group of the formula: -P(O)OR¹⁵- can be prepared, for example, by the following method. 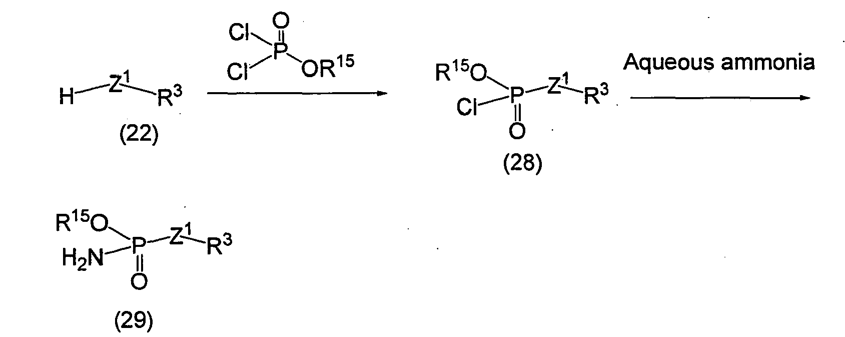 (wherein, R³, R¹⁵, R¹⁶ and Z¹ are as defined above)

The compound of the formula (22) can be reacted with, for example, 1.0 to 3.0 equivalents or more of dichlorophosphate in the presence of 1.0 to 3.0 equivalents or more of sodium hydride, etc. at a temperature of from 0°C to room temperature to convert into the compound of the formula (28).

Then the compound of the formula (28) can be reacted with, for example, an excess amount of aqueous ammonia in an ether solvent such as tetrahydrofuran (THF), etc. to convert into the compound of the formula (29).

### Procedure (F)

The compound of the formula (14) or the formula (19) wherein Z is a group of the formula: -(CH₂)_{q}- and Y is a group of the formula: - P(O)OR¹⁵- can be prepared, for example, by the following method. 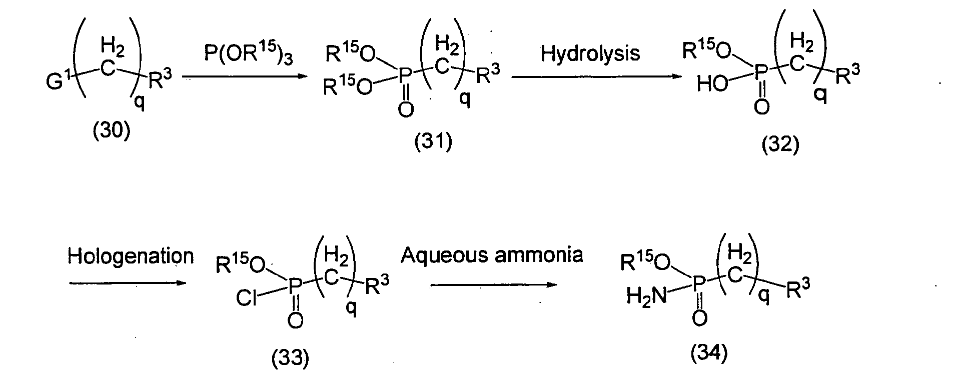 (wherein, R³, R¹⁵, q and G¹ are as defined above)

The compound of the formula (30) can be reacted with, for example, an amount as solvent of a phosphorus reagent such as trimethyl phosphite, etc. without solvent at a temperature of from 0°C to 150°C to give the compound of the formula (31).

The compound of the formula (31) can be reacted, for example, in an alcohol solvent such as methanol, etc. in the presence of a base such as sodium hydroxide, etc. at a temperature of from room temperature to a boiling point of the solvent to be used to convert into the compound of the formula (32).

The compound of the formula (32) can be reacted with, for example, 1.0 to 3.0 equivalents or more of oxalyl chloride, etc. in a polar solvent such as N,N-dimethylformamide, etc. at a temperature of 0°C to room temperature to give the acid chloride (33), and then which is reacted with an excess amount of aqueous ammonia to convert into the compound of the formula (34).

### Procedure (G)

The compound of the formula (14) or (19) wherein Z is a group of the formula: -(CH₂)_{q}- and Y is a group of the formula: C(O)- can be prepared, for example, by the following method. 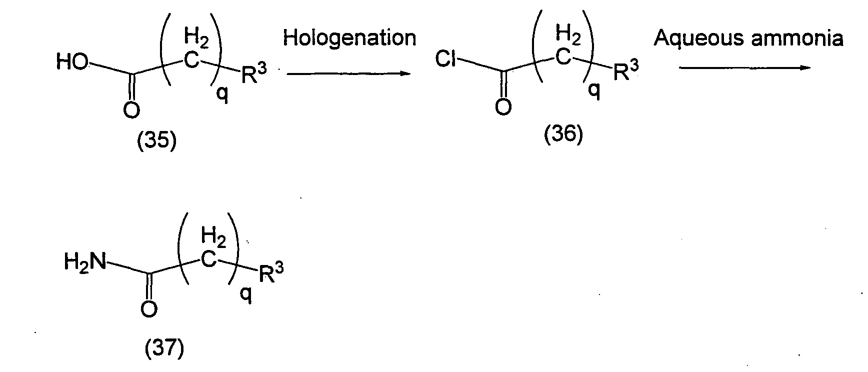 (wherein, R³ and q are as defined above)

The compound of the formula (35) can be reacted with, for example, 1.0 to 3.0 equivalents or more of oxalyl chloride, etc. in a halogenated hydrocarbon solvent such as dichloromethane, etc. at a temperature of 0°C to room temperature to give acid chloride thereof (36) and then which is reacted with an excess amount of aqueous ammonia to convert into the compound of the formula (37).

The compound of the formula (11) used in the above procedure (A) or (B) is a well-known compound or can be prepared from a well-known compound by using a well-known technique. For example, these compounds can be prepared in the following manner.

### Procedure (H)

Among the compound of the formula (11), the compound of the formula (111) or the formula (113) can be prepared, for example, as follows. 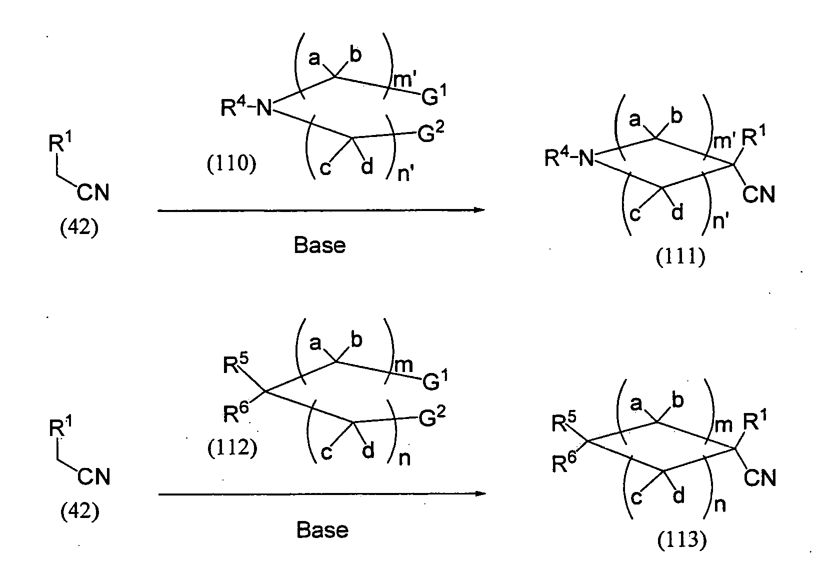 (wherein, each of m' and n' is an integer of 1 to 4 with the proviso that 3 ≦ m' + n' ≦ 8; G¹ and G² are independently a leaving group such as an iodine atom, a bromine atom, a chlorine atom, p-toluenesulfonyl group, etc.; R¹, a, b, c, d, m, n, R⁴, R⁵ and R⁶ are as defined above)

The compound of the formula (42) can be reacted with, for example, the compound of the formula (110) or the formula (112) in an amount of 1 to 3 equivalents to the compound of the formula (42) in a solvent at - 30 to -10°C in the presence of 2 to 5 equivalents of a base such as sodium hydride, etc. to give the compound of the formula (111) or the formula (113) respectively.

The above reaction is usually performed in a solvent and the solvent may be any solvent which does not disturb the reaction, for example, ethers (e.g., diethylether, diisopropylether, tetrahydrofuran, or 1,4-dioxane, etc.), aromatic hydrocarbons (e.g., benzene, toluene, or xylene, etc.), esters (e.g., methyl acetate, ethyl acetate, propyl acetate, etc.), halogenated hydrocarbons (e.g., dichloromethane, chloroform, dichloroethane, chlorobenzene, dichlorobenzene, etc.), ketones (e.g., acetone, methyl ethyl ketone, etc.), nitriles (e.g., acetonitrile, isobutyronitrile, etc.), N,N-dimethylformamide, and dimethyl sulfoxide.

### Procedure (I)

Alternatively, among the compound of the formula (11), the compound of the formula (52) can be prepared, for example, as follows. 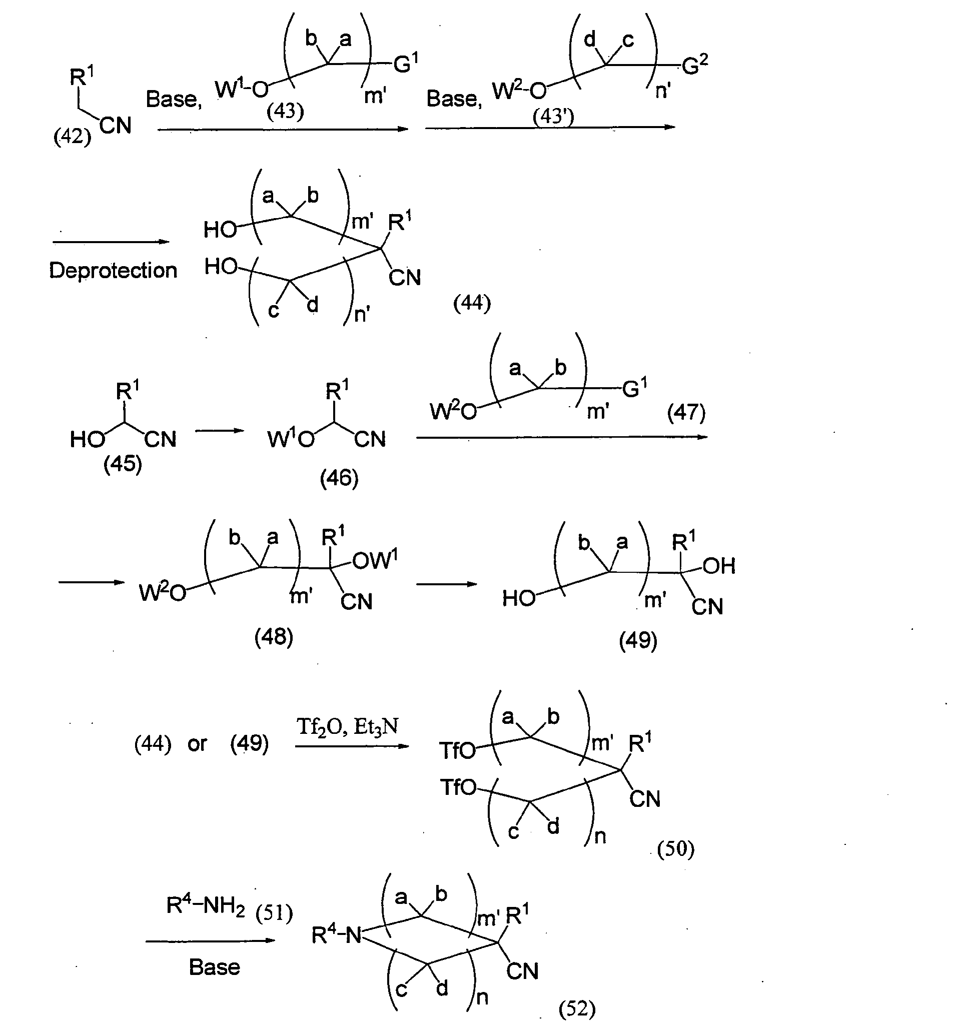 (wherein, W¹ and W² are a protecting group of a hydroxy group; Tf is a trifluoromethanesulfonyl group; G¹, G², m', n', R¹, n, a, b, c, d and R⁴ are as defined above)

The compound of the formula (52) wherein n is an integer of 1 to 4 can be prepared, for example, from the compound of the formula (44), etc. Specifically, the compound of the formula (42) can be reacted with, for example, 1 to 1.5 equivalents of the compound of the formula (43) in the presence of 1 to 1.5 equivalents or more of a base such as sodium hydride, etc. at a temperature of from room temperature to a boiling point of the solvent to be used and then which is reacted with the compound of the formula (43') in the presence of 1 to 1.5 equivalents or more of sodium hydride, etc. at a temperature of from room temperature to a boiling point of the solvent to be used, and then the protecting group of the hydroxy group is subjected to a deprotection reaction to give the compound of the formula (44).

The above reaction is usually performed in a solvent and the solvent may be any solvent which does not disturb the reaction, for example, ethers (e.g., diethyl ether, diisopropylether, tetrahydrofuran, or 1,4-dioxane, etc.), aromatic hydrocarbons (e.g., benzene, toluene, or xylene, etc.), esters (e.g., methyl acetate, ethyl acetate, propyl acetate, etc.), halogenated hydrocarbons (e.g., dichloromethane, chloroform, dichloroethane, chlorobenzene, dichlorobenzene, etc.), ketones (e.g., acetone, methyl ethyl ketone, etc.), nitriles (e.g., acetonitrile, isobutyronitrile, etc.), N,N-dimethylformamide, and dimethyl sulfoxide.

The compound of the formula (52) wherein n is 0, can be prepared, for example, from the compound of the formula (49), etc. Specifically, the hydroxy group in the compound of the formula (45) can be protected to convert into the compound of the formula (46) and which then is reacted with, for example, 1 to 1.5 equivalents or more of the compound of the formula (47) in an ether solvent such as tetrahydrofuran (THF), under basic conditions such as one in the presence of 1 to 1.5 equivalents of lithium diisopropylamide at a temperature of from -78°C to room temperature or one in the presence of 1 to 1.5 equivalents of sodium hexamethyldisilazide at a temperature of from 0°C to a heat under reflux, etc., and then is subjected to a deprotection reaction to give the compound of the formula (49).

The protecting group of a hydroxy group may be any protecting group which does not disturb the reaction, preferably, for example, silyl group such as tert-butyldimethylsilyl group, etc.

A deprotection reaction can be performed by using the technique, for example described in the literature (PROTECTIVE GROUPS IN ORGANIC SYNTHESIS, 3rd ed., JOHN WILEY & SONS, INC.: New York (1999), etc.).

The compound of the formula (44) or the formula (49) thus obtained can be reacted with 2 to 5 equivalents of anhydrous trifluoromethane sulfonic acid in a solvent at a temperature of from - 30°C to -10°C in the presence of 2 to 5 equivalents of a base to give the compound of the formula (50) and usually without isolating in situ, thereto was added 1 to 3 equivalents of the compound of the formula (51) and 1 to 3 equivalents of a base such as triethylamine, etc. to give the compound of the formula (52).

The above reaction is usually performed in a solvent and the solvent may be any solvent which does not disturb the reaction, for example, includes the solvent described above, etc.

### Procedure (I')

Among the compound of the formula (11), the compound of the formula (204) wherein a and c may combine to form an alkylene group can be prepared, for example, as follows. 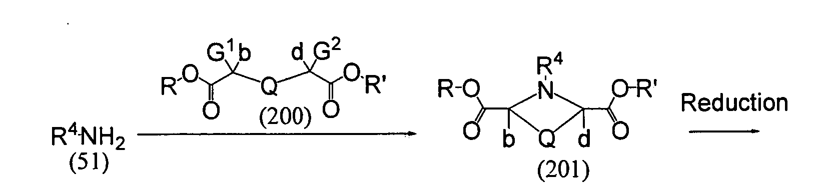 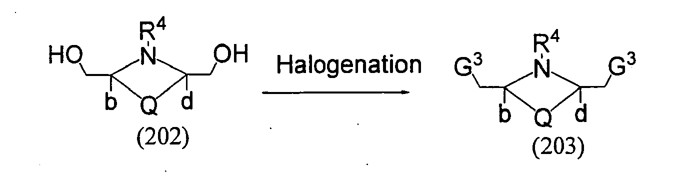 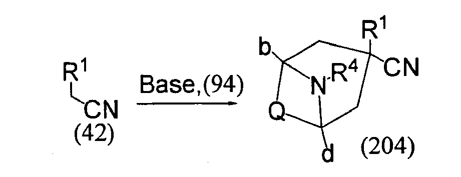 (wherein, R¹, R⁴, b, d, G¹ and G² are as defined above; G³ is a leaving group such as a chlorine atom, a bromine atom, etc.; and Q is an alkylene group)

The synthesis of the compound of the formula (204) may be done by a route of the synthesis of the compound of the formula (203). Specifically, the compound of the formula (200) can be reacted with 1.0 to 2.0 equivalents of the compound of the formula (51) at a temperature of from room temperature to a boiling point of the solvent to be used to synthesize the compound of the formula (201), and the resulting compound of the formula (201) is reacted in the presence of 1 to 1.5 equivalents or more of a reducing agent such as lithium aluminum hydride, etc. at a temperature of from room temperature to a boiling point of the solvent to be used to synthesize the compound of the formula (202), and further the resulting compound of the formula (203) is reacted with 2.0 to 3.0 equivalents or more of thionyl chloride at a temperature of from 0°C to a boiling point of the solvent to be used to synthesize the compound of the formula (203).

The compound of the formula (42) can be reacted with 1.0 to 2.0 equivalents of the compound of the formula (203) obtained above in a solvent in the presence of 2.0 to 4.0 equivalents of a base such as sodium hydride, etc. at a temperature of from -10°C to 50°C to synthesize the compound of the formula (204).

The above reaction is usually performed in a solvent and the solvent may be any solvent which does not disturb the reaction, for example, ethers (e.g., diethylether, diisopropylether, tetrahydrofuran, or dioxane, etc.), aromatic hydrocarbons (e.g., benzene, toluene, or xylene, etc.), esters (e.g., methyl acetate, ethyl acetate, propyl acetate, etc.), halogenated hydrocarbons (e.g., dichloromethane, chloroform, dichloroethane, chlorobenzene, dichlorobenzene, etc.), ketones (e.g., acetone, methyl ethyl ketone, etc.), nitriles (e.g., acetonitrile, isobutyronitrile, etc.), N,N-dimethylformamide, and dimethyl sulfoxide.

### Procedure (J)

Alternatively, among the compound of the formula (11), the compound of the formula (104), the formula (105) or the formula (106) can be prepared, for example, as follows. In these formulae, R²² is a hydrogen atom, a substituted or unsubstituted alkyl group or a substituted or unsubstituted aryl group. 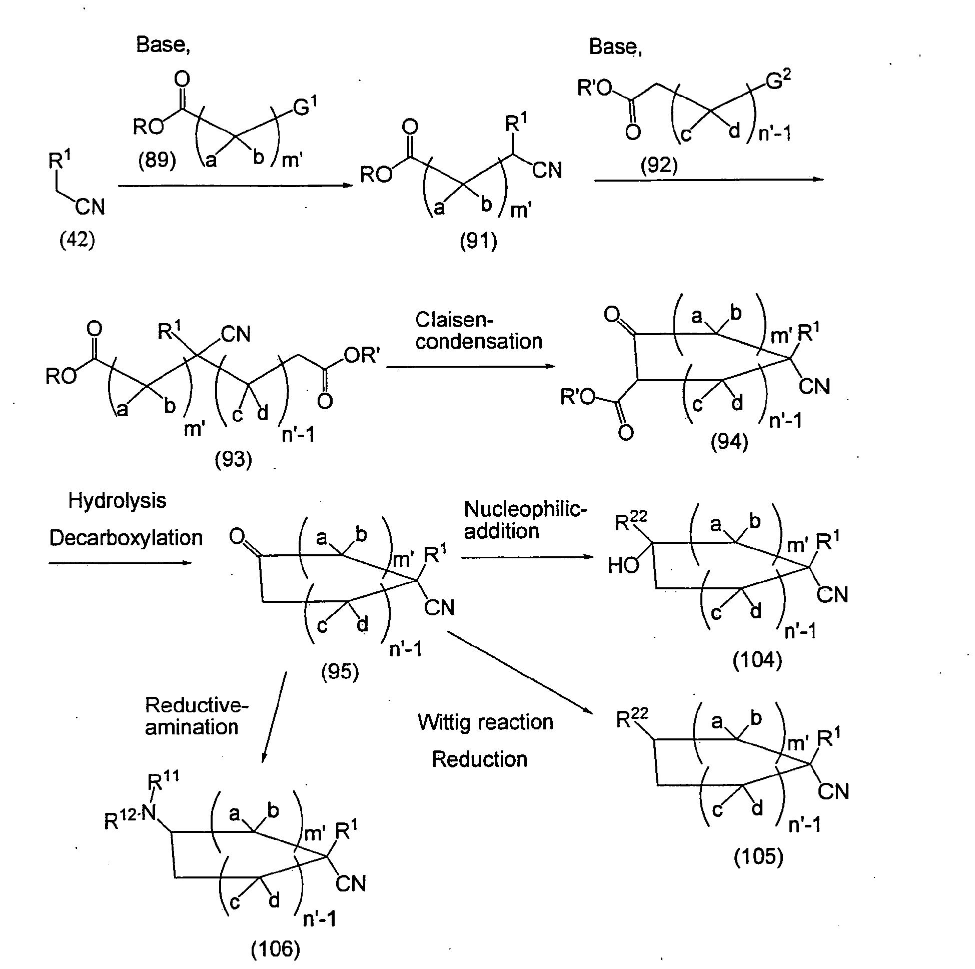 (wherein, R and R' are independently an alkyl group; R¹, m', n', a, b, c, d, R¹¹, R¹², G¹ and G² are defined above; and R²² is defined above)

The compound of the formula (42) can be reacted with, for example, 1 to 5 equivalents of the compound of the formula (89) in an ether solvent such as tetrahydrofuran, etc. at a temperature of from ice-cooling to 100°C in the presence of 1 to 5 equivalents or more of a base such as sodium hydride, etc. to synthesize the compound of the formula (91). Then, under a similar condition as described above, the compound of the formula (91) can be reacted with the compound of the formula (92) to give the compound of the formula (93).

The compound of the formula (93) can be reacted in an alcohol solvent such as ethanol, etc., in the presence of a base such as sodium ethoxide etc., at a temperature of from room temperature to a boiling point of the solvent to be used to convert into the compound of the formula (94).

The compound of the formula (94) can be reacted in an alcohol solvent such as ethanol, etc., in the presence of a base such as sodium hydroxide, etc., at a temperature of from room temperature to a boiling point of the solvent to be used to convert into the compound of the formula (95).

The compound of the formula (95) can be reacted with, for example, 1.0 to 5.0 equivalents of a nucleophilic reagent, in an ether solvent such as tetrahydrofuran, etc., at a temperature of from 0°C to 120°C, preferably at a temperature of from room temperature to a boiling point of the solvent to be used to convert into the compound of the formula (104). A nucleophilic reagent includes, for example, a Grignard reagent, etc. such as R²²MgBr, etc.

The compound of the formula (95) can be reacted with, for example, 1.0 to 5.0 equivalents of a nucleophilic reagent such as Wittig reagent, etc. in an ether solvent such as trahydrofuran, etc. at a temperature of from 0°C to 120°C, preferably at a temperature of from room temperature to a boiling point of the solvent to be used and then followed by subjecting to a hydrogenation reaction at room temperature by using a conventional method to convert into the compound of the formula (105).

The compound of the formula (95) can be reacted with, for example, 1.0 to 5.0 equivalents of a reducing reagent such as sodium triacetoxyborohydride, etc. and 1.0 to 5.0 equivalents of the compound of the formula; HNR¹¹R¹² (R¹¹ and R¹² are as defined above) in a halogenated hydrocarbon solvent such as dichloroethane, etc. at a temperature of from 0°C to 120°C, preferably at a temperature of from room temperature to a boiling point of the solvent to be used to convert into the compound of the formula (106).

### Procedure (J')

Further, the following compounds can be prepared from the compound of the formula (104) thus obtained. 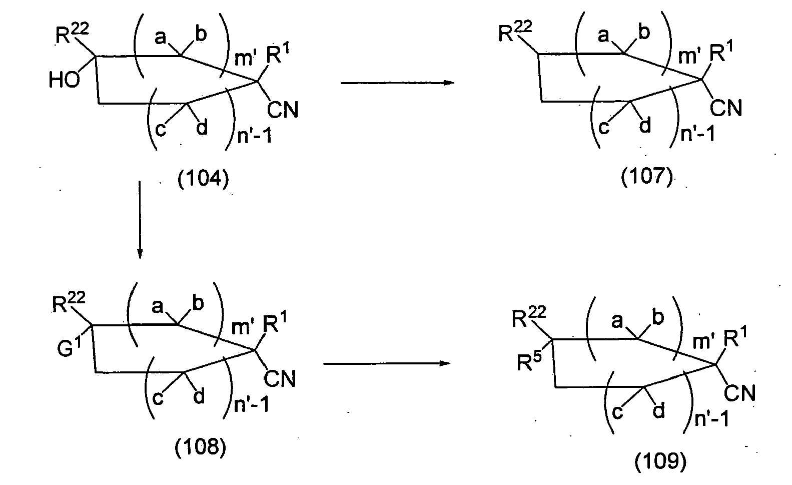 (wherein, R¹, m', n', a, b, c, d, R⁵, R²² and G¹ are as defined above)

The compound of the formula (104) can be reduced with, for example, 1.0 to 5.0 equivalents of triethyl silane, etc., in a solvent such as trifluoroacetic acid, etc. at a temperature of from 0°C to 120°C, preferably at a temperature of from room temperature to a boiling point of the solvent to be used to synthesize the compound of the formula (107).

The compound of the formula (104) also can be converted into the compound of the formula (108) by using a conventional halogenation or a transformation into a methanesulfonate. The compound of the formula (104) can be reacted with, for example, 2.0 to 3.0 equivalents or more of methanesulfonyl chloride, in a halogenated hydrocarbon solvent such as dichloromethane, etc., in the presence of 2.0 to 3.0 equivalents or more of a base such as triethylamine, etc. at a temperature of from room temperature to a boiling point of the solvent to be used to give the compound of the formula (108).

The compound of the formula (108) obtained thus can be reacted with the compound of the formula: R⁵M (M is, for example, Li or MgBr, etc.) by using a conventional method to give the compound of the formula (109).

### Procedure (J-2)

Among the compound of the formula (11) or the formula (12), the compound of the formula (407) or the formula (411) can be prepared, for example, as follows. 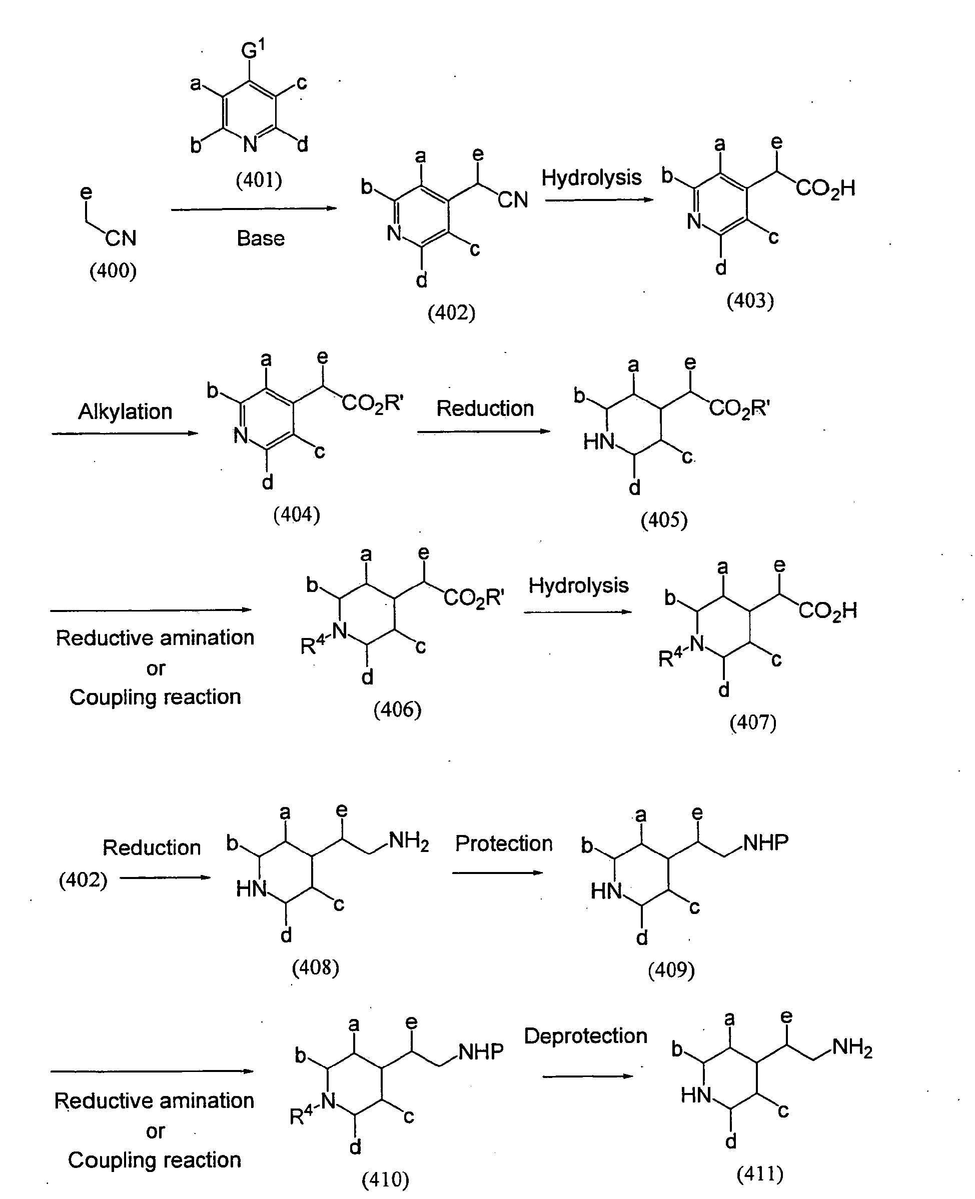 (wherein, R' is an alkyl group; R¹, m', n', a, b, c, d, e and G¹ are as defined above; and P is a protecting group)

The compound of the formula (400) can be reacted with, for example, 1 to 5 equivalents of the compound of the formula (401) in an ether solvent such as tetrahydrofuran, etc., in the presence of 1 to 5 equivalents or more of a base such as butyllithium, etc. at a temperature of from ice-cooling to 100°C to give the compound of the formula (402).

The compound of the formula (403) can be hydrolyzed with acid, etc. such as conc. hydrochloric acid, etc. to convert into the compound of the formula (403).

The compound of the formula (403) can be alkylated with alkylhalide in a polar solvent such as N,N-dimethylformamide, etc. in the presence of a base such as potassium carbonate, etc. at a temperature of from room temperature to a boiling point of the solvent to be used to convert into the compound of the formula (404).

The compound of the formula (404) can be subjected to a hydrogenation reaction under an atmosphere of hydrogen under normal pressure to 4 atmospheres in an acidic solvent such as acetic acid, etc., in the presence of a catalyst such as platinum oxide, etc. at a temperature of from room temperature to a boiling point of the solvent to be used to convert into the compound of the formula (405).

The compound of the formula (405) can be subjected to a coupling reaction using a palladium catalyst in the presence of a base, a coupling reaction using a base, or a reductive amination by using a conventional method to synthesize the compound of the formula (406).

The coupling reaction using a palladium catalyst can be performed in the presence of a base such as sodium tert-butoxide, by using a zero valence palladium catalyst such as tris(dibenzylideneacetone)-dipalladium or tetrakis(triphenylphosphine)palladium, etc., and a phosphorus ligand such as 2,2'-bis(diphenylphosphino)-1,1'-binaphtyl or 1,1'-bis(diphenylphosphino)ferrocene.

The solvent may be any solvent which does not disturb the reaction, but the reaction can be preferably performed in a nonpolar solvent such as toluene, etc.

The coupling reaction using a base can be performed with, for example, a base such as sodium hydride or triethylamine, etc. by using a conventional method.

The solvent may be any solvent which does not disturb the reaction, but the reaction can be preferably performed in a polar solvent such as dimethyl sulfoxide, etc.

The reductive amination can be performed with, for example, a reducing agent such as sodium triacetoxyborohydride, etc. by using a conventional method.

The solvent may be any solvent which does not disturb the reaction, but the reaction can be preferably performed in halogenated hydrocarbons such as dichloromethane, etc.

Then the compound of the formula (406) can be hydrolyzed, for example, with a base such as aqueous potassium hydroxide solution, etc. to convert into the compound of the formula (407).

The compound of the formula (408) can also be prepared from the compound of the formula (402) by a hydrogenation, similarly as in the case of the compound of the formula (405).

The compound of the formula (408) can be, if necessary, protected with a protecting group such as tert-butylcarbamate, etc. to convert into the compound of the formula (409), followed by subjecting to a coupling reaction using a palladium catalyst in the presence of a base, a coupling using a base, or a reductive amination, similarly as a method for synthesizing the compound of the formula (406), to convert into the compound of the formula (410).

The protecting group in the compound of the formula (410) can be, if necessary, subjected to a deprotection to convert into the compound of the formula (411).

The compound of the formula (110) or the formula (112) used in the above procedure (H) is a well-known compound or can be prepared from a well-known compound by using a well-known technique. For example, these compounds can be prepared in the following manner.

### Procedure (K)

Among the compound of the formula (110), the compound of the formula (58) or the formula (58') can be prepared, for examples, by the following method. 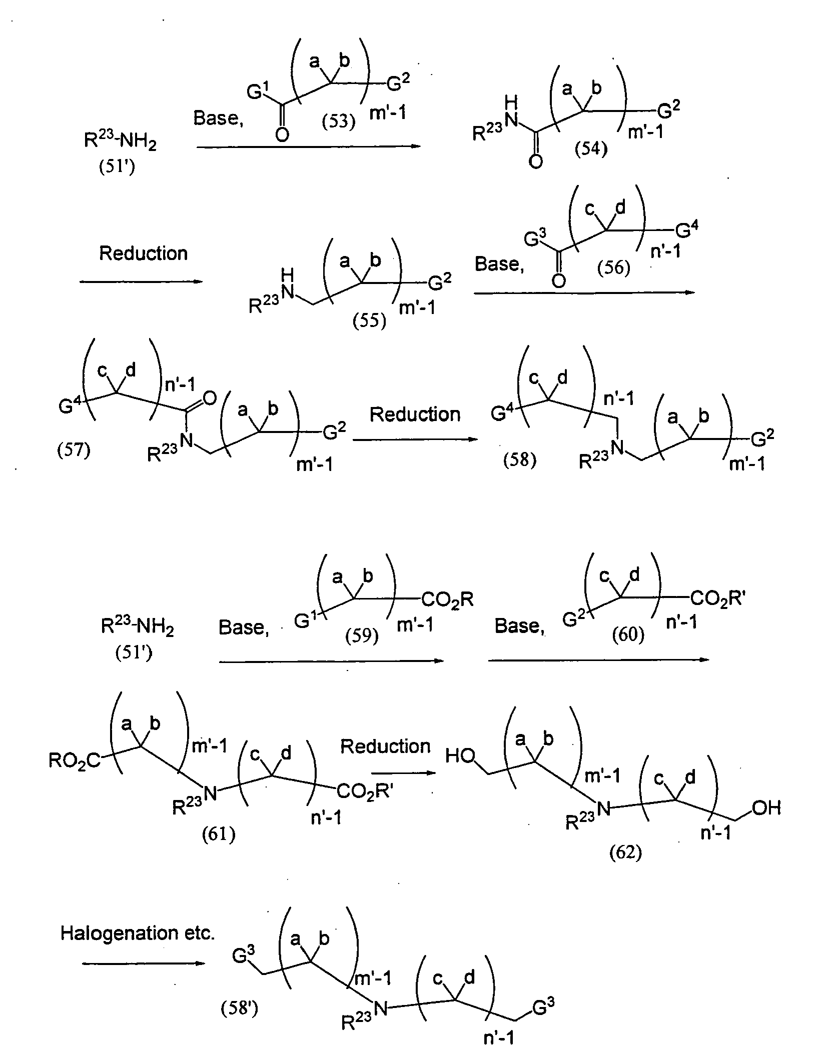 (wherein, R and R' are independently an alkyl group; R²³ is a substituted or unsubstituted alkyl group, a substituted or unsubstituted alkenyl group, a substituted or unsubstituted alkynyl group, a substituted or unsubstituted cycloalkyl group, or a substituted or unsubstituted aryl group; G¹, G², G³ and G⁴ are independently, for example, a leaving group such as an iodine atom, a bromine atom, a chlorine atom or p-toluenesulfonyl group, etc.; R¹, m', n', a, b, c and d are as defined above)

The compound of the formula (51') can be reacted with, for example, 1 to 1.5 mole equivalents of the compound of the formula (53) in the presence of 1 to 1.5 equivalents or more of a base such as triethylamine, etc. at a temperature of from room temperature to a boiling point of the solvent to be used to give the compound of the formula (54).

The compound of the formula (54) can be reacted, for example, in the presence of 1 to 1.5 equivalents or more of a reducing reagent such as borane tetrahydrofuran complex (BH₃ THF) at a temperature of from room temperature to a boiling point of the solvent to be used to give the compound of the formula (55).

The compound of the formula (55) can be reacted with, for example, 1 to 1.5 equivalents or more of the compound of the formula (56) in the presence of a base such as triethylamine, etc. at a temperature of from room temperature to a boiling point of the solvent to be used to give the compound of the formula (57).

The compound of the formula (57) can be reacted, for example, in the presence of 1 to 1.5 equivalents or more of a reducing agent such as borane tetrahydrofuran complex (BH₃· THF) at a temperature of from room temperature to a boiling point of the solvent to be used to give the compound of the formula (58).

The above reaction is usually performed in a solvent and the solvent may be any solvent which does not disturb the reaction, for example, ethers (e.g., diethyl ether, diisopropylether, tetrahydrofuran, or 1,4-dioxane, etc.), aromatic hydrocarbons (e.g., benzene, toluene, or xylene, etc.), esters (e.g., methyl acetate, ethyl acetate, propyl acetate, etc.), halogenated hydrocarbons (e.g., dichloromethane, chloroform, dichloroethane, chlorobenzene, dichlorobenzene, etc.), ketones (e.g., acetone, methyl ethyl ketone, etc.), nitriles (e.g., acetonitrile, isobutyronitrile, etc.), N,N-dimethylformamide, and dimethyl sulfoxide. The compound of the formula (51') can be reacted with, for example, 1 to 1.5 equivalents of the compound of the formula (59) without solvent, in the presence of 1 to 1.5 equivalents or more of a base such as sodium carbonate, etc., at a temperature of from 100 to 150°C and then the resultant can be reacted with, for example, the compound of the formula (60) without solvent, in the presence of 1 to 1.5 equivalents or more of a base such as sodium carbonate at a temperature of from 100 to 150°C to give the compound of the formula (61).

The compound of the formula (61) can be reacted, for example, in the presence of 1 to 4.0 equivalents or more of a reducing agent such as lithium aluminum hydride, etc. at a temperature of from room temperature to a boiling point of the solvent to be used to give the compound of the formula (62).

The compound of the formula (62) can be converted into the compound of the formula (58') by using a conventional halogenation or a transformation into a methanesulfonate. The compound of the formula (62) can be subjected to an elimination reaction, for example, by reacting with 2.0 to 3.0 equivalents or more of methanesulfonyl chloride, in the presence of 2.0 to 3.0 or more equivalents of a base such as triethylamine, etc., in a halogenated hydrocarbon solvent such as dichloromethane, etc. at a temperature of from room temperature to a boiling point of the solvent to be used to give the compound of the formula (58').

### Procedure (L)

Among the compound of the formula (112), the compound of the formula (81), the formula (86) or the formula (87) can be prepared, for example, by the following method. 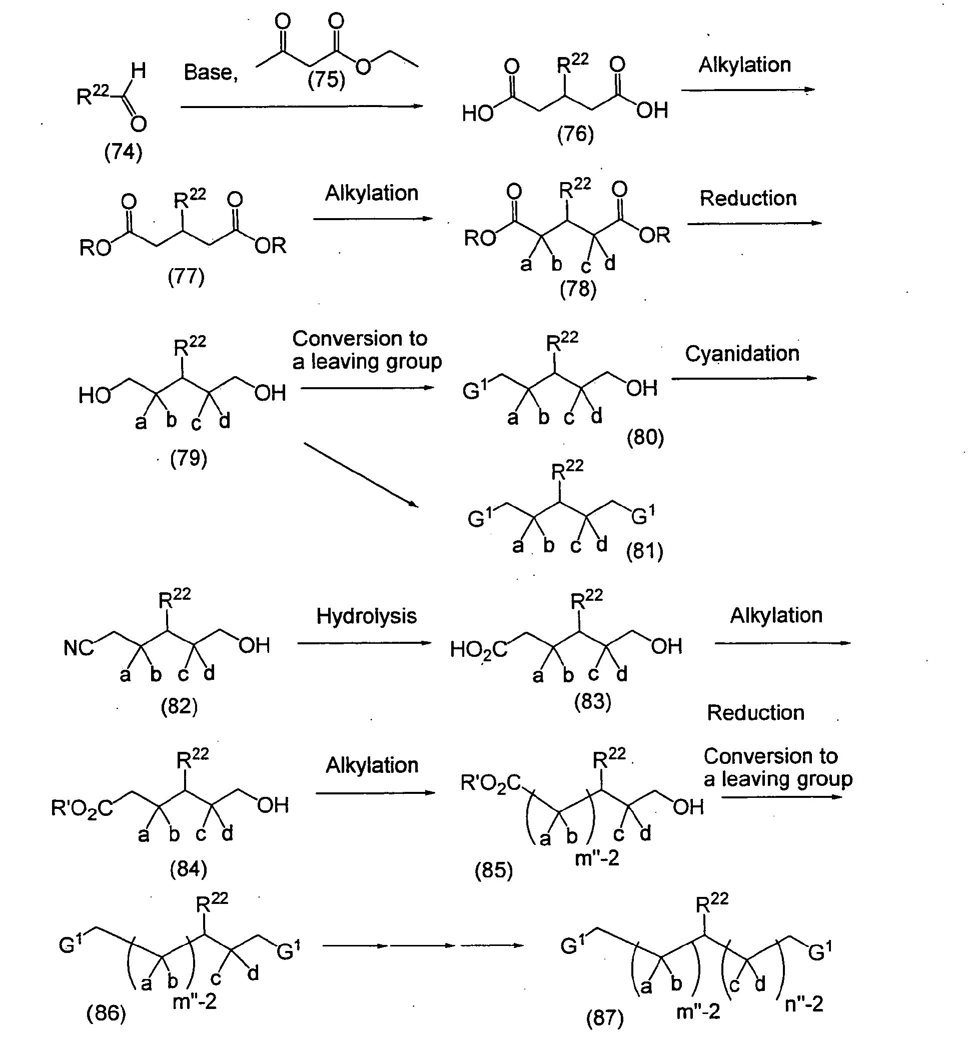 (wherein, R and R' are an alkyl group; m" and n" are 3 or 4; Y, G¹, R²², a, b, c and d are as defined above)

The compound of the formula (74) can be reacted with, for example, 2 to 5 equivalents of the compound of the formula (75) without solvent in the presence of a catalytic amount to 1.5 equivalents or more of a base such as piperidine, etc. at a temperature of from room temperature to 100°C to give the compound of the formula (76).

The compound of the formula (76) can be reacted with, for example, 2.0 to 5.0 equivalents of an alkyl halide in a polar solvent such as N,N-dimethylformamide, etc. in the presence of a base such as potassium carbonate, etc., at a temperature of from 0°C to 120°C, preferably at a temperature of from room temperature to a boiling point of the solvent to be used to convert into the compound of the formula (77).

The compound of the formula (77) can be reacted with, for example, 1.0 to 1.2 equivalents of the compound of the formula: a-G¹ in a polar solvent such as N,N-dimethylformamide, etc. in the presence of a base such as sodium hydride, etc., at a temperature of from 0°C to 120°C, preferably at a temperature of from room temperature to a boiling point of the solvent to be used, followed by, if necessary, with the compounds of the formulae: b-G¹, c-G¹ and d-G¹ under the similar conditions as described above to convert into the compound of the formula (78). The compounds of the formulae: a-G¹, b-G¹, c-G¹ and d-G¹ include, for example, an alkyl halide, etc.

The compound of the formula (78) can be reduced with, for example, lithium aluminum hydride, etc. by using a conventional method, to convert into the compound of the formula (79).

The hydroxy group in the compound of the formula (79) can be converted to a leaving group by using a conventional method to give the compounds of the formula (80) and the formula (81). The compound of the formula (79) can be reacted with, for example, 0.5 to 3.0 equivalents or more of a sulfonyl chloride such as p-toluenesulfonyl chloride, etc. in a solvent such as pyridine, etc. at a temperature of from room temperature to a boiling point of the solvent to be used to give the compounds of the formula (80) and the formula (81).

The compound of the formula (80) can be reacted with, for example, 1.0 to 3.0 equivalents or more of a cyanide salt such as potassium cyanide, etc., in a polar solvent such as dimethyl sufoxide, etc. at a temperature of from room temperature to 150°C to convert into the compound of the formula (82).

The compound of the formula (82) can be hydrolyzed by using a conventional method to convert into the compound of the formula (83).

The compound of the formula (83) can be reacted with, for example, 1.0 to 5.0 equivalents of alkyl halide in a polar solvent such as N,N-dimethylformamide, etc., in the presence of a base such as sodium hydride, etc., at a temperature of from 0°C to 120°C, preferably at a temperature of from room temperature to a boiling point of the solvent to be used to convert into the compound of the formula (84).

The compound of the formula (84) can be reacted with, for example, 1.0 to 1.2 equivalents of the compound of the formula: a-G¹ in a polar solvent such as N,N-dimethylformamide, etc., in the presence of a base such as potassium carbonate, etc., at temperature of from 0°C to 120°C, preferably at a temperature of from room temperature to a boiling point of the solvent to be used, followed by with 1.0 to 1.2 equivalents of the compound of the formula: b-G¹ under a similar condition as described above to convert into the compound of the formula (85). The compounds of the formulae: a-G¹ and b-G¹ include, for example, an alkyl halide, etc.

If necessary, the reaction procedures from the compound of the formula (78) to that of the formula (85) can be performed repeatedly to convert into the compound of the formula (86), or further into the compound of the formula (87).

A part of the structure of the compounds obtained by the above method can be converted into other partial structure to give other compounds of the formula (1) having a wide variety of partial structures.

### Procedure (M), Conversion of R⁴

Among the compound of the formula (11), the compound of the formula (63) can be converted into the compounds having a wide variety of R⁴ thereof, for example, by the following method. 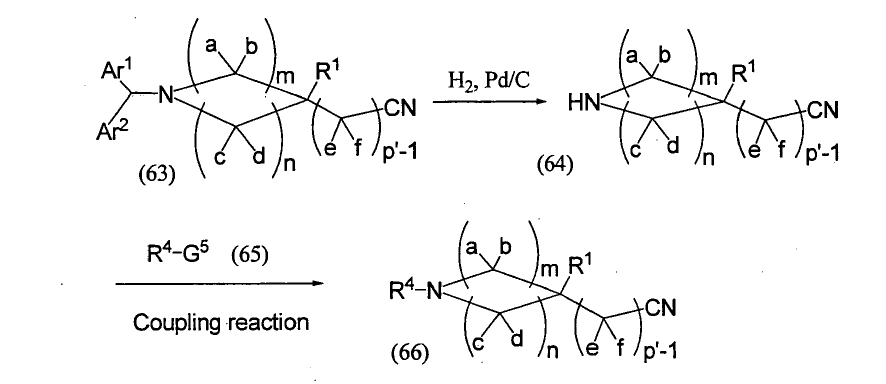 (wherein, m, n, p', R¹, a, b, c, d, e, f and R⁴ are as defined above; G⁵ is a leaving group such as an iodine atom, a bromine atom, a chlorine atom, etc.; Ar¹ is a substituted or unsubstituted phenyl group, Ar² is a hydrogen atom, or a substituted or unsubstituted phenyl group, and the substituents includes, for example, an alkyl group or an alkoxy group, etc.)

The compound of the formula (63) can be subjected to a hydrogenation reaction by using a conventional method to convert into the compound of the formula (64).

The compound of the formula (65) can be subjected to a coupling reaction using a palladium catalyst in the presence of a base or a coupling reaction using a base to synthesize the compound of the formula (66).

The coupling reaction using a palladium catalyst can be performed, for example, in the presence of sodium tert-butyrate, etc., using, for example, a zero valence palladium catalyst such as tris(dibenzylideneacetone)dipalladium or tetrakis(triphenylphosphine)palladium, etc., and, for example, a phosphorus ligand such as 2,2'-bis(diphenylphosphino)-1,1'-binaphtyl or 1,1'-bis(diphenylphosphino)ferrocene.

The solvent may be any solvent which does not disturb the reaction, but the reaction can be preferably performed in a nonpolar solvent such as toluene, etc.

The coupling reaction using a base can be performed with, for example, a base such as sodium hydride or triethylamine, etc. by using a conventional method.

The solvent may be any solvent which does not disturb the reaction, but the reaction can be preferably performed in a polar solvent such as dimethyl sulfoxide, etc.

### Procedure (M'), Conversion of R¹¹ and R¹²

Among the compound of the formula (11), the compound of the formula (302) can be converted into the compound having a wide variety of R¹¹ and R¹² thereof, for example, by the following method. 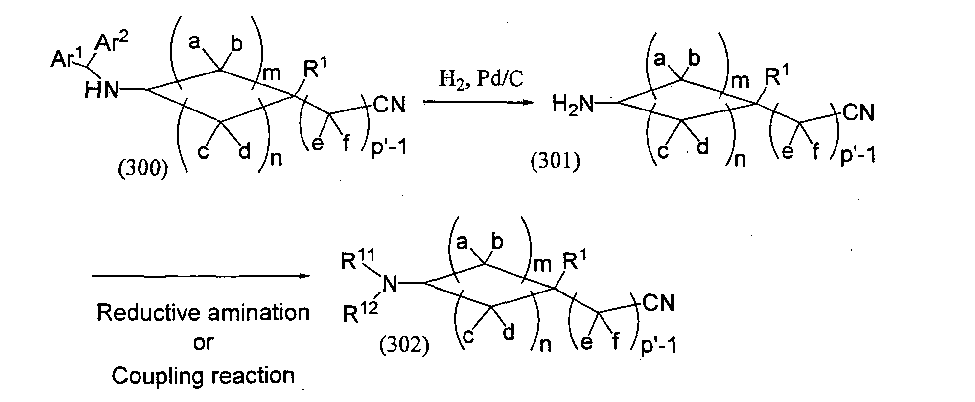 (wherein, m, n, p', R¹, R¹¹, R¹², a, b, c, d, e, f, Ar¹, Ar² and R⁴ are as defined above)

The compound of the formula (300) can be subjected to a hydrogenation reaction by using a conventional method to convert into the compound of the formula (301).

The compound of the formula (301) can be subjected to a coupling reaction using a palladium catalyst in the presence of a base, a coupling reaction using a base, or a reductive amination by using a conventional method to synthesize the compound of the formula (302).

A coupling reaction using a palladium catalyst can be performed in the presence of a base such as sodium tert-butoxide, etc., using, for example, a zero valence palladium catalyst such as tris(dibenzylideneacetone)dipalladium or tetrakis(triphenylphosphine)-palladium, etc. and for example, a phosphorus ligand such as 2,2'-bis(diphenylphosphino)-1,1'-binaphtyl or 1,1'-bis(diphenylphosphino)-ferrocene, etc.

The solvent may be any solvent which does not disturb the reaction, but the reaction can be preferably performed in a nonpolar solvent such as toluene, etc.

The coupling reaction using a base can be performed with, for example, a base such as sodium hydride or triethylamine, etc. by using a conventional method.

The solvent may be any solvent which does not disturb the reaction, but the reaction can be preferably performed in a polar solvent such as dimethyl sulfoxide, etc.

The reductive amination can be performed with, for example, a reducing agent such as sodium triacetoxyborohydride, etc. by using a conventional method.

The solvent may be any solvent which does not disturb the reaction, but the reaction can be preferably performed in halogenated hydrocarbons such as dichloromethane, etc.

### Procedure (N)

Among the compound of the formula (11), the compound of the formula (90) can also be converted into the compound of the formula (71). For example, the method includes the following one. 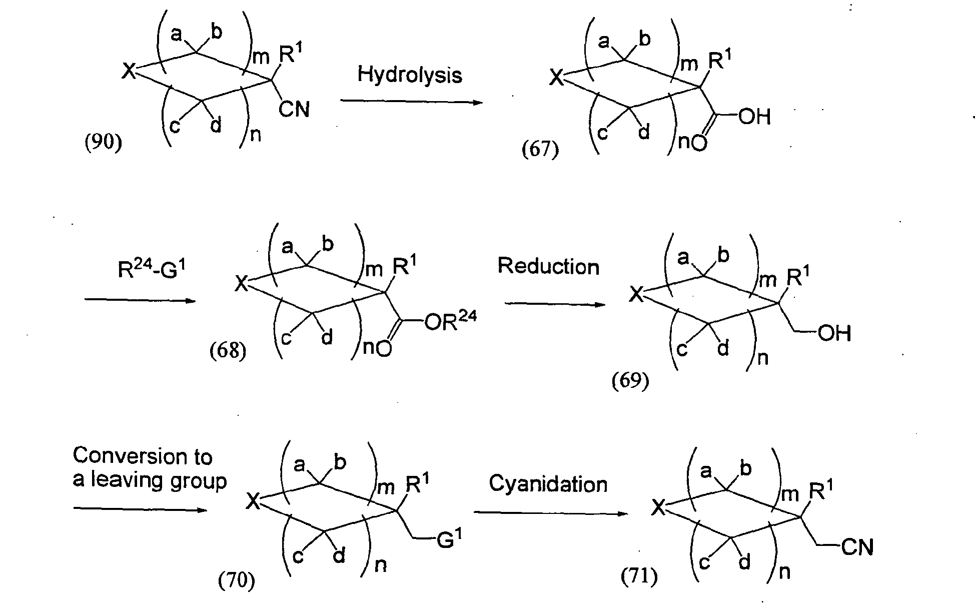 (wherein, m, n, R¹, a, b, c, d, X and G¹ are as defined above; R²⁴ is a substituted or unsubstituted alkyl group or a substituted or unsubstituted arylalkyl group)

The compound of the formula (90) can be hydrolyzed, for example, with an acid such as 48% aqueous hydrobromic acid solution, etc. to convert into the compound of the formula (67).

The compound of the formula (67) can be reacted with, for example, 1.0 to 3.0 equivalents of the compound of the formula: R²⁴-G¹ in a polar solvent such as N,N-dimethylformamide, etc., at a temperature of from 0°C to 120°C, preferably at a temperature of from room temperature to a boiling point of the solvent to be used, to convert into the compound of the formula (68).

The compound of the formula (68) can be reduced with, for example, lithium aluminum hydride, etc. by using a conventional method to convert into the compound of the formula (69).

When X is the formula: NR⁴ and R⁴ is the group of the formula: - C(=O)R⁷, the compound of the formula (67) can be reacted with, for example, 1 to 3 eqivalents of acetyl chloride in halogenated hydrocarbons solvent such as dichloromethane, at a temperature of from 0°C to room temperature to convert to a mixed acid anhydride, followed by reducing with a reducing agent such as sodium borohydride, etc. by using a conventional method, to synthesize the compound of the formula (69), without reducing a carbonyl group in the group of the formula: -C(=O)R⁷.

The compound of the formula (69) can be reacted with, for example, 2.0 to 3.0 equivalents or more of an arylsulfonyl chloride such as p-toluenesulfonyl chloride, etc., using 2.0 to 3.0 equivalents or more of pyridine as a solvent, at a temperature from room temperature to a boiling point of the solvent to be used, to give the compound of the formula (70).

The compound of the formula (70) can be reacted with, for example, 1.0 to 3.0 equivalents or more of a cyanide salt such as potassium cyanide, in a polar solvent such as dimethyl sulfoxide, etc. at a temperature from room temperature to 150°C to give the compound of the formula (71).

The above reaction is usually performed in a solvent and the solvent may be any solvent which does not disturb the reaction, for example, ethers (e.g., diethylether, diisopropylether, tetrahydrofuran, or 1,4-dioxane, etc.), aromatic hydrocarbons (e.g., benzene, toluene, or xylene, etc.), esters (e.g., methyl acetate, ethyl acetate, propyl acetate, etc.), halogenated hydrocarbons (e.g., dichloromethane, chloroform, dichloroethane, chlorobenzene, dichlorobenzene, etc.), ketones (e.g., acetone, methyl ethyl ketone, etc.), nitriles (e.g., acetonitrile, isobutyronitrile, etc.), N,N-dimethylformamide, and dimethyl sulfoxide.

A series of the reaction procedures from the compound of the formula (90) to that of the formula (71) can be performed repeatedly to increase the number of methylene group corresponding to the partial structure: -{C(e)(f)}- in the compound of the formula (11).

### Procedure (O)

Each of the following compounds can be converted from the compound of the formula (90), for example, by the following method. 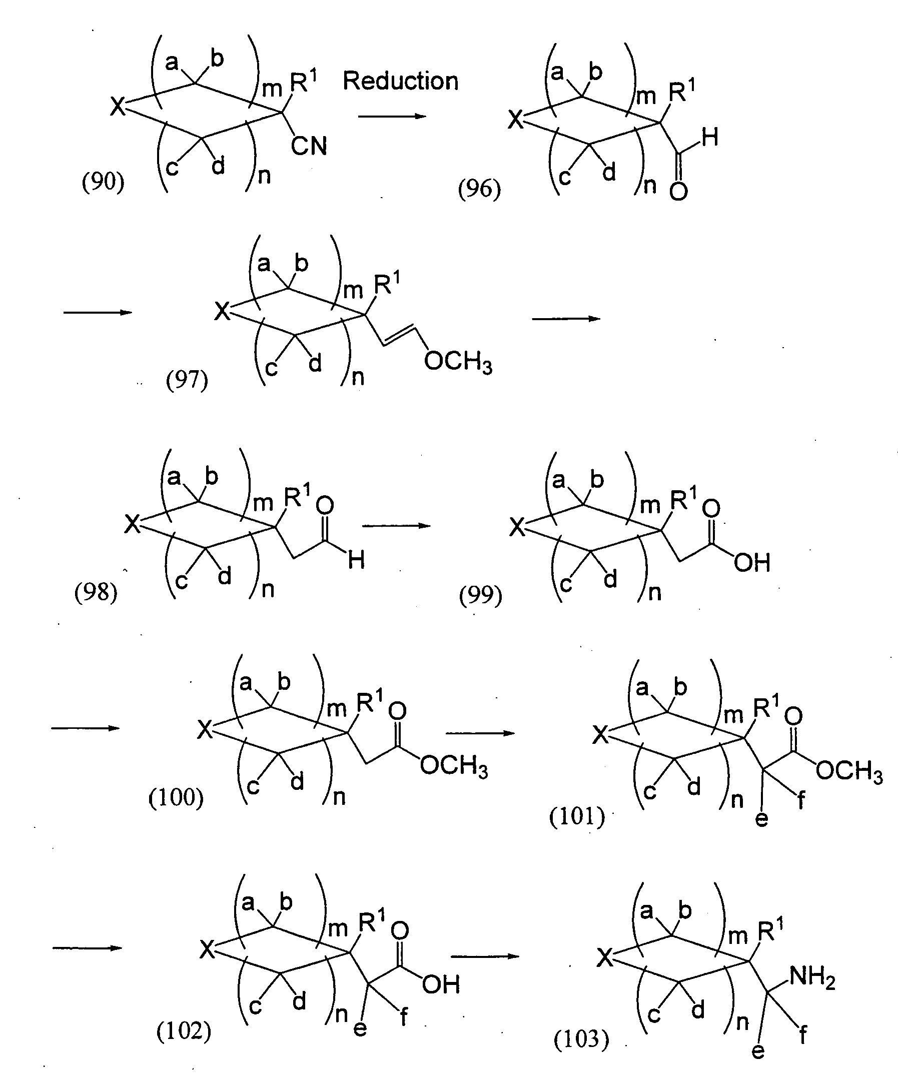 (wherein, X, m, n, R¹, a, b, c, d, e and f are as defined above)

The compound of the formula (90) can be reduced with, for example, 1 to 1.5 equivalents of diisobutylaluminum hydride (DIBAL), etc. in a halogenated hydrocarbon solvent such as dichloromethane, etc. at a temperature of from ice-cooling to room temperature, to convert into the compound of the formula (96).

Then the resulting compound can be reacted with, for example, the 1 to 1.5 equivalents of Wittig reagent such as (methoxymethyl)triphenylphosphonium chloride at an ether solvent such as tetrahydrofuran, etc. in the presence of 1 to 1.5 equivalents of a strong base such as sodium amide (NaNH₂), etc. at a temperature of from -78°C to room temperature, to convert into the compound of the formula (97).

The resulting compound can be reacted, for example, in a 1:1 mixture of a halogenated hydrocarbon solvent and trifluoroacetic acid, at a temperature of from ice-cooling to room temperature, to convert into the compound of the formula (98).

The resulting compound can be oxidized with, for example, 1 to 3 equivalents of an oxidizing agent such as potassium permanganate (KMnO₄), etc., at a temperature of from room temperature to -40°C, to convert into the compound of the formula (99).

The resulting compound can be reacted with, for example, 1 to 1.5 equivalents of methyl iodide, in a solvent such as N,N-dimethylformamide, etc. in the presense of a base such as potassium carbonate in about 3 equivalents at a temperature of from room temperature to a heat under reflux to convert into the compound of the formula (100).

The resulting compound can be reacted with, for example, 1 to 1.5 equivalents of the compound of the formula: e-G¹ in an ether solvent such as tetrahydrofuran, etc. in the presense of a strong base such as lithiumdiisopropylamide, etc. in 1 to 1.5 equivalents, at a temperature of from -78°C to room temperature, followed by, for example, with 1 to 1.5 equivalents of a compound of the formula: f-G² in the presense of a strong base such as lithiumdiisopropylamide, etc. to convert into the compound of the formula (101) (wherein, G¹, G², e and f are as defined above).

The resulting compound can be hydolysed with, for example, 1 to 3 equivalents of sodium hydroxide, etc., in a 1 : 4 mixed solvent of water - ethanol at a temperature of from room temperatue to a heat under reflux to convert into the compound of the formula (102).

Then, the resulting compound can undergo a curtius rearrangement, for example, with 1 to 1.5 equivalents of diphenylphosphorylazide and 1 to 1.5 equivalents of triethylamine, etc. in a solvent such as N,N-dimethylformamide, etc. at a temperature of from ice-cooling to 60°C to convert into the compound of the formula (103).

### Procedure (P)

In the compound of the formula (101), a carboxylic acid ester group can be converted to a cyanomethyl group, in a similar route as showed in the above procedure (N) that convert from the compound of the formula (68) to the compound of the formula (71), thus to give the compound of the formula (71'), as showed in the following reaction scheme. This compound can be hydrolysed, in a similar manner as showed in the above procedure (N) that convert from the compound of the formula (90) to the compound of the formula (67), thus to give the compound of the formula (67'), and further which is performed in a similar manner as showed in the above procedure (O) that convert from the compound of the formula (99) to the compound of the formula (101), thus to convert into the compound of the formula (101'). 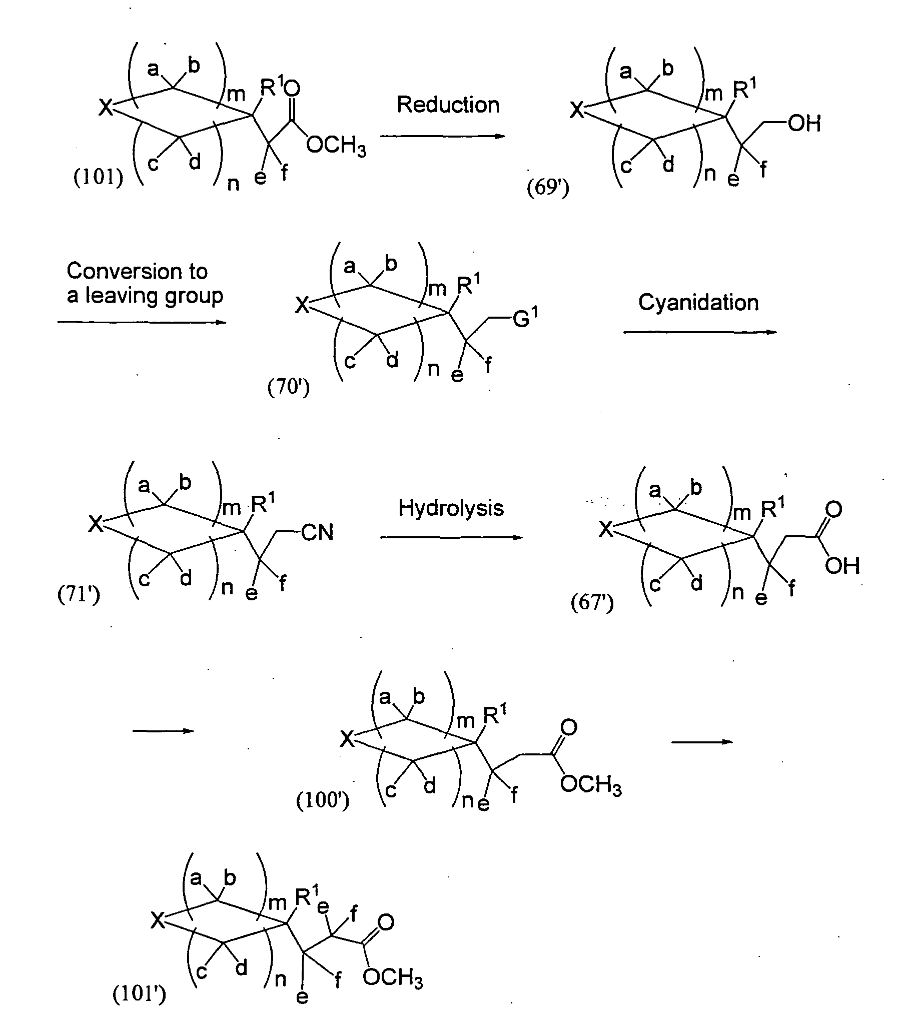 (wherein, X, m, n, R¹, a, b, c, d, e and f are as defined above)

Each of the starting material of the reaction explained above is a well-known compound or can be prepared from a well-known compound by using a method known to those skilled in the art or a similar method as thereto.

Each of the compounds obtained by the above procedure can be isolated and purified by the conventional isolation mehod including a recrystallization method, a purification method using a chromatography, an extraction with solvent, or a reprecipitaion, etc.

The products that can be obtained in any procedure can be a salt form or a free form depending on the reaction conditions. These products can be converted into a desirable salt form or a free form by using a conventional method.

The present invention is illustrated in more detail by the following Reference Examples and Examples and Testing Examples, but should not be construed to be limited thereto.

In this specification, the following abbreviations can be used for sake of simplicity of description.
THF: tetrahydrofuran
WSC: 1-(3-dimethylaminopropyl)-3-ethyl carbodiimide
rac-BINAP: racemi-2,2'-bis(diphenylphosphino)-1,1'-binaphtyl
Burgess reagent: (methoxycarbonylsulfamoyl)triethylammonium hydroxide

The conditions for performing the High performance liquid chromatography Analysis which decsribe a retention time in the following articles of Examples and Reference Examples are as follows:
Column: octadecyl group-chamically bonded type silica (ODS), particle size 5 µm, pore size 12 nm, column length 50 mm, column internal diameter 4.6 mm (Trade name: YMC CombiScreen ODS-A (S-5 µm, 12 nm) 50×4.6 mm (YMC Co., Ltd.))
Flow rate: 3.5 mL/ min
Detection wavelength: 220 nm
Mobile phase:
A phase; 0.05% aqueous trifluoroacetic acid solution
B phase; 0.035% trifluoroacetic acid-acetonitrile solution Time program:

| Step | time (min.) | A phase : B phase |
|---|---|---|
| 1 | 0.0-0.5 | 90 : 10 |
| 2 | 0.5-4.2 | 90: 10 → 1 : 99 |
| 3 | 4.2-4.4 | 1 : 99 → 99 : 1 |

### Reference Example 1-1

### Synthesis of phenyl sulfamate

To a solution of phenol (0.439 mL, 5.00 mmol) in heptane (20 mL) was added dropwise slowly chlorosulfonyl isocyanate (0.479 mL, 5.50 mmol) at room temperature and the mixture was heated under reflux for 10 hours. After cooling to room temerature, thereto was further added water (0.700 mL) and the mixture was heated under reflux for 2 hours. Thereto was further added water to quench the reaction and the mixture was extracted with ethyl acetate and the organic layer was washed twice with water. The organic layer was dried over anhydrous magnesium sulfate and filtered, and the solvent was evaporated under reduced pressure. The resulting residue was crystallized from hexane to give the title compound 536 mg as white crystals.
¹H-NMR δ (DMSO-d₆); 7.26-7.34 (3H, m), 7.43-7.48 (2H, m), 7.98 (2H, br).

### Reference Example 1-2

### Synthesis of 2-methylphenyl sulfamate

In the same manner as in Reference Example 1-1, the title compound was synthesized. The title compound was purified by silica gel column chromatography.
¹H-NMR δ (DMSO-d₆); 2.28 (3H, s), 7.17-7.31 (4H, m), 8.01 (2H, br).

### Reference Example 1-3

### Synthesis of 2-isopropylphenyl sulfamate

In the same manner as in Reference Example 1-1, the title compound was synthesized.
¹H-NMR δ (DMSO-d₆); 1.16 (6H, d, J = 6.06 Hz), 3.36 (1H, m), 7.25-7.38 (4H, m), 8.05 (2H, br).

### Reference Example 1-4

### Synthesis of 2,6-dimethylphenyl sulfamate

In the same manner as in Reference Example 1-1, the title compound was synthesized.
¹H-NMR δ (DMSO-d₆); 2.30 (6H, s), 7.06-7.08 (3H, m), 8.05 (1H, s).

### Reference Example 1-5

### Synthesis of mesityl sulfamate

In the same manner as in Reference Example 1-1, the title compound was synthesized. The title compound was purified by silica gel column chromatography.
¹H-NMR δ (DMSO-d₆); 2.20 (6H, s), 2.25 (3H, s), 6.88 (2H, s), 7.97 (2H, s).

### Reference Example 1-6

### Synthesis of 2,4-difluorophenyl sulfamate

In the same manner as in Reference Example 1-1, the title compound was synthesized. The title compound was purified by silica gel column chromatography.
¹H-NMR δ (DMSO-d₆); 7.14-7.21 (1H, m), 7.42-7.53 (1H, m), 8.26 (2H, br).

### Reference Example 1-7

### Synthesis of 2-tert-butylphenyl sulfamate

In the same manner as in Reference Example 1-1, the title compound was synthesized.
¹H-NMR δ (DMSO-d₆); 7.13-7.18 (1H, m), 7.23-7.28 (1H, m), 7.36-7.41 (1H, m), 7.51-7.58 (1H, m), 8.22 (2H, br).

### Reference Example 1-8

### Synthesis of 2-(trifluoromethyl)phenyl sulfamate

In the same manner as in Reference Example 1-1, the title compound was synthesized. The title compound was purified by silica gel column chromatography.
¹H-NMR δ (DMSO-d₆); 7.48 (1H, t, J = 7.5 Hz), 7.69-7.81 (4H, m), 8.42 (2H, br).

### Reference Example 1-9

### Synthesis of 2,6-diisopropylphenyl sulfamate

In the same manner as in Reference Example 1-1, the title compound was synthesized.
¹H-NMR δ (DMSO-d₆); 1.24 (12H, d, J = 6.96 Hz), 3.40-3.49 (2H, m), 5.07 (2H, br), 7.17-7.28 (3H, m).

### Reference Example 2-1

### Synthesis of 2-(bromomethyl)-1,3-dimethylbenzene

To a solution of 2,6-dimethylbenzyl alcohol (456 mg, 3.35 mmol) and triethylamine (0.560 mL, 4.02 mmol) in dichloromethane (CH₂Cl₂, 15 mL) was added methanesulfonyl chloride (0.258 mL, 3.68 mmol) under ice-cooling and the mixture was stirred for one hour. Thereto was subsequently added lithium bromide (LiBr, 582 mg, 6.70 mmol) under ice-cooling, and the mixture was warmed to room temperature and the mixture was stirred for 2 hours. Thereto was added water to quench the reaction and the reactant was extracted with ethyl acetate and the organic layer was washed twice with water. The organic layer was dried over anhydrous magnesium sulfate and filtered, and the solvent was evaporated under reduced pressure. The resulting residue was purified by silica gel column chromatography to give the title compound 364 mg as colorless oils.
¹H-NMR δ (DMSO-d₆); 2.37 (6H, s), 4.71 (2H, s), 7.04-7.16 (3H, m).

### Reference Example 2-2

### Synthesis of (2,6-dimethylphenyl)methanesulfonic acid

A solution of 2-(bromomethyl)-1,3-dimethylbenzene (300 mg, 1.52 mmol) and sodium sulfite (Na₂SO₃, 191 mg, 1.52 mmol) in 1,4-dioxane (5 mL) -water (5 mL) was heated under reflux for 5 hours. The solvent was evaporated, and the resulting sulfonate was washed with ethyl acetate and dissolved in 4N hydrogen chloride/ 1,4-dioxane solution (10 mL). The precipitated salt was collected by filtration and the solvent was evaporated under reduced pressure to give the title compound 136 mg as pale brown crystals.
¹H-NMR δ (DMSO-d₆); 2.38 (6H, s), 3.36 (1H, s), 3.88 (2H, s), 6.92-6.94 (3H, m).

### Reference Example 2-3

### Synthesis of 1-(2,6-dimethylphenyl)methanesulfonamide

To a solution of (2,6-dimethylphenyl)methanesulfonic acid (100 mg, 0.500 mmol) in N,N-dimethylformamide (DMF, 10 mL) was added dropwise thionyl chloride (SOCl₂ , 0.0802 mL, 1.10 mmol) under ice-cooling, and the mixture was stirred at such temperature for 2 hours. Then 28% aqueous ammonia (2.0 mL) was added under ice-cooling, and the mixture was warmed to room temperature and the mixture was stirred for 2 hours. The mixture was acidified with 3N hydrochloric acid and extracted with ethyl acetate, and the organic layer was washed once with 3N hydrochloric acid. The organic layer was dried over anhydrous magnesium sulfate and filtered, and the solvent was evaporated under reduced pressure. The resulting residue was purified by preparative thin layer chromatography (preparative TLC) to give the title compound 41.9 mg as white crystals.
¹H-NMR δ (DMSO-d₆); 2.39 (6H, s), 4.42 (2H, s), 7.01-7.12 (5H, m).

### Reference Example 2-4

### Synthesis of 1-(2-methylphenyl)methanesulfonamide

In the same manner as in Reference Examples 2-1 to 2-3, the title compound was synthesized.
¹H-NMR δ (DMSO-d₆); 2.37 (3H, s), 4.29 (2H, s), 6.90 (2H, s), 7.12-7.29 (4H, m).

### Reference Example 2-5

### Synthesis of 2,4,6-triisopropylbenzenesulfonamide

To a solution of 2,4,6-triisopropylbenzyl chloride (300 mg, 0.990 mmol) in 1,4-dioxane (10 mL) was added aqueous ammonia (5 mL) under ice-cooling. The mixture was warmed to room temperature and stirred for 2 hours. The solvent was evaporated under reduced pressure and the mixture was extracted with ethyl acetate, and the organic layer was washed twice with saturated aqueous ammonium chloride solution. The organic layer was dried over magnesium sulfate and filtered, and the solvent was evaporated under reduced pressure. The resulting residue was crystallized from hexane to give the title compound 193 mg as white crystals.
¹H-NMR δ (DMSO-d₆); 1.18 (18H, d, J = 6.75 Hz), 2.88 (1H, tt, J = 6.75 Hz), 4.10 (2H, d, J = 6.75 Hz), 7.17 (2H, s), 7.29 (2H, s).

### Reference Example 3

### Synthesis of methyl 2-methylphenyl amidophosphate

To a suspension of sodium hydride (213 mg, 5.34 mmol) in tetrahydrofuran (THF, 20 mL) was added o-crezol (524 mg, 4.85 mmol) at room temperature and the mixture was stirred for one hour. The reaction solution was added to a solution of methyl dichlorophosphonate (0.550 mL, 5.81 mmol) in tetrahydrofuran (THF, 20 mL) at room temperature and the mixture was stirred for 2 hours. The reaction solution was added to 28% aqueous ammonia (10 mL)-tetrahydrofuran (THF, 10 mL) at room temperature and the mixture was stirred at such temperature overnight. The reactant was extracted with ethyl acetate, and the organic layer washed twice with water. The organic layer was dried over anhydrous magnesium sulfate and filtered, and the solvent was evaporated under reduced pressure. The resulting residue was purified by silica gel column chromatography to give the title compound 197 mg as white crystals.
¹H-NMR δ (DMSO-d₆); 2.24 (3H, s), 3.66 (3H, d, J = 11.5 Hz), 5.01 (2H, d, J = 6.6 Hz), 7.02-7.28 (4H, m).

### Reference Example 4-1

### Synthesis of dimethyl (2-methylbenzyl)phosphate

2-methylbenzyl bromide (3.45 g, 18.6 mmol) was heated in trimethylphosphite (P(OMe)₃, 15mL) at 150°C for 10 hours. To water was added the reaction mixture to quench the reaction, and the solvent was evaporated, and the resulting sulfonate was washed with ethyl acetate. The reactant was extracted with ethyl acetate and the organic layer was washed twice with water. The organic layer was dried over anhydrous magnesium sulfate and filtered, and the solvent was evaporated under reduced pressure. The resulting residue was purified by silica gel column chromatography to give the title compound 3.28 g as colorless oils.
¹H-NMR δ (DMSO-d₆); 2.31 (3H, d, J = 1.65 Hz), 3.22 (2H, d, J = 21.6 Hz), 3.54 (3H, s), 3.59 (3H, s), 7.10-7.19 (4H, m).

### Reference Example 4-2

### Synthesis of methyl hydrogen (2-methylbenzyl)phosphate

To a solution of dimethyl (2-methylbenzyl)phosphate (2.00 g, 9.34 mmol) in ethanol (30 mL) -water (15 mL) was added sodium hydroxide (1.87 g, 46.7 mmol) at room temperature and the mixture was heated under reflux for 10 hours. Thereto was further added 2N aqueous lithium hydroxide solution (0.149 mL, 0.284 mmol) at room temperature, and the mixture was heated under reflux for 4 hours. The solvent was evaporated and the resulting phosphate was washed with ethyl acetate. After the phosphate was dissolved in 4N hydrogen chloride/1,4-dioxane solution (14 mL), the precipitated salt was collected by filtration. The solvent was evaporated under reduced pressure to give the title compound 1.71 g as white crystals.
¹H-NMR δ (DMSO-d₆); 2.32 (3H, s), 3.01 (2H, d, J = 21.6 Hz), 3.46 (3H, d, J = 10.8 Hz), 7.06-7.21 (4H, m).

### Reference Example 4-3

### Synthesis of methyl P-(2-methylbenzyl)phosphonamidate

To methyl hydrogen (2-methylbenzyl) phosphate (200 mg, 0.999 mmol) in N,N-dimethylformamide (DMF, 20 mL) under ice-cooling was added dropwise thionyl chloride (SOCl₂ , 0.160 mL, 2.20 mmol) and the mixture was stirred at such temperature for 5 hours. Then, thereto was added 28% aqueous ammonia (4.0 mL) under ice-cooling, and the mixture was warmed to room temperature and the mixture was stirred overnight. The reaction was quenched with 10% hydrochloric acid, and the reactant was extracted with ethyl acetate, and the organic layer was washed twice with water. The organic layer was dried over anhydrous magnesium sulfate, and filtered, and the solvent was evaporated under reduced pressure to give the title compound 62.0 mg as colorless oils.
¹H-NMR δ (DMSO-d₆); 2.33 (3H, s), 2.95-3.12 (2H, m), 3.47 (3H, d, J = 11.0 Hz), 4.31 (2H, d, J = 4.77 Hz), 7.07-7.23 (4H, m).

### Reference Example 5

### Synthesis of 2-(2-methylphenyl)acetamide

To a solution of o-tolylacetic acid (1.00 g, 6.66 mmol) in dichloromethane (CH₂Cl₂ , 20 mL) was added dropwise oxalyl chloride ((COCl)₂ , 0.717 mL, 7.99 mmol) at room temperature, followed by addition of N,N-dimethylformamide (DMF, one drop) and the mixture was stirred at such temperature for 4 hours. Then thereto was added 28% aqueous ammonia (5 mL) under ice-cooling, and the mixture was warmed to room temperature and stirred for 3 hours. The reaction was quenched with 10% hydrochloric acid, and the mixture was extracted with ethyl acetate, and the organic layer was washed once with water.

The organic layer was dried over anhydrous magnesium sulfate and filtered, and the solvent was evaporated under reduced pressure. The resulting residue was crystallized from a mixture of diethylether-hexane to give the title compound 701 mg as white crystals.
¹H-NMR δ (DMSO-d₆); 2.25 (3H, s), 3.40 (2H, s), 6.87 (1H, br), 7.08-7.19 (4H, m), 7.36 (1H, br).

### Reference Example 6

### Synthesis of 4-hydroxy-2-(2-hydroxyethyl)-2-(3-methoxyphenyl)-butanenitrile

To a solution of sodium hydride (1.79 g, 44.8 mmol) in dimethyl sulfoxide (DMSO, 60 mL) was added a solution of (3-methoxyphenyl)acetonitrile (2.50 mL, 17.9 mmol) and 2-bromoethyl tert-butyl dimethylsilylether (9.22 mL, 43.0 mmol) in diethylether (20 mL) at room temperature, and the mixture was stirred overnight.

Thereto was added water and the mixture was extracted twice with diethylether, and the organic layer was washed three times with water.

The organic layer was dried over anhydrous magnesium sulfate and filtered, and the solvent was evaporated under reduced pressure.

The residue was dissolved in tetrahydrofuran (THF, 100 mL), and thereto was added tetrabutylammonium fluoride (TBAF, 12.2 g, 46.5 mmol), and the mixture was stirred at room temperature overnight. Thereto was added water and the mixture was extracted twice with ethyl acetate, and the organic layer was washed twice with water. The organic layer was dried over anhydrous magnesium sulfate and filtered, and the solvent was evaporated under reduced pressure. The resulting residue was purified by silica gel column chromatography to give the title compound 2.91 g as colorless oils.
¹H-NMR δ (DMSO-d₆); 1.97-2.02 (4H, m), 3.12-3.22 (2H, m), 3.32-3.45 (2H, m), 3.76 (3H, s), 4.59 (2H, t, J = 5.13 Hz), 6.87-7.03 (3H, m), 7.31-7.36 (1H, m).

### Reference Example 7-1

### Synthesis of 1-benzhydryl-4-(3-methoxyphenyl)piperidine-4-carbonitrile

To a solution of 4-hydroxy-2-(2-hydroxyethyl)-2-(3-methoxyphenyl)butanenitrile (1.80 g, 7.65 mmol) in acetonitrile (180 mL) were added successively anhydrous trifluoromethane sulfonic acid (2.83 mL, 16.8 mmol) and triethylamine (2.34 mL, 16.8 mmol) at -30 to -20°C and the mixture was stirred for 15 min. Thereto were added aminodiphenylmethane (1.68 g, 9.18 mmol) and triethylamine (2.34 mL, 16.8 mmol) at -30 to -20°C, and the mixture was warmed to room temperature slowly and stirred for 2 hours. Thereto was added water to quench the reaction, and the mixture was extracted with ethyl acetate, and the organic layer was washed twice with water. The organic layer was dried over anhydrous magnesium sulfate, and filtered, and evaporated under reduced pressure. The resulting residue was purified by silica gel column chromatography to give the title compound 2.45 g as white crystals.
¹H-NMR δ (DMSO-d₆); 2.08 (4H, m), 2.18-2.26 (2H, m), 2.88-2.93 (2H, m), 3.78 (3H, s), 4.45 (1H, s), 6.91-6.94 (1H, m), 7.03-7.05 (1H, m), 7.09-7.12 (1H, m), 7.16-7.21 (2H, m), 7.27-7.37 (5H, m), 7.44-7.47 (4H, m).

### Reference Example 7-2

### Synthesis of 1-(2-methoxyphenyl)-4-(3-methoxyphenyl)piperidine-4-carbonitrile

In the same manner as in Reference Example 7-1, the title compound was synthesized.
¹H-NMR δ (DMSO-d₆); 2.16-2.20 (4H, m), 2.85-2.93 (2H, m), 3.49-3.53 (2H, m), 3.79 (6H, m), 6.87-7.02 (5H, m), 7.08-7.17 (2H, m), 7.35-7.40 (1H, m).

### Reference Example 7-3

### Synthesis of 1-(2-methoxyphenyl)-4-phenylpiperidine-4-carbonitrile

In the same manner as in Reference Example 7-1, the title compound was synthesized.
High performance liquid chromatography/ Mass spectrometry
m/z 293.3 (M+H)
Retention time: 3.21 minute (min.)

### Reference Example 8

### Synthesis of 4-(3-methoxyphenyl)piperidine-4-carbonitrile

A suspension of 1-benzhydryl-4-(3-methoxyphenyl)piperidine-4-carbonitrile (34.3 mg, 0.090 mmol), acetic acid (0.005 mL, 0.090 mmol) and 10% palladium hydroxide (3.4 mg) in methanol (20 mL) was stirred under an atmosphere of hydrogen for 3 hours. The mixture was filtered on celite and the solvent was evaporated. Thereto was added saturated aqueous sodium hydrogen carbonate solution, and the mixture was extracted with ethyl acetate and the organic layer was washed once with saturated aqueous sodium hydrogen carbonate solution. The organic layer was dried over anhydrous magnesium sulfate, and filtered, and the solvent was evaporated under reduced pressure. The resulting residue was purified by preparative thin layer chromatography to give the title compound 12.9 mg as oils.
¹H-NMR δ (DMSO-d₆); 1.86-2.06 (4H, m), 2.79-2.87 (2H, m), 3.07-3.11 (2H, m), 3.77 (3H, s), 6.92-6.96 (1H, m), 7.00-7.08 (2H, m), 7.33-7.39 (1H, m).

### Reference Example 9

### Synthesis of 1-[3-(methoxy)pyridin-2-yl]-4-(3-methoxyphenyl)piperidine-4-carbonitrile

To a solution of tris(dibenzylideneacetone)dipalladium (Pd₂ (dba)₃, 419 mg, 10 mol%) and 2,2'-bis(diphenylphosphino)-1,1'-binaphtyl (BINAP, 570 mg, 20 mol%) in toluene (30 mL) were added 2-bromo-3-methoxypyridine (1.30 g, 6.87 mmol), 4-(3-methoxyphenyl)piperidine-4-carbonitrile (990 mg, 4.58 mmol) and sodium tert-butyrate (1.54 g, 13.7 mmol) at room temperature, and the mixture was heated under reflux for 3 hours. Thereto was added saturated aqueous sodium chloride solution to quench the reaction, and the mixture was extracted with ethyl acetate, and the organic layer was washed twice with saturated aqueous sodium chloride solution. The organic layer was dried over anhydrous magnesium sulfate and filtered, and the solvent was evaporated under reduced pressure. The resulting residue was purified by silica gel column chromatography to give the title compound 920 mg as yellow crystals.
High performance liquid chromatography/Mass spectrometry
m/z 324.1 (M+H)
Retention time: 2.48 min.

### Reference Example 10-1

### Synthesis of 4-(3-methoxyphenyl)-1-pyrimidin-2-ylpiperidine-4-carbonitrile

To a suspension of sodium hydride (18.5 mg, 0.555 mmol) in dimethyl sulfoxide (DMSO, 15 mL) was added 4-(3-methoxyphenyl)piperidine-4-carbonitrile (200 mg, 0.462 mmol) at room temperature, followed by addition of 2-bromopyrimidine (88.2 mg, 0.555 mmol) at room temperature and the mixture was stirred overnight. Thereto was added water to quench the reaction, and the mixture was extracted with ethyl acetate, and the organic layer was washed twice with water. The organic layer was dried over anhydrous magnesium sulfate and filtered, and the solvent was evaporated under reduced pressure. The resulting residue was purified by preparative thin layer chromatography to give the title compound 55.8 mg as colorless oils.
High performance liquid chromatography/ Mass spectrometry
m/z 295.3 (M+H)
Retention time: 3.42 min.

### Reference Example 10-2

### Synthesis of 1-(1,3-benzoxazol-2-yl)-4-(3-methoxyphenyl)piperidine-4-carbonitrile

In the same manner as in Reference Example 10-1, the title compound was synthesized.
High performance liquid chromatography/Mass spectrometry
m/z 334.5 (M+H)
Retention time: 3.51 min.

### Reference Example 10-3

### Synthesis of 1-(1,3-benzothiazol-2-yl)-4-(3-methoxyphenyl)piperidine-4-carbonitrile

In the same manner as in Reference Example 10-1, the title compound was synthesized.
High performance liquid chromatography/Mass spectrometry
m/z 350.4 (M+H)
Retention time : 3.53 min.

### Reference Example 11

### Synthesis of 4-(3-methoxyphenyl)-1-(2,2,2-trifluoroethyl)piperidine-4-carbonitrile

To a solution of 4-(3-methoxyphenyl)piperidine-4-carbonitrile (250 mg, 1.162 mmol) and triethylamine (0.243 mL, 17.4 mmol) in dichloromethane (CH₂Cl₂, 15 mL) was added 2,2,2-trifluoroethyl trifluoromethanesulfonate (322 mg, 1.39 mmol) under ice-cooling, and the mixture was warmed to room temperature and stirred overnight. Thereto was further added 2,2,2-trifluoroethyl trifluoromethanesulfonate (322 mg, 1.39 mmol) under ice-cooling, and the mixture was warmed to room temperature and stirred overnight. Thereto was added water to quench the reaction, and the mixture was extracted with ethyl acetate, and the organic layer was washed twice with water. The organic layer was dried over anhydrous magnesium sulfate and filtered, and the solvent was evaporated under reduced pressure. The resulting residue was purified by silica gel column chromatography to give the title compound 315 mg as colorless oils.
High performance liquid chromatography/Mass spectrometry
m/z 299.2 (M+H)
Retention time: 3.67 min.

### Reference Example 12

### Synthesis of 1-benzyl-4-(3-methoxyphenyl)piperidine-4-carbonitrile

To a solution of 4-(3-methoxyphenyl)piperidine-4-carbonitrile (1.00 g, 4.62 mmol) and potassium carbonate (1.92 g, 13.9 mmol) in N,N-dimethylformamide (DMF, 20 mL) was added benzyl bromide (0.665 mL, 5.09 mmol) at room temperature, and the mixture was stirred at such temperature overnight. Thereto was added water to quench the reaction, and the mixture was extracted with ethyl acetate, and the organic layer was washed twice with water. The organic layer was dried over anhydrous magnesium sulfate and filtered, and the solvent was evaporated under reduced pressure. The resulting residue was purified by silica gel column chromatography to give the title compound 1.21 g as colorless oils.
¹H-NMR δ (DMSO-d₆); 1.97-2.11 (4H, m), 2.27-2.35 (2H, m), 2.91-2.95 (2H, m), 3.55 (2H, s), 3.77 (3H, s), 6.90-6.94 (1H, m), 7.02-7.04 (1H, m), 7.08-7.11 (1H, m), 7.24-7.37 (6H, m).

### Reference Example 13

### Synthesis of 1-[2-(2-methoxyethoxy)phenyl]-4-(3-methoxyphenyl)-piperidine-4-carbonitrile

### Synthesis of 1-(2-hydroxyphenyl)-4-(3-methoxyphenyl)piperidine-4-carbonitrile

To a solution of 2-aminophenol (2.00 g, 18.3 mmol) and triethylamine (3.32 mL, 23.8 mmol) in N,N-dimethylformamide (DMF, 100 mL) was added trimethylsilylchloride (2.79 mL, 22.0 mmol) under ice-cooling, and the mixture was warmed to room temperature and stirred for 2 hours. Thereto was added water to quench the reaction, and the mixture was extracted with diethylether, and the organic layer was washed twice with water. The organic layer was dried over anhydrous magnesium sulfate and filtered, and the solvent was evaporated under reduced pressure to give the reaction mixture 3.31g.

On the other hand, to a solution of 4-hydroxy-2-(2-hydroxyethyl)-2-(3-methoxyphenyl)butanenitrile (400 mg, 1.70 mmol) in acetonitrile (50 mL) were added successively anhydrous trifluoromethanesulfonic acid (0.629 mL, 3.74 mmol) and triethylamine (0.521 mL, 3.74 mmol) at -30 to -20°C, and the mixture was stirred for 15 min, and at -30 to -20°C, thereto were added 400 mg of the above-obtained reaction mixture 3.31g and triethylamine (0.616 mL, 4.42 mmol), and the mixture was warmed to room temperature slowly and stirred for one hour. Thereto was added water to quench the reaction, and the mixture was extracted with diethylether, and the organic layer was washed once with water. The organic layer was dried over anhydrous magnesium sulfate and filtered, and the solvent was evaporated under reduced pressure to give the reaction mixture 625 mg.

Then, the resulting reaction mixture was dissolved in tetrahydrofuran (20 mL), and thereto was added tetrabutylammonium fluoride (943 mg, 3.61 mmol) at room temperature, and the mixture was stirred for 3 hours. Thereto was added saturated aqueous ammonium chloride solution to quench the reaction, and the mixture was extracted with ethyl acetate, and the organic layer was washed once with saturated aqueous ammonium chloride solution and once with a saturated aqueous sodium bicarbonate solution. The organic layer was dried over anhydrous magnesium sulfate and filtered, and the solvent was evaporated under reduced pressure. The resulting residue was crystallized from diethylether to give the title compound 255 mg as pale brown powder.
High performance liquid chromatography/ Mass spectrometry
m/z 309.6 (M+H)
Retention time: 2.92 min.

### 1-[2-(2-methoxyethoxy)phenyl]-4-(3-methoxyphenyl)piperidine-4-carbonitrile

To a suspension of sodium hydride (31.2 mg, 0.779 mmol) in N,N-dimethylformamide (DMF, 20 mL) was added 1-(2-hydroxyphenyl)-4-(3-methoxyphenyl)piperidine-4-carbonitrile (200 mg, 0.649 mmol) at room temperature, followed by addition of 2-bromoethylmethylether (0.073 mL, 0.779 mmol), and the mixture was stirred for 4 hours. Thereto was added saturated aqueous ammonium chloride solution to quench the reaction, and the mixture was extracted with ethyl acetate, and the organic layer was washed twice with saturated aqueous ammonium chloride solution and once with saturated aqueous sodium bicarbonate solution. The organic layer was dried over anhydrous magnesium sulfate and filtered, and the solvent was evaporated under reduced pressure. The resulting residue was purified by silica gel column chromatography to give the title compound 221 mg as colorless oils.
High performance liquid chromatography/ Mass spectrometry
m/z 367.3 (M+H)

### Reference Example 14

### Synthesis of 1-(2-methoxyphenyl)-4-(3-methoxyphenyl)azepane-4-carbonitrile

### N-(3-chloropropyl)-2-methoxyaniline

To a suspension of o-anisidine (3.00 mL, 26.6 mmol) and potassium carbonate (11.0 g, 79.8 mmol) in N,N-dimethylformamide (DMF, 50 mL) was added 1-bromo-3-chloropropane (2.37 mL, 23.9 mmol) at room temperature, and the mixture was warmed to 55°C and stirred for 5 hours. Thereto was added water to quench the reaction, and the mixture was extracted with ethyl acetate, and the organic layer was washed twice with water. The organic layer was dried over anhydrous magnesium sulfate and filtered, and the solvent was evaporated under reduced pressure. The resulting residue was purified by silica gel column chromatography to give the title compound 2.00 g as colorless oils.
¹H-NMR δ (DMSO-d₆); 1.99 (2H, t, J = 6.57 Hz), 3.17 (2H, t, J = 6.57 Hz), 3.70 (2H, t, J = 6.57 Hz), 3.75 (3H, s), 4.91 (1H, t, J = 5.85 Hz), 6.50-6.56 (2H, m), 6.73-6.80 (2H, m).

### 2-chloro-N-(3-chloropropyl)-N-(2-methoxyphenyl)acetamide

To a solution of N-(3-chloropropyl)-2-methoxyaniline (1.00 g, 5.00 mmol) and triethylamine (0.906 mL, 6.50 mmol) in dichloromethane (CH₂Cl₂ , 20 mL) was added chloroacetylchloride (0.478 mL, 6.00 mmol) under ice-cooling, and the mixture was warmed to room temperature and stirred overnight. Thereto was added water to quench the reaction, and the mixture was extracted with ethyl acetate, and the organic layer was washed twice with water. The organic layer was dried over anhydrous magnesium sulfate and filtered, and the solvent was evaporated under reduced pressure. The resulting residue was purified by silica gel column chromatography to give the title compound 1.27 g as colorless oils.
¹H-NMR δ (DMSO-d₆); 2.01 (2H, m), 3.56 (2H, m), 3.76-3.82 (4H, m), 3.86 (3H, s), 6.99-7.05 (2H, m) 7.18-7.21 (1H, m), 7.36-7.42 (1H, m).

### N-(2-chloroethyl)-N-(3-chloropropyl)-2-methoxyaniline

To a solution of 2-chloro-N-(3-chloropropyl)-N-(2-methoxyphenyl)-acetamide (847 mg, 3.07 mmol) in tetrahydrofuran (THF, 20 mL) was added a solution of borane-tetrahydrofuran in tetrahydrofuran (1.13 M, 4.07 mL, 4.60 mmol) under ice-cooling, and the mixture was warmed to room temperature and stirred overnight. Thereto was added methanol to quench the reaction and the solvent was evaporated under reduced pressure. The residue was extracted with ethyl acetate, and the organic layer was washed twice with saturated aqueous ammonium chloride solution. The organic layer was dried over anhydrous magnesium sulfate and filtered, and the solvent was evaporated under reduced pressure. The resulting residue was purified by silica gel column chromatography to give the title compound 370 mg as colorless oils.
¹H-NMR δ (DMSO-d₆); 1.79 (2H, tt, J = 6.78, 6.78 Hz), 3.25 (2H, t, J = 6.78 Hz), 3.34 (2H, t, J = 6.78 Hz), 3.58 (2H, t, J = 6.78 Hz), 3.77 (3H, s) 6.82-6.88 (1H, m), 6.94-7.00 (3H, m).

### 1-(2-methoxyphenyl)-4-(3-methoxyphenyl)azepane-4-carbonitrile

To a suspension of sodium hydride (494 mg, 12.3 mmol) in dimethyl sulfoxide (DMSO, 300 mL) was added a solution of 2-chloro-N-(3-chloropropyl)-N-(2-methoxyphenyl)acetamide (1.47 g, 5.61 mmol) and (3-methoxyphenyl)acetonitrile (0.783 mL, 5.61 mmol) in dimethyl sulfoxide (DMSO, 50 mL) at room temperature, and the mixture was stirred overnight. Thereto was added saturated aqueous ammonium chloride solution to quench the reaction and the mixture was extracted with ethyl acetate, and the organic layer was washed once with saturated aqueous ammonium chloride solution and once with saturated aqueous sodium bicarbonate solution. The organic layer was dried over anhydrous magnesium sulfate and filtered, and the solvent was evaporated under reduced pressure. The resulting residue was purified by silica gel column chromatography to give the title compound 1.00 g as colorless oils.
¹H-NMR δ (DMSO-d₆); 2.15-2.60 (6H, m), 3.21-3.62 (4H, m), 3.83 (6H, s), 6.83-7.03 (5H, m), 7.09-7.16 (2H, m), 7.28-7.34.

### Reference Example 15

### Synthesis of N,N-bis(2-chloroethyl)aniline

To a solution of N-phenyldiethanolamine (10.0 g, 55.2 mmol) in toluene (200 mL) was added thionyl chloride (9.70 mL, 132 mmol) at room temperature, and the mixture was warmed to 100°C and stirred for one hour. Thereto was added saturated aqueous sodium bicarbonate solution to quench the reaction, and the mixture was extracted twice with diethylether, and the organic layer was washed twice with water. The organic layer was dried over anhydrous magnesium sulfate and filtered, and the solvent was evaporated under reduced pressure. The resulting residue was purified by silica gel column chromatography to give the title compound 5.86 g as colorless oils.
¹H-NMR δ (DMSO-d₆); 3.60-3.66 (4H, m), 3.71-3.76 (4H, m), 6.68-6.71 (2H, m), 6.75-6.80 (1H, m), 7.24-7.29 (2H, m).

### Reference Example 16

### Synthesis of 1-(3-methoxyphenyl)-4-phenylcyclohexanecarbonitrile

To a suspension of sodium hydride (100 mg, 2.62 mmol) in dimethylsulfoxide (DMSO, 50 mL) was added a solution of [3-chloro-1-(2-chloroethyl)propyl]benzene (400 mg, 1.31 mmol) and (3-methoxyphenyl)acetonitrile (0.182 mL, 1.28 mmol) in dimethylsulfoxide (DMSO, 5 mL) at room temperature and the mixture was stirred overnight. Thereto was added saturated aqueous ammonium chloride solution to quench the reaction and the mixture was extracted with ethyl acetate, and the organic layer was washed once with saturated aqueous ammonium chloride solution and once with saturated aqueous sodium bicarbonate solution. The organic layer was dried over anhydrous magnesium sulfate and filtered, and the solvent was evaporated under reduced pressure. The resulting residue was purified by preparative TLC to give the title compound 305 mg as colorless oils.
¹H-NMR δ (DMSO-d₆); 1.77-2.20 (8H, m), 2.67-2.76 (1H, m), 3.77 (3H, s), 6.92-6.97 (1H, m), 7.07-7.39(8H, m).

### Reference Example 17-1

### Synthesis of methyl 1-(2-methoxyphenyl)-4-(3-methoxyphenyl)-piperidine-4-carboxylate

A suspension of 1-(2-benzyloxyphenyl)-4-(3-methoxyphenyl)-piperidine-4-carbonitrile (1.00g, 3.24 mmol) in 48% aqueous hydrogen bromide solution (20 mL) was heated under reflux for 12 hours. The solvent was evaporated under reduced pressure, and the precipitated solid was washed with ethyl acetate. The resulting reaction mixture was dissolved in N,N-dimethylformamide (DMF, 20 mL), and thereto were added iodomethane (0.666 mL, 10.7 mmol) and potassium carbonate (5.38 g, 38.9 mmol) at room temperature, and the mixture was warmed to 55°C and then stirred for 4 hours. Thereto was added water to quench the reaction, and the mixture was extracted with ethyl acetate, and the organic layer was washed twice with water. The organic layer was dried over anhydrous magnesium sulfate and filtered, and the solvent was evaporated under reduced pressure and the resulting residue was purified by silica gel column chromatography. The title compound was obtained in 990 mg as colorless oils.
¹H-NMR δ (DMSO-d₆); 1.96-2.02 (2H, m), 2.62-2.69 (6H, m), 3.60 (3H, s), 3.75 (3H, s), 3.76 (3H, s), 6.84-6.98 (7H, m), 7.26-7.31 (1H, m).

### Reference Example 17-2

### Synthesis of methyl 4-(3-methoxyphenyl)-l-pyrimidin-2-ylpiperidine-4-carboxylate

4-(3-methoxyphenyl)-1-pyrimidin-2-ylpiperidine-4-carbonitrile (70 mg, 0.238 mmol) was added to 12N hydrochloric acid (5 mL), and this resulting suspension was heated under reflux for 12 hours. The solvent was evaporated under reduced pressure, and the precipitated solid was washed with ethyl acetate. The resulting reaction mixture was dissolved in N,N-dimethylformamide (DMF, 10 mL), and thereto were added iodomethane (0.0593 mL, 0.952 mmol) and potassium carbonate (263 mg, 1.90 mmol) at room temperature, and the mixture was warmed to 35°C and then stirred for 4 hours. Thereto was added water to quench the reaction, and the mixture was extracted with ethyl acetate, and the organic layer was washed twice with water. The organic layer was dried over anhydrous magnesium sulfate and filtered, and the solvent was evaporated under reduced pressure and, the resulting residue was purified by preparative thin layer chromatography. The title compound was obtained in 30.4 mg as colorless oils.
High performance liquid chromatography/ Mass spectrometry
m/z 328.3 (M+H)
Retention time: 3.24 min.

### Reference Example 17-3

### Synthesis of methyl 4-(3-methoxyphenyl)-1-(3-methoxypyridin-2-yl)-piperidine-4-carboxylate

In the same manner as in Reference Example 17-1, the title compound was synthesized.
High performance liquid chromatography/Mass spectrometry
m/z 357.2 (M+H)

### Retention time: 2.76 min.

### Reference Example 17-4

### Synthesis of methyl 1-(2-methoxyphenyl)-4-phenylpiperidine-4-carboxylate

In the same manner as in Reference Example 17-1, the title compound was synthesized.
High performance liquid chromatography/Mass spectrometry
m/z 326.4 (M+H)

### Retention time: 4.11 min.

### Reference Example 17-5

### Synthesis of methyl 1-benzyl-4-(3-methoxyphenyl)piperidine-4-carboxylate

In the same manner as in Reference Example 17-1, the title compound was synthesized.
High performance liquid chromatography/Mass spectrometry
m/z 340.4 (M+H)

### Retention time: 2.82 min.

### Reference Example 17-6

### Synthesis of methyl 4-(3-methoxyphenyl)-1-(2,2,2-trifluoroethyl)-piperidine-4-carboxylate

### methyl 4-(3-hydroxyphenyl)-1-(2,2,2-trifluoroethyl)piperidine-4-carboxylate

A suspension of 4-(3-methoxyphenyl)piperidine-4-carbonitrile (115 mg, 0.386 mmol) in 47% aqueous hydrogen bromide solution (500 mL) was heated under reflux for 4 hours and then the solvent was evaporated under reduced pressure. The precipitated solid was washed with ethyl acetate. To a suspension of the above solid and potassium carbonate (K₂ CO₃, 267 mg, 1.93 mmol) in N,N-dimethylformamide (15 mL) was added methyl iodide (0.060 mL, 0.964 mmol) at room temperature, and the mixture was warmed to 55°C and heated for 5 hours. Thereto was added water to quench the reaction and then the mixture was extracted with ethyl acetate and the organic layer was washed twice with water. The solvent was evaporated under reduced pressure and then the resulting residue was purified by preparative thin layer chromatography to give the title compound 46 mg as colorless oils.
High performance liquid chromatography/ Mass spectrometry
m/z 340.4 (M+H)
Retention time: 2.82 min.

### methyl 4-(3-methoxyphenyl)-1-(2,2,2-trifluoroethyl)piperidine-4-carboxylate

To a solution of methyl 4-(3-hydroxyphenyl)-1-(2,2,2-trifluoroethyl)piperidine-4-carboxylate (46.0 mg, 0.145 mmol) and methyl iodide (0.0118 mL, 0.188 mmol) in tetrahydrofuran (100 mL) was added sodium hydride (NaH, 8.70 mg, 0.218 mmol) at room temperature, and the mixture was stirred overnight. Thereto was added saturated aqueous ammonium chloride solution to quench the reaction and the mixture was extracted with ethyl acetate, and the organic layer was washed twice with water. The organic layer was dried over anhydrous magnesium sulfate and filtered, and the solvent was evaporated under reduced pressure. The resulting residue was purified by preparative thin layer chromatography to give the title compound 44.9 mg as colorless oils.
High performance liquid chromatography/ Mass spectrometry
m/z 332.5 (M+H)

### Retention time: 2.92 min.

### Reference Example 17-7

### Synthesis of methyl 1-(3-methoxyphenyl)-4-phenylcyclohexane carboxylate

In the same manner as in Reference Example 17-1, the title compound was synthesized.
¹H-NMR δ (DMSO-d₆); 1.50-1.58 (2H, m), 1.66-1.88 (4H, m), 3.65 (3H, s), 3.74 (3H, s), 6.82-6.94 (3H, m), 7.14-7.30 (6H, m).

### Reference Example 17-8

### Synthesis of methyl 4-(3-methoxyphenyl)-1-phenylpiperidine-4-carboxylate

In the same manner as in Reference Example 17-2, the title compound was synthesized.
High performance liquid chromatography/ Mass spectrometry
m/z 326.4 (M+H)

### Retention time: 3.98 min.

### Reference Example 17-9

### Synthesis of methyl 4-(3-methoxyphenyl)-1-pyrimidin-2-ylpiperidine-4-carboxylate

In the same manner as in Reference Example 17-2, the title compound was synthesized.

High performance liquid chromatography/ Mass spectrometry
m/z 328.3 (M+H)

### Retention time: 3.08 min.

### Reference Example 17-10

### Synthesis of methyl 1-(1,3-benzoxazole-2-yl)-4-(3-methoxyphenyl)-piperidine-4-carboxylate

In the same manner as in Reference Example 17-2, the title compound was synthesized.
High performance liquid chromatography/ Mass spectrometry
m/z 367.0 (M+H)

### Retention time: 3.24 min.

### Reference Example 17-11

### Synthesis of methyl 1-(1,3-benzothiazol-2-yl)-4-(3-methoxyphenyl)-piperidine-4-carboxylate

In the same manner as in Reference Example 17-2, the title compound was synthesized.
High performance liquid chromatography/ Mass spectrometry
m/z 383.2 (M+H)

### Retention time: 3.32 min.

### Reference Example 17-12

### Synthesis of methyl 1-[2-(2-methoxyethoxy)phenyl]-4-(3-methoxyphenyl)piperidine-4-carboxylate

In the same manner as in Reference Example 17-2, the title compound was synthesized.
High performance liquid chromatography/ Mass spectrometry
m/z 400.3 (M+H)

### Retention time: 2.80 min.

### Reference Example 17-13

### Synthesis of methyl 1-(2-methoxyphenyl)-4-(3-methoxyphenyl)azepane-4-carboxylate

In the same manner as in Reference Example 17-2, the title compound was synthesized.
High performance liquid chromatography/ Mass spectrometry
m/z 370.3 (M+H)

### Retention time: 2.73 min.

### Reference Example 18

### Synthesis of [1-(2-methoxyphenyl)-4-(3-methoxyphenyl)piperidin-4-yl]acetonitrile

### [1-(2-methoxyphenyl)-4-(3-methoxyphenyl)piperidin-4-yl]methanol

To a suspension of lithium aluminum hydride (51.4 mg, 1.35 mol) in tetrahydrofuran (THF, 30 mL) was added slowly a solution of methyl 1-(2-methoxyphenyl)-4-(3-methoxyphenyl)piperidine-4-carboxylate (500 mg, 1.35 mol) in tetrahydrofuran (THF, 10 mL) under ice-cooling, and the mixture was warmed to room temperature and stirred overnight. Thereto was added 14.5N aqueous ammonia (0.200 mL) under ice-cooling to quench the reaction and the precipitated salt was collected by filtration on celite. The solvent was evaporated under reduced pressure, and the resulting residue was purified by silica gel column chromatography. The title compound was obtained in 388 mg as colorless oils.
High performance liquid chromatography/ Mass spectrometry
m/z 328.3 (M+H)
Retention time: 2.38 min.

### [1-(2-methoxyphenyl)-4-(3-methoxyphenyl)piperidin-4-yl]methyl 4-methylbenzenesulfonate

To a solution of [1-(2-methoxyphenyl)-4-(3-methoxyphenyl)-piperidin-4-yl]methanol (88.0 mg, 0.269 mmol) in pyridine (10 mL) was added p-toluenesulfonyl chloride (56.4 mg, 0.296 mmol) at room temperature, and the mixture was warmed to 50°C and stirred for 4 hours. The solvent was evaporated under reduced pressure, and the mixture was extracted with ethyl acetate, and the organic layer was washed twice with saturated aqueous ammonium chloride solution. The organic layer was dried over anhydrous magnesium sulfate and filtered, and the solvent was evaporated under reduced pressure. The resulting residue was purified by thin layer chromatography to give the title compound 33.3 mg as colorless oils.
High performance liquid chromatography/Mass spectrometry
m/z 482.4 (M+H)
Retention time: 3.11 min.

### [1-(2-methoxyphenyl)-4-(3-methoxyphenyl)piperidin-4-yl]acetonitrile

A suspension of [1-(2-methoxyphenyl)-4-(3-methoxyphenyl)-piperidin-4-yl]methyl 4-methylbenzenesulfonate (33.3 mg, 0.0690 mmol) and potassium cyanide (9.00 mg, 0.138 mmol) in dimethyl sulfoxide (DMSO, 10 mL) was warmed to 80°C and the mixture was stirred for 4 hours. Thereto was added water to quench the reaction, and the mixture was extracted with ethyl acetate and the organic layer was washed twice with water. The organic layer was dried over anhydrous magnesium sulfate and filtered, and the solvent was evaporated under reduced pressure. The resulting residue was purified by preparative thin layer chromatography to give the title compound 20.1 mg as colorless oils.
High performance liquid chromatography/Mass spectrometry
m/z 337.6 (M+H)

### Retention time: 3.07 min.

### Reference Example 19

### Synthesis of methyl 1-(3-methoxyphenyl)-4-oxocyclohexanecarboxylate

### Synthesis of dimethyl 1-(3-methoxyphenyl)-4-oxocyclohexane-1,3-dicarboxylate

To a suspension of sodium hydride (277 mg, 6.93 mol) in N,N-dimethylformamide (DMF, 20 mL) was added dropwise a solution of methyl 3-methoxyphenylacetonitrile (0.500 mL, 3.15 mmol) and methyl acrylate (0.710 mL, 7.88 mmol) in N,N-dimethylformamide (DMF, 10 mL) under ice-cooling, and then the mixture was warmed to room temperature and stirred for 3 hours. The reaction was quenched with saturated aqueous ammonium chloride solution and the mixture was extracted with ethyl acetate, and the organic layer was washed twice with saturated aqueous ammonium chloride solution. The organic layer was dried over anhydrous magnesium sulfate and filtered, and the solvent was evaporated under reduced pressure. The resulting residue was purified by silica gel column chromatography to give the title compound 647 mg as colorless oils.
¹H-NMR δ (DMSO-d₆); 2.16-2.33 (4H, m), 2.59 (1H, d, J = 15.8 Hz), 2.94 (1H, d, J = 15.8 Hz), 3.56 (3H, s), 4.03 (3H, s), 4.05 (3H, s), 6.79-6.88 (3H, m), 7.25-7.30 (1H, m), 12.0 (1H, s).

### 1-(3-methoxyphenyl)-4-oxocyclohexanecarboxylic acid

A solution of dimethyl 1-(3-methoxyphenyl)-4-oxocyclohexane-1,3-dicarboxylate (250 mg, 0.780 mmol) and potassium hydroxide (175 mg, 3.12 mmol) in a mixture of 1,4-dioxane (5 mL) -water (5 mL) was heated under reflux for 5 hours. Then thereto was added 3N hydrochloric acid to acidify the reaction solution and the mixture was heated at 60°C for 4 hours. The mixture was extracted with ethyl acetate, and the organic layer was washed once with 3N hydrochloric acid. The organic layer was dried over anhydrous magnesium sulfate and filtered, and the solvent was evaporated under reduced pressure. The resulting residue was purified by silica gel column chromatography to give the title compound 146 mg as white crystals.
¹H-NMR δ (DMSO-d₆); 2.24-2.34 (2H, m), 2.39-2.48 (2H, m), 2.52-2.62 (2H, m), 2.70-2.76 (2H, m), 3.81 (3H, s), 6.84-6.88 (1H, m), 7.01-7.07 (2H, m), 7.29-7.35 (1H, m).

### methyl 1-(3-methoxyphenyl)-4-oxocyclohexanecarboxylate

To a solution of 1-(3-methoxyphenyl)-4-oxocyclohexanecarboxylic acid (146 mg, 0.588 mmol) in N,N-dimethylformamide (DMF, 15 mL) were added iodomethane (0.0549 mL, 0.882 mmol) and potassium carbonate (244 mg, 1.76 mmol) at room temperature, and the mixture was warmed to 35 °C and then stirred for 4 hours. Thereto was added water to quench the reaction, and the mixture was extracted with ethyl acetate, and the organic layer was washed twice with water. The organic layer was dried over anhydrous magnesium sulfate and filtered, and the solvent was evaporated under reduced pressure. The resulting residue was purified by silica gel column chromatography to give the title compound 120 mg as white crystals.
¹H-NMR δ (DMSO-d₆); 2.19-2.29 (2H, m), 2.40-2.59 (4H, m), 2.72-2.77 (2H, m), 3.72 (3H, s), 3.81 (3H, s), 6.82-6.85 (1H, m), 6.95-7.01 (2H, m), 7.26-7.32 (1H, m).

### Reference Example 20

### Synthesis of methyl 4-hydroxy-1-(3-methoxyphenyl)cyclohexanecarboxylate

To a solution of sodium borohydride (86.5 mg, 2.29 mol) in methanol (20 mL) was added dropwise a solution of methyl 1-(3-methoxyphenyl)-4-oxocyclohexanecarboxylate (500 mg, 1.91 mmol) in methanol (10 mL) at room temperature, and the mixture was stirred at such temperature overnight. Thereto was added saturated aqueous ammonium chloride solution to quench the reaction and the mixture was extracted with ethyl acetate, and the organic layer was washed twice with saturated aqueous ammonium chloride solution. The organic layer was dried over anhydrous magnesium sulfate and filtered, and the solvent was evaporated under reduced pressure. The resulting residue was purified by silica gel column chromatography to give the stereoisomers of the title compound: 104 mg (TLC development; hexane: ethyl acetate = 2 : 1, Rf = 0.6) and 364 mg (Rf = 0.5) as white crystals respectively.
¹H-NMR δ (DMSO-d₆); 1.43-1.60 (4H, m), 1.98-2.19 (4H, m), 3.54 (3H, s), 3.68-3.72 (1H, m), 3.73 (3H, s), 4.56 (1H, d, J = 4.59 Hz), 6.81-6.86 (2H, m), 6.92-6.94 (1H, m), 7.23-7.29 (1H, m).
Stereoisomer
¹H-NMR δ (DMSO-d₆); 1.17-1.30 (2H, m), 1.56-1.65 (2H, m), 1.77-1.82 (2H, m), 2.38-2.42 (2H, m), 3.38-3.47 (1H, m), 3.59 (3H, s), 3.72 (3H, s), 4.41 (1H, d, J = 3.66 Hz), 6.79-6.89 (3H, m), 7.21-7.26 (1H, m).

### Reference Example 21-1

### Synthesis of methyl 1-(3-methoxyphenyl)-4-phenoxycyclohexane-carboxylate

To methyl 4-hydroxy-1-(3-methoxyphenyl)cyclohexanecarboxylate (100 mg, 0.378 mmol) (TLC development; hexane : ethyl acetate = 2 : 1, Rf = 0.5), triphenyl phosphine (PPh₃, 149 mg, 0.567 mmol) and phenol (0.0664 mL, 0.756 mmol) in tetrahydrofuran (THF, 15 mL) was added dropwise diethyl azodicarboxylate (DEAD, 0.223 mL, 0.567 mmol) at room temperature and the mixture was stirred at such temperature for 2 hours. Thereto was added water to quench the reaction and the mixture was extracted with ethyl acetate, and the organic layer was washed twice with water. The organic layer was dried over anhydrous magnesium sulfate and filtered, and the solvent was evaporated under reduced pressure. The resulting residue was purified by silica gel column chromatography to give the title compound 77.3 mg as colorless oils.
¹H-NMR δ (DMSO-d₆); 1.72-1.76 (4H, m), 2.03-2.11 (2H, m), 2.19-2.23 (2H, m), 3.58 (3H, s), 3.74 (3H, s), 4.56 (1H, m), 6.81-6.98 (6H, m), 7.22-7.31 (3H, m).

### Reference Example 21-2

### Synthesis of methyl 1-(3-methoxyphenyl)-4-phenoxycyclohexane-carboxylate (stereoisomer of Reference Example 21-1)

In the same manner, the above stereoisomer was synthesized using methyl 4-hydroxy-1-(3-methoxyphenyl)cyclohexanecarboxylate (TLC development; hexane : ethyl acetate = 2 : 1, Rf = 0.6) as a starting material.
¹H-NMR δ (DMSO-d₆); 1.39-1.50 (2H, m), 1.79-1.84 (2H, m), 1.98-2.06 (2H, m), 2.45-2.50 (2H, m), 3.62 (3H, s), 3.74 (3H, s), 4.36 (1H, m), 6.72-6.94 (5H, m), 7.11-7.30 (4H, m).

### Reference Example 22

### Synthesis of methyl 4-benzylidene-1-(3-methoxyphenyl)cyclohexanecarboxylate

To a suspension of benzyl triphenylphosphonium chloride (574 mg, 1.48 mmol) in tetrahydrofuran (THF, 20 mL) was added a solution of 1.01 M sodium bis(trimethylsilyl)amide in tetrahydrofuran (THF, 1.34 mL) at room temperature and the mixture was stirred at such temperature for 30 min. Then, thereto was added methyl 1-(3-methoxyphenyl)-4-oxocyclohexanecarboxylate (300 mg, 1.13 mmol) at room temperature, and the mixture was heated under reflux for 2 hours. The mixture was extracted with ethyl acetate, and the organic layer was washed once with saturated aqueous ammonium chloride solution and once with saturated aqueous sodium bicarbonate solution. The organic layer was dried over anhydrous magnesium sulfate and filtered, and the solvent was evaporated under reduced pressure. The resulting residue was purified by silica gel column chromatography to give the title compound 62.6 mg as colorless oils.
High performance liquid chromatography/Mass spectrometry
m/z 337.3 (M+H)

### Retention time: 4.28 min.

### Reference Example 23-1

### Synthesis of 1-(2-methoxyphenyl)-4-(3-methoxyphenyl)piperidine-4-carboxylic acid

To a solution of methyl 1-(2-methoxyphenyl)-4-(3-methoxyphenyl)-piperidine-4-carboxylate (1.00 g, 4.62 mmol) in ethanol (5 mL) was added 2N aqueous lithium hydroxide solution (0.071 mL, 0.142 mmol) at room temperature, and the mixture was heated under reflux for 4 hours. Thereto was further added 2N aqueous lithium hydroxide solution (0.149 mL, 0.284 mmol) at room temperature, and the mixture was heated under reflux for 4 hours. The solvent was evaporated, and the residue was suspended in 1,4-dioxane (3 mL) -water (2 mL) and the mixture was heated under reflux for additional 5 hours. Thereto was added saturated aqueous ammonium chloride solution to quench the reaction and the mixture was extracted with ethyl acetate, and the organic layer was washed twice with saturated aqueous ammonium chloride solution. The organic layer was dried over anhydrous magnesium sulfate and filtered, and the solvent was evaporated under reduced pressure. The resulting residue was crystallized from a mixture of ethyl acetate-hexane to give the title compound 35.2 mg as white crystals.
High performance liquid chromatography/ Mass spectrometry
m/z 342.3 (M+H)

### Retention time: 2.37 min.

### Reference Example 23-2

### Synthesis of 4-(3-methoxyphenyl)-1-phenylpiperidine-4-carboxylic acid

In the same manner as in Reference Example 23-1, the title compound was synthesized.
High performance liquid chromatography/ Mass spectrometry
m/z 312.4 (M+H)
Retention time: 2.55 min.

### Reference Example 23-3

### Synthesis of 4-(3-methoxyphenyl)-1-(3-methoxypyridin-2-yl)piperidine-4-carboxylic acid

In the same manner as in Reference Example 23-1, the title compound was synthesized.
High performance liquid chromatography/ Mass spectrometry
m/z 343.2 (M+H)

### Retention time: 2.44 min.

### Reference Example 23-4

### Synthesis of 4-(3-methoxyphenyl)-1-pyrimidin-2-ylpiperidine-4-carboxylic acid

In the same manner as in Reference Example 23-1, the title compound was synthesized.
High performance liquid chromatography/ Mass spectrometry
m/z 314.3 (M+H)

### Retention time: 2.78 min.

### Reference Example 23-5

### Synthesis of 1-(1,3-benzoxazol-2-yl)-4-(3-methoxyphenyl)piperidine-4-carboxylic acid

In the same manner as in Reference Example 23-1, the title compound was synthesized.
High performance liquid chromatography/ Mass spectrometry
m/z 353.2 (M+H)

### Retention time: 3.05 min.

### Reference Example 23-6

### Synthesis of 1-(1,3-benzothiazol-2-yl)-4-(3-methoxyphenyl)piperidine-4-carboxylic acid

In the same manner as in Reference Example 23-1, the title compound was synthesized.
High performance liquid chromatography/ Mass spectrometry
m/z 368.9 (M+H)

### Retention time: 3.07 min.

### Reference Example 23-7

### Synthesis of 1-(2-methoxyphenyl)-4-phenylpiperidine-4-carboxylic acid

In the same manner as in Reference Example 23-1, the title compound was synthesized.
High performance liquid chromatography/Mass spectrometry
m/z 312.4 (M+H)

### Retention time: 2.26 min.

### Reference Example 23-8

### Synthesis of 1-benzyl-4-(3-methoxyphenyl)piperidine-4-carboxylic acid

In the same manner as in Reference Example 23-1, the title compound was synthesized.
High performance liquid chromatography/ Mass spectrometry
m/z 326.4 (M+H)

### Retention time: 2.26 min.

### Reference Example 23-9

### Synthesis of 1-[2-(2-methoxyethoxy)phenyl]-4-(3-methoxyphenyl)-piperidine-4-carboxylic acid

In the same manner as in Reference Example 23-1, the title compound was synthesized.
High performance liquid chromatography/Mass spectrometry
m/z 386.3 (M+H)

### Retention time: 2.53 min.

### Reference Example 23-10

### Synthesis of 4-(3-methoxyphenyl)-1-(2,2,2-trifluoroethyl)piperidine-4-carboxylic acid

In the same manner as in Reference Example 23-1, the title compound was synthesized.
High performance liquid chromatography/Mass spectrometry
m/z 318.2 (M+H)

### Retention time: 2.40 min.

### Reference Example 23-11

### Synthesis of 1-(2-methoxyphenyl)-4-(3-methoxyphenyl)azepane-4-carboxylic acid

In the same manner as in Reference Example 23-1, the title compound was synthesized.
High performance liquid chromatography/ Mass spectrometry
m/z 356.1 (M+H)

### Retention time: 2.46 min.

### Reference Example 23-12

### Synthesis of 1-(3-methoxyphenyl)-4-phenoxycyclohexanecarboxylic acid

In the same manner as in Reference Example 23-1, the title compound was synthesized.
¹H-NMR δ (DMSO-d₆); 1.70-1.80 (4H, m), 1.98-2.20 (4H, m), 3.74 (3H, s), 4.56-4.57 (1H, m), 6.82-7.01 (6H, m), 7.22-7.30 (3H, m).

### Reference Example 23-13

### Synthesis of 1-(3-methoxyphenyl)-4-phenoxycyclohexanecarboxylic acid (stereoisomer of Reference Example 23-12)

In the same manner as in Reference Example 23-1, the title compound was synthesized.
¹H-NMR δ (DMSO-d₆); 1.44-1.55 (2H, m), 1.73-1.80 (2H, m), 1.98-2.05 (2H, m), 2.43-2.50 (2H, m), 3.74 (3H, s), 4.33-4.40 (1H, m), 6.81-6.98 (6H, m), 7.12-7.29 (3H, m).

### Reference Example 23-14

### Synthesis of [1-(2-methoxyphenyl)-4-(3-methoxyphenyl)piperidin-4-yl]acetic acid

In the same manner as in Reference Example 23-1, the title compound was synthesized.
High performance liquid chromatography/ Mass spectrometry
m/z 356.3 (M+H)

### Retention time: 2.67 min.

### Reference Example 23-15

### Synthesis of 1-(3-methoxyphenyl)-4-phenylcyclohexanecarboxylic acid

In the same manner as in Reference Example 23-1, the title compound was synthesized.
¹H-NMR δ (DMSO-d₆); 1.56-1.71 (4H, m), 1.84-1.88 (2H, m), 2.50-2.58 (3H, m), 3.74 (3H, s), 6.82-7.00 (3H, m), 7.15-7.31 (6H, m).

### Reference Example 24

### Synthesis of 4-(3-methoxyphenyl)-1-pyrimidin-2-ylpiperidine-4-carbonyl chloride hydrochloride

To a solution of 4-(3-methoxyphenyl)-1-pyrimidin-2-ylpiperidine-4-carboxylic acid (0.700 g, 2.23 mmol) in toluene (50 mL) was added dimethylformamide (0.020 mL) at 0° C, followed by addition of oxalyl chloride (3.12 mL, 35.8 mL) and the mixture was stirred at room temperature for 10 hours. The solvent of reaction solution was evaporated under reduced pressure to give the title compound 0.734 g as white solids.

### Reference Example 25

### Synthesis of sulfamoyl chloride

To a solution of chlorosulfonyl isocyanate (5 mL, 57.4 mmol) in dichloromethane (18 mL) was added formic acid (2.22 mL, 59.1 mmol) at 0°C, and the mixture was stirred at room temperature for 3 hours and then refluxed for 3 hours. The reaction solution was concentrated under reduced pressure to give the title compound 6.53 g as colorless oils.

### Reference Example 26-1

### Synthesis of N-methyl-N-phenylsulfamide

To a solution of N-methylaniline (139 mg, 1.30 mmol) in N-methylpyrrolidinone (5.20 mL) was added sulfamoyl chloride (300 mg, 2.60 mmol) at 0°C and the mixture was stirred at room temperature for 4 hours. The reaction solution was poured into water and then the mixture was extracted with ethyl acetate. The organic layer was dried over anhydrous magnesium sulfate and filtered, and then the solvent was evaporated under reduced pressure and the resulting residue was purified by silica gel column chromatography to give the title compound 180 mg as white solids.
¹H-NMR δ (DMSO-d₆); 3.09 (3H, s), 7.00 (2H, s), 7.19-7.26 (1H, m), 7.30-7.39 (4H, m).

### Reference Example 26-2

### Synthesis of N-(2,6-diisopropylphenyl)sulfamide

In the same manner as in Reference Example 26-1, the title compound was synthesized using 2,6-diisopropylaniline as a starting material.
High performance liquid chromatography/ Mass spectrometry
m/z 257 (M+H)

### Retention time: 3.24 min.

### Reference Example 27

### Synthesis of N-phenylsulfamide

To a solution of aniline (500 mg, 5.36 mmol) in 1,2-dimethoxyethane (10 mL) was added sulfamide (2.57 g, 26.84 mmol) and the mixture was stirred at 90°C for 24 hours. The reaction solution was poured into saturated aqueous sodium chloride solution and thereto was added ethyl acetate, and the mixture was extracted with ethyl acetate. The organic layer was washed with saturated aqueous sodium chloride solution and then dried over anhydrous magnesium sulfate and filtered. The filtrate was concentrated under reduced pressure, and the resulting residue was purified by silica gel column chromatography to give the title compound 699 mg as white solids.
¹H-NMR δ (DMSO-d₆); 6.93-6.98 (2H, m), 7.07 (2H, brs), 7.13-7.15 (2H, m), 7.23-7.27 (2H, m), 9.47 (2H, s).

### Reference Example 28-1

### Synthesis of N-benzyl-N-methylsulfamide

### Synthesis of tert-butyl {[benzyl(methyl)amino]sulfonyl}carboxamide

To a solution of N-benzylmethylamine (147 mg, 1.21 mmol) in dichloromethane (3.60 mL) was added (tert-butoxycarbonyl){[4-(dimethyliminino)pyridin-1(4H)-yl]sulfonyl}azanide (400 mg, 1.33 mmol) at room temperature and the mixture was stirred at room temperature for 3 hours. The solvent of the reaction solution was evaporated under reduced pressure, and then the resulting residue was purified by silica gel column chromatography to give the title compound 174 mg as white solids.

### Synthesis of N-benzyl-N-methylsulfamide

Tert-butyl {[benzyl(methyl)amino]sulfonyl}carboxamide (156 mg, 0.519 mmol) was dissolved in 4N hydrogen chloride/dioxane (4.00 mL) and the mixture was stirred at room temperature for 5 hours. The solvent of the reaction solution was evaporated under reduced pressure to give the title compound 104 mg as white solids.
¹H-NMR δ (DMSO-d₆); 2.49 (3H, s), 4.06 (2H, s), 6.88 (2H, s), 7.26-7.39 (5H, m).

### Reference Example 28-2

### Synthesis of N-(1-phenylethyl)sulfamide

In the same manner as in Reference Example 28-1, the title compound was synthesized using α-methylbenzylamine as a starting material.
¹H-NMR δ (DMSO-d₆); 1.39 (3H, d, J=6.9 Hz), 4.35-4.47 (1H, m), 6.52 (2H, s), 7.08 (1H, d, J=8.3 Hz), 7.17-7.25 (1H, m), 7.26-7.39 (4H, m).

### Reference Example 28-3

### Synthesis of N-(tert-butyl)sulfamide

In the same manner as in Reference Example 28-1, the title compound was synthesized using tert-butylamine as a starting material. ¹H-NMR δ (DMSO-d₆); 1.22 (9H, s), 6.31 (1H, s), 6.41 (2H, s).

### Reference Example 29

### Synthesis of N-benzylsulfamide

To a solution of benzylamine (139 mg, 1.30 mmol) in N,N-dimethylformamide (12.0 mL) was added diisopropylethylamine (1.02 mL, 5.85 mmol) at room temperature, followed by addition of sulfamoyl chloride (300 mg, 2.60 mol) at -60°C, and the mixture was stirred for 2 hours with warming to -30°C. The solvent of the reaction solution was evaporated under reduced pressure, and then the residue was taken to pH 7 to 8 with saturated aqueous sodium hydrogen carbonate solution, and the resulting residue was purified by reverse phase column chromatography to give the title compound 41.1 mg as white solids.
¹H-NMR δ (DMSO-d₆); 4.05 (2H, d, J=6.4 Hz), 6.62 (2H, s), 7.04 (1H, t, J=6.4 Hz), 7.20-7.37 (5H, m).

### Reference Example 30

### Synthesis of (tert-butoxycarbonyl){[4-(dimethyliminio)pyridin-1(4H)-yl] sulfonyl}azanide

To a solution of t-butanol (1.57 g, 0.0212 mol) in dichloromethane (15 mL) was added dropwise chlorosulfonyl isocyanate (3.0 g, 0.0212 mol) at 0°C, and the contents was rinsed with dichloromethane (1.0 mL) thoroughly. After 5 min., thereto was added N,N-dimethylaminopyridine (5.18 g, 0.0424 mol) and the mixture was stirred at room temperature for one hour. After diluting with chloroform, this mixture was washed with water several times. This organic layer was dried over anhydrous magnesium sulfate and was filtered, and then the solvent was evaporated under reduced pressure. The resulting residue was crystallized from acetonitrile to give the title compound 2.50 g as white solids.
¹H-NMR δ (DMSO-d₆); 1.25 (9H, s), 3.21 (6H, s), 6.95 (2H, d, J=8.0Hz), 8.44 (2H, d, J=8.0Hz).

### Reference Example 31

### Synthesis of acetylsulfamoyl chloride

To acetic acid (2.0 mL, 0.0349 mol) was added dropwise chlorosulfonyl isocyanate (3.04 mL, 0.0349 mol) at 0°C, and after 10 min., the mixture was warmed to room temperature. Thereto was added hexane and the resulting crystals was filtered under reduced pressure to give the title compound 5.37 g as white solids.

### Reference Example 32

### Synthesis of 4-(bromomethyl)benzenesulfonamide

To a solution of 4-bromomethylbenzenesulfonyl chloride (500 mg, 1.85 mmol) in tetrahydrofuran (10 mL) was added dropwise saturated aqueous ammonia (300 µL) at 0°C and the mixture was stirred overnight. Thereto was added 1N hydrochloric acid to quench the reaction, and the reactant was extracted with ethyl acetate. The ethyl acetate layer was washed with 1N hydrochloric acid again, followed by washing with saturated aqueous sodium chloride solution, and then dried over anhydrous magnesium sulfate. After filtering, the solvent was evaporated under reduced pressure, and the resulting residue was repulped with ethyl acetate/hexane to give the title compound 187 mg as white solids.
¹H-NMR (DMSO-d₆) δ; 4.75 (2H, s), 7.40 (2H, s), 7.63 (2H, d, J=8.5Hz), 7.78 (2H, d, J=8.5Hz).

### Reference Example 33

### Synthesis of cis-4-[(diphenylmethyl)amino]-1-(3-methoxyphenyl)-cyclohexanecarbonitrile, and trans-4-[(diphenylmethyl)amino]-1-(3-methoxyphenyl)cyclohexanecarbonitrile

### Synthesis of methyl 5-cyano-2-hydroxy-5-(3-methoxyphenyl)cyclohexa-1-en-1-carboxylate

To a suspension of sodium hydride (31.5 g, 722 mmol) in dimethylformamide (DMF, 720 mL) was added dropwise a solution of (3-methoxyphenyl)acetonitrile and methyl acrylate (73.8 mL, 820 mmol) in dimethylformamide (DMF, 120 mL) at 0°C and the mixture was stirred at room temperature for 2 hours. The reaction solution was poured into water, and then thereto was added conc. hydrochloric acid to adjust to pH 4. After extracted with ethyl acetate (2000 mL, 300 mL), the organic layer was washed with saturated aqueous ammonium chloride solution (1000 mL × 2) and saturated aqueous sodium chloride solution, and the organic layer was dried over anhydrous magnesium sulfate and filtered, and the solvent was evaporated under reduced pressure, and then the resulting residue was crystallized from diethylether (110 mL) to give the title compound 70.7 g as white solids.

### Synthesis of 1-(3-methoxyphenyl)-4-oxocyclohexanecarbonitrile

To a solution of methyl 5-cyano-2-hydroxy-5-(3-methoxyphenyl)-cyclohexa-1-en-1-carboxylate (29.6 g, 103 mmol) in dimethyl sulfoxide (DMSO, 92.6 mL) was added water (5.68 mL, 315 mmol) at room temperature and the mixture was stirred at 127°C for 9 hours. The reaction solution was poured into water (1200 mL), and the mixture was stirred at 0°C for one hour, and then the precipitated product was collected by filtration. The resulting solid was crystallized from diethylether (35 mL) to give the title compound 20.0 g as white solids. Synthesis of cis-4-[(diphenylmethyl)amino]-1-(3-methoxyphenyl)-cyclohexanecarbonitrile, and trans-4-[(diphenylmethyl)amino]-1-(3-methoxyphenyl)cyclohexanecarbonitrile

To a solution of 1-(3-methoxyphenyl)-4-oxocyclohexanecarbonitrile (18.4 g, 80.4 mmol) and benzhydrylamine (22.1 g, 121 mmol) in 1,2-dichloroethane (307 mL) was added sodium triacetoxyborohydride (NaBH(OAc)₃, 25.6 g, 121 mmol) at 0°C, and the mixture was warmed from 0°C to room temperature gradually and the mixture was stirred overnight. The reaction solution was poured into saturated aqueous sodium hydrogen carbonate solution (300 mL), and the mixture was extracted with chloroform (300 mL). The organic layer was washed with saturated aqueous sodium hydrogen carbonate solution (300 mL × 2), and then dried over anhydrous magnesium sulfate and filtered. The solvent was evaporated under reduced pressure, and then the resulting residue was crystallized from acetone (360 mL) and the precipitated crystals were collected by filtration to give the title compound cis-4-[(diphenylmethyl)amino]-1-(3-methoxyphenyl)cyclohexanecarbonitrile 17.7 g as white solids. The solvent of filtrate was evaporated under reduced pressure and then the resulting residue was crystallized from diethylether (210 mL) and filtered to remove the solid. The solvent of the filtrate was evaporated under reduced pressure, and then the resulting residue was recrystallized from methanol (250 mL) to give the title compound trans-4-[(diphenylmethyl)amino]-1-(3-methoxyphenyl)-cyclohexanecarbonitrile 9.64 g as white solids.
cis-4-[(diphenylmethyl)amino]-1-(3-methoxyphenyl)cyclohexane-carbonitrile
High performance liquid chromatography/Mass spectrometry
m/z 397.2 (M+H)
Retention time: 2.94 min.
trans-4-[(diphenylmethyl) amino]-1-(3-methoxyphenyl)cyclohexane-carbonitrile
High performance liquid chromatography/Mass spectrometry
m/z 397.5 (M+H)

### Retention time: 3.11 min.

### Reference Example 34-1

### Synthesis of cis-4-amino-1-(3-methoxyphenyl)cyclohexanecarbonitrile

To a suspension of cis-4-[(diphenylmethyl)amino]-1-(3-methoxyphenyl)cyclohexanecarbonitrile (15.0 g, 37.8 mmol) in ethanol (450 mL) were added 10%-palladium/carbon (1.43 g) and ammonium formate (23.8 g, 378 mmol) at room temperature and the mixture was refluxed for 3 hours. The reaction solution was filtered on celite, and then the solvent was evaporated under reduced pressure and the resulting residue was purified by silica gel column chromatography to give the title compound 8.13 g as white solids.
High performance liquid chromatography/Mass spectrometry
m/z 231.4 (M+H)
Retention time: 0.25 min., 2.23 min.

### Reference Example 34-2

### Synthesis of trans-4-amino-1-(3-methoxyphenyl)cyclohexane-carbonitrile

In the same manner as in Reference Example 34-1, the title compound was synthesized using trans-4-[(diphenylmethyl)amino]-1-(3-methoxyphenyl)cyclohexanecarbonitrile as a starting material.
High performance liquid chromatography/ Mass spectrometry
m/z 231.4 (M+H)
Retention time: 0.29 min., 2.17 min.

### Reference Example 35

### Synthesis of {trans-4-[(diphenylmethyl)amino]-1-(3-methoxyphenyl)-cyclohexyl}acetonitrile

### Synthesis of {cis-4-[(diphenylmethyl)amino]-1-(3-methoxyphenyl)-cyclohexyl}methanol

To a suspension of lithium aluminum hydride (523 mg, 13.95 mmol) in tetrahydrofuran (60 mL) was added a solution of methyl cis-4-[(diphenylmethyl)amino]-1-(3-methoxyphenyl)cyclohexanecarboxylate (2 g, 4.65 mmol) in tetrahydrofuran (20 mL) at 0°C and the mixture was stirred at about 0 to 15°C for 4 hours. To the reaction solution was added successively water (0.52 mL), 1N aqueous sodium hydroxide solution (0.52 mL) and water (1.5 mL) and then the mixture was filtered and concentrated. The resulting residue was purified by crystallization to give the title compound 1.76 g as white solids.
High performance liquid chromatography/ Mass spectrometry
m/z 402 (M+H)

### Retention time: 2.80 min.

### Synthesis of {cis-4-[(diphenylmethyl)amino]-1-(3-methoxyphenyl)-cyclohexyl}methylmethane sulfonate

To a solution of {cis-4-[(diphenylmethyl)amino]-1-(3-methoxyphenyl)cyclohexyl}methanol (1.41 g, 3.51 mmol) in dichloromethane (28 mL) were added triethylamine (0.538 mL, 3.86 mol) and methanesulfonyl chloride (0.298 mL, 3.86 mmol) and the mixture was stirred at room temperature for 3 hours and a half. During of the reaction, thereto was added methanesulfonyl chloride (0.027 mL, 0.351 mmol). The reaction solution was poured into saturated aqueous sodium bicarbonate solution and the mixture was extracted with ethyl acetate. The organic layer was washed with saturated aqueous sodium chloride solution, and then dried over anhydrous magnesium sulfate and filtered. The filtrate was concentrated under reduced pressure to give the title compound 1.87 g as colorless oils.
High performance liquid chromatography/ Mass spectrometry
M/z 480 (M+H)

### Retention time: 2.96 min.

### Synthesis of {trans-4-[(diphenylmethyl)amino]-1-(3-methoxyphenyl)-cyclohexyl}acetonitrile

To a solution of {cis-4-[(diphenylmethyl)amino]-1-(3-methoxyphenyl)cyclohexyl}methylmethanesulfonate (1.67 g, 3.48 mmol) in dimethyl sulfoxide (34 mL) were added potassium cyanide (2.26 g, 34.8 mmol) and 18-crown-6-ether (9.19 g, 34.8 mmol) and the mixture was stirred at 100°C for 10 hours and a half. The reaction solution was poured into saturated aqueous sodium bicarbonate solution and the mixture was extracted with ethyl acetate. The organic layer was washed with saturated aqueous sodium chloride solution, and then dried over anhydrous magnesium sulfate and filtered. The resulting residue was purified by silica gel column chromatography to give the title compound 0.80 g as white solids.
High performance liquid chromatography/ Mass spectrometry
m/z 411 (M+H)
Retention time: 2.90 min.

### Reference Example 36

### Synthesis of cis-1-(3-methoxyphenyl)-4-(piperidin-4-ylamino)-cyclohexanecarbonitrile dihydrochloride

### Synthesis of tert-butyl 4-{[cis-4-cyano-4-(3-methoxyphenyl)cyclohexyl]-amino}piperidin-1-carboxylate

To a solution of cis-4-amino-1-(3-methoxyphenyl)cyclohexane-carbonitrile (1.00 g, 4.34 mmol) and 1-tert-butoxycarbonyl-4-piperidone (952 mg, 4.78 mmol) in 1,2-dichloroethane (20 mL) at room temperature was added sodium triacetoxyborohydride (NaHB(OAc)₃, 1.38 g, 6.51 mmol) at room temperature and then the mixture was stirred overnight. Thereto was added saturated aqueous sodium bicarbonate solution to quench the reaction, and then the mixture was extracted with ethyl acetate and the organic layer was washed twice with saturated aqueous sodium bicarbonate solution. The organic layer was dried over anhydrous magnesium sulfate and filtered, and then the solvent was evaporated under reduced pressure. The resulting residue was purified by silica gel column chromatography, followed by crystallization from hexane-ethyl acetate to give the title compound 1.34 g as white crystals.
High performance liquid chromatography/ Mass spectrometry
m/z 414.3 (M+H)
Retention time: 2.69 min.

### Synthesis of cis-1-(3-methoxyphenyl)-4-(piperidin-4-ylamino)-cyclohexanecarbonitrile dihydrochloride

Tert-butyl 4-{[cis-4-cyano-4-(3-methoxyphenyl)cyclohexyl]amino} piperidine-1-carboxylate (1.00 g, 2.42 mmol) was dissolved in 4M hydrogen chloride/1,4-dioxane solution (10 mL), and then the mixture was stirred at room temperature overnight. The solvent was evaporated under reduced pressure, and the resulting residue was crystallized from ethyl acetate to give the title compound 1.45 g as white solids.
High performance liquid chromatography/Mass spectrometry
m/z 314.3 (M+H)
Retention time: 1.80 min.

### Reference Example 37

### Synthesis of trans-1-(3-methoxyphenyl)-4-(piperidin-4-ylamino)-cyclohexanecarbonitrile dihydrochloride

To a solution of trans-4-amino-1-(3-methoxyphenyl)cyclohexane-carbonitrile (500 g, 2.17 mmol) and 1-tert-butoxycarbonyl-4-piperidone (476 mg, 2.39 mmol) in 1,2-dichloroethane (10 mL) was added sodium triacetoxyborohydride (NaHB(OAc)₃, 690 mg, 3.26 mmol) at room temperature and then the mixture was stirred overnight. Thereto was added saturated aqueous sodium bicarbonate solution to quench the reaction, and then the mixture was extracted with ethyl acetate and the organic layer was washed twice with saturated aqueous sodium bicarbonate solution. The organic layer was dried over anhydrous magnesium sulfate and filtered, and then the solvent was evaporated under reduced pressure. The resulting residue was purified by silica gel column chromatography, followed by crystallization from hexane-ethyl acetate to give the oil and the resulting oil was dissolved in 4M hydrogen chloride/1,4-dioxane solution (5 mL) and then the mixture was stirred at room temperature overnight. The solvent was evaporated under reduced pressure, and then the resulting residue was crystallized from ethyl acetate to give the title compound 750 mg as white solids.
High performance liquid chromatography/Mass spectrometry
m/z 314.3 (M+H)
Retention time: 0.29 min.

### Reference Example 38

### Synthesis of 4-(3-methoxyphenyl)-1,4'-bipiperidine-4-carbonitrile dihydrochloride

### Synthesis of tert-butyl 4-cyano-4-(3-methoxyphenyl)-1,4'-bipiperidine-1'-carboxylate

To a solution of 4-(3-methoxyphenyl)piperidine-4-carbonitrile (2.47 g, 11.4 mmol) and 1-tert-butoxycarbonyl-4-piperidone (2.50 g, 12.5 mmol) in 1,2-dichloroethane (50 mL) was added sodium triacetoxyborohydride (NaHB(OAc)₃, 3.62 g, 17.1 mmol) at room temperature and then the mixture was stirred overnight. Thereto was added saturated aqueous sodium bicarbonate solution to quench the reaction, and then the mixture was extracted with chloroform and the organic layer was washed twice with saturated aqueous sodium bicarbonate solution. The organic layer was dried over anhydrous magnesium sulfate and filtered, and then the solvent was evaporated under reduced pressure. The resulting residue was purified by silica gel column chromatography to give the title compound 1.51 g as colorless oils.
High performance liquid chromatography/ Mass spectrometry
m/z 400.3 (M+H)
Retention time: 2.61 min.

### Synthesis of 4-(3-methoxyphenyl)-1,4'-bipiperidine-4-carbonitrile dihydrochloride

Tert-butyl 4-cyano-4-(3-methoxyphenyl)-1,4'-bipiperidine-1'-carboxylate (1.51 g, 3.78 mmol) was dissolved in 4M hydrogen chloride/ 1,4-dioxane solution (15 mL) and then the mixture was stirred at room temperature overnight. The solvent was evaporated under reduced pressure, and then the resulting residue was crystallized from ethyl acetate to give the title compound 1.18 g as white solids.
High performance liquid chromatography/Mass spectrometry
m/z 300.3 (M+H)
Retention time: 0.28 min.

### Reference Example 39

### Synthesis of trans-1-(3-methoxyphenyl)-4-piperazin-l-ylcyclohexane-carbonitrile dihydrochloride, and cis-1-(3-methoxyphenyl)-4-piperazin-1-ylcyclohexanecarbonitrile dihydrochloride

### Synthesis of tert-butyl 4-[trans-4-cyano-4-(3-methoxyphenyl)-cyclohexyl]piperazine-1-carboxylate, and tert-butyl 4-[cis-4-cyano-4-(3-methoxyphenyl) cyclohexyl]piperazine-1-carboxylate

To a solution of 1-(3-methoxyphenyl)-4-oxocyclohexanecarbonitrile (2.00 g, 8.72 mmol) and t-butyl 1-piperazinecarboxylate (1.79 g, 9.59 mmol) in 1,2-dichloroethane (50 mL) was added sodium triacetoxyborohydride (NaHB(OAc)₃, 2.77 g, 13.1 mmol) at room temperature and then the mixture was stirred overnight. Thereto was added saturated aqueous sodium bicarbonate solution to quench the reaction, and the mixture was extracted with chloroform, and the organic layer was washed twice with saturated aqueous sodium bicarbonate solution. The organic layer was dried over anhydrous magnesium sulfate and filtered, and then the solvent was evaporated under reduced pressure. The resulting residue was purified by silica gel column chromatography to give the title compound 4-[trans-4-cyano-4-(3-methoxyphenyl)cyclohexyl]piperazine-1-carboxylate 740 mg as white crystals and tert-butyl 4-[cis-4-cyano-4-(3-methoxyphenyl)-cyclohexyl]piperazine-1-carboxylate 3.26 g as white crystals respectively. tert-butyl 4-[trans-4-cyano-4-(3-methoxyphenyl)cyclohexyl]piperazin-1-carboxylate
m/z 400.3 (M+H)
Retention time: 2.61 min.
tert-butyl 4-[cis-4-cyano-4-(3-methoxyphenyl)cyclohexyl]piperazine-1-carboxylate
High performance liquid chromatography/Mass spectrometry
m/z 400.3 (M+H)
Retention time: 2.65 min.

### Reference Example 40-1

### Synthesis of trans-1-(3-methoxyphenyl)-4-piperazin-1-yl-cyclohexane-carbonitrile dihydrochloride

Tert-butyl 4-[trans-4-cyano-4-(3-methoxyphenyl)cyclohexyl]-piperazine-1-carboxylate (500 mg, 1.25 mmol) was dissolved in 4M hydrogen chloride/1,4-dioxane solution (20 mL), and then the mixture was stirred at room temperature for 5 hours. The solvent was evaporated under reduced pressure and then therero was added ethyl acetate. The precipitated crystals were collected by filtration to give the title compound 440 mg as white crystals.
High performance liquid chromatography/Mass spectrometry
m/z 300.3 (M+H)
Retention time: 1.67 min.

### Reference Example 40-2

### Synthesis of cis-1-(3-methoxyphenyl)-4-piperazin-1-ylcyclohexane-carbonitrile dihydrochloride

In the same manner as in Reference Example 40-1, the title compound was synthesized using tert-butyl 4-[cis-4-cyano-4-(3-methoxyphenyl)cyclohexyl]piperazine-l-carboxylate in 2.00 g as a starting material to give the title compound 1.04 g as white crystals.
High performance liquid chromatography/Mass spectrometry
m/z 300.3 (M+H)
Retention time: 1.94 min.

### Reference Example 41

### Synthesis of cis-4-[1-benzylpiperidin-4-yl]amino]-1-(3-methoxyphenyl)-cyclohexanecarbonitrile

To a suspension of cis-1-(3-methoxyphenyl)-4-(piperidin-4-ylamino)cyclohexanecarbonitrile (50 mg, 0.129 mmol) and potassium carbonate (89.1 mg, 0.645 mmol) in N,N-dimethylformamide (DMF, 1.0 mL) was added benzyl bromide (0.020 mL, 0.168 mmol) under ice-cooling and then the mixture was warmed to room temperature and stirred overnight. Thereto was added water to quench the reaction, and then the mixture was extracted with ethyl acetate and the organic layer was washed once with saturated aqueous sodium chloride solution. The organic layer was dried over anhydrous magnesium sulfate and filtered, and then the solvent was evaporated under reduced pressure. The resulting residue was purified by silica gel column chromatography to give the title compound 30.8 mg as pale yellow solids.
High performance liquid chromatography/ Mass spectrometry
m/z 404.5 (M+H)
Retention time: 2.00 min.

### Reference Example 42

### Synthesis of tert-butyl [cis-4-amino-4-(3-methoxyphenyl)cyclohexyl]-carbamate

### Synthesis of methyl cis-4-[(diphenylmethyl)amino]-1-(3-methoxyphenyl)-cyclohexanecarboxylate, and methyl trans-4-[(diphenylmethyl)arnino]-1-(3-methoxyphenyl)cyclohexanecarboxylate

To a solution of methyl 1-(3-methoxyphenyl)-4-oxocyclohexane-carboxylate) (100 mg, 0.381 mmol) in methanol (2.0 mL) were added benzohydrylamine (72.0 µL, 0.419 mmol), acetic acid (44.0 µL, 0.762 mmol) and sodium cyanoborohydride (29.0 mg, 0.457 mmol) at 0°C and the mixture was stirred at room temperature for 3 days. Thereto was added saturated aqueous sodium hydrogen carbonate solution to quench the reaction, and the reactant was extracted with ethyl acetate. The mixture was dried over anhydrous magnesium sulfate and filtered, and then the solvent was evaporated under reduced pressure. The resulting residue was purified by silica gel column chromatography to give the title compound: methyl cis-4-[(diphenylmethyl)amino]-1-(3-methoxyphenyl)cyclohexanecarboxylate in 74.0 mg and trans-4-[(diphenylmethyl)amino]-1-(3-methoxyphenyl)cyclohexanecarboxylate in 72.3 mg as white solids respectively.
cis-4-[(diphenylmethyl)amino]-1-(3-methoxyphenyl)cyclohexanecarboxylate
High performance liquid chromatography/ Mass spectrometry
m/z 430 (M+H)
Retention time: 2.92 min.
trans-4-[(diphenylmethyl)amino]-1-(3-methoxyphenyl)cyclohexanecarboxylate
High performance liquid chromatography/ Mass spectrometry
m/z 430 (M+H)
Retention time: 3.01 min.

### Synthesis of methyl cis-4-[(tert-butoxycarbonyl)amino]-1-(3-methoxyphenyl)cyclohexanecarboxylate

To a solution of methyl cis-4-[(diphenylmethyl)amino]-1-(3-methoxyphenyl)cyclohexanecarboxylate (1.20 g, 2.79 mmol) in methanol (24 mL) and tetrahydrofuran (12 mL) were added 10%-palladium-carbon (240 mg) and di-tert-butyl carbonate (963 µL, 4.19 mmol) at room temperature and the mixture was stirred under an atmosphere of hydrogen (0.3 MPa) for 3 hours. The mixture was filtered on celite and the solvent was evaporated under reduced pressure. This residue was purified by silica gel column chromatography to give the title compound 0.987 g as white solids.
High performance liquid chromatography/Mass spectrometry
m/z 364 (M+H)
Retention time: 3.92 min.

### Synthesis of cis-4-[(tert-butoxycarbonyl)amino]-1-(3-methoxyphenyl)-cyclohexanecarboxylic acid

To a solution of methyl cis-4-[(tert-butoxycarbonyl)amino]-1-(3-methoxyphenyl)cyclohexanecarboxylate (800 mg, 2.20 mmol) in dimethyl sulfoxide (8.0 mL) was added dropwise 6N- aqueous potassium hydroxide solution (550 µL, 3.30 mmol) at room temperature, and the mixture was stirred at 50°C for 1.5 hours. The reaction solution was cooled to room temperature and diluted with water and taken to pH = 4 with aqueous hydrochloric acid. This mixture was filtered under reduced pressure and washed with water to give a crude material. This material was purified by silica gel column chromatography to give the title compound 439mg as white solids.
¹H-NMR δ (DMSO-d₆); 1.31 (2H, m), 1.36 (9H, s), 1.53 (2H, m), 1.75 (2H, m), 2.39 (2H, m), 3.25 (1H, m), 3.72 (3H, s), 6.76 (1H, s), 6.79 (1H, dd, J=8.1, 2.4Hz), 6.87 (1H, m), 6.95 (1H, d, J=8.1Hz), 7.23 (1H, dd, J=8.1, 8.41Hz), 12.44 (1H, brs.).

### Synthesis of benzyl tert-butyl [cis-1-(3-methoxyphenyl)cyclohexan-1,4-diyl]biscarbamate

To a solution of cis-4-[(tert-butoxycarbonyl)amino]-1-(3-methoxyphenyl)cyclohexanecarboxylic acid (1.2 g, 3.43 mmol) in toluene (24 mL) were added triethylamine (1.43 mL, 10.29 mmol) and diphenylphosphoryl azide (0.885 mL, 4.11 mmol) and the mixture was stirred at 80°C for 8 hours. Then thereto was added benzyl alcohol (1.77 mL, 17.15 mmol) at room temperature and the mixture was stirred at 100°C for 8 hours and a half. The reaction solution was poured into water and the mixture was extracted with ethyl acetate. The organic layer was washed with saturated aqueous sodium chloride solution, and then dried over anhydrous magnesium sulfate and filtered. The filtrate was concentrated under reduced pressure, and the resulting residue was purified by silica gel column chromatography to give the title compound 1.45 g as white solids.
High performance liquid chromatography/ Mass spectrometry
m/z 455 (M+H)
Retention time: 3.84 min.

### Synthesis of tert-butyl [cis-4-amino-4-(3-methoxyphenyl)cyclohexyl]-carbamate

To a solution of benzyl tert-butyl [cis-1-(3-methoxyphenyl)-cyclohexan-1,4-diyl]biscarbamate (1.34 g, 2.94 mmol) in methanol (26 mL) was added 10% palladium-carbon (270 mg) and the mixture was stirred at room temperature for 3 hours and a half. The reaction solution was concentrated under reduced pressure and the resulting residue was purified by silica gel column chromatography to give the title compound 945 mg as white solids.
High performance liquid chromatography/Mass spectrometry
m/z 321 (M+H)
Retention time: 2.46 min.

### Reference Example 43

### Synthesis of cis-4-[(biphenyl-4-ylmethyl)amino]-1-(3-methoxyphenyl)-cyclohexanecarbonitrile

To a solution of cis-4-amino-1-(3-methoxyphenyl)cyclohexane-carbonitrile (1.5 g, 6.51 mmol) in 1,2-dichloroethane (60 mL) were added 4-biphenylaldehyde (1.18 g, 6.61 mmol) and sodium triacetoxyborohydride (2.06 g, 9.765 mmol) and the mixture was stirred at room temperature for 22 hours. The reaction solution was poured into saturated aqueous sodium bicarbonate solution and the mixture was extracted with ethyl acetate. The organic layer was washed with saturated aqueous sodium chloride solution and then dried over anhydrous sodium sulfate. The filtrate was concentrated under reduced pressure and the resulting residue was purified by silica gel column chromatography to give the title compound 2.0 g as white solids. High performance liquid chromatography/Mass spectrometry
m/z 397 (M+H)
Retention time: 2.99 min.

### Example 1-1

### Synthesis of phenyl {[1-(2-methoxyphenyl)-4-(3-methoxyphenyl)-piperidin-4-yl]carbonyl}sulfamate

To a solution of 1-(2-methoxyphenyl)-4-(3-methoxyphenyl)-piperidine-4-carboxylic acid (50 mg, 0.146 mmol) in dichloromethane (CH₂Cl₂ , 5 mL) was added dropwise oxalyl chloride ((COCl)₂ , 0.0280 mL, 0.321 mmol) under ice-cooling, and the mixture was warmed to room temperature and stirred overnight. The reaction solution was evaporated under reduced pressure, and the residue was dissolved in dichloromethane (CH₂Cl₂, 5 mL), and thereto were added phenyl sulfamate (30.3 mg, 0.175 mmol) and triethylamine (0.00610 mL, 0.438 mmol) at room temperature and the mixture was stirred at such temperature overnight. Thereto was added water to quench the reaction, and the mixture was extracted with ethyl acetate, and the organic layer was washed twice with saturated aqueous sodium chloride solution. The organic layer was dried over anhydrous magnesium sulfate and filtered, and the solvent was evaporated under reduced pressure. The resulting residue was purified by preparative thin layer chromatography (preparative TLC) to give the title compound 45.5 mg as amorphous.
High performance liquid chromatography/ Mass spectrometry
m/z 497.5 (M+H)
Retention time: 3.05 min.

### Example 1-2

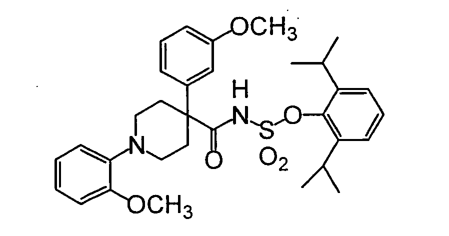

### Synthesis of 2,6-diisopropylphenyl {[1-(2-methoxyphenyl)-4-(3-methoxyphenyl)piperidin-4-yl]carbonyl}sulfamate

In the same manner as in Example 1-1, the title compound was synthesized.
High performance liquid chromatography/Mass spectrometry
m/z 581.3 (M+H)
Retention time: 4.80 min.

### Example 1-3

### Synthesis of 2-isopropylphenyl {[1-(2-methoxyphenyl)-4-(3-methoxyphenyl)piperidin-4-yl]carbonyl}sulfamate

In the same manner as in Example 1-1, the title compound was synthesized.
High performance liquid chromatography/ Mass spectrometry
m/z 539.2 (M+H)
Retention time: 3.28 min.

### Example 1-4

### Synthesis of 2,6-dimethylphenyl {[1-(2-methoxyphenyl)-4-(3-methoxyphenyl)piperidin-4-yl]carbonyl}sulfamate

In the same manner as in Example 1-1, the title compound was synthesized.
High performance liquid chromatography/Mass spectrometry
m/z 525.2 (M+H)
Retention time: 3.13 min.

### Example 1-5

### Synthesis of 2-methylphenyl {[1-(2-methoxyphenyl)-4-(3-methoxyphenyl)piperidin-4-yl]carbonyl}sulfamate

In the same manner as in Example 1-1, the title compound was synthesized.
High performance liquid chromatography/ Mass spectrometry
m/z 511.5 (M+H)
Retention time: 3.17 min.

### Example 1-6

### Synthesis of mesityl {[1-(2-methoxyphenyl)-4-(3-methoxyphenyl)-piperidin-4-yl]carbonyl}sulfamate

In the same manner as in Example 1-1, the title compound was synthesized.
High performance liquid chromatography/Mass spectrometry
m/z 539.2 (M+H)
Retention time: 3.40 min.

### Example 1-7

### Synthesis of 2,4-difluorophenyl {[1-(2-methoxyphenyl)-4-(3-methoxyphenyl)piperidin-4-yl]carbonyl}sulfamate

In the same manner as in Example 1-1, the title compound was synthesized.
High performance liquid chromatography/ Mass spectrometry
m/z 533.1 (M+H)
Retention time: 3.19 min.

### Example 1-8

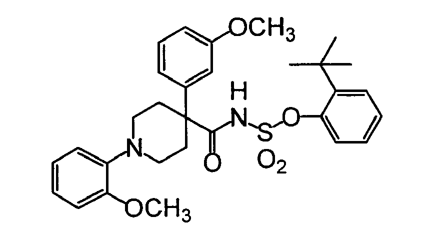

### Synthesis of 2-tert-butylphenyl {[1-(2-methoxyphenyl)-4-(3-methoxyphenyl)piperidin-4-yl]carbonyl}sulfamate

In the same manner as in Example 1-1, the title compound was synthesized.
High performance liquid chromatography/Mass spectrometry
m/z 533.2 (M+H)
Retention time: 3.39 min.

### Example 1-9

### Synthesis of 2-(trifluoromethyl)phenyl {[1-(2-methoxyphenyl)-4-(3-methoxyphenyl)piperidin-4-yl]carbonyl}sulfamate

In the same manner as in Example 1-1, the title compound was synthesized.
High performance liquid chromatography/Mass spectrometry
m/z 565.3 (M+H)
Retention time: 3.32 min.

### Example 1-10

### Synthesis of 1-(2-methoxyphenyl)-4-(3-methoxyphenyl)-N-[(2-methoxyphenyl)sulfonyl]piperidine-4-carboxamide

In the same manner as in Example 1-1, the title compound was synthesized.
High performance liquid chromatography/Mass spectrometry
m/z 495.5 (M+H)
Retention time: 2.96 min.

### Example 1-11

### Synthesis of 1-(2-methoxyphenyl)-4-(3-methoxyphenyl)-N-[(2,4,6-triisopropylphenyl)sulfonyl]piperidine-4-carboxamide

In the same manner as in Example 1-1, the title compound was synthesized.
High performance liquid chromatography/ Mass spectrometry
m/z 607.3 (M+H)
Retention time: 3.64 min.

### Example 1-12

### Synthesis of 2,6-diisopropylphenyl {[1-(2-methoxyphenyl)-4-(3-methoxyphenyl)piperidin-4-yl]acetyl}sulfamate

In the same manner as in Example 1-1, the title compound was synthesized.
High performance liquid chromatography/ Mass spectrometry
m/z 595.3 (M+H)
Retention time: 3.84 min.

### Example 1-13

### Synthesis of 1-(2-methoxyphenyl)-4-(3-methoxyphenyl)-N-[(2-methoxybenzyl)sulfonyl]piperidine-4-carboxamide

In the same manner as in Example 1-1, the title compound was synthesized.
High performance liquid chromatography/ Mass spectrometry
m/z 509.5 (M+H)
Retention time: 3.11 min.

### Example 1-14

### Synthesis of N-[(2,6-dimethylbenzyl)sulfonyl]-1-(2-methoxyphenyl)-4-(3-methoxyphenyl)piperidine-4-carboxamide

In the same manner as in Example 1-1, the title compound was synthesized.
High performance liquid chromatography/Mass spectrometry
m/z 523.6 (M+H)
Retention time: 3.17 min.

### Example 1-15

### Synthesis of 2,6-diisopropylphenyl {[4-(3-methoxyphenyl)-1-(3-methoxy-pyridin-2-yl)piperidin-4-yl]carbonyl}sulfamate

In the same manner as in Example 1-1, the title compound was synthesized.
High performance liquid chromatography/Mass spectrometry
m/z 582.1 (M+H)
Retention time: 3.47 min.

### Example 1-16

### Synthesis of 2,6-diisopropylphenyl {[4-(3-methoxyphenyl)-1-phenyl-piperidin-4-yl]carbonyl}sulfamate

In the same manner as in Example 1-1, the title compound was synthesized.
High performance liquid chromatography/ Mass spectrometry
m/z 551.6 (M+H)
Retention time: 3.90 min.

### Example 1-17

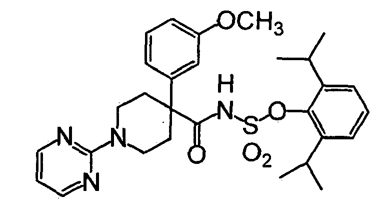

### Synthesis of 2,6-diisopropylphenyl {[4-(3-methoxyphenyl)-1-pyrimidin-2-ylpiperidin-4-yl] carbonyl}sulfamate

In the same manner as in Example 1-1, the title compound was synthesized.
High performance liquid chromatography/Mass spectrometry
m/z 553.2 (M+H)
Retention time: 4.11 min.

### Example 1-18

### 2,6-diisopropylphenyl {[1-(1,3-benzoxazole-2-yl)-4-(3-methoxyphenyl)-piperidin-4-yl]carbonyl}sulfamate

In the same manner as in Example 1-1, the title compound was synthesized.
High performance liquid chromatography/ Mass spectrometry
m/z 592.5 (M+H)
Retention time: 4.09 min.

### Example 1-19

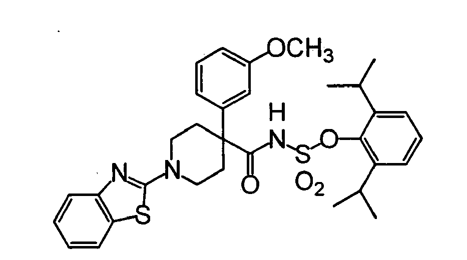

### Synthesis of 2,6-diisopropylphenyl {[1-(1,3-benzothiazol-2-yl)-4-(3-methoxyphenyl)piperidin-4-yl]carbonyl}sulfamate

In the same manner as in Example 1-1, the title compound was synthesized.
High performance liquid chromatography/ Mass spectrometry
m/z 608.4 (M+H)
Retention time: 4.01 min.

### Example 1-20

### Synthesis of 2,6-diisopropylphenyl {[1-benzyl-4-(3-methoxyphenyl)-piperidin-4-yl]carbonyl}sulfamate

In the same manner as in Example 1-1, the title compound was synthesized.
High performance liquid chromatography/ Mass spectrometry
m/z 565.3 (M+H)
Retention time: 3.51 min.

### Example 1-21

### Synthesis of 2,6-diisopropylphenyl {[1-[2-(2-methoxyethoxy)phenyl]-4-(3-methoxyphenyl)piperidin-4-yl]carbonyl}sulfamate

In the same manner as in Example 1-1, the title compound was synthesized.
High performance liquid chromatography/Mass spectrometry
m/z 625.5 (M+H)
Retention time: 3.59 min.

### Example 1-22

### Synthesis of 2,6-diisopropylphenyl {[4-(3-methoxyphenyl)-1-(2,2,2-trifluoroethyl)piperidin-4-yl]carbonyl}sulfamate

In the same manner as in Example 1-1, the title compound was synthesized.
High performance liquid chromatography/Mass spectrometry
m/z 557.4 (M+H)
Retention time: 2.86 min.

### Example 1-23

### Synthesis of 2,6-diisopropylphenyl 1[1-(2-methoxyphenyl)-4-phenyl-piperidin-4-yl]carbonyl}sulfamate

In the same manner as in Example 1-1, the title compound was synthesized.
High performance liquid chromatography/Mass spectrometry
m/z 551.3 (M+H)
Retention time: 3.69 min.

### Example 1-24

### Synthesis of 2,6-diisopropylphenyl {[1-(2-methoxyphenyl)-4-(3-methoxyphenyl) azepan-4-yl] acetyl}sulfamate

In the same manner as in Example 1-1, the title compound was synthesized.
High performance liquid chromatography/Mass spectrometry
m/z 595.3 (M+H)
Retention time: 3.63 min.

### Example 1-25

### Synthesis of 2,6-diisopropylphenyl {[1-(3-methoxyphenyl)-4-phenoxy-cyclohexyl]carbonyl}sulfamate

In the same manner as in Example 1-1, the title compound was synthesized.
High performance liquid chromatography/ Mass spectrometry
m/z 566.4 (M+H)
Retention time: 5.18 min.

### Example 1-26

### Synthesis of 2,6-diisopropylphenyl {[1-(3-methoxyphenyl)-4-phenoxy-cyclohexylicarbonyllsulfamate (stereoisomer of Example 1-25)

In the same manner as in Example 1-1, the title compound was synthesized.
High performance liquid chromatography/Mass spectrometry
m/z 566.4 (M+H)
Retention time: 4.61 min.

### Example 1-27

### Synthesis of 2,6-diisopropylphenyl {[1-(3-methoxyphenyl)-4-phenylcyclohexyl]carbonyl}sulfamate

In the same manner as in Example 1-1, the title compound was synthesized.
High performance liquid chromatography/ Mass spectrometry
m/z 550.4 (M+H)
Retention time: 4.93 min.

### Example 1-28

### Synthesis of 2,6-diisopropylphenyl {[1-(3-methoxyphenyl)-4-oxo-cyclohexyl]carbonyl}sulfamate

In the same manner as in Example 1-1, the title compound was synthesized.
High performance liquid chromatography/ Mass spectrometry
m/z 488.3 (M+H)
Retention time: 4.13 min.

### Example 2-1

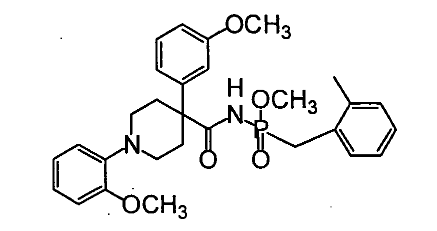

### Synthesis of methyl N-{[1-(2-methoxyphenyl)-4-(3-methoxyphenyl)piperidin-4-yl]carbonyl}-P-(2-methylbenzyl)-phosphonamidate

To a solution of 1-(2-methoxyphenyl)-4-(3-methoxyphenyl)-piperidine-4-carboxylic acid (50 mg, 0.146 mmol) in dichloromethane (CH₂Cl₂, 5 mL) was added dropwise oxalyl chloride ((COCl)₂, 0.0280 mL, 0.321 mmol) under ice-cooling and the mixture was warmed to room temperature and then stirred for 3 hours. The reaction solution was evaporated under reduced pressure to give acid chloride as white powder.

On the other hand, to a solution of sodium hydride (21.7 mg, 0.542 mmol) in tetrahydrofuran (THF, 5 mL) was added methyl P-(2-methylbenzyl)phosphonamidate (50 mg, 0.249 mmol) in tetrahydrofuran (THF, 0.5 mL) at room temperature, and the mixture was stirred for 30 min. To the reaction solution was added the above acid chloride at room temperature, and the mixture was stirred at such temperature for 2 hours. Thereto was added 10% hydrochloric acid to take the reaction solution to be weak acidic, and thereto was added sodium chloride to make the reaction solution to be saturated. The mixture was extracted twice with ethyl acetate, and the organic layer was dried over anhydrous magnesium sulfate and filtered, and the solvent was evaporated under reduced pressure. The resulting residue was purified by preparative thin layer chromatography (preparative TLC) to give the title compound 31.5 mg as amorphous.
High performance liquid chromatography/ Mass spectrometry
m/z 523.6 (M+H)
Retention time: 3.05 min.

### Example 2-2

### Synthesis of 1-(2-methoxyphenyl)-4-(3-methoxyphenyl)-N-[(2-methoxyphenyl)acetyl]piperidine-4-carboxamide

In the same manner as in Example 2-1, the title compound was synthesized.
High performance liquid chromatography/ Mass spectrometry
m/z 473.4 (M+H)
Retention time: 3.09 min.

### Example 2-3

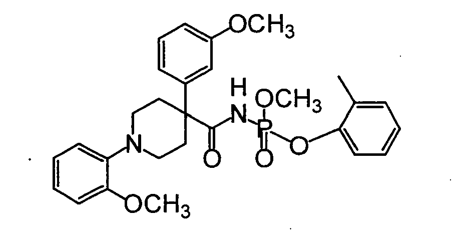

### Synthesis of 2-methylphenyl methyl {[1-(2-methoxyphenyl)-4-(3-methoxyphenyl)piperidin-4-yl]carbonyl}amidophosphate

In the same manner as in Example 2-1, the title compound was synthesized.
High performance liquid chromatography/ Mass spectrometry
m/z 525.5 (M+H)
Retention time: 2.90 min.

### Example 3-1

### Synthesis of 2,6-diisopropylphenyl {[4-anilino-1-(3-methoxyphenyl)-cyclohexyl]carbonyl}sulfamate

To a solution of 2,6-diisopropylphenyl {[1-(3-methoxyphenyl)-4-oxocyclohexyl]carbonyl}sulfamate (100 mg, 0.205 mmol), aniline (0.0411 mL, 0.451 mmol) and acetic acid (0.001 mL, 10 mol%) in 1,2-dichloroethane (ClCH₂CH₂Cl, 10 mL) was added sodium triacetoxyborohydride (NaBH(OAc)₃, 130 mg, 0.615 mmol) at room temperature and then the mixture was stirred at such temperature overnight. The reaction was quenched with water and the mixture was extracted with ethyl acetate and the organic layer was washed twice with water. The organic layer was dried over anhydrous magnesium sulfate and filtered, and the solvent was evaporated under reduced pressure. The resulting residue was purified by silica gel column chromatography to give the title compound 41.7 mg as white powder.
High performance liquid chromatography/ Mass spectrometry
m/z 565.6 (M+H)
Retention time: 3.65 min.

### Example 3-2

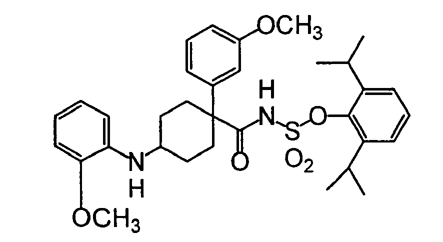

### Synthesis of 2,6-diisopropylphenyl ({1-(3-methoxyphenyl)-4-[(2-methoxyphenyl)amino]cyclohexyl}carbonyl)sulfamate

In the same manner as in Example 3-1, the title compound was synthesized.
High performance liquid chromatography/ Mass spectrometry
m/z 595.6 (M+H)
Retention time: 4.28 min.

### Example 4

### Synthesis of 2,6-diisopropylphenyl {[4-hydroxy-1-(3-methoxyphenyl) cyclohexyl]carbonyl}sulfamate

To a solution of 2,6-diisopropylphenyl {[1-(3-methoxyphenyl)-4-oxocyclohexyl]carbonyl}sulfamate (20.0 mg, 0.041 mmol) in tetrahydrofuran (THF, 5 mL) was added sodium borohydride (NaBH₄, 3.14 mg, 0.090 mmol) under ice-cooling and then the mixture was warmed to room temperature and the mixture was stirred overnight. The reaction was quenched with saturated aqueous ammonia and the mixture was extracted with ethyl acetate, and the organic layer was washed twice with saturated aqueous ammonia. The mixture was dried over anhydrous magnesium sulfate and filtered, and the solvent was evaporated under reduced pressure to give the title compound 20.0 mg as amorphous.
High performance liquid chromatography/Mass spectrometry
m/z 490.2 (M+H)
Retention time: 3.88 min.

### Example 5

### Synthesis of 2,6-diisopropylphenyl {[4-hydroxy-1-(3-methoxyphenyl)-4-phenylcyclohexyl] carbonyl}sulfamate

To a solution of 2,6-diisopropylphenyl {[1-(3-methoxyphenyl)-4-oxocyclohexyl]carbonyl}sulfamate (20.0 mg, 0.041 mmol) in tetrahydrofuran (THF, 5 mL) was added 0.94 M phenyllithium solution (PhLi, 0.0957 mL) at -78°C and the mixture was warmed to room temperature and stirred for 2 hours. Thereto was further added a 0.94M phenyllithium solution (PhLi, 0.0957 mL) at -78°C, and the mixture was warmed to room temperature and the mixture was stirred for 2 hours. Thereto was added saturated aqueous ammonia to quench the reaction and the mixture was extracted with ethyl acetate and the organic layer was washed twice with saturated aqueous ammonia. The organic layer was dried over anhydrous magnesium sulfate and filtered, and the solvent was evaporated under reduced pressure. The resulting residue was purified by preparative thin layer chromatography (preparative TLC) to give the title compound 15.2 mg as white powder.
High performance liquid chromatography/Mass spectrometry
m/z 588.2 (M+H)
Retention time: 4.30 min.

### Example 6

### Synthesis of 2,6-diisopropylphenyl {[1,4-diphenylpiperidin-4-yl]-carbonyl}sulfamate

To a suspension of sodium hydride (382 mg, 9.55 mmol) in dimethyl sulfoxide (50 mL) was added a solution of phenylacetonitrile (0.500 mL, 4.34 mmol) and N,N-bis(2-chloroethyl)aniline (1.04 g, 4.77 mmol) in dimethyl sulfoxide (10 mL) at room temperature. The mixture was stirred overnight, and then thereto was added water to quench the reaction. The mixture was extracted with ethyl acetate, and the organic layer was washed twice with water. The organic layer was dried over anhydrous magnesium sulfate and filtered, and the solvent was evaporated under reduced pressure. The resulting residue was purified by silica gel column chromatography to give yellow crystals 500mg, and 100mg of them was suspended in 48% aqueous hydrogen bromide (5 mL), and the mixture was heated under reflux for 4 hours and the solvent was evaporated under reduced pressure. The resulting residue was dissolved in dichloromethane (10 mL), and thereto was added oxalyl chloride (0.0682mL, 0.782 mmol) at room temperature and the mixture was heated under reflux for 3 hours. The solvent was evaporated under reduced pressure, and the residue was dissolved in dichloromethane (10 mL), and thereto were added 2,6-diisopropylphenyl sulfamate (110 mg, 0.426 mmol) and triethylamine (0.148 mL, 1.07 mmol) at room temperature and the mixture was stirred for 2 hours. Thereto was added saturated aqueous sodium chloride solution to quench the reaction and the mixture was extracted with ethyl acetate, and the organic layer was washed once with water. The solvent was evaporated under reduced pressure, and the residue was purified by preparative thin layer chromatography to give the title compound 78.8 mg as amorphous.
High performance liquid chromatography/ Mass spectrometry
m/z 521.3 (M+H)
Retention time: 4.45 min.

### Example 7

### Synthesis of 2,6-diisopropylphenyl {[4-benzylidene-1-(3-methoxyphenyl)cyclohexyl]carbonyl}sulfamate

To a solution of methyl 4-benzylidene-1-(3-methoxyphenyl)-cyclohexanecarboxylate (62.6 mg, 0.186 mmol) in 1,4-dioxane (8 mL)-water (2 mL) was added lithium hydroxide (22.3 mg, 0.930 mmol) and the mixture was heated under reflux for 5 hours. The solvent was evaporated, and thereto was added 10% hydrochloric acid to take the mixture to be weak acidic. The mixture was extracted with ethyl acetate, and the organic layer was washed once with 10% hydrochloric acid. The organic layer was dried over anhydrous magnesium sulfate and filtered, and the solvent was evaporated under reduced pressure to give white powder. The resulting white powder was dissolved in dichloromethane (10 mL) and thereto was added oxalyl chloride (0.0311 mL, 0.347 mmol) at room temperature. Thereto was added one drop of N,N-dimethylformamide, and the mixture was stirred at room temperature for 2 hours. The solvent was evaporated under reduced pressure, and the residue was dissolved in dichloromethane (10 mL), and thereto were added 2,6-diisopropylphenyl sulfamate (57.9 mg, 0.225 mmol) and triethylamine (0.0723 mL, 0.519 mmol) at room temperature and the mixture was stirred overnight. Thereto was added saturated aqueous ammonium chloride solution to quench the reaction and the mixture was extracted with ethyl acetate, and the organic layer was washed twice with saturated aqueous ammonium solution. The organic layer was dried over anhydrous magnesium sulfate and filtered, and the solvent was evaporated under reduced pressure. The resulting residue was purified by preparative thin layer chromatography to give the title compound 37.3 mg as amorphous.
High performance liquid chromatography/ Mass spectrometry
m/z 562.5 (M+H)
Retention time: 4.80 min.

### Example 8

### Synthesis of 2,6-diisopropylphenyl {[1-(2-methoxyphenyl)-4-(3-methoxyphenyl)piperidin-4-yl]carbonyl}sulfamate hydrochloride

To a solution of 2,6-diisopropylphenyl 1[1-(2-methoxyphenyl)-4-(3-methoxyphenyl)piperidin-4-yl]carbonyl}sulfamate (100 mg, 0.172 mmol) in methanol (MeOH, 10 mL) was added 1M hydrogen chloride/diethylether solution (Et₂O, 0.224 mL) at room temperature and the mixture was stirred at such temperature for 15 min. The solvent was evaporated under reduced pressure, and the resulting residue was crystallized from diethylether to give the title compound 98.9 mg as white powder.
Elementary Analysis: (Anal. Calcd for C₃₂H₄₁ClN₂O₆S·H₂O: C, 60.51; H, 6.82; Cl, 5.58; N, 4.41; S, 5.05. Found: C, 60.02; H, 6.74; Cl, 5.63; N, 4.33; S, 5.22.)

### Example 9

### Synthesis of 2,6-diisopropylphenyl {[1-(2-methoxyphenyl)-4-(3-methoxyphenyl)piperidin-4-yl]carbonyl}sulfamate sodium salt

To a solution of 2,6-diisopropylphenyl {[1-(2-methoxyphenyl)-4-(3-methoxyphenyl)piperidin-4-yl]carbonyl}sulfamate (100 mg, 0.172 mmol) in methanol (MeOH, 10 mL) was added sodium t-butoxide (NaO^{t} Bu, 16.5 mg, 0.172 mmol) at room temperature and the mixture was stirred at such temperature for 15 min. The solvent was evaporated under reduced pressure and the resulting residue was crystallized from diethylether to give the title compound 83.6 mg as white powder.
Elementary Analysis: (Anal. Calcd for C₃₂H₃₉N₂NaO₆S · 1/2 H₂O: C, 62.83; H, 6.59; N, 4.58; Na, 3.76; S, 5.24. Found: C, 62.90; H, 6.48; N, 4.44; Na, 3.74; S, 5.30.)

### Example 10-1

### Synthesis of N-{[cis-1-(3-methoxyphenyl)-4-piperazin-1-ylcyclohexyl]-methyl}methanesulfonamide dihydrochloride

To a solution of cis-1-(3-methoxyphenyl)-4-piperazin-1-yl-cyclohexanecarbonitrile dihydrochloride (300 mg, 0.806 mmol) and triethylamine (0.449 mL, 3.22 mmol) in dichloromethane (5.0 mL) was added trityl chloride (269 mg, 0.966 mmol) under ice-cooling and then the mixture was warmed to room temperature and stirred overnight. Thereto was added water to quench the reaction and then the mixture was extracted with ethyl acetate and the organic layer was washed twice with water. The organic layer was dried over anhydrous magnesium sulfate and filtered, and then the solvent was evaporated under reduced pressure to give the reaction mixture 321 mg as colorless amorphous. A 150mg of the resulting reaction mixture was added to a suspension of lithium aluminum hydride (15.8 mg, 0.416 mmol) in THF (5 mL) under ice-cooling, and then the mixture was warmed to room temperature and stirred overnight. Thereto was added 28% aqueous ammonia to quench the reaction. The mixture was filtered on celite and the solvent was evaporated under reduced pressure to give white crystal. The resulting crystals were dissolved in dichloromethane (5 mL) and thereto was added triethylamine (0.0580 mL, 0.416 mmol). Then thereto was added methanesulfonyl chloride (0.0257 mL, 0.332 mmol) at -10°C and then the mixture was warmed to room temperature and the mixture was stirred overnight. Thereto was added water to quench the reaction and then the mixture was extracted with ethyl acetate and the organic layer was washed twice with water. The organic layer was dried over anhydrous magnesium sulfate and filtered, and then the solvent was evaporated under reduced pressure. The resulting residue was purified by silica gel column chromatography to give 105 mg as colorless amorphous. To the resulting amorphous was added 4M hydrogen chloride/1,4-dioxane solution (5 mL), and the mixture was stirred at room temperature overnight. The solvent was evaporated under reduced pressure and then the resulting residue was crystallized from ethyl acetate to give the title compound 49.6 mg as white solids.
High performance liquid chromatography/Mass spectrometry
m/z 382.4 (M+H)
Retention time: 2.02 min.

### Example 10-2

### Synthesis of N-{[trans-1-(3-methoxyphenyl)-4-piperazin-1-ylcyclohexyl]-methyl}methanesulfonamide dihydrochloride

In the same manner as in Example 10-1, the title compound as white crystals was synthesized using trans-1-(3-methoxyphenyl)-4-(piperidin-4-ylamino)-cyclohexanecarbonitrile dihydrochloride as a starting material.
High performance liquid chromatography/Mass spectrometry
m/z 382.4 (M+H)
Retention time: 0.61 min.

### Example 10-3

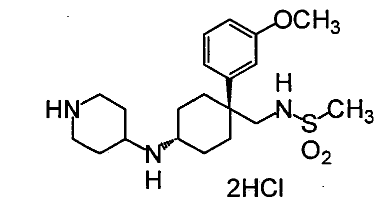

### Synthesis of N-{[cis-1-(3-methoxyphenyl)-4-(piperidin-4-ylamino)-cyclohexyl]methyl}methanesulfonamide dihydrochloride

In the same manner as in Example 10-1, the title compound as white crystals was synthesized using cis-1-(3-methoxyphenyl)-4-(piperidin-4-ylamino)cyclohexanecarbonitrile dihydrochloride as a starting material.
m/z 396.1 (M+H)
Retention time: 0.81 min.

### Example 10-4

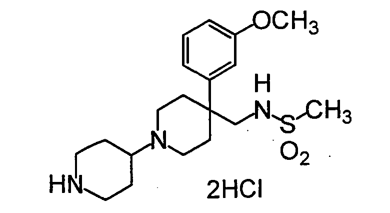

### Synthesis of N-{[4-(3-methoxyphenyl)-1,4'-bipiperidin-4-yl]methyl} methanesulfonamide dihydrochloride

In the same manner as in Example 10-1, the title compound as white crystals was synthesized using 4-(3-methoxyphenyl)-1,4'-bipiperidine-4-carbonitrile dihydrochloride as a starting material.
High performance liquid chromatography/Mass spectrometry
m/z 382.4 (M+H)
Retention time: 0.51 min.

### Example 11

### Synthesis of N-{[cis-4-amino-l-(3-methoxyphenyl)cyclohexyl]methyl} methanesulfonamide

### Synthesis of N-{[cis-4-[(diphenylmethyl)amino]-1-(3-methoxyphenyl)-cyclohexyl]methyl}methanesulfonamide

To a suspension of lithium aluminum hydride (71.8 mg, 1.89 mmol) in THF (10 mL) was added cis-4-[(diphenylmethyl)amino]-1-(3-methoxyphenyl)cyclohexanecarbonitrile (500 mg, 1.26 mmol) under ice-cooling, and then the mixture was warmed to room temperature and stirred overnight. Thereto was added 28% aqueous ammonia to quench the reaction. The mixture was filtered on celite and then the solvent was evaporated under reduced pressure to give white crystals in 299 mg. The resulting crystal 280 mg was dissolved in dichloromethane (5 mL) and thereto was added triethylamine (0.118 mL, 0.839 mmol). Then thereto was added methanesulfonyl chloride (0.0630 mL, 0.769 mmol) at -10°C, and then the mixture was warmed to room temperature and stirred overnight. Thereto was added water to quench the reaction and then the mixture was extracted with ethyl acetate and the organic layer was washed twice with water. The organic layer was dried over anhydrous magnesium sulfate and filtered, and then the solvent was evaporated under reduced pressure. The resulting residue was purified by silica gel column chromatography to give the title compound 155 mg as colorless oils.
High performance liquid chromatography/ Mass spectrometry
m/z 479.3 (M+H)
Retention time: 2.84 min.

### Synthesis of N-{[cis-4-amino-1-(3-methoxyphenyl)cyclohexyl]methyl}-methanesulfonamide

A suspension of N-{[cis-4-[(diphenylmethyl)amino]-1-(3-methoxyphenyl)cyclohexyl]methyl}methanesulfonamide (155 mg, 0.324 mmol), palladium/ carbon (31 mg) and ammonium formate (204 mg, 3.24 mmol) in ethanol (5 mL) was heated under reflux for 5 hours. Thereto were further added palladium/ carbon (93 mg) and ammonium formate (612 mg, 9.72 mmol) and the mixture was heated under reflux for 8 hours. The mixture was filtered on celite and then the solvent was evaporated under reduced pressure. The resulting residue was purified by silica gel column chromatography to give the title compound 61.6 mg as colorless oils.
High performance liquid chromatography/ Mass spectrometry
m/z 313.2 (M+H)
Retention time: 1.41 min.

### Example 12-1

### Synthesis of N-(4-{[(cis-4-(3-methoxyphenyl)-4-{[(methylsulfonyl)amino]-methyl}cyclohexyl)amino]methyl}phenyl)acetamide

To a solution of N-{[cis-4-amino-1-(3-methoxyphenyl)cyclohexyl]-methyl}methanesulfonamide (30 mg, 0.0960 mmol) and 4-acetamide benzaldehyde (17.3 mg, 0.106 mmol) in 1,2-dichloroethane (2 mL) was added sodium triacetoxyborohydride (NaHB(OAc)₃, 17.3 mg, 0.106 mmol) at room temperature and then the mixture was stirred overnight. Thereto was added saturated aqueous sodium bicarbonate solution to quench the reaction, and then the mixture was extracted with ethyl acetate, and the organic layer was washed twice with saturated aqueous sodium bicarbonate solution. The organic layer was dried over anhydrous magnesium sulfate and filtered, and then the solvent was evaporated under reduced pressure. The resulting residue was purified by silica gel column chromatography to give the title compound 25.2 mg as colorless amorphous.
High performance liquid chromatography/ Mass spectrometry
m/z 460.3 (M+H)
Retention time: 2.34 min.

### Example 12-2

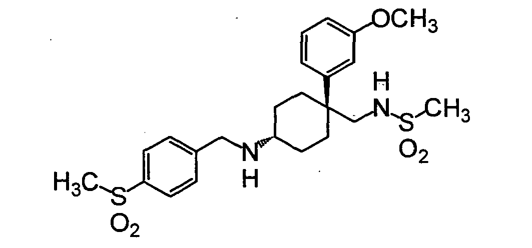

### Synthesis of N-[(cis-1-(3-methoxyphenyl)-4-{[4-(methylsulfonyl)benzyl]-amino}cyclohexyl)methyl]methanesulfonamide

In the same manner as in Example 12-1, the title compound was synthesized using 4-methylsulfonyl benzaldehyde as an aldehyde.
High performance liquid chromatography/Mass spectrometry
m/z 481.3 (M+H)
Retention time: 2.28 min.

### Example 13-1

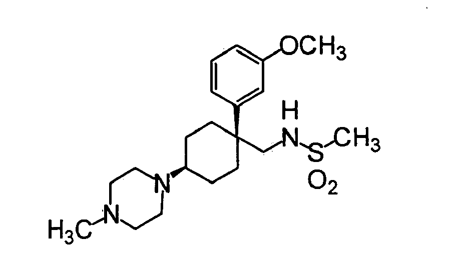

### Synthesis of N-{[trans-1-(3-methoxyphenyl)-4-(4-methylpiperazin-1-yl)cyclohexyl]methyl}methanesulfonamide

A solution of N-{[trans-1-(3-methoxyphenyl)-4-piperazin-1-ylcyclohexyl]methyl}methanesulfonamide dihydrochloride (19.3 mg, 0.0425 mmol), iodomethane (0.00317, 0.051 mmol) and potassium carbonate (23.5 mg, 0.17 mmol) in N,N-dimethylformamide (DMF, 1 mL) was stirred at room temperature overnight. Thereto was added water to quench the reaction, and then the mixture was extracted with ethyl acetate, and the organic layer was washed once with water. The organic layer was dried over anhydrous magnesium sulfate and filtered, and then the solvent was evaporated under reduced pressure to give the title compound 3.34 mg as amorphous.
High performance liquid chromatography/ Mass spectrometry
m/z 396.1 (M+H)
Retention time: 0.29 min.

### Example 13-2

### Synthesis of N-{[4-(3-methoxyphenyl)-1'-methyl-1,4'-bipiperidin-4-yl]-methyl}methane sulfonamide

In the same manner as in Example 13-1, the title compound was synthesized using N-{[4-(3-methoxyphenyl)-1,4'-bipiperidin-4-yl]methyl}-methanesulfonamide dihydrochloride as an starting material.
High performance liquid chromatography/Mass spectrometry
m/z 396.1 (M+H)
Retention time: 0.37 min.

### Example 14-1

### Synthesis of N-{[cis-4-[(1-isopropylpiperidin-4-yl)amino]-1-(3-methoxyphenyl)cyclohexyl]methyl}methanesulfonamide

A solution of N-{[cis-1-(3-methoxyphenyl)-4-(piperidin-4-ylamino)-cyclohexyl]methyl}methanesulfonamide dihydrochloride (50.0 mg, 0.107 mmol), 2-iodopropane (0.013 mL, 0.128 mmol) and potassium carbonate (59.2 mg, 0.428 mmol) in N,N-dimethylformamide (DMF, 2 mL) was stirred at room temperature overnight. Thereto was added saturated aqueous sodium chloride solution to quench the reaction and then the mixture was extracted with ethyl acetate and the organic layer was washed once with saturated aqueous sodium chloride solution. The organic layer was dried over anhydrous magnesium sulfate and filtered, and then the solvent was evaporated under reduced pressure. The resulting residue was purified by silica gel column chromatography to give the title compound 30.1 mg as colorless oils.
High performance liquid chromatography/ Mass spectrometry
m/z 0.38 (M+H)
Retention time: 438.4 min.

### Example 14-2

### Synthesis of N-{[cis-4-[(1-methylpiperidin-4-yl)amino]-1-(3-methoxyphenyl)cyclohexyl]methyl}methanesulfonamide

In the same manner as in Example 14-1, the title compound was synthesized using iodomethane as an alkylating agent.
High performance liquid chromatography/Mass spectrometry
m/z 2.17 (M+H)
Retention time: 410.1 min.

### Example 15-1

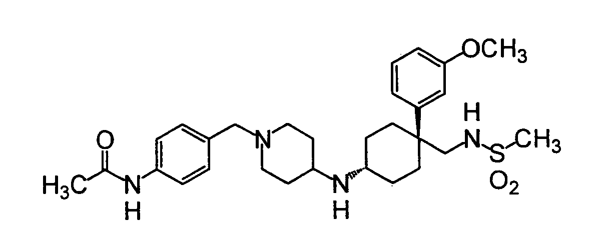

### Synthesis of N-[4-({4-[(cis-4-(3-methoxyphenyl)-4-{[(methylsulfonyl)-amino]methyl}cyclohexyl)amino]piperidin-1-yl}methyl)phenyl]acetamide

To solution of N-{[cis-1-(3-methoxyphenyl)-4-(piperidin-4-ylamino)-cyclohexyl]methyl}methanesulfonamide dihydrochloride (50.0 mg, 0.107 mmol) and 4-acetamide benzaldehyde (20.9 mg, 0.128 mmol) in 1,2-dichloroethane (2 mL) was added sodium triacetoxyborohydride (NaHB(OAc)₃, 29.5mg, 0.139 mmol) at room temperature and then the mixture was stirred overnight. Thereto was added saturated aqueous sodium bicarbonate solution to quench the reaction and the mixture was extracted with ethyl acetate and the organic layer was washed twice with saturated aqueous sodium chloride solution. The organic layer was dried over anhydrous magnesium sulfate and filtered, and then the solvent was evaporated under reduced pressure. The resulting residue was purified by silica gel column chromatography to give the title compound 13.7 mg as colorless oils.
High performance liquid chromatography/ Mass spectrometry
m/z 543.4 (M+H)
Retention time: 1.94 min.

### Example 15-2

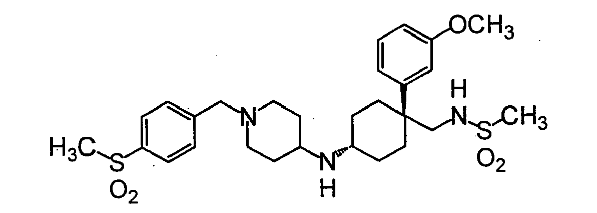

### Synthesis of N-[(cis-1-(3-methoxyphenyl)-4-{1-[4-(methylsulfonyl)-benzyl]piperidin-4-ylamino}cyclohexyl)methyl]methanesulfonamide

In the same manner as in Example 15-1, the title compound was synthesized using 4-methylsulfonyl benzaldehyde as an aldehyde.
High performance liquid chromatography/ Mass spectrometry
m/z 564.5 (M+H)
Retention time: 2.36 min.

### Example 16

### Synthesis of N-[cis-4-[(biphenyl-4-ylmethyl)amino]-1-(3-methoxyphenyl)cyclohexyl]methanesulfonamide

### Synthesis of tert-butyl {cis-4-(3-methoxyphenyl)-4-[(methylsulfonyl)-amino]cyclohexyl}carbamate

To a solution of tert-butyl [cis-4-amino-4-(3-methoxyphenyl)-cyclohexyl]carbamate (100 mg, 0.312 mmol) and triethylamine (0.057 mL, 0.406 mmol) in dichloromethane (2.0 mL) was added methanesulfonyl chloride (0.030 mL, 0.374 mmol) under ice-cooling and then the mixture was warmed to room temperature and stirred overnight. Thereto was added water to quench the reaction and the mixture was extracted with ethyl acetate and the organic layer was washed twice with water. The organic layer was dried over anhydrous magnesium sulfate and filtered, and then the solvent was evaporated under reduced pressure. The resulting residue was purified by silica gel column chromatography to give the title compound 117 mg as pale yellow solids.
High performance liquid chromatography/Mass spectrometry
m/z 399.2 (M+H)
Retention time: 3.28 min.

### Synthesis of N-[cis-4-amino-1-(3-methoxyphenyl)cyclohexyl]methanesulfonamide hydrochloride

A solution of tert-butyl {cis-4-(3-methoxyphenyl)-4-[(methylsulfonyl)amino]cyclohexyl}carbamate (117 mg, 0.293 mmol) in 4M hydrogen chloride/1,4-dioxane solution (5 mL) was stirred at room temperature overnight. The solvent was evaporated under reduced pressure, and then the resulting residue was crystallized from ethyl acetate to give the title compound 39.1 mg as white solids.
High performance liquid chromatography/Mass spectrometry
m/z 299.5 (M+H)
Retention time: 2.11 min.

### Synthesis of N-[cis-4-[(biphenyl-4-ylmethyl)amino]-1-(3-methoxyphenyl)cyclohexyl]methanesulfonamide

To a solution of N-[cis-4-amino-1-(3-methoxyphenyl)cyclohexyl]-methanesulfonamide hydrochloride (18.5 mg, 0.055 mmol) and 4-biphenylcarboxyaldehyde (12.0 mg, 0.066 mmol) in 1,2-dichloroethane (1 mL) was added sodium triacetoxyborohydride (NaHB(OAc)₃, 15.3 mg, 0.072 mmol) at room temperature and then the mixture was stirred overnight. Thereto was added saturated aqueous sodium bicarbonate solution to quench the reaction and the mixture was extracted with ethyl acetate and the organic layer was washed twice with saturated aqueous sodium chloride solution. The organic layer was dried over anhydrous magnesium sulfate and filtered, and then the solvent was evaporated under reduced pressure. The resulting residue was purified by preparative thin layer chromatography to give the title compound 9.00 mg as colorless oils.
¹H-NMR (400MHz, DMSO-d₆) δ 1.67 (6H, m), 2.10 (2H, m), 2.53 (3H, m), 2.95 (1H, m), 3.81 (3H, m), 3.94 (2H, m), 6.05 (1H, m), 6.76-6.80 (1H, m), 6.91-6.92 (1H, m), 6.96-6.98 (1H, m), 7.19-7.22 (1H, m), 7.34-7.36 (1H, m), 7.41-7.46 (4H, m), 7.56-7.60 (4H, m).

### Example 17

### Synthesis of N-[cis-1-(3-methoxyphenyl)-4-(piperidin-4-ylamino)-cyclohexyl]methanesulfonamide dihydrochloride

To a solution of N-[cis-4-amino-1-(3-methoxyphenyl)cyclohexyl]-methanesulfonamide hydrochloride (18.0 mg, 0.055 mmol) and 1-tert-butoxycarbonyl-4-piperidone (12.2 mg, 0.066 mmol) in 1,2-dichloroethane (1 mL) was added sodium triacetoxyborohydride (NaHB(OAc)₃, 15.3 mg, 0.072 mmol) at room temperature and then the mixture was stirred overnight. Thereto was added saturated aqueous sodium bicarbonate solution to quench the reaction and the mixture was extracted with ethyl acetate, and the organic layer was washed twice with saturated aqueous sodium chloride solution. The organic layer was dried over anhydrous magnesium sulfate and filtered, and then the solvent was evaporated under reduced pressure. The resulting residue was purified by preparative thin layer chromatography and then the resulting product was dissolved in 4M hydrogen chloride/1,4-dioxane solution (1 mL) and the mixture was stirred at room temperature overnight. The solvent was evaporated under reduced pressure, and then the resulting residue was crystallized from ethyl acetate to give the title compound 4.50 mg as white solids.
High performance liquid chromatography/Mass spectrometry
m/z 387.4 (M+H)
Retention time: 2.80 min.

### Example 18-1

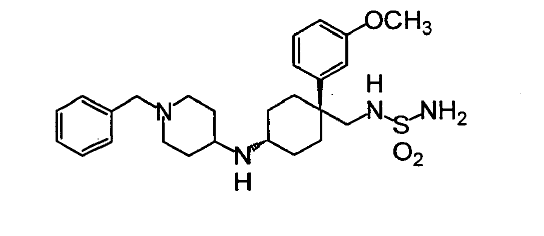

### Synthesis of N-{[cis-4-[(1-benzylpiperidin-4-yl)amino]-1-(3-methoxyphenyl)cyclohexyl]methyl}sulfamide

To a solution of tert-butyl [({2-[cis-4-amino-1-(3-methoxyphenyl)-cyclohexyl]methyl}amino)sulfonyl]carbamate (40 mg, 0.0967 mmol) and 1-benzyl-4-piperidone (22.0 mg, 0.116 mmol) in 1,2-dichloroethane (2 mL) was added sodium triacetoxyborohydride (NaHB(OAc)₃, 71.7 mg, 0.338 mmol) at room temperature and then the mixture was stirred overnight. Thereto was added saturated aqueous sodium bicarbonate solution to quench the reaction and the mixture was extracted with ethyl acetate, and the organic layer was washed twice with saturated aqueous sodium chloride solution. The organic layer was dried over anhydrous magnesium sulfate and filtered, and then the solvent was evaporated under reduced pressure. The resulting residue was purified by preparative thin layer chromatography to give a product. The resulting product was dissolved in trifluoroacetic acid (2.5 mL)-dichloromethane (0.5 mL) and the mixture was stirred overnight. Thereto was added saturated aqueous sodium bicarbonate solution to quench the reaction and then the mixture was salted out and extracted with ethyl acetate, and the organic layer was washed twice with saturated aqueous sodium chloride solution. The organic layer was dried over anhydrous magnesium sulfate and filtered, and then the solvent was evaporated under reduced pressure to give the title compound 3.00 mg as amorphous.
High performance liquid chromatography/Mass spectrometry
m/z 487.4 (M+H)
Retention time: 2.17 min.

### Example 18-2

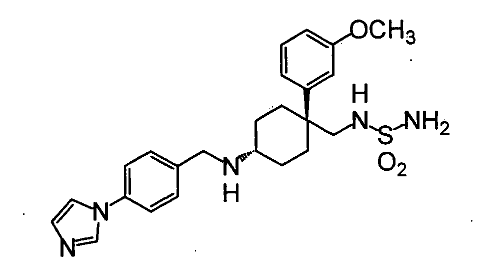

### Synthesis of N-{[cis-4-{[4-(1H-imidazol-1-yl)benzyl]amino}-1-(3-methoxyphenyl)cyclohexyl]methyl}sulfamide

In the same manner as in Example 18-1, the title compound was synthesized using 4-(1H-imidazol-1-yl)benzaldehyde as a reaction reagent.
High performance liquid chromatography/ Mass spectrometry
m/z 470.3 (M+H)
Retention time: 2.05 min.

### Example 18-3

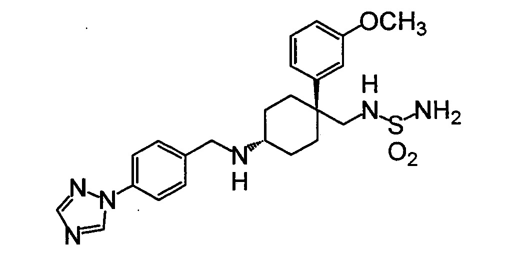

### Synthesis of N-[(cis-1-(3-methoxyphenyl)-4-{[4-(1H-1,2,4-triazol-1-yl)-benzyl]amino}cyclohexyl)methyl]sulfamide

In the same manner as in Example 18-1, the title compound was synthesized using 4-(1H-1,2,4-triazol-1-yl)benzaldehyde as a reaction reagent.
High performance liquid chromatography/Mass spectrometry
m/z 471.2 (M+H)
Retention time: 2.36 min.

### Example 18-4

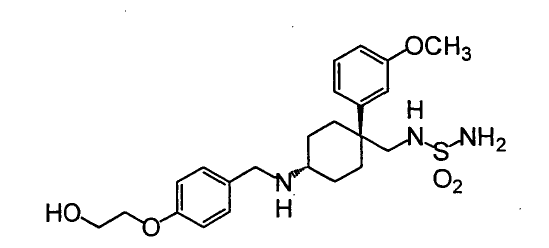

### Example 18-4

### Synthesis of N-{[cis-4-{[4-(2-hydroxyethoxy)benzyl]amino}-1-(3-methoxyphenyl)cyclohexyl]methyl}sulfamide

In the same manner as in Example 18-1, the title compound was synthesized using 4-(2-hydroxyethoxy)benzaldehyde as a reaction reagent.
High performance liquid chromatography/Mass spectrometry
m/z 464.2 (M+H)
Retention time: 2.36 min.

### Example 18-5

### Synthesis of N-[4-({[cis-4-{[(aminosulfonyl)amino]methyl}-4-(3-methoxyphenyl)cyclohexyl]amino}methyl)phenyl]acetamide

In the same manner as in Example 18-1, the title compound was synthesized using 4-acetamidobenzaldehyde as a reaction reagent.
High performance liquid chromatography/ Mass spectrometry
m/z 450.2 (M+H)
Retention time: 2.51 min.

### Example 18-6

### Synthesis of N-({cis-1-(3-methoxyphenyl)-4-[(quinolin-4-ylmethyl)-amino] cyclohexyl}methyl) sulfamide

In the same manner as in Example 18-1, the title compound was synthesized using 4-quinolinecarboxyaldehyde as a reaction reagent.
High performance liquid chromatography/ Mass spectrometry
m/z 455.2 (M+H)
Retention time: 2.17 min.

### Example 18-7

### Synthesis of N-{[cis-4-(benzylamino)-1-(3-methoxyphenyl)cyclohexyl]-methyl}sulfamide

In the same manner as in Example 18-1, the title compound was synthesized using benzaldehyde as a reaction reagent.
High performance liquid chromatography/ Mass spectrometry
m/z 404.5 (M+H)
Retention time: 2.78 min.

### Example 18-8

### Synthesis of N-({cis-1-(3-methoxyphenyl)-4-[(4-morpholin-4-ylbenzyl)-amino]cyclohexyl}methyl)sulfamide

In the same manner as in Example 18-1, the title compound was synthesized using 4-morpholin-4-ylbenzaldehyde as a reaction reagent.
High performance liquid chromatography/ Mass spectrometry
m/z 489.4 (M+H)
Retention time: 2.71 min.

### Example 18-9

### Synthesis of N-{[cis-4-[(4-butoxybenzyl)amino]-1-(3-methoxyphenyl) cyclohexyl]methyl}sulfamide

In the same manner as in Example 18-1, the title compound was synthesized using 4-butoxybenzaldehyde as a reaction reagent.
High performance liquid chromatography/ Mass spectrometry
m/z 476.2 (M+H)
Retention time: 3.19 min.

### Example 18-10

### Synthesis of N-{[cis-4-[(1,3-benzodioxol-4-ylmethyl)amino]-1-(3-methoxyphenyl)cyclohexyl]methyl}sulfamide

In the same manner as in Example 18-1, the title compound was synthesized using 2,3-(methylenedioxy)benzaldehyde as a reaction reagent.
High performance liquid chromatography/ Mass spectrometry
m/z 448.2 (M+H)
Retention time: 2.50 min.

### Example 18-11

### Synthesis of N-{[cis-4-{[(5-chloro-1,2,3-thiadiazol-4-yl)methyl]amino}-1-(3-methoxyphenyl) cyclohexyl] methyl} sulfamide

In the same manner as in Example 18-1, the title compound was synthesized using 5-chloro-1,2,3-thiadiazol-4-carbaldehyde as a reaction reagent.
High performance liquid chromatography/ Mass spectrometry
m/z 552.1 (M+H)
Retention time: 3.03 min.

### Example 18-12

### Synthesis of N-[(cis-1-(3-methoxyphenyl)-4-{[(1-methyl-1H-indol-3-yl)methyl]amino}cyclohexyl)methyl]sulfamide

In the same manner as in Example 18-1, the title compound was synthesized using 1-methylindol-3-carbaldehyde as a reaction reagent.
High performance liquid chromatography/ Mass spectrometry
m/z 408.2 (M+H)
Retention time: 0.94 min.

### Example 18-13

### Synthesis of N-{[cis-4-[(1-benzofuran-2-ylmethyl)amino]-1-(3-methoxyphenyl)cyclohexyl]methyl}sulfamide

In the same manner as in Example 18-1, the title compound was synthesized using 2-benzofurancarbaldehyde as a reaction reagent.
High performance liquid chromatography/ Mass spectrometry
m/z 444.3 (M+H)
Retention time: 2.65 min.

### Example 19-1

### Synthesis of N-({cis-1-(3-methoxyphenyl)-4-[(1-methylpiperidin-4-yl)-amino]cyclohexyl}methyl) sulfamide

To a solution of tert-butyl [({2-[cis-4-amino-1-(3-methoxyphenyl)-cyclohexyl]methyl}amino)sulfonyl]carbamate (60 mg, 0.145 mmol) and 1-methyl-4-piperidone (0.027 mL, 0.218 mmol) in 1,2-dichloroethane (2 mL) was added sodium triacetoxyborohydride (NaHB(OAc)₃, 108 mg, 0.508 mmol) at room temperature and then the mixture was stirred overnight. Thereto was added saturated aqueous sodium bicarbonate solution to quench the reaction and then the mixture was extracted with chloroform, and the organic layer was washed twice with saturated aqueous sodium chloride solution. The organic layer was dried over anhydrous magnesium sulfate and filtered, and then the solvent was evaporated under reduced pressure. The resulting residue was purified by preparative thin layer chromatography to give a product. The resulting product was dissolved in 4M hydrogen chloride/dioxane solution (0.5 mL) and the mixture was stirred overnight. The solvent was evaporated under reduced pressure to give the title compound 2.76 mg as white powder.
High performance liquid chromatography/ Mass spectrometry
m/z 411.5 (M+H)
Retention time: 1.56 min.

### Example 19-2

### Synthesis of N-{[cis-4-[(2-hydroxy-4-methoxybenzyl)aminol-1-(3-methoxyphenyl)cyclohexyl]methyl}sulfamide

In the same manner as in Example 19-2, the title compound was synthesized using 2-hydroxy-4-methoxybenzaldehyde as a reaction reagent.
High performance liquid chromatography/ Mass spectrometry
m/z 450.2 (M+H)
Retention time: 2.51 min.

### Example 20

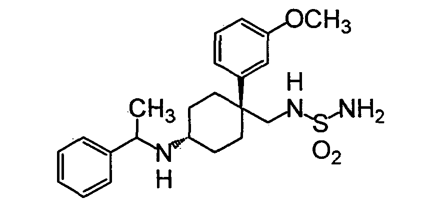

### Synthesis of N-({cis-1-(3-methoxyphenyl)-4-[(1-phenylethyl)amino]-cyclohexyl}methyl) sulfamide

A solution of tert-butyl [({2-[cis-4-amino-1-(3-methoxyphenyl)-cyclohe3iyl]methyl}amino)sulfonyl]carbamate (50.0 mg, 0.121 mmol), (1-chloroethyl)benzene (0.025 mL, 0.145 mmol) and potassium carbonate (50.2 mg, 0.363 mmol) in N,N-dimethylformamide (DMF, 5 mL) was stirred at 60°C for 24 hours. Thereto was added water to quench the reaction and the mixture was extracted with chloroform, and the organic layer was washed once with water. The organic layer was dried over anhydrous magnesium sulfate and filtered, and then the solvent was evaporated under reduced pressure. The resulting residue was purified by silica gel column chromatography to give a product. The resulting product was dissolved in trifluoroacetic acid (2.0 mL) and the mixture was stirred overnight. Thereto was added saturated aqueous sodium bicarbonate solution to quench the reaction, and then the mixture was salted out and extracted with ethyl acetate, and the organic layer was washed twice with saturated aqueous sodium chloride solution. The organic layer was dried over anhydrous magnesium sulfate and filtered, and then the solvent was evaporated under reduced pressure to give the title compound 4.20 mg as orange-white solids.
High performance liquid chromatography/Mass spectrometry
m/z 418.2 (M+H)
Retention time: 2.63 min.

### Example 21

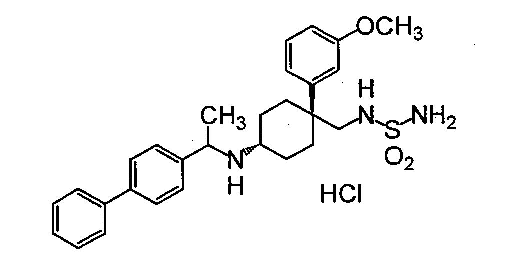

### Synthesis of N-{[cis-4-[(1-biphenyl-4-ylethyl)amino]-1-(3-methoxyphenyl)cyclohexyl]methyl}sulfamide hydrochloride

### Synthesis of cis-4-[(1-biphenyl-4-ylethyl)amino]-1-(3-methoxy-phenyl)-cyclohexanecarbonitrile

To a solution of cis-4-amino-1-(3-methoxyphenyl)cyclohexane-carbonitrile (250 mg, 1.09 mmol) and 4-acetoxybiphenyl (276.9 mg, 1.41 mmol) in 1,2-dichloroethane (10 mL) was added sodium triacetoxyborohydride (NaHB(OAc)₃, 345 mg, 1.63 mmol) at room temperature and then the mixture was stirred overnight. Thereto was added saturated aqueous sodium bicarbonate solution to quench the reaction and then the mixture was extracted with chloroform, and the organic layer was washed twice with saturated aqueous sodium chloride solution. The organic layer was dried over anhydrous magnesium sulfate and filtered, and then the solvent was evaporated under reduced pressure. The resulting residue was purified by silica gel column chromatography to give the title compound 108 mg as colorless oils.
High performance liquid chromatography/Mass spectrometry
m/z 411.2 (M+H)
Retention time: 3.07 min.

### Synthesis of tert-butyl [({[cis-4-[(1-biphenyl-4-ylethyl)amino]-1-(3-methoxyphenyl)cyclohexyl]methyl}amino)sulfonyl]carbamate

To a suspension of lithium aluminum hydride (18.5 mg, 0.487 mmol) in tetrahydrofuran was added dropwise a solution of cis-4-[(1-biphenyl-4-ylethyl) amino]-1-(3-methoxyphenyl)cyclohexanecarbonitrile (80.0 mg, 1.09 mmol) in tetrahydrofuran (10 mL) under heating at reflux, and the mixture was stirred at such temperature for 3 hours. Thereto was added aqueous ammonia under ice-cooling to quench the reaction and then the precipitated solid was collected by filtration on celite. The solvent was evaporated under reduced pressure and then the reactant was dissolved in dichloromethane (5.0 mL) and thereto was added (tert-butoxycarbonyl){[4-(dimethyliminio)pyridin-1 (4H)-yl]-sulfonyl}azanide (58.8 mg, 0.195 mmol) at room temperature. After stirring overnight, thereto was added water to quench the reaction and the mixture was extracted with ethyl acetate, and the organic layer was washed twice with water. The organic layer was dried over anhydrous magnesium sulfate and filtered, and then the solvent was evaporated under reduced pressure. The resulting residue was crystallized from diethylether to give the title compound 56.6 mg as white solids.
High performance liquid chromatography/ Mass spectrometry
m/z 594.4 (M+H)
Retention time: 3.32 min.

### Synthesis of N-{[cis-4-[(1-biphenyl-4-ylethyl)amino]-1-(3-methoxyphenyl)cyclohexyl]methyl}sulfamide

A solution of tert-butyl [({[cis-4-[(1-biphenyl-4-ylethyl)amino]-1-(3-methoxyphenyl)cyclohexyl]methyl}amino)sulfonyl]carbamate (40.0 mg, 0.0674 mmol) in 4M hydrogen chloride/1,4-dioxane (1 mL) was stirred at room temperature overnight. The solvent was evaporated under reduced pressure and then the resulting residue was crystallized from diethylether to give the title compound 34.0 mg as white solids.
High performance liquid chromatography/Mass spectrometry
m/z 494.4 (M+H)
Retention time: 3.01 min.

### Example 22

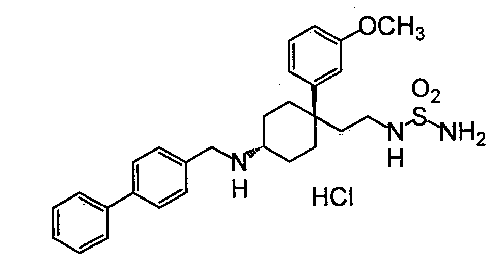

### Synthesis of N-{2-[trans-4-[(biphenyl-4-ylmethyl)amino]-1-(3-methoxyphenyl)cyclohexyl]ethyl}sulfamide hydrochloride

### Synthesis of tert-butyl [({2-[trans-4-[(diphenylmethyl)amino]-1-(3-methoxyphenyl)cyclohexyl]ethyl}amino)sulfonyl]carbamate

To a suspension of lithium aluminum hydride (46.2 mg, 1.22 mmol) in tetrahydrofuran (5.0 mL) was added dropwise [trans-4-[(diphenylmethyl)amino]-1-(3-methoxyphenyl)acetonitrile (200 mg, 0.487 mmol) in tetrahydrofuran (2.0 mL) under heating at reflux, and the mixture was stirred at such temperature for 3 hours. Thereto was added aqueous ammonia under ice-cooling to quench the reaction and then the precipitated solid was collected by filtration on celite. The solvent was evaporated under reduced pressure and then the reactant was dissolved in dichloromethane (5.0 mL), and thereto was added (tert-butoxycarbonyl){[4-(dimethyliminio)pyridine-1 (4H)-yl]sulfonyl}azanide (146 mg, 0.487 mmol) at room temperature. After stirring overnight, thereto was added water to quench the reaction and the mixture was extracted with ethyl acetate, and the organic layer was washed twice with water. The organic layer was dried over anhydrous magnesium sulfate and filtered, and then the solvent was evaporated under reduced pressure. The resulting residue was purified by silica gel column chromatography to give the title compound 269 mg as colorless oils.
High performance liquid chromatography/Mass spectrometry
m/z 594.4 (M-NHSO₂NH₂)
Retention time: 3.36 min.

### Synthesis of tert-butyl [({2-[trans-4-amino-1-(3-methoxyphenyl)-cyclohexyl]ethyl}amino)sulfonyl]carbamate

A suspension of tert-butyl [({2-[trans-4-[(diphenylmethyl)amino]-1-(3-methoxyphenyl)cyclohexyl]ethyl}amino)sulfonyl]carbamate (169 mg, 0.285 mmol) and palladium hydroxide (32 mg) in methanol (10 mL) was stirred under an atmosphere of hydrogen in 0.3 MPa at room temperature for 3 hours. The mixture was filtered on celite to remove the catalyst and then the solvent was evaporated under reduced pressure. The resulting residue was crystallized from ethyl acetate to give the title compound 75.3 mg as white solids.
High performance liquid chromatography/ Mass spectrometry
m/z 428.3 (M+H)
Retention time: 2.82 min.

### Synthesis of tert-butyl [({2-[trans-4-[(biphenyl-4-ylmethyl)amino]-1-(3-methoxyphenyl)cyclohexyl]ethyl}amino)sulfonyl]carbamate

To a solution of tert-butyl [({2-[trans-4-amino-1-(3-methoxyphenyl)-cyclohexyl]ethyl}amino)sulfonyl]carbamate (55.3 mg, 0.129 mmol) and 4-acetoxybiphenyl (35.4 mg, 0.194 mmol) in 1,2-dichloroethane (2 mL) was added sodium triacetoxyborohydride (NaHB(OAc)₃, 95.6 mg, 0.452 mmol) at room temperature and then the mixture was stirred overnight. Thereto was added saturated aqueous sodium bicarbonate solution to quench the reaction and then the mixture was extracted with chloroform, and the organic layer was washed twice with saturated aqueous sodium chloride solution. The organic layer was dried over anhydrous magnesium sulfate and filtered, and then the solvent was evaporated under reduced pressure. The resulting residue was purified by silica gel column chromatography to give the title compound 15.9 mg as amorphous.
High performance liquid chromatography/Mass spectrometry
m/z 594.4 (M+H)
Retention time: 3.42 min.

### Synthesis of N-{2-[trans-4-[(biphenyl-4-ylmethyl)amino]-1-(3-methoxyphenyl)cyclohexyl]ethyl}sulfamide hydrochloride

A solution of tert-butyl [({2-[trans-4-[(biphenyl-4-ylmethyl)amino]-1-(3-methoxyphenyl)cyclohexyl]ethyl}amino)sulfonyl]carbamate (13.9 mg, 0.0234 mmol) in 4M hydrogen chloride/1,4-dioxane (1 mL) was stirred at room temperature overnight. The solvent was evaporated under reduced pressure, and then the resulting residue was crystallized from ethyl acetate to give the title compound 6.49 mg as white solids.
High performance liquid chromatography/ Mass spectrometry
m/z 494.4 (M+H)
Retention time: 3.22 min.

### Example 23

### Synthesis of N-[cis-4-[(biphenyl-4-ylmethyl)amino]-1-(3-methoxyphenyl)cyclohexyl]sulfamide

### Synthesis of tert-butyl ({[cis-4-[(tert-butoxycarbonyl)amino]-1-(3-methoxyphenyl)cyclohexyl]amino}sulfonyl)carbamate

To a solution of tert-butanol (0.0549 mL, 0.601 mmol) in dichloromethane (5.0 mL) was added chlorosulfonyl isocyanate (0.0475 mL, 0.546mmol) under ice-cooling and then the mixture was stirred at room temperature overnight. The solvent was evaporated under reduced pressure and the reactant was dissolved in dichloromethane (5.0 mL) and then thereto were added tert-butyl [cis-4-amino-4-(3-methoxyphenyl)cyclohexyl]carbamate (175 mg, 0.546 mmol) and triethylamine (0.183 mL, 1.31 mmol) under ice-cooling and the mixture was warmed to room temperature and the mixture was stirred overnight. Thereto was added saturated aqueous ammonium chloride solution to quench the reaction and then the mixture was extracted with ethyl acetate, and the organic layer was washed twice with saturated aqueous ammonium chloride solution. The organic layer was dried over anhydrous magnesium sulfate and filtered, and then the solvent was evaporated under reduced pressure. The resulting residue was purified by preparative thin layer chromatography to give the title compound 167 mg as white solids.
¹H-NMR (400MHz, DMSO-d₆) δ 1.33 (9H, s), 1.37 (9H, s), 1.59-1.66 (6H, m), 2.38-2.40 (2H, m), 3.17 (1H, brd.), 3.72 (3H, s), 6.70-6.76 (2H, m), 6.98-7.05 (2H, m), 7.14-7.20 (1H, m), 7.84 (1H, s), 10.4 (1H, s).

### Synthesis of N-[cis-4-amino-1-(3-methoxyphenyl)cyclohexyl]sulfamide hydrochloride

A solution of tert-butyl ({[cis-4-[(tert-butoxycarbonyl)aminol-1-(3-methoxyphenyl)cyclohexyl]amino}sulfonyl)carbamate (165 mg, 0.330 mmol) in 4M hydrogen chloride/ 1,4-dioxane (3 mL) was stirred at room temperature overnight. The solvent was evaporated under reduced pressure, and then the resulting residue was crystallized from ethyl acetate to give the title compound 67.4 mg as white solids.
High performance liquid chromatography/ Mass spectrometry
m/z 204.2 (M-NHSO₂NH₂)
Retention time: 2.24 min.

### Synthesis of N-[cis-4-[(biphenyl-4-ylmethyl)amino]-1-(3-methoxyphenyl)cyclohexyl]sulfamide

To a solution of N-[cis-4-amino-1-(3-methoxyphenyl)cyclohexyl]-sulfamide hydrochloride (50.0 mg, 0.149 mmol) and 4-phenylbenzaldehyde (40.6 mg, 0.223 mmol) in 1,2-dichloroethane (10 mL) was added sodium triacetoxyborohydride (NaHB(OAC)₃, 110 mg, 0.522 mmol) at room temperature and then the mixture was stirred overnight. Thereto was added saturated aqueous sodium bicarbonate solution to quench the reaction and then the mixture was extracted with chloroform, and the organic layer was washed twice with saturated aqueous sodium chloride solution. The organic layer was dried over anhydrous magnesium sulfate and filtered, and then the solvent was evaporated under reduced pressure. The resulting residue was purified by preparative thin layer chromatography to give the title compound 12.7 mg as white solids.
¹H-NMR (400MHz, DMSO-d₆) δ 1.66 (3H, brdm), 2.13 (2H, brdm), 2.53 (3H, brdm), 2.94 (1H, brdm), 3.82 (3H, s), 3.93 (2H, s), 6.06 (1H, brd), 6.76 (1H, m), 6.92-6.99 (2H, m), 7.20-7.22 (1H, m), 7.31-7.36 (1H, m), 7.41 (5H, m), 7.56-7.61 (5H, m).

### Example 24

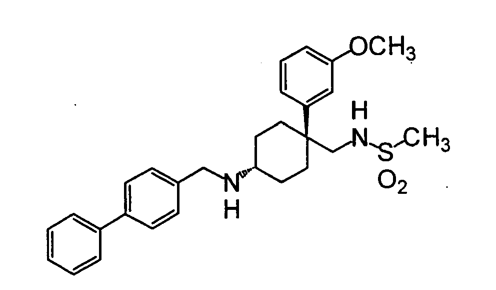

### Synthesis of N-{[cis-4-[(biphenyl-4-ylmethyl)amino]-1-(3-methoxyphenyl)cyclohexyl]methyl}methanesulfonamide

To a solution of cis-4-(aminomethyl)-N-(biphenyl-4-ylmethyl)-4-(3-methoxyphenyl)cyclohexanamine (50 mg, 0.125 mmol) and triethylamine (0.088 mL, 0.625 mmol) in dichloromethane (10 mL) was added methanesulfonyl chloride (0.012 mL, 0.138 mmol) under ice-cooling and then the mixture was warmed to room temperature and stirred overnight. Thereto was added water to quench the reaction and the mixture was extracted with ethyl acetate, and the organic layer was washed once with water. The organic layer was dried over anhydrous magnesium sulfate and filtered, and then the solvent was evaporated under reduced pressure. The resulting residue was purified by silica gel column chromatography to give the title compound 56.6 mg as colorless solids.
High performance liquid chromatography/ Mass spectrometry
m/z 479.3 (M+H)
Retention time: 2.88 min.

### Example 25

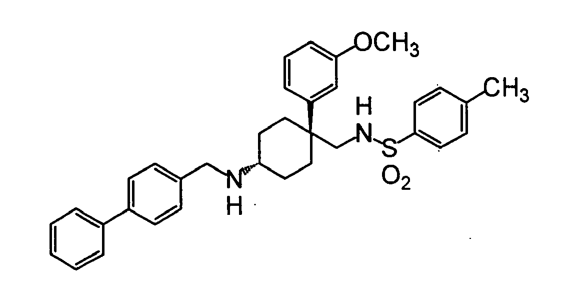

### Synthesis of N-{[cis-4-[(biphenyl-4-ylmethyl)amino]-1-(3-methoxyphenyl)cyclohexyl]methyl}-4-methylbenzenesulfonamide

To a solution of cis-4-(aminomethyl)-N-(biphenyl-4-ylmethyl)-4-(3-methoxyphenyl)cyclohexanamine (50 mg, 0.125 mmol) and triethylamine (0.088 mL, 0.625 mmol) in dichloromethane (1.0 mL) was added p-toluenesulfonyl chloride (26.3 mg, 0.138 mmol) under ice-cooling and then the mixture was warmed to room temperature and stirred overnight. Thereto was added water to quench the reaction and the mixture was extracted with ethyl acetate, and the organic layer was washed once with water. The organic layer was dried over anhydrous magnesium sulfate and filtered, and then the solvent was evaporated under reduced pressure. The resulting residue was purified by silica gel column chromatography to give the title compound 69.9 mg as colorless solids.
High performance liquid chromatography/Mass spectrometry
m/z 555.2 (M+H)
Retention time: 3.19 min.

### Example 26

### Synthesis of N-{[cis-4-[(biphenyl-4-ylmethyl)amino]-1-(3-methoxyphenyl)cyclohexyl]methyl}benzenesulfonamide

To a solution of cis-4-(aminomethyl)-N-(biphenyl-4-ylmethyl)-4-(3-methoxyphenyl)cyclohexanamine (50 mg, 0.125 mmol) and triethylamine (0.023 mL, 0.163 mmol) in dichloromethane (2.0 mL) was added benzenesulfonyl chloride (0.019 mL, 0.150 mmol) under ice-cooling and then the mixture was warmed to room temperature and stirred overnight. Thereto was added water to quench the reaction and the mixture was extracted with ethyl acetate, and the organic layer was washed twice with water. The organic layer was dried over anhydrous magnesium sulfate and filtered, and then the solvent was evaporated under reduced pressure. The resulting residue was purified by silica gel column chromatography to give the title compound 44.2 mg as white solids.
High performance liquid chromatography/ Mass spectrometry
m/z 541.5 (M+H)
Retention time: 3.38 min.

### Example 27-1

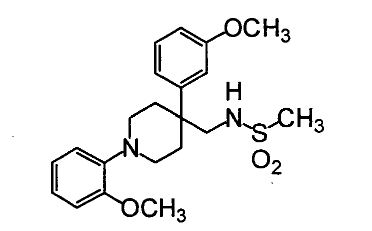

### Synthesis of N-{[1-(2-methoxyphenyl)-4-(3-methoxyphenyl)piperidin-4-yl]methyl}methanesulfonamide

To a solution of 1-(2-methoxyphenyl)-4-(3-methoxyphenyl)-piperidine-4-carbonitrile (1.00g, 3.10 mmol) in diethylether (50 mL) was added lithium aluminum hydride (353 mg, 9.30 mol) at 0°C and the mixture was stirred at room temperature for 5 hours. To the reaction solution was added 30% aqueous ammonia and then the mixture was filtered on celite and the filtrate was concentrated under reduced pressure. To a solution of the resulting residue (100 mg, 0.306 mmol) in dichloromethane (2 mL) were added triethylamine (0.042 mL, 0.306 mmol) and methanesulfonyl chloride (0.023 mL, 0.306 mmol) at 0°C and the mixture was stirred at room temperature for 2 hours and a half. The reaction solution was poured into saturated aqueous sodium bicarbonate solution, and the mixture was extracted with ethyl acetate. The organic layer was washed with saturated aqueous sodium chloride solution and then dried over anhydrous sodium sulfate and filtered. The filtrate was concentrated under reduced pressure and the resulting residue was purified by silica gel column chromatography to give the title compound 108 mg as white solids.
High performance liquid chromatography/Mass spectrometry
m/z 405 (M+H)
Retention time: 2.25 min.

### Example 27-2

### Synthesis of N-{[1-(2-methoxyphenyl)-4-(3-methoxyphenyl)piperidin-4-yl] methyl}-4-methylbenzene sulfonamide

In the same manner as in Example 27-1, the title compound was synthesized.
High performance liquid chromatography/ Mass spectrometry
m/z 481 (M+H)
Retention time: 2.78 min.

### Example 28-1

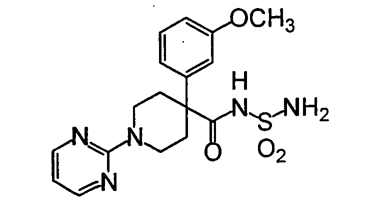

### Synthesis of N-(aminosulfonyl)-4-(3-methoxyphenyl)-1-pyrimidin-2-ylpiperidine-4-carboxamide

To a solution of sulfamide (39.1 mg, 0.407 mmol) in tetrahydrofuran (2.00 mL) was added sodium hydride (26.8 mg, 0.614 mmol) at 0°C and the mixture was stirred at room temperature for 15 min. Then thereto was added 4-(3-methoxyphenyl)-1-pyrimidin-2-ylpiperidine-4-carbonyl chloride hydrochloride (50.0 mg, 0.136 mmol) at room temperature and the mixture was stirred at room temperature for 6 hours. The reaction solution was poured into saturated aqueous ammonium chloride solution and then the mixture was extracted with ethyl acetate. The organic layer was dried over anhydrous magnesium sulfate and filtered, and then the solvent was evaporated under reduced pressure, and the resulting residue was purified by thin layer chromatography to give the title compound 39.3 mg as white solids.
¹H-NMR δ (DMSO-d₆) 1.77 (1H, t, J= 11.3 Hz), 2.57 (2H, d, J= 13.6 Hz), 3.15 (2H, t, J= 11.3 Hz), 3.74 (3H, s), 4.45 (2H, d, J= 4.8 Hz), 6.84 (1H, d, J= 8.6 Hz), 6.88-6.95 (2H, m), 7.27 (1H, t, J= 7.7 Hz), 7.40 (2H, s), 8.34 (2H, d, J= 4.8 Hz), 11.11 (1H, s).

### Example 28-2

### Synthesis of 4-(3-methoxyphenyl)-N-{[methyl(phenyl)amino]sulfonyl}-1-pyrimidin-2-ylpiperidine-4-carboxamide

In the same manner as in Example 28-1, the title compound was synthesized using N-methyl-N-phenylsulfamide.
¹H-NMR δ (DMSO-d₆); 1.76 (2H, t, J= 10.6 Hz), 2.50-2.58 (2H, m), 2.93 (2H, J= 13.5 Hz), 3.24 (3H, s), 3.73 (3H, s), 4.28 (2H, d, J= 13.5 Hz), 6.61 (1H, t, J= 4.2 Hz), 6.86-6.93 (3H, m), 7.10-7.24 (1H, t, J= 7.7 Hz), 8.84 (2H, d, J= 4.7 Hz), 11.30 (1H, s).

### Example 28-3

### Synthesis of N-{[(2,6-diisopropylphenyl)amino]sulfonyl}-4-(3-methoxyphenyl)-1-pyrimidin-2-ylpiperidine-4-carboxamide

In the same manner as in Example 28-1, the title compound was synthesized using N-(2,6-diisopropylphenyl)sulfamide.
High performance liquid chromatography/ Mass spectrometry
m/z 552.1 (M+H)
Retention time: 3.74 min.

### Example 28-4

### Synthesis of N-[(benzylamino)sulfonyl]-4-(3-methoxyphenyl)-1-pyrimidin-2-ylpiperidine-4-carboxamide

In the same manner as in Example 28-1, the title compound was synthesized using N-benzylsulfamide.
High performance liquid chromatography/ Mass spectrometry
m/z 482.4 (M+H)
Retention time: 3.17 min.

### Example 28-5

### Synthesis of N-{[benzyl(methyl)amino]sulfonyl}-4-(3-methoxyphenyl)-1-pyrimidin-2-ylpiperidine-4-carboxamide

In the same manner as in Example 28-1, the title compound was synthesized using N-benzyl-N-methylsulfamide.
¹H-NMR δ (DMSO-d₆); 1.80-1.92 (2H, m), 2.54-2.64 (2H, m), 2.58 (3H, s), 3.16-3.27 (2H, m), 3.76 (3H, s), 4.29 (2H, s), 4.41 (2H, d, J= 13.9 Hz), 6.61 (1H, t, J= 4.8 Hz), 6.88 (1H, d, J= 8.0 Hz), 6.92-7.02 (2H, m), 7.23-7.38 (6H, m), 8.35 (2H, d, J= 4.6 Hz), 11.30 (1H, s).

### Example 28-6

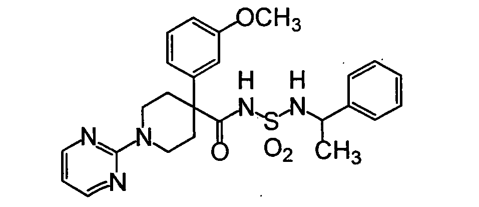

### Synthesis of 4-(3-methoxyphenyl)-N-{[(1-phenylethyl)amino]sulfonyl}-1-pyrimidin-2-ylpiperidine-4-carboxamide

In the same manner as in Example 28-1, the title compound was synthesized using N-(1-phenylethyl)sulfamide.
¹H-NMR δ (DMSO-d₆); 1.20 (3H, d, J=6.8 Hz), 1.70-1.95 (2H, m); 2.37 (2H, t, J=17.4 Hz), 2.91 (1H, t, J=10.8 Hz), 3.16 (1H, t, J=10.2 Hz), 3.74 (3H, s), 4.25-4.40 (1H, m), 6.61 (1H, t, J=4.6 Hz), 6.82-7.00 (4H, m), 7.16 (2H, t, J=7.3 Hz), 7.22-7.33 (3H, m), 8.35 (2H, d, J=9.8 Hz), 8.41 (1H, d, J= 7.9 Hz), 10.96 (1H, s).

### Example 29

### Synthesis of N-(aminosulfonyl)-4-(3-methoxyphenyl)-1-pyrimidin-2-ylpiperidine-4-carboxamide sodium salt

To a solution of N-(aminosulfonyl)-4-(3-methoxyphenyl)-1-pyrimidin-2-ylpiperidine-4-carboxamide (50.0 mg, 0.128 mmol) in methanol (5 mL) was added sodium tert-butoxide (12.1 mg, 0.126 mmol) at 0°C and the mixture was stirred at room temperature for 1.5 hours. The solvent of reaction solution was evaporated under reduced pressure to give the title compound 51 mg as white solids.
¹H-NMR δ (DMSO-d₆); 1.43-1.55 (2H, m), 2.44-2.52 (2H, m), 3.17 (2H, t, J=11.4 Hz), 3.69 (3H, s), 4.44 (2H, d, J=13.0 Hz), 5.46 (2H, t, J=4.6 Hz), 6.63-6.72 (1H, m), 6.90-6.98 (2H, m), 7.13 (1H, t, J=8.3 Hz), 8.30 (2H, d, J=4.7 Hz).

### Example 30

### Synthesis of 1-(2-methoxyphenyl)-4-(3-methoxyphenyl)-N-[(4-methylphenyl)sulfonyl]piperidine-4-carboxamide

To a solution of 1-(2-methoxyphenyl)-4-(3-methoxyphenyl)-piperidine-4-carboxylic acid (3g, 8.78 mmol) in dichloroethane (60 mL) were added dimethylformamide (2 drops) and oxalyl chloride (1.57 mL, 17.57 mmol) at 0°C, and the mixture was stirred at room temperature for 3 hours and then the reaction solution was concentrated to dryness.

To a solution of p-toluenesulfonamide (64 mg, 0.378 mmol) in tetrahydrofuran (1 mL) was added sodium hydride (16 mg, 0.378 mmol) at 0°C and the mixture was stirred at room temperature for 15 min.

Then thereto was added the above-obtained residue (50 mg, 0.126 mmol) at room temperature and the mixture was stirred for 2 hours and a half. The reaction solution was poured into saturated aqueous ammonium chloride solution and thereto was added ethyl acetate and the mixture was extracted with ethyl acetate. The organic layer was washed with saturated aqueous sodium chloride solution and then the mixture was dried over anhydrous magnesium sulfate and filtered. The filtrate was concentrated under reduced pressure and the resulting residue was purified by thin layer chromatography to give the title compound 48 mg as white solids.
High performance liquid chromatography/ Mass spectrometry
m/z 495 (M+H)
Retention time: 2.88 min.

### Example 31

### Synthesis of N-(anilinosulfonyl)-1-(2-methoxyphenyl)-4-(3-methoxyphenyl)piperidine-4-carboxamide

To a solution of 1-(2-methoxyphenyl)-4-(3-methoxyphenyl)-piperidine-4-carboxylic acid (3g, 8.78 mmol) in 1,2-dichloroethane (60 mL) were added dimethylformamide (2 drops) and oxalyl chloride (1.57 mL, 17.57 mmol) at 0°C, and the mixture was stirred at room temperature for 3 hours and then the reaction solution was concentrated to dryness.

To a solution of N-phenylsulfamide (65 mg, 0.378 mmol) in tetrahydrofuran (2 mL) was added sodium hydride (16 mg, 0.378 mmol), at 0°C and the mixture was stirred at room temperature for 15 min.

Then thereto was added the above-obtained residue (50 mg, 0.126 mmol) at room temperature and the mixture was stirred for 2 hours. The reaction solution was poured into ice-water, and thereto was added ethyl acetate and the mixture was extracted with ethyl acetate. The organic layer was washed with saturated aqueous sodium chloride solution, and then dried over anhydrous magnesium sulfate and filtered. The filtrate was concentrated under reduced pressure, and the resulting residue was purified by silica gel column chromatography to give the title compound 39 mg as white solids.
High performance liquid chromatography/ Mass spectrometry
m/z 496 (M+H)
Retention time: 2.73 min.

### Example 32

### Synthesis of tert-butyl ({[4-(3-methoxyphenyl)-1-pyrimidin-2-ylpiperidin-4-ylJamino}sulfonyl)carbamate

### a) Synthesis of 4-(3-methoxyphenyl)-1-pyrimidin-2-ylpiperidine-4-amine

To a solution of 4-(3-methoxyphenyl)-1-pyrimidin-2-ylpiperidine-4-carboxylic acid (200 mg, 0.638mmol) in toluene (4.0 mL) were added dropwise triethylamine (98 µL, 0.702 mmol) and diphenylphosphoryl azide (142 µL, 0.657 mmol) at room temperature, and the mixture was stirred at 80°C for 1.5 hours. Thereto were added triethylamine (44 µL, 0.319 mmol) and diphenylphosphoryl azide (28 µL, 0.128 mmol), and the mixture was heated with stirring for another one hour. To the reaction solution was added 20%-aqueous hydrochloric acid (4.0 mL) and the mixture was stirred at 100°C for 4 hours. The mixture was cooled to room temperature and toluene was evaporated under reduced pressure, and the resulting aqueous layer was taken to pH = 8 with aqueous sodium hydroxide solution. The precipitated solid was filtered under reduced pressure and then dried to give a crude material. This material was purified by silica gel column chromatography to give the title compound 153 mg as colorless oils.
¹H-NMR (DMSO-d₆) δ; 1.62 (2H, m), 1.84 (2H, m), 1.92 (2H, s), 3.44 (2H, m), 3.73 (3H, s), 4.37 (2H, m), 6.55 (1H, dd, J=4.7, 4.7Hz), 6.76 (1H, dd, J=8.0, 2.2Hz), 7.06 (1H, d, J=8.0Hz), 7.09 (1H, d, J=2.2Hz), 7.20 (1H, dd, J=8.0, 8.0Hz), 8.32 (2H, d, J=4.7Hz).

### b) Synthesis of tert-butyl ({[4-(3-methoxyphenyl)-1-pyrimidin-2-ylpiperidin-4-yl]amino}sulfonyl)carbamate

To a solution of 4-(3-methoxyphenyl)-1-pyrimidin-2-ylpiperidine-4-amine (30.0 mg, 0.106 mmol) in dichloromethane (1.0 mL) was added (tert-butoxycarbonyl){[4-(dimethyliminio)pyridin-1(4H)-yl]sulfonyl}-azanide (32.0 mg, 0.106 mmol) at room temperature and the mixture was stirred overnight. Then the mixture was stirred under reflux for 7 hours, and thereto was added (tert-butoxycarbonyl){[4-(dimethyliminio)-pyridin-1(4H)-yl]sulfonyl}azanide (16.0 mg, 0.0528 mmol) and the mixture was stirred for another 8 hours. Thereto was added saturated aqueous ammonium chloride solution to quench the reaction, and the reactant was extracted with chloroform. This organic layer was dried over anhydrous magnesium sulfate and filtered, and the solvent was evaporated under reduced pressure. The resulting residue was purified by silica gel column chromatography to give the title compound 35.0 mg as white solids.
¹H-NMR (DMSO-d₆) δ; 1.29 (9H, s), 1.80 (2H, m), 2.35 (2H, m), 3.29 (2H, m), 3.73 (3H, s), 4.32 (2H, m), 6.59 (1H, dd, J=4.7, 4.7Hz), 6.79(1H, m), 7.02 (2H, m), 7.20 (1H, dd, J=8.1, 8.1Hz), 8.16 (1H, s), 8.33 (2H, d, J=4.7Hz), 10.67 (1H, s).

### Example 33

### Synthesis of N-({[4-(3-methoxyphenyl)-1-pyrimidin-2-ylpiperidin-4-yl]amino}sulfonyl)-2,2-dimethylpropanamide

### a) Synthesis of N-[4-(3-methoxyphenyl)-1-pyrimidin-2-ylpiperidin-4-yl]sulfamide

To a solution of tert-butyl ({[4-(3-methoxyphenyl)-1-pyrimidin-2-ylpiperidin-4-yl]amino}sulfonyl)carbamate (240 mg, 0.518 mmol) obtained in Example 32 in methanol (2.4 mL) was added 4M hydrogen chloride/dioxane (2.4 mL) at 0°C and the mixture was stirred at room temperature for 4 hours. The solvent was evaporated under reduced pressure and then the residue was diluted with chloroform, and the mixture was washed with saturated aqueous sodium hydrogen carbonate solution and saturated aqueous sodium chloride solution successively. This organic layer was dried over anhydrous magnesium sulfate and filtered, and the solvent was evaporated under reduced pressure. This residue was purified by silica gel column chromatography to give the title compound 197 mg as white solids.
¹H-NMR (DMSO-d₆) δ; 1.75 (2H, m), 2.29 (2H, m), 3.45 (2H, m), 3.73 (3H, s), 4.34 (2H, m), 6.35 (2H, s), 6.57 (1H, dd, J=4.8, 4.8Hz), 6.77 (1H, m), 7.05 (2H, m), 7.18 (1H, dd, J=7.9, 7.9Hz), 7.23 (1H, s), 8.32 (2H, d, J=4.8Hz).

### b) Synthesis of N-({[4-(3-methoxyphenyl)-1-pyrimidin-2-ylpiperidin-4-yl]amino}sulfonyl)-2,2-dimethylpropanamide

To a suspension of 55%-sodium hydride (4.8 mg, 0.110 mmol) in N,N-dimethylformamide (0.5 mL) was added N-[4-(3-methoxyphenyl)-1-pyrimidin-2-ylpiperidin-4-yl]sulfamide (20.0 mg, 0.0550 mmol) at 0°C and the mixture was stirred for 15 min. Thereto was added pivaloyl chloride (7.5 µL, 0.0605 mmol) and the mixture was stirred at room temperature overnight. Thereto was added water to quench the reaction and the mixture was taken to pH = 6 with 1M-aqueous hydrochloric acid and then the reactant was extracted with ethyl acetate. This organic layer was washed with saturated aqueous sodium chloride solution, and then dried over anhydrous magnesium sulfate and filtered, and the solvent was evaporated under reduced pressure. This residue was purified by preparative thin layer chromatography to give the title compound 9.2 mg as white solids.
¹H-NMR (DMSO-d₆) δ; 0.88 (9H, s), 1.75 (2H, m), 2.40 (2H, m), 3.30 (2H, m), 3.73 (3H, s), 4.33 (2H, m), 6.58 (1H, dd, J=4.7, 4.7Hz), 6.78 (1H, m), 6.98 (1H, d, J=7.9Hz), 7.02 (1H, m), 7.18 (1H, d, J=7.9, 7.9Hz), 8.20 (1H, s), 8.32 (2H, d, J=4.7Hz), 10.63 (1H, s).
High performance liquid chromatography/ Mass spectrometry
m/z 448.2 (M+H)
Retention time: 2.84 min.

### Example 34

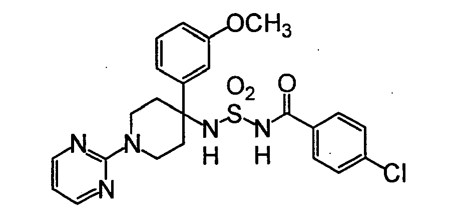

### Synthesis of 4-chloro-N-({[4-(3-methoxyphenyl)-1-pyrimidin-2-yl-piperidin-4-yl]amino}sulfonyl)benzamide

To a suspension of 55%-sodium hydride (4.8 mg, 0.110 mmol) in N,N-dimethylformamide (0.5 mL) was added N-[4-(3-methoxyphenyl)-1-pyrimidin-2-ylpiperidin-4-yl]sulfamide obtained in Example 33a) (20.0 mg, 0.0550 mmol) at 0°C and the mixture was stirred for 5 min. Thereto was added p-chlorobenzoyl chloride (10.5 µL, 0.0825 mmol) and the mixture was stirred at room temperature for 3.5 hours. Thereto was added water to quench the reaction and the mixture was taken to pH = 4 with 1M-aqueous hydrochloric acid. The precipitated crystals was filtered under reduced pressure and washed with water. The resulting residue was washed with methanol/diethylether to give the title compound 20.0 mg as white solids.
¹H-NMR (DMSO-d₆) δ; 1.76 (2H, m), 2.55 (2H, m), 3.38 (2H, m), 3.55 (3H, s), 4.31 (2H, m), 6.47 (1H, m), 6.58 (1H, dd, J=4.6, 4.6Hz), 6.94 (2H, m), 7.02 (1H, dd, J=8.1, 8.1Hz), 7.46 (2H, d, J=8.6Hz), 7.56 (2H, d, J=8.6Hz), 8.31 (2H, d, J=4.6Hz), 8.40 (1H, s), 11.39 (1H, s).

### Example 35

### Synthesis of N-(aminosulfonyl)-4-(3-methoxyphenyl)-1-[4-(trifluoromethyl)pyrimidin-2-yl]piperidine-4-carboxamide

### a) Synthesis of N-[(tert-butylamino)sulfonyl]-1-(diphenylmethyl)-4-(3-methoxyphenyl)piperidine-4-carboxamide

To a suspension of 1-(diphenylmethyl)-4-(3-methoxyphenyl)-piperidine-4-carboxylic acid (250 mg, 0.623 mmol) in dichloromethane (5.0 mL) was added dropwise thionyl chloride (91 µL, 1.25 mmol) at 0°C and the mixture was stirred under reflux for 3 hours. The reaction solution was concentrated under reduced pressure and thereto was added toluene and the solvent was evaporated azeotropically with toluene several times to give the residue. Separately, to a suspension of 55%-sodium hydride (82 mg, 1.87 mmol) in tetrahydrofuran (6.0 mL) was added N-(tert-butyl)sulfamide (190 mg, 1.25 mmol) at 0°C and the mixture was stirred for 10 min to give the suspension. To a solution of the above-obtained residue in tetrahydrofuran (5.0 mL) was added dropwise the above-obtained suspension and the mixture was stirred overnight. Thereto was added saturated aqueous ammonium chloride solution to quench the reaction and the reactant was extracted with ethyl acetate. The organic layer was dried over anhydrous magnesium sulfate and filtered, and the solvent was evaporated under reduced pressure. This residue was purified by silica gel column chromatography to give the title compound 263 mg as white solids.
¹H-NMR (DMSO-d₆) δ; 0.94 (9H, s), 1.95 (2H, m), 2.10 (2H, m), 3.73 (3H, s), 4.19 (1H, s), 6.83 (1H, d, J=8.3Hz), 6.91 (2H, m), 7.16 (2H, m), 7.26 (6H, m), 7.38 (4H, m), 10.68 (1H, s).

### b) Synthesis of N-[(tert-butylamino)sulfonyl]-4-(3-methoxyphenyl)-1-[4-(trifluoromethyl)pyrimidin-2-yl]piperidine-4-carboxamide

To a solution of N-[(tert-butylamino)sulfonyl]-1-(diphenylmethyl)-4-(3-methoxyphenyl)piperidine-4-carboxamide (40 mg, 0.0747 mmol) in ethanol (1.2 mL) were added ammonium formate (120 mg) and 10%-palladium-carbon 50% wet (8.0 mg) and the mixture was stirred under reflux for 2 hours. The reaction solution was filtered on celite under reduced pressure, and the solvent was evaporated under reduced pressure and the residue was dried.

The resulting residue was diluted in N,N-dimethylformamide (0.5 mL), and thereto were added 2-chloro-4-trifluoromethyl pyrimidine (10.8 µL, 0.0896 mmol) and potassium carbonate (31 mg, 0.224 mmol), and the mixture was stirred at 60°C for 2 hours. Thereto was added water to quench the reaction and the reactant was extracted with ethyl acetate. The organic layer was washed with saturated aqueous sodium chloride solution and then dried over anhydrous magnesium sulfate and filtered, and the solvent was evaporated under reduced pressure. This residue was purified by preparative thin layer chromatography to give the title compound 31.5 mg as white solids.
¹H-NMR (CDCl₃) δ; 1.16 (9H, s), 2.14 (2H, m), 2.43 (2H, m), 3.82 (3H, s), 3.88 (2H, m), 4.08 (2H, m), 5.17 (1H, s), 6.75 (1H, d, J=4.7Hz), 6.88 (1H, dd, J=8.0, 2.2Hz), 6.91 (1H, m), 6.97 (1H, m), 7.35 (1H, dd, J=8.0, 8.0Hz), 7.74 (1H, brs.), 8.47 (1H, d, J=4.7Hz).

### c) Synthesis of N-(aminosulfonyl)-4-(3-methoxyphenyl)-1-[4-(trifluoromethyl)pyrimidin-2-yl]piperidine-4-carboxamide

To N-[(tert-butylamino)sulfonyl]-4-(3-methoxyphenyl)-1-[4-(trifluoromethyl)pyrimidin-2-yl]piperidine-4-carboxamide (28.0 mg, 0.0543 mmol) was added trifluoroacetic acid (0.5 mL) at room temperature and the mixture was stirred at room temperature overnight. The solvent was evaporated under reduced pressure, and the resulting residue was diluted with water, and then the mixture was taken to pH = 4 to 5 with sodium bicarbonate aqueous solution, and the reactant was extracted with chloroform. This organic layer was dried over anhydrous magnesium sulfate and filtered, and the solvent was evaporated under reduced pressure. This residue was repulped with diethylether/hexane to give the title compound 15.6 mg as white solids.
¹H-NMR (DMSO-d₆) δ; 1.84 (2H, m), 2.58 (2H, m), 3.23 (2H, m), 3.74 (3H, s), 4.43 (2H, m), 6.87 (1H, m), 6.91 (2H, m), 7.00 (1H, d, J=4.9Hz), 7.28 (1H, dd, J=8.1, 8.1Hz), 7.42 (2H, s), 8.67 (1H, d, J=4.9Hz), 11.15 (1H, s).

### Example 36

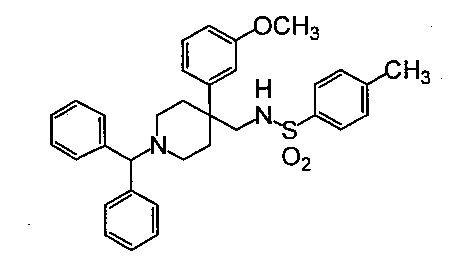

### Synthesis of N-{[1-(diphenylmethyl)-4-(3-methoxyphenyl)piperidin-4-yl]methyl}-4-methylbenzenesulfonamide

To a solution of 1-(diphenylmethyl)-4-(3-methoxyphenyl)piperidine-4-carbonitrile (200 mg, 0.523 mmol) in tetrahydrofuran (10 mL) was added lithium aluminum hydride (79 mg, 2.09 mmol) at room temperature and the mixture was stirred under reflux for 2 hours. The mixture was cooled to room temperature and thereto were added water, 2N-aqueous sodium hydroxide solution and water successively to quench the reaction and then the mixture was filtered on celite. The filtrate was evaporated under reduced pressure to give the mixture of amine derivative. The resulting amine derivative was dissolved in dichloromethane (6.0 mL), and thereto were added triethylamine (146 µL, 1.05 mmol) and p-toluenesulfonyl chloride (110 mg, 0.575 mmol) at room temperature, and the mixture was stirred overnight. Thereto were added saturated aqueous sodium hydrogen carbonate solution to quench the reaction and the reactant was extracted with chloroform. This organic layer was dried over anhydrous magnesium sulfate and filtered, and then the solvent was evaporated under reduced pressure. This residue was purified by silica gel column chromatography to give the title compound 262 mg as pale yellow amorphous.
¹H-NMR (CDCl₃) δ; 1.83 (2H, m), 2.05 (2H, m), 2.19 (2H, m), 2.40 (3H, s), 2.45 (2H, m), 3.01 (2H, d, J=6.6Hz), 3.77 (3H, s), 3.85 (1H, t, J=6.6Hz), 4.09 (1H, s), 6.71 (1H, m), 6.78 (2H, m), 7.14 (2H, m), 7.24 (7H, m), 7.35 (4H, m), 7.55 (2H, d, J=8.3Hz).

### Example 37

### Synthesis of N-(aminosulfonyl)-1-(diphenylmethyl)-4-(3-methoxyphenyl)-piperidine-4-carboxamide

### a) Synthesis of 1-(diphenylmethyl)-4-(3-methoxyphenyl)piperidine-4-carboxamide

A solution of 1-(diphenylmethyl)-4-(3-methoxyphenyl)piperidine-4-carbonitrile (4.0 g, 0.0105 mol) in dimethyl sulfoxide (20 mL) was warmed to 50°C, and then thereto was added dropwise 6N- aqueous potassium hydroxide solution (20 mL), and the mixture was stirred at 100°C for one hour. Thereto was added dimethyl sulfoxide (10 mL) and after 30 min., the mixture was warmed to 120°C. After 5.5 hours, the reaction solution was cooled to 0°C, and then thereto was added water (100 mL) and the mixture was taken to pH = 8 with conc. hydrochloric acid. This mixture was filtered under reduced pressure to give the crude material of the title compound 4.79 g as white solids. This material was repulped with diethylether to give the crude material of the title compound 4.59 g as white solids.
¹H-NMR (DMSO-d₆) δ; 1.77 (2H, m), 2.03 (2H, m), 2.35 (2H, m), 2.49 (2H, m), 3.68 (3H, s), 4.17 (1H, s), 6.75 (1H, m), 6.85 (3H, m), 7.00 (1H, brs.), 7.09-7.25 (7H, m), 7.34 (4H, m).

### b) Synthesis of 1-(diphenylmethyl)-4-(3-methoxyphenyl)piperidine-4-carboxylic acid

To 1-(diphenylmethyl)-4-(3-methoxyphenyl)piperidine-4-carboxamide (4.0 g, 0.999 mmol) was added conc. hydrochloric acid (40 mL) at room temperature and the mixture was stirred at 90°C for 18 hours. The reaction solution was concentrated under reduced pressure. The resulting residue was diluted with water, and then the mixture was taken to pH = 6 with aqueous sodium hydroxide solution, and the mixture was extracted with chloroform. The organic layer was washed with saturated aqueous sodium chloride solution and then dried over anhydrous magnesium sulfate and filtered, and the solvent was evaporated under reduced pressure. The resulting residue was repulped with chloroform to give the first crystals of the title compound 2.11 g as white solids. Also the filtrate was concentrated under reduced pressure, and the resulting residue was purified by silica gel column chromatography to give the second crystals of the title compound 361 mg as white solids.
¹H-NMR (DMSO-d₆) δ; 1.85 (2H, m), 2.04 (2H, m), 2.34 (2H, m), 2.63 (2H, m), 3.72 (3H, s), 4.26 (1H, s), 6.83 (1H, dd, 8.1, 2.0Hz), 6.86 (1H, d, J=2.0Hz), 6.92 (1H, d, J=8.1Hz), 7.16 (2H, m), 7.27 (5H, m), 7.39 (4H, m), 12.43(1H, brs.).

### c) Synthesis of N-(aminosulfonyl)-1-(diphenylmethyl)-4-(3-methoxyphenyl)piperidine-4-carboxamide

To a solution of 1-(diphenylmethyl)-4-(3-methoxyphenyl)piperidine-4-carboxylic acid (1.0 g, 2.49 mmol) in dichloromethane (20 mL) was added dropwise thionyl chloride (363 µL, 4.98 mol) at room temperature, and the mixture was stirred under reflux for 3 hours. The reaction solution was concentrated under reduced pressure and thereto was added toluene and the solvent was evaporated azeotropically with toluene several times to give a residue.

Separately, to a suspension of 55%-sodium hydride (326 mg, 7.47 mmol) in tetrahydrofuran (5.0 mL) was added sulfamide (718 mg, 7.47 mmol) at 0°C and the mixture was stirred for 30 min to give the suspension. To a solution of the above-obtained residue in tetrahydrofuran (15 mL) was added dropwise the above-obtained suspension and the mixture was stirred overnight. Thereto was added saturated aqueous ammonium chloride solution to quench the reaction and the reactant was extracted with ethyl acetate. The organic layer was dried over anhydrous magnesium sulfate and filtered, and the solvent was evaporated under reduced pressure. Thereto residue was added methanol to remove the insoluble matter, and the solvent was evaporated under reduced pressure. This residue was purified by silica gel column chromatography to give the title compound 357 mg as white solids.
High performance liquid chromatography/ Mass spectrometry
m/z 480.1 (M+H)
Retention time: 2.63 min.

### Example 38

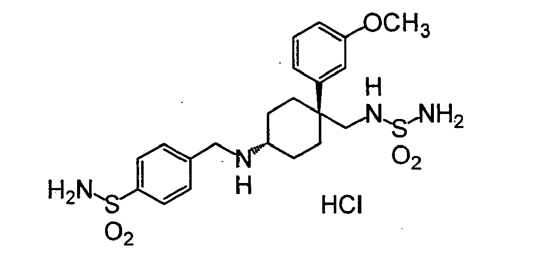

### Synthesis of 4-({[cis-4-{[(aminosulfonyl)amino]methyl}-4-(3-methoxyphenyl)cyclohexyl]amino}methyl)benzenesulfonamide hydrochloride

### a) Synthesis of tert-butyl {[cis-4-[(diphenylmethyl)amino]-1-(3-methoxyphenyl)cyclohexyl]methyl}carbamate

To a solution of cis-4-(aminomethyl)-N-(diphenylmethyl)-4-(3-methoxyphenyl)cyclohexanamine (1.5 g, 3.74 mmol) in tetrahydrofuran (30 mL) was added dropwise a solution of triethylamine (626 µL, 4.49 mmol) and t-butyl dicarbonate (834 mg; 3.82 mmol) in tetrahydrofuran (10 mL) at 0°C, and the mixture was stirred for one hour. Thereto was added water to quench the reaction and the reactant was extracted with ethyl acetate. The ethyl acetate layer was washed with saturated aqueous sodium chloride solution, and then the organic layer was dried over anhydrous magnesium sulfate and filtered, and the solvent was evaporated under reduced pressure. The resulting residue was purified by silica gel column chromatography to give the title compound 1.69 g as white solids.
¹H-NMR (DMSO-d₆) δ; 1.15-1.26 (9H, m), 1.52 (6H, m), 1.95 (2H, m), 2.31 (2H, m), 3.13 (2H, d, J=5.7Hz), 3.69 (3H, s), 4.94 (1H, d, J=4.8Hz), 6.01-6.16 (1H, m), 6.72 (1H, m), 6.79 (1H, m), 6.87 (1H, d, J=7.9Hz), 7.15 (3H, m), 7.26 (4H, m), 7.39 (4H, m).

### b) Synthesis of tert-butyl {[cis-4-amino-1-(3-methoxyphenyl)-cyclohexyl] methyl}carbamate

To a solution of tert-butyl {[cis-4-[(diphenylmethyl)amino]-1-(3-methoxyphenyl)cyclohexyl]methyl}carbamate (1.65 g, 3.30 mmol) in tetrahydrofuran (15 mL) and methanol (15 mL) was added 10%-palladium hydroxide-carbon (80 mg), and the mixture was stirred under an atmosphere of hydrogen (0.3 MPa) for 3 hours. The mixture was stirred under an atmosphere of hydrogen (0.4 MPa) for another 2.5 hours. The mixture was filtered on celite and the solvent was evaporated under reduced pressure. The resulting residue was purified by silica gel column chromatography to give the title compound 1.05 g as white solids.
¹H-NMR (DMSO-d₆) δ; 1.05-1.27 (9H, m), 1.31-1.58 (6H, m), 1.92 (2H, m), 2.60 (1H, m), 3.15 (2H, d, J=6.0Hz), 3.71 (3H, s), 5.99-6.10 (1H, m), 6.74 (1H, m), 6.82 (1H, m), 6.89 (1H, m), 7.18 (1H, dd, J=8.0, 8.0Hz).

### c) Synthesis of tert-butyl {[cis-4-{[4-(aminosulfonyl)benzyl]amino}-1-(3-methoxyphenyl)cyclohexyl]methyl}carbamate

To a solution of tert-butyl {[cis-4-amino-1-(3-methoxyphenyl)-cyclohexyl]methyl}carbamate (100 mg, 0.299 mmol) in N,N-dimethylformamide (2.0 mL) were added 4-(bromomethyl)benzenesulfonamide (75 mg, 0.299 mmol) and potassium carbonate (41 mg, 0.299 mmol), and the mixture was stirred at room temperature for 2 hours. Thereto was added 4-(bromomethyl)benzenesulfonamide (37 mg, 0.149 mmol) and the mixture was stirred for 1.5 hours. Thereto was added water to quench the reaction and the reactant was extracted with ethyl acetate. The organic layer was washed with saturated aqueous sodium chloride solution, and then dried over anhydrous magnesium sulfate and filtered, and the solvent was evaporated under reduced pressure. The resulting residue was purified by silica gel column chromatography to give the title compound 100 mg as white solids.
High performance liquid chromatography/ Mass spectrometry
m/z 504.2 (M+H)
Retention time: 2.78 min.

### d) Synthesis of 4-({[cis-4-(aminomethyl)-4-(3-methoxyphenyl)-cyclohexyl]amino}methyl)benzenesulfonamide dihydrochloride

To a solution of tert-butyl {[cis-4-{[4-(aminosulfonyl)benzyl]amino}-1-(3-methoxyphenyl)cyclohexyl]methyl}carbamate (90.0 mg, 0.179 mmol) in methanol (1.0 mL) was added dropwise 4N hydrogen chloride/dioxane (1.0 mL), and the mixture was stirred at room temperature for 1.5 hours. The reaction solution was concentrated under reduced pressure, and the resulting residue was repulped with methanol/ diethylether to give the title compound 82 mg as white solids.
¹H-NMR (DMSO-d₆) δ; 1.64 (2H, m), 1.98 (4H, m), 2.24 (2H, m), 3.14 (1H, m), 3.21 (2H, s), 3.76 (3H, s), 4.32 (2H, s), 6.89 (1H, m), 6.95 (1H, m), 7.00 (1H, d, J=7.9Hz), 7.31 (1H, dd, J=7.9, 7.9Hz), 7.46 (2H, s), 7.62 (3H, s), 7.79 (2H, d, J=8.6Hz), 7.84 (2H, d, J=8.6Hz), 9.52 (2H, br).

### e) Synthesis of tert-butyl [({[cis-4-{[4-(aminosulfonyl)benzyl]amino}-1-(3-methoxyphenyl)cyclohexyl]methyl}amino)sulfonylcarbamate

To a solution of 4-({[cis-4-(aminomethyl)-4-(3-methoxyphenyl)-cyclohexyl]amino}methyl)benzenesulfonamide dihydrochloride (30 mg, 0.0630 mmol) in dichloromethane (1.0 mL) was added (tert-butoxycarbonyl){[4-(dimethyliminio)pyridin-1(4H)-yl]sulfonyl}azanide (18.9 mg, 0.0630 mmol) at 0°C, and the mixture was stirred at room temperature overnight. Thereto was added saturated aqueous sodium chloride solution to quench the reaction and the reactant was extracted with chloroform. The chloroform layer was dried over anhydrous magnesium sulfate and filtered, and the solvent was evaporated under reduced pressure. The resulting residue was purified by silica gel column chromatography to give the title compound 19.4 mg as white solids.
High performance liquid chromatography/ Mass spectrometry
m/z 583.2 (M+H)
Retention time: 2.76 min.

### f) Synthesis of 4-({[cis-4-{[(aminosulfonyl)amino]methyl}-4-(3-methoxyphenyl)cyclohexyl]amino}methyl)benzenesulfonamide hydrochloride

To a solution of tert-butyl [({[cis-4-{(4-(aminosulfonyl)benzyl]amino}-1-(3-methoxyphenyl)cyclohexyl]methyl}amino)sulfonylcarbamate (16.5 mg, 0.0283 mmol) in methanol (0.5 mL) was added dropwise 4N hydrogen chloride/dioxane (0.5 mL), and the mixture was stirred at room temperature for 3 hours. The reaction solution was concentrated under reduced pressure, and the resulting residue was repulped with diethylether/hexane to give the title compound 11.6 mg as white solids.
¹H-NMR (DMSO-d₆) δ; 1.57 (2H, m), 1.78 (2H, m), 1.98 (2H, m), 2.18 (2H, m), 3.10 (1H, m), 3.24 (2H, d, J=6.8Hz), 3.74 (3H, s), 4.25 (2H, br), 5.35 (1H, t, J=6.8Hz), 6.36 (2H, s), 6.81 (1H, dd, J=8.0, 2.2Hz), 6.86 (1H, m), 6.94 (1H, d, J=8.0Hz), 7.24 (1H, dd, J=8.0, 8.0Hz), 7.43 (2H, s), 7.75 (2H, d, J=8.3Hz), 7.85 (2H, d, J=8.3Hz), 9.14 (2H, br).

### Example 39

### Synthesis of tert-butyl ({[(cis-1-(3-methoxyphenyl)-4-{[4-(methylsulfonyl)benzyl]amino}cyclohexyl)methyl]amino}sulfonyl)carbamate

### a) Synthesis of cis-4-(aminomethyl)-N-(diphenylmethyl)-4-(3-methoxyphenyl)cyclohexanamine

To a solution of cis-4-[(diphenylmethyl)amino]-1-(3-methoxyphenyl)cyclohexanecarbonitrile (1.0 g, 2.52 mmol) in tetrahydrofuran (20 mL) was added lithium aluminum hydride (191 mg, 5.04 mmol) at 0°C, and the mixture was stirred at room temperature for one hour. Subsequently, the mixture was stirred under reflux for 2 hours and thereto were added water, 2N-aqueous sodium hydroxide solution and water successively to quench the reaction. The reaction solution was filtered on celite and the solvent was evaporated under reduced pressure to give the title compound 1.05 g as white solids.
¹H-NMR (CDCl₃) δ; 1.43 (2H, m), 1.64 (2H, m), 1.83 (2H, m), 1.99 (2H, m), 2.51 (1H, m), 2.86 (2H, s), 3.79 (3H, s), 5.00 (1H, s), 6.75 (1H, dd, J=8.0, 2.5Hz), 6.84 (1H, m), 6.91 (1H, d, J=8.0Hz), 7.19 (3H, m), 7.26 (6H, m), 7.31 (4H, m).

### b) Synthesis of tert-butyl [({[cis-4-[(diphenylmethyl)amino]-1-(3-methoxyphenyl)cyclohexyl]methyl}amino)sulfonyl]carbamate

To a solution of cis-4-(aminomethyl)-N-(diphenylmethyl)-4-(3-methoxyphenyl)cyclohexanamine (1.0 g, 2.50 mmol) in dichloromethane (20 mL) was added (tert-butoxycarbonyl){[4-(dimethyliminio)pyridin-1(4H)-yl]sulfonyl}azanide (752 mg, 2.50 mmol) and the mixture was stirred for 5 hours. Thereto was added water to quench the reaction and the reactant was extracted with chloroform. The organic layer was dried over anhydrous magnesium sulfate and filtered, and the solvent was evaporated under reduced pressure. The resulting residue was purified by silica gel column chromatography to give the title compound 1.48 g as white solids.
¹H-NMR (DMSO-d₆) δ; 1.35 (9H, s), 1.47 (4H, m), 1.65 (2H, m), 2.04 (2H, m), 2.38 (1H, m), 3.06 (2H, d, J=6.4Hz), 3.70 (3H, s), 5.03 (1H, m), 6.05 (1H, br.), 6.76 (1H, dd, J=8.2, 2.3Hz), 6.82 (1H, s), 6.89 (1H, d, J=8.2Hz), 7.16 (3H, m), 7.28 (4H, m), 7.40 (4H, m).

### c) Synthesis of tert-butyl [({[cis-4-amino-1-(3-methoxyphenyl)-cyclohexyl]methyl}amino)sulfonyl]carbamate

To tert-butyl [({[cis-4-[(diphenylmethyl)amino]-1-(3-methoxyphenyl)cyclohexyl]methyl}amino)sulfonyl]carbamate (960 mg, 1.66 mmol) in tetrahydrofuran (9.5 mL) and methanol (9.5 mL) was added 10%-palladium hydroxide-carbon (190 mg), and the mixture was stirred under an atmosphere of hydrogen (0.48 MPa) for 2 hours. Thereto was added N,N-dimethylformamide (10 mL), and tetrahydrofuran and methanol was evaporated under reduced pressure. The residue was filtered on celite, and the solvent was evaporated under reduced pressure. The resulting residue was crystallized from methanol to give the title compound 368 mg as gray solids.
¹H-NMR (DMSO-d₆) δ; 1.36 (9H, s), 1.67 (6H, m), 2.08 (2H, m), 3.05 (1H, m), 3.15 (2H, d, J=5.2Hz), 3.73 (3H, s), 6.19 (1H, m), 6.82 (1H, dd, J=8.2, 2.4Hz), 6.86 (1H, m), 6.92 (1H, d, J=8.2Hz), 7.24 (1H, dd, J=8.2, 8.2Hz), 7.93 (3H, s), 10.87 (1H, s).

### d) Synthesis of tert-butyl ({[(cis-1-(3-methoxyphenyl)-4-{[4-(methylsulfonyl)benzyl]amino}cyclohexyl)methyl]amino}sulfonyl)carbamate

To a solution of tert-butyl [({[cis-4-amino-1-(3-methoxyphenyl)-cyclohexyl]methyl}amino)sulfonyl]carbamate (60 mg, 0.145 mmol) in methanol (1.2 mL) and dichloroethane (1.0 mL) were added dropwise 4-methanesulfonylbenzaldehyde (29.4 mg, 0.160 mmol) and acetic acid (16.6 µL, 0.290 mmol) at 0°C, and the mixture was stirred for 30 min. Subsequently, thereto was added sodium cyanoborohydride (18.2 mg, 0.290 mmol), and the mixture was stirred at room temperature overnight. Thereto was added saturated aqueous sodium hydrogen carbonate solution to quench the reaction and the mixture was taken to pH = 6 to 7 with aqueous hydrochloric acid, and the reactant was extracted with chloroform. After washing with saturated aqueous sodium chloride solution, the organic layer was dried over anhydrous magnesium sulfate and filtered, and the solvent was evaporated under reduced pressure. The resulting residue was purified by silica gel column chromatography to give the title compound 14.9 mg as white solids.
¹H-NMR (DMSO-d₆) δ; 1.33 (9H, s), 1.57 (4H, m), 1.71 (2H, m), 2.08 (2H, m), 2.63 (1H, m), 3.03 (2H, d, J=6.2Hz), 3.19 (3H, s), 3.72 (3H, s), 3.96 (2H, m), 6.79 (1H, dd, J=8.0, 2.0Hz), 6.85 (1H, m), 6.91 (1H, d, J=8.0Hz), 7.22 (1H, dd, J=8.0, 8.0Hz), 7.67 (2H, d, J=8.4Hz), 7.88 (2H, d, J=8.4Hz).

### Example 40

### Synthesis of N-{[cis-4-[(biphenyl-4-ylmethyl)amino]-1-(3-ethoxyphenyl)-cyclohexyl]methyl}methanesulfonamide

### a) Synthesis of N-[cis-4-cyano-4-(3-methoxyphenyl)cyclohexyl]biphenyl-4-carboxamide

To a solution of cis-4-amino-1-(3-methoxyphenyl)cyclohexane-carbonitrile (300 mg, 1.30 mmol) in N,N-dimethylformamide (6.0 mL) were added 4-phenylbenzoic acid (284 mg, 1.43 mmol), triethylamine (218 µL, 1.56 mmol), WSC-hydrochloride (275 mg, 1.43 mmol) and 1-hydroxy benzotriazole (194 mg, 1.43 mmol) at room temperature, and the mixture was stirred at room temperature overnight. Thereto was added saturated aqueous sodium hydrogen carbonate solution to quench the reaction and the precipitated solid was collected by filtration under reduced pressure. This crude material was repulped with diethylether to give the title compound 509 mg as white solids.
¹H-NMR (DMSO-d₆) δ; 1.82 (2H, m), 1.98 (2H, m), 2.12 (4H, m), 3.78 (3H, s), 4.04 (1H, m), 6.95 (1H, dd, J=8.0, 2.3Hz), 7.11 (1H, m), 7.17 (1H, m), 7.35 (1H, dd, J=8.0, 8.0Hz), 7.40 (1H, m), 7.49 (2H, m), 7.72 (2H, d, J=7.1Jz), 7.78 (2H, d, J=8.3Hz), 7.98 (2H, d, J=8.3Hz), 8.53 (1H, d, J=8.1Hz).

### b) Synthesis of N-[cis-4-cyano-4-(3-hydroxyphenyl)cyclohexyl]biphenyl-4-carboxamide

To a solution of N-[cis-4-cyano-4-(3-methoxyphenyl)cyclohexyl]-biphenyl-4-carboxamide (410 mg, 0.999 mmol) in dichloroethane (3.0 mL) was added dropwise 1M-boron tribromide/dichloromethane (3.00 mL, 3.00 mmol) at 0°C, and the mixture was stirred at room temperature overnight. Thereto was added ice to quench the reaction and the mixture was taken to pH > 12 with aqueous sodium hydroxide solution and the mixture was stirred for 30 min. This mixture was taken to pH = 1 with aqueous hydrochloric acid and the precipitated solid was collected by filtration to give the title compound 378 mg as white solids.
¹H-NMR (DMSO-d₆) δ; 1.81 (2H, m), 1.97-2.13 (6H, m), 3.98 (1H, m), 6.75 (1H, m), 6.96 (2H, in), 7.22 (1H, dd, J=7.9, 7.9Hz), 7.41 (1H, m), 7.49 (2H, m), 7.72 (2H, m), 7.78 (2H, d, J=8.5Hz), 7.97 (2H, d, J=8.5Hz), 8.53 (1H, d, J=7.9Hz), 9.63 (1H, s).

### c) Synthesis of N-[cis-4-cyano-4-(3-ethoxyphenyl)cyclohexyl]biphenyl-4-carboxamide

To a solution of N-[cis-4-cyano-4-(3-hydroxyphenyl)cyclohexyl]-biphenyl-4-carboxamide (150 mg, 0.378 mmol) in N,N-dimethylformamide (1.5 mL) were added ethyl iodide (33.2 µL, 0.416 mmol) and potassium carbonate (78 mg, 0.567 mmol) and the mixture was stirred at room temperature for 2 hours. Thereto was further added ethyl iodide (15.1 µL, 0.189 mmol) and the mixture was stirred overnight. Thereto was added water to quench the reaction and the precipitated solid was collected by filtration to give the title compound 158 mg as white solids.
¹H-NMR (DMSO-d₆) δ; 1.33 (3H, t, J=7.0Hz), 1.84 (2H, m), 1.98 (2H, m), 2.11 (4H, m), 4.00 (1H, m), 4.06 (2H, q, J=7.0Hz), 6.92 (1H, dd, J=8.1, 2.4Hz), 7.10 (1H, m), 7.15 (1H, d, J=8.1Hz), 7.33 (2H, dd, J=8.1, 8.1Hz), 7.40 (1H, m), 7.49 (2H, m), 7.72 (2H, d, J=7.0Hz), 7.78 (2H, d, J=8.5Hz), 7.97 (2H, d, J=8.5Hz), 8.53 (1H, d, J=5.9Hz).

### d) Synthesis of N-{[cis-4-[(biphenyl-4-ylmethyl)amino]-1-(3-ethoxyphenyl)cyclohexyl]methyl}methanesulfonamide

To a solution of N-[cis-4-cyano-4-(3-ethoxyphenyl)cyclohexyl]-biphenyl-4-carboxamide (75 mg, 0.177 mmol) in tetrahydrofuran (2.0 mL) was added lithium aluminum hydride (27 mg, 0.707 mmol) at 0°C and the mixture was stirred under reflux for 4 hours. Thereto was further added lithium aluminum hydride (13.5 mg, 0.354 mmol) and the mixture was stirred for 10 hours. Thereto was further added lithium aluminum hydride (13.5mg, 0.354mmol) and the mixture was stirred for 9 hours. Thereto were added water, 2N-aqueous sodium hydroxide solution and water successively to quench the reaction. The reaction solution was filtered on celite and the solvent was evaporated under reduced pressure to give the amino product as a crude material.

The resulting crude material was dissolved in dichloromethane (1.5 mL) and thereto were added triethylamine (38 µL, 0.265 mmol) and methanesulfonyl chloride (16.8 µL, 0.211 mmol) at 0°C and the mixture was stirred at room temperature overnight. Thereto was added aqueous sodium hydrogen carbonate to quench the reaction and the reactant was extracted with chloroform. The chloroform layer was dried over anhydrous magnesium sulfate and filtered, and the solvent was evaporated under reduced pressure. The resulting residue was purified by silica gel column chromatography to give a crude material. This material was repulped with ethyl acetate/hexane to give the title compound 48.5 mg as white solids.
¹H-NMR (DMSO-d₆) δ; 1.31 (3H, t, J=6.8Hz), 1.56 (2H, m), 1.78 (2H, m), 1.94 (2H, m), 2.17 (2H, m), 2.64 (3H, s), 3.01 (1H, m), 3.27 (2H, d, J=6.4Hz), 3.99 (2H, q, J=6.8Hz), 4.16 (2H, br), 6.42 (1H, t, J=6.4Hz), 6.79 (1H, d, J=7.9Hz), 6.86 (1H, s), 6.93 (1H, d, J=7.9Hz), 7.23 (1H, dd, J=7.9, 7.9Hz), 7.38 (1H, m), 7.47 (2H, m), 7.63 (2H, m), 7.70 (4H, m), 9.06 (1H, br).

### Example 41

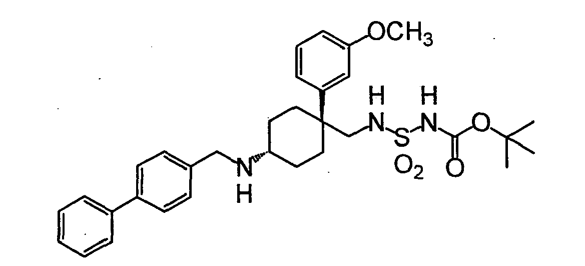

### Synthesis of tert-butyl [({[cis-4-[(biphenyl-4-ylmethyl)amino]-1-(3-methoxyphenyl)cyclohexyl]methyl}amino)sulfonyl]carbamate

To a solution of cis-4-(aminomethyl)-N-(biphenyl-4-ylmethyl)-4-(3-methoxyphenyl)cyclohexanamine (70.0 mg, 0.175 mmol) in dichloromethane (2.0 mL) was added (tert-butoxycarbonyl){[4-(dimethyl-iminio)pyridin-l(4H)-yl]sulfonyl}azanide obtained in Reference Example 30 (53 mg, 0.175 mmol) and the mixture was stirred at room temperature overnight. Thereto was added water to quench the reaction and the reactant was extracted with chloroform. This organic layer was dried over anhydrous magnesium sulfate and filtered, and the solvent was evaporated under reduced pressure. This residue was purified by silica gel column chromatography and the resulting crude material was washed with diethylether to give the title compound 55.5 mg as white solids.
¹H-NMR (DMSO-d₆) δ; 1.28 (9H, s), 1.57 (4H, m), 1.5 (2H, m), 2.16 (2H, m), 2.87 (1H, m), 3.04 (2H, d, J=6.4Hz), 3.73 (3H, s), 4.04 (2H, s), 6.79 (1H, dd, J=8.1, 2.1Hz), 6.88 (2H, m), 7.22 (1H, dd, J=8.1, 8.1Hz), 7.36 (1H, m), 7.44-7.54 (4H, m), 7.68 (4H, m).

### Example 42

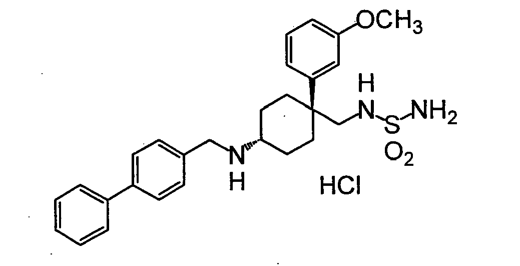

### Synthesis of N-{[cis-4-[(biphenyl-4-ylmethyl)amino]-1-(3-methoxyphenyl)cyclohexyl]methyl}sulfamide hydrochloride

To tert-butyl [({[cis-4-[(biphenyl-4-ylmethyl)amino]-1-(3-methoxyphenyl)cyclohexyl]methyl}amino)sulfonyl]carbamate obtained in Example 41 (40.0 mg, 0.0690 mmol) was added 4M-hydrogen chloride/dioxane (1.0 mL) at room temperature, and the mixture was stirred at room temperature for 2 hours. Thereto was added methanol (0.5 mL), and the mixture was stirred for additional one hour. The solvent was evaporated under reduced pressure and the residue was washed with ethyl acetate to give the title compound 35.9 mg as white solids.
¹H-NMR (DMSO-d₆) δ; 1.57 (2H, m), 1.79 (2H, m), 1.98 (2H, m), 2.18 (2H, m), 3.10 (1H, m), 3.24 (2H, d, J=6.8Hz), 3.74 (3H, s), 4.21 (2H, brs.), 5.39 (1H, t, J=6.8Hz), 6.40 (2H, s), 6.81 (1H, dd, J=8.1, 2.0Hz), 6.88 (1H, d, J=2.0Hz), 6.94 (1H, d, J=8.1Hz), 7.24 (1H, dd, J=8.1, 8.1Hz), 7.38 (1H, m), 7.48 (2H, m), 7.63-7.76 (6H, m), 9.09 (2H, brs.).

### Example 43

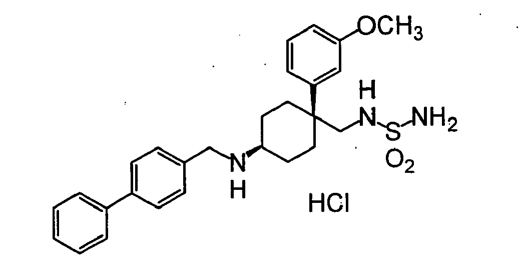

### Synthesis of N-{[trans-4-[(biphenyl-4-ylmethyl)amino]-1-(3-methoxyphenyl)cyclohexyl]methyl}sulfamide hydrochloride

### a) Synthesis of trans-4-(aminomethyl)-N-(biphenyl-4-ylmethyl)-4-(3-methoxyphenyl)cyclohexanamine

To a suspension of lithium aluminum hydride (19.1 mg, 0.504 mmol) in tetrahydrofuran (2.0 mL) was added dropwise trans-4-[(biphenyl-4-ylmethyl)amino]-1-(3-methoxyphenyl)cyclohexane-carbonitrile (100 mg, 0.252 mmol) in tetrahydrofuran (2.0 mL) under reflux and the mixture was stirred for 4 hours. Thereto were added water, 2N-aqueous sodium hydroxide solution and water successively to quench the reaction. The reaction solution was filtered on celite and the solvent was evaporated under reduced pressure. The resulting residue was purified by silica gel column chromatography to give the title compound 90.5 mg as colorless oils.
¹H-NMR (CDCl₃) δ; 1.22 (2H, m), 1.39 (2H, m), 1.86 (2H, m), 2.34 (2H, m), 2.61 (1H, m), 2.61 (2H, s), 3.80 (2H, s), 3.81 (3H, s), 6.76 (1H, dd, J=8.1, 2.0Hz), 6.89 (1H, m), 6.94 (1H, m), 7.26 (1H, dd, J=8.1, 8.1Hz), 7.35 (3H, m), 7.42 (2H, m), 7.54 (4H, m).

### b) Synthesis of tert-butyl [({[trans-4-[(biphenyl-4-ylmethyl)amino]-1-(3-methoxyphenyl) cyclohexyl] methyl}amino) sulfonyl] carbamate

To a solution of trans-4-(aminomethyl)-N-(biphenyl-4-ylmethyl)-4-(3-methoxyphenyl)cyclohexanamine (70 mg, 0.175 mmol) in dichloromethane (1.5 mL) was added (tert-butoxycarbonyl){[4-(dimethyliminio)pyridin-1(4H)-yl]sulfonyl}azanide (52.7 mg, 0.175 mmol) at 0°C and the mixture was stirred at room temperature for 2 hours. The reaction solution was concentrated under reduced pressure, and the mixture was repulped with water/methanol. The resulting mixture was repulped with methanol/ ethyl acetate again to give the title compound 55.0 mg as white solids.
High performance liquid chromatography/ Mass spectrometry
m/z 580.1 (M+H)
Retention time: 3.07 min.

### c) Synthesis of N-{[trans-4-[(biphenyl-4-ylmethyl)amino]-1-(3-methoxyphenyl)cyclohexyl]methyl}sulfamide hydrochloride

To a solution of tert-butyl [({[trans-4-[(biphenyl-4-ylmethyl)amino]-1-(3-methoxyphenyl)cyclohexyl]methyl}amino)sulfonyl]carbamate (35 mg, 0.0604 mmol) in methanol (0.5 mL) was added dropwise 4N hydrogen chloride/dioxane (0.5 mL), and the mixture was stirred at room temperature for 4 hours. The reaction solution was concentrated under reduced pressure and the resulting residue was repulped with ethyl acetate/diethylether to give the title compound 31.3 mg as white solids.
¹H-NMR (DMSO-d₆) δ; 1.33 (2H, m), 1.62 (2H, m), 2.00 (2H, m), 2.26 (2H, m), 2.76 (2H, d, J=7.1Hz), 3.05 (1H, m), 3.76 (3H, s), 4.12 (2H, s), 6.26 (1H, t, J=7.1Hz), 6.41 (2H, s), 6.85 (1H, m), 6.91 (1H, m), 7.00 (1H, d, J=8.0Hz), 7.33 (1H, dd, J=8.0, 8.0Hz), 7.37 (1H, m), 7.46 (2H, m), 7.56 (2H, d, J=8.2Hz), 7.66 (2H, m), 7.69 (2H, d, J=8.2Hz), 8.72 (2H, br.).

### Example44

### Synthesis of N-{[cis-4-[(biphenyl-4-ylmethyl)amino]-1-(3-ethoxyphenyl)-cyclohexyl]methyl}sulfamide hydrochloride

### a) Synthesis of tert-butyl [({[cis-4-[(biphenyl-4-ylmethyl)amino]-1-(3-ethoxyphenyl)cyclohexyl]methyl}amino)sulfonyl]carbamate

In the same manner as in Example 39a) b), the title compound was synthesized in 60.3 mg as white solids.
¹H-NMR (DMSO-d₆) δ; 1.29 (9H, s), 1.30 (3H, t, J=6.9Hz), 1.57 (4H, m), 1.86 (2H, m), 2.17 (2H, m), 2.95 (1H, m), 3.04 (2H, d, J=6.3Hz), 3.98 (2H, q, J=6.9Hz), 4.06 (2H, s), 6.78 (1H, m), 6.83 (1H, m), 6.89 (1H, d, J=8.1Hz), 7.20 (1H, dd, J=8.1, 8.1Hz), 7.37 (1H, m), 7.47 (2H, m), 7.55 (2H, d, J=8.0Hz), 7.69 (4H, m).

### b) Synthesis of N-{[cis-4-[(biphenyl-4-ylmethyl)amino]-1-(3-ethoxyphenyl)cyclohexyl]methyl}sulfamide hydrochloride

In the same manner as in Example 38d), the title compound was synthesized in 36.9 mg as white solids.
¹H-NMR (DMSO-d₆) δ; 1.31 (3H, t, J=7.0Hz), 1.57 (2H, m), 1.78 (2H, m), 1.98 (2H, m), 2.18 (2H, m), 3.10 (1H, m), 3.23 (2H, d, J=6.6Hz), 3.99 (2H, q, J=7.0Hz), 4.22 (2H, s), 5.38 (1H, t, J=6.6Hz), 6.40 (2H, s), 6.79 (1H, m), 6.86 (1H, m), 6.92 (1H, d, J=7.9Hz), 7.22 (1H, dd, J=7.9, 7.9Hz), 7.41 (1H, m), 7.48 (2H, m), 7.65 (2H, d, J=8.2Hz), 7.68 (2H, m), 7.74 (2H, d, J=8.2Hz), 9.06 (2H, br).

### Example 45

### Synthesis of N-[({[cis-4-[(biphenyl-4-ylmethyl)amino]-1-(3-methoxyphenyl)cyclohexyl]methyl}amino)sulfonyl]acetamide

To a solution of cis-4-(aminomethyl)-N-(biphenyl-4-ylmethyl)-4-(3-methoxyphenyl)cyclohexanamine (30 mg, 0.0749 mmol) in dichloromethane (1.0 mL) were added pyridine (12.1 µL, 0.150 mmol) and acetylsulfamoyl chloride (12.4 mg, 0.0786 mmol) obtained in Reference Example 31 at 0°C, and the mixture was stirred at room temperature overnight. Thereto was added water to quench the reaction and the reactant was extracted with chloroform. The organic layer was dried over anhydrous magnesium sulfate and filtered, and the solvent was evaporated under reduced pressure. The resulting residue was purified by thin layer chromatography, and followed by repulping with chloroform/hexane to give the title compound 15.7 mg as white solids.
¹H-NMR (DMSO-d₆) δ; 1.57 (2H, m), 1.67 (2H, m), 1.84 (3H, s), 1.90 (2H, m), 2.14 (2H, m), 2.98 (1H, m), 3.18 (2H, d, J=5.9Hz), 3.74 (3H, s), 4.14 (2H, s), 6.21 (1H, br.), 6.80 (1H, dd, J=8.1, 2.2Hz), 6.85 (1H, m), 6.91 (1H, m), 7.23 (1H, dd, J=8.1, 8.1Hz), 7.37 (1H, m), 7.47 (2H, m), 7.60 (2H, d, J=8.3Hz), 7.69 (4H, m).

### Example 46

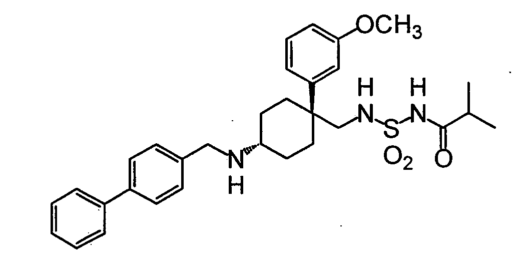

### Synthesis of N-[({[cis-4-[(biphenyl-4-ylmethyl)amino]-1-(3-methoxyphenyl)cyclohexyl]methyl}amino)sulfonyl]-2-methylpropionamide

In the same manner as in Reference Example 31 and Example 45, the title compound was synthesized in 25.9 mg as white solids.
¹H-NMR (DMSO-d₆) δ; 1.00 (6H, d, J=6.9Hz), 1.58 (2H, m), 1.72 (2H, m), 1.97 (2H, m), 2.17 (2H, m), 2.44 (1H, sep, J=6.9Hz), 3.08 (1H, m), 3.25 (2H, d, J=5.9Hz), 3.74 (3H, s), 4.21 (2H, s), 6.41 (1H, m), 6.81 (1H, dd; J=8.0, 2.0Hz), 6.85 (1H, m), 6.91 (1H, d, J=8.0Hz), 7.23 (1H, dd, J=8.0, 8.0Hz), 7.38 (1H, m), 7.48 (2H, m), 7.64 (2H, d, J=8.2Hz), 7.67 (2H, m), 7.73 (2H, d, J=8.2Hz), 8.97 (1H, br.), 11.32 (1H, br.).
High performance liquid chromatography/ Mass spectrometry
m/z 550.7 (M+H)
Retention time: 3.26 min.

### Example 47

### Synthesis of methyl [({[cis-4-[(biphenyl-4-ylmethyl)amino]-1-(3-methoxyphenyl)cyclohexyl]methyl}amino)sulfonyl]carbamate

To a solution of cis-4-(aminomethyl)-N-(biphenyl-4-ylmetliyl)-4-(3-methoxyphenyl)cyclohexanamine (30.0 mg, 0.0749 mmol) in dichloromethane (1.0 mL) was added Burgess reagent (35.7 mg, 0.150 mmol) and the mixture was stirred at room temperature for 2 hours. Thereto was added water to quench the reaction, and the reactant was extracted with chloroform. This organic layer was dried over anhydrous magnesium sulfate and filtered, and the solvent was evaporated under reduced pressure. This residue was purified by preparative thin layer chromatography and the resulting crude material was repulped with ethyl acetate/hexane to give the title compound 17.5 mg as white solids.
¹H-NMR (DMSO-d₆) δ; 1.54 (2H, m), 1.70 (2H, m), 1.96 (2H, m), 2.17 (2H, m), 3.05 (1H, m), 3.09 (2H, m), 3.37 (3H, s), 3.74 (3H, s), 4.20 (2H, s), 6.80 (1H, dd, J=8.1, 2.4Hz), 6.85 (1H, m), 6.91 (1H, m), 7.23 (1H, dd, J=8.1, 8.1Hz), 7.38 (1H, m), 7.48 (2H, m), 7.59 (2H, d, J=8.3Hz), 7.68 (2H, m), 7.74 (2H, d, J=8.3Hz).

### Example 48

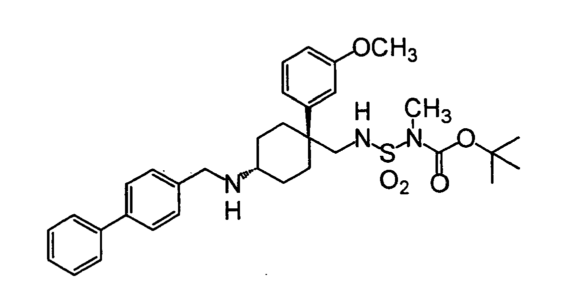

### Synthesis of tert-butyl [({[cis-4-[(biphenyl-4-ylmethyl)amino]-1-(3-methoxyphenyl)cyclohexyl]methyl}amino)sulfonyl]methylcarbamate

To a solution of tert-butyl [({[cis-4-[(biphenyl-4-ylmethyl)aminol-1-(3-methoxyphenyl)cyclohexyl]methyl}amino)sulfonyl]carbamate obtained in Example 41 (800 mg, 1.38 mmol) in tetrahydrofuran (20 mL) were added dropwise methanol (67.3 µL, 1.66 mmol), triphenyl phosphine (434 mg, 1.66 mmol) and 40%-diethyl azodicarboxylate/toluene solution (753 µL, 1.66 mmol) at 0°C, and the mixture was stirred for 30 min. After stirring at room temperature overnight, thereto was added saturated aqueous sodium hydrogen carbonate solution to quench the reaction and the reactant was extracted with ethyl acetate. The ethyl acetate layer was washed with saturated aqueous sodium chloride solution, and then dried over anhydrous magnesium sulfate and filtered, and the solvent was evaporated under reduced pressure. The resulting residue was purified by silica gel column chromatography to give the crude material. This material was repulped with methanol to give the title compound 515 mg as white solids.
¹H-NMR (DMSO-d₆) δ; 1.35 (9H, s), 1.42 (2H, m), 1.60 (4H, m), 2.07 (2H, m), 2.45 (1H, m), 3.02 (3H, s), 3.16 (2H, s), 3.72 (3H, s), 3.73 (2H, s), 6.18 (1H, m), 6.81 (1H, dd, J=8.0, 2.2Hz), 6.89 (1H, m), 6.95 (1H, d, J=8.0Hz), 7.24 (1H, dd, J=8.0, 8.0Hz), 7.33 (1H, m), 7.44 (4H, m), 7.57 (2H, d, J=8.3Hz), 7.62 (2H, m).

### Example 49

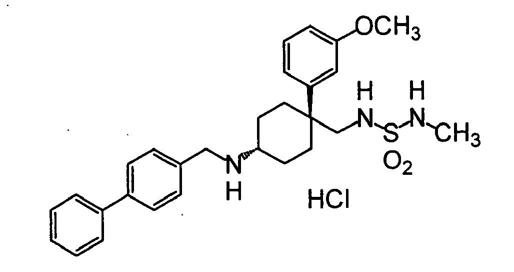

### Synthesis of N-{[cis-4-[(biphenyl-4-ylmethyl)amino]-1-(3-methoxyphenyl)cyclohexyl]methyl}-N'-methylsulfamide hydrochloride

To a solution of tert-butyl [({[cis-4-[(biphenyl-4-ylmethyl)amino]-1-(3-methoxyphenyl)cyclohexyl]methyl}amino)sulfonyl]methylcarbamate obtained in Example 48 (18.3 mg, 0.0308 mmol) in methanol (0.5 mL) was added dropwise 4N hydrogen chloride/dioxane (0.5 mL), and the mixture was stirred at room temperature for 4 hours. The reaction solution was concentrated under reduced pressure and the resulting residue was repulped with methanol/diethylether to give the title compound 16.5 mg as white solids.
¹H-NMR (DMSO-d₆) δ; 1.55 (2H, m), 1.83 (2H, m), 1.98 (2H, m), 2.11 (3H, d, J=5.1Hz), 2.20 (2H, m), 3.10 (1H, m), 3.15 (2H, m), 3.74 (3H, s); 4.22 (2H, s), 6.20 (1H, t, J=6.6Hz), 6.54 (1H, q, J=5.1Hz), 6.79 (1H, dd, J=8.1, 2.2Hz), 6.89 (1H, m), 6.95 (1H, d, J=8.1Hz), 7.23 (1H, dd, J=8.1, 8.1Hz), 7.38 (1H, m), 7.48 (2H, m), 7.65 (2H, d, J=8.2Hz), 7.68 (2H, m), 7.74 (2H, d, J=8.2Hz), 9.04 (2H, br.).

### Example 50

### Synthesis of tert-butyl [({[cis-1-(3-methoxyphenyl)-4-(4-methylpiperazin-1-yl) cyclohexyl] methyl}amino) sulfonyl] carbamate

To a suspension of lithium aluminum hydride (97.2 mg, 2.57 mmol) in tetrahydrofuran (10 mL) under reflux was added dropwise tert-butyl 4-[cis-4-cyano-4-(3-methoxyphenyl)cyclohexyl]piperazine-1-carboxylate (500 mg, 1.28 mmol) in tetrahydrofuran (10 mL). After the completion of addition, the contents was rinsed with tetrahydrofuran (2.0 mL) thoroughly and stirred for one hour. Thereto were added water, 2N-aqueous sodium hydroxide solution and water successively to quench the reaction. The reaction solution was filtered on celite and the solvent was evaporated under reduced pressure to give the amine product 400 mg.

The resulting amine product (300 mg, 0.762 mmol) was dissolved in dichloromethane (6.0 mL) and thereto was added (tert-butoxycarbonyl){[4-(dimethyliminio)pyridin-1 (4H)-yl] sulfonyl}azanide obtained in Reference Example 30 (230 mg, 0.762 mmol) at 0°C, and the mixture was stirred at room temperature for 9 hours. Thereto was added saturated aqueous sodium chloride solution was added to quench the reaction and the reactant was extracted with chloroform. The chloroform layer was dried over anhydrous magnesium sulfate and filtered, and the solvent was evaporated under reduced pressure. The resulting residue was purified by silica gel column chromatography to give the crude material. This material was repulped with diethylether to give the title compound 186 mg as white solids.
¹H-NMR (DMSO-d₆) δ; 1.32 (9H, s), 1.49 (4H, m), 1.66 (2H, m), 2.10 (2H, m), 2.29 (4H, m), 2.59 (8H, m), 3.06 (2H, d, J=6.6Hz), 3.72 (3H, s), 5.48 (1H, m), 6.79 (1H, dd, J=8.0, 2.2Hz), 6.85 (1H, m), 6.91 (1H, d, J=8.0Hz), 7.21 (1H, dd, J=8.0, 8.0Hz).
High performance liquid chromatography/Mass spectrometry
m/z 497.2 (M+H)
Retention time: 2.38 min.

### Example 51

### Synthesis of N-{[cis-1-(3-methoxyphenyl)-4-(4-methylpiperazin-1-yl)-cyclohexyl]methyl}sulfamide dihydrochloride

To a solution of tert-butyl [({[cis-1-(3-methoxyphenyl)-4-(4-methyl-piperazin-1-yl)cyclohexyl]methyl}amino)sulfonyl]carbamate obtained in Example 50 (10 mg, 0.101 mmol) in methanol (0.5 mL) was added dropwise 4N-hydrogen chloride/dioxane (1.0 mL), and the mixture was stirred at room temperature for 2 hours. The reaction solution was concentrated under reduced pressure and the resulting residue was repulped with ethyl acetate to give the title compound 58 mg as white solids.
¹H-NMR (DMSO-d₆) δ; 1.52 (2H, m), 1.80 (2H, m), 2.00 (2H, m), 2.27 (2H, m), 2.82 (3H, s), 3.20 (2H, d, J=5.9Hz), 3.30 (1H, m), 3.50 (8H, m), 3.74 (3H, s), 5.51 (1H, m), 6.42 (2H, s), 6.80 (1H, dd, J=8.1, 2.4Hz), 6.87 (1H, m), 6.93 (1H, d, J=8.1Hz), 7.23 (1H, dd, J=8.1, 8.1Hz).
High performance liquid chromatography/ Mass spectrometry
m/z 397.5 (M+H)
Retention time: 1.57 min.

### Example 52

### Synthesis of N-{[trans-4-[(1-benzylpiperidin-4-yl)amino]-1-(3-methoxyphenyl)cyclohexyl]methyl}methanesulfonamide

### a) Synthesis of N-[trans-4-(aminomethyl)-4-(3-methoxyphenyl)-cyclohexyl]- -1-benzylpiperidine-4-amine

To a suspension of lithium aluminum hydride (348 mg, 9.17 mmol) in tetrahydrofuran (28 mL) was added dropwise a solution of trans-4-[(1-benzylpiperidin-4-yl)-1-(3-methoxyphenyl)cyclohexanecarbonitrile (1.85 g, 4.58 mmol) in tetrahydrofuran (10 mL) under reflux. After a completion of addition, the contents was rinsed with tetrahydrofuran (2.0 mL) thoroughly and stirred for 2 hours. Thereto were added water, 2N-aqueous sodium hydroxide solution and water successively to quench the reaction. The reaction solution was filtered on celite and the solvent was evaporated under reduced pressure to give the title compound 1.57 g as white solids.
¹H-NMR (CDCl₃) δ; 1.12 (2H, m), 1.28 (2H, m), 1.38 (2H, m), 1.76 (4H, m), 1.97 (2H, m), 2.32 (2H, m), 2.53 (1H, m), 2.60 (2H, s), 2.67 (1H, m), 2.80 (2H, m), 3.48 (2H, s), 3.80 (3H, s), 6.74 (1H, m), 6.85 (1H, m), 6.92 (1H, m), 7.25 (6H, m).

### b) Synthesis of N-{[trans-4-[(1-benzylpiperidin-4-yl)amino]-1-(3-methoxyphenyl)cyclohexyl]methyl}methanesulfonamide

To a solution of N-[trans-4-(aminomethyl)-4-(3-methoxyphenyl)-cyclohexyl]-1-benzylpiperidine-4-amine (750 mg, 1.84 mmol) in dichloromethane (15 mL) were added triethylamine (385 µL, 2.76 mmol) and methanesulfonyl chloride (157 µL, 2.02 mmol) at 0°C and the mixture was stirred at room temperature overnight. Thereto was added aqueous sodium hydrogen carbonate solution to quench the reaction and the reactant was extracted with chloroform. The chloroform layer was dried over anhydrous magnesium sulfate and filtered, and the solvent was evaporated under reduced pressure. The resulting residue was purified by silica gel column chromatography to give the title compound 474 mg as white amorphous.
¹H-NMR (DMSO-d₆) δ; 0.89 (2H, m), 1.12 (2H, m), 1.45 (2H, m), 1.63 (4H, m), 1.87 (2H, m), 2.42 (1H, m), 2.50 (1H, m), 2.56 (3H, s), 2.66 (2H, m), 2.86 (2H, d, J=6.8Hz), 3.38 (2H, s), 3.73 (3H, s), 6.73 (1H, t, J=6.8Hz), 6.79 (1H, m), 6.86 (1H, m), 6.95 (1H, m), 7.25 (6H, m).

### Example 53

### Synthesis of 4-({4-[(cis-4-(3-methoxyphenyl)-4-{[(methylsulfonyl)amino]-methyl}cyclohexyl)amino]piperidin-1-yl}methyl)benzenesulfonamide

N-{[cis-1-(3-methoxyphenyl)-4-(piperidin-4-ylamino)cyclohexyl]-methyl}methanesulfonamide dihydrochloride (48.0 mg, 0.102 mmol) was dissolved in N,N-dimethylformamide (1.0 mL) and thereto were added potassium carbonate (42.0 mg, 0.306 mmol) and 4-(bromomethyl)-benzenesulfonamide obtained in Reference Example 32 (31mg, 0.122mmol) and the mixture was stirred at room temperature overnight. Thereto was added water to quench the reaction and the reactant was extracted with ethyl acetate. The ethyl acetate layer was washed with saturated aqueous sodium chloride solution, and then the organic layer was dried over anhydrous magnesium sulfate and filtered, and the solvent was evaporated under reduced pressure. The resulting residue was repulped with ethyl acetate to give the crude material. This material was purified by preparative thin layer chromatography to give the title compound 8.4 mg as white solids.
¹H-NMR (DMSO-d₆) δ; 1.23 (3H, m), 1.38 (2H, m), 1.56 (4H, m), 1.74 (2H, m), 1.98 (4H, m), 2.58 (1H, m), 2.61 (3H, s), 2.70 (2H, m), 3.15 (2H, d, J=6.4Hz), 3.49 (3H, s), 3.72 (3H, s), 6.36 (1H, t, J=6.4Hz), 6.78 (1H, dd, J=8.0, 2.2Hz), 6.86 (1H, m), 6.93 (1H, d, J=8.0Hz), 7.22 (1H, dd, J=8.0, 8.0Hz), 7.31 (2H, s), 7.47 (2H, d, J=8.3Hz), 7.74 (2H, d, J=8.3Hz).
High performance liquid chromatography/Mass spectrometry
m/z 565.3 (M+H)
Retention time: 2.02 min.

### Example 54

### Synthesis of N-{[cis-1-(3-methoxyphenyl)-4-(piperidin-4-ylamino)-cyclohexyl]methyl}sulfamide dihydrochloride

### a) Synthesis of cis-1-(3-methoxyphenyl)-4-[(1-tritylpiperidin-4-yl)amino]-cyclohexanecarbonitrile

To a solution of cis-1-(3-methoxyphenyl)-4-(piperidin-4-ylamino)-cyclohexanecarbonitrile dihydrochloride (311 mg, 0.805 mmol) and triethylamine (0.449 mL, 3.22 mmol) in dichloromethane (5.0 mL) was added trityl chloride (269 mg, 0.966 mmol) at room temperature and the mixture was warmed to room temperature and the mixture was stirred overnight. Thereto was added water to quench the reaction, and then the mixture was extracted with ethyl acetate and the organic layer was washed twice with water. The organic layer was dried over anhydrous magnesium sulfate, and filtered, and then the solvent was evaporated under reduced pressure to give the title compound 458 mg as colorless amorphous.

### b) Synthesis of tert-butyl {[({cis-1-(3-methoxyphenyl)-4-[(1-trityl-piperidin-4-yl) amino] cyclohexyl}methyl) amino] sulfonyl}carbamate

To a solution of cis-1-(3-methoxyphenyl)-4-[(1-tritylpiperidin-4-yl)-amino]cyclohexanecarbonitrile (80 mg, 0.144 mmol) in tetrahydrofuran (4.0 mL) was added lithium aluminum hydride (16.4 mg, 0.216 mmol) at 0°C, and the mixture was stirred under reflux for 2 hours. Thereto were added water, 2N-aqueous sodium hydroxide solution and water successively to quench the reaction. The reaction solution was filtered on celite and the solvent was evaporated under reduced pressure to give the amino product as crude material.

The resulting crude material was dissolved in dichloromethane (3.0 mL), and thereto was added (tert-butoxycarbonyl){[4-(dimethyliminio)-pyridin-1(4H)-yl]sulfonyl}azanide (43.3 mg, 0.144 mmol) at 0°C and the mixture was stirred at room temperature for 3 days. Thereto was added saturated aqueous sodium chloride solution to quench the reaction and the reactant was extracted with chloroform. The chloroform layer was dried over anhydrous magnesium sulfate and filtered, and the solvent was evaporated under reduced pressure. The resulting residue was purified by silica gel column chromatography to give the crude material in 77.0 mg. The resulting crude material was dissolved in dichloromethane (1.0 mL), and thereto was added 4-aminobutanol (5.1 µL) and the mixture was stirred overnight. Thereto was further added 4-aminobutanol (5.1 µL) and the mixture was stirred overnight. Thereto was added saturated aqueous sodium chloride solution to quench the reation and the reactant was extracted with chloroform. The chloroform layer was dried over anhydrous magnesium sulfate and filtered, and the solvent was evaporated under reduced pressure. The resulting residue was purified by silica gel column chromatography to give the title compound 62.1 mg as white solids.
¹H-NMR (DMSO-d₆) δ; 1.24 (9H, s), 1.33 (2H, m), 1.52 (4H, m), 1.79 (4H, m), 1.93 (2H, m), 2.13 (2H, m), 2.99 (6H, m), 3.71 (3H, s), 6.78 (1H, m), 6.80 (1H, m), 6.86 (1H, m) 7.17-7.33 (16H, m).

### c) Synthesis of N-{[cis-1-(3-methoxyphenyl)-4-(piperidin-4-ylamino)-cyclohexyl]methyl}sulfamide dihydrochloride

To a solution of tert-butyl {[({cis-1-(3-methoxyphenyl)-4-[(1-trityl-piperidin-4-yl)amino]cyclohexyl}methyl)amino]sulfonyl}carbamate (50.0 mg, 0.0677 mmol) in methanol (1.0 mL) was added dropwise 4N-hydrogen chloride/dioxane (1.0 mL), and the mixture was stirred at room temperature for 2 hours. The reaction solution was concentrated under reduced pressure and the resulting residue was repulped with methanol/ethyl acetate to give the title compound 25.1 mg as white solids.
¹H-NMR (DMSO-d₆) δ; 1.56 (2H, m), 1.73-1.98 (6H, m), 2.16 (4H, m), 2.92 (2H, m), 3.20 (1H, m), 3.23 (2H, d, J=6.8Hz), 3.37 (2H, m), 3.47 (1H, m), 3.73 (3H, s), 5.44 (1H, t, J=6.8Hz), 6.40 (2H, s), 6.80 (1H, d, J=8.1Hz), 6.87 (1H, s), 6.93 (1H, d, J=8.1Hz), 7.24 (1H, dd, J=8.1, 8.1Hz), 8.99 (2H, br), 9.11 (2H, br).
High performance liquid chromatography/ Mass spectrometry
m/z 397.5 (M+H)
Retention time: 0.90 min.

### Example 55

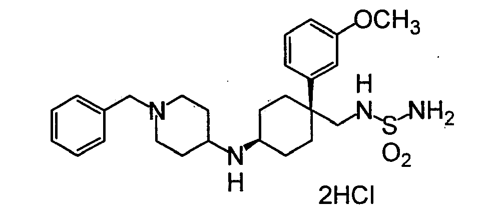

### Synthesis of N-{[trans-4-[(1-benzylpiperidin-4-yl)amino]-1-(3-methoxyphenyl)cyclohexyl]methyl}sulfamide dihydrochloride

### a) Synthesis of tert-butyl [({[trans-4-[(1-benzylpiperidin-4-yl)amino]-1-(3-methoxyphenyl)cyclohexyl]methyl}amino)sulfonyl]carbamate

To a solution of N-[trans-4-(aminomethyl)-4-(3-methoxyphenyl)-cyclohexyl]-1-benzylpiperidine-4-amine obtained in Example 52a) (750 mg, 1.84 mmol) in dichloromethane (15 mL) was added (tert-butoxycarbonyl){[4-(dimethyliminio)pyridin-1 (4H)-yl]sulfonyl}azanide obtained in Reference Example 30 (555 mg, 1.84 mmol) at 0°C and the mixture was stirred at room temperature overnight. The solvent was evaporated under reduced pressure and the resulting residue was repulped with methanol/diethylether to give the title compound 974 mg as white solids.
¹H-NMR (DMSO-d₆) δ; 1.16 (2H, m), 1.24 (9H, s), 1.34 (2H, m), 1.51 (2H, m), 1.79-1.95 (6H, m), 2.28 (2H, m), 2.58 (2H, d, J=6.8Hz), 2.76 (2H, m), 2.93 (1H, m), 3.06 (1H, m), 3.43 (2H, s), 3.73 (3H, s), 6.83 (2H, m), 6.91 (1H, d, J=7.5Hz), 7.28 (6H, m).

### b) Synthesis of N-{[trans-4-[(1-benzylpiperidin-4-yl)amino]-1-(3-methoxyphenyl)cyclohexyl]methyl}sulfamide dihydrochloride

In the same manner as in Example 49, the title compound was synthesized as white solids in 50.7 mg.
¹H-NMR (DMSO-d₆) δ; 1.17 (2H, m), 1.61 (2H, m), 1.90-2.08 (6H, m), 2.27 (2H, m), 2.76 (2H, d, J=7.2Hz), 2.90 (2H, m), 3.12 (1H, m), 3.28 (1H, m), 3.33 (2H, m), 3.74 (3H, s), 4.23 (2H, m), 6.24 (1H, t, J=7.2Hz), 6.39 (2H, s), 6.83 (1H, m), 6.87 (1H, m), 6.97 (1H, m), 7.30 (1H, dd, J=8.0, 8.0Hz), 7.46 (3H, m), 7.57 (2H, m), 8.74 (2H, br), 11.03 (1H, br).
High performance liquid chromatography/Mass spectrometry
m/z 487.4 (M+H)
Retention time: 1.69 min.

### Example 56

### Synthesis of 2,6-diisopropylphenyl {[8-(2-methoxyphenyl)-3-(3-methoxyphenyl)-8-azabicyclo[3.2.1]oct-3-yl]carbonyl}sulfamate

### a) Synthesis of diethyl cis-1-(2-methoxyphenyl)pyrrolidine-2,5-dicarboxylate

A solution of diethyl 2,5-dibromoadipic acid (8.0 g, 0.0222 mol) in toluene (25 mL) was heated to 80°C and after stopping the heating, thereto was added dropwise 2-methoxyaniline (4.1 g, 0.333 mol) over 40 min., and the mixture was stirred at 100°C for 11 hours again. The reaction solution was filtered under reduced pressure and the filtrate was concentrated under reduced pressure and the resulting residue was purified by silica gel column chromatography to give the title compound 2.08 g as white solids.
¹H-NMR (CDCl₃) δ; 1.25 (6H, t, J=7.2Hz), 2.21 (4H, m), 3.71 (3H, s), 4.21 (4H, m), 4.36 (2H, m), 6.70 (1H, m), 6.82 (3H, m).

### b) Synthesis of [cis-1-(2-methoxyphenyl)pyrrolidin-2,5-diyl]dimethanol

To a suspension of lithium aluminum hydride (354 mg, 9.33 mmol) in tetrahydrofuran (30 mL) was added dropwise a solution of diethyl cis-1-(2-methoxyphenyl)pyrrolidine-2,5-dicarboxylate (2.0 g, 6.22 mmol) in tetrahydrofuran (15 mL) over 15 min. at 0°C and then the contents was rinsed with tetrahydrofuran (5 mL) thoroughly and the mixture was stirred at room temperature for 3 hours. Thereto were added water (350 µL), 2N-aqueous sodium hydroxide solution (700 µL) and water (1.05 mL) successively and the mixture was stirred for 2 hours. After filtering on celite, the solvent was evaporated under reduced pressure. The resulting residue was purified by silica gel column chromatography to give the title compound 1.47 g as colorless oils.
¹H-NMR (CDCl₃) δ; 2.01 (4H, m), 3.08 (2H, m), 3.38 (4H, m), 3.41 (2H, m), 6.96 (1H, dd, J=8.3, 1.3Hz), 7.01 (1H, ddd, J=7.7, 7.7, 1.3Hz), 7.18 (1H, m), 7.28 (1H, dd, J=7.7, 1.7Hz).

### c) Synthesis of cis-2,5-bis(chloromethyl)-1-(2-methoxyphenyl)pyrrolidine hydrochloride

To a solution of [cis-1-(2-methoxyphenyl)pyrrolidin-2,5-diyl]-dimethanol (1.4 g, 5.90 mmol) in chloroform (14 mL) was added dropwise slowly thionyl chloride (1.54 g, 0.0130 mol) at 0°C and then the mixture was heated under reflux for 30 min. The reaction solution was concentrated under reduced pressure and the resulting residue was recrystallized from 2-propanol (7 mL) to give the the title compound 1.06 g as white crystals.
¹H-NMR (DMSO-d₆) δ; 1.86 (2H, m), 2.07 (2H, m), 3.39 (2H, dd, J=10.5, 8.5Hz), 3.54 (2H, dd, J=10.5, 3.3Hz), 3.68 (2H, m), 3.80 (3H, s), 6.86 (1H, m), 6.96-7.11(3H, m).

### d) Synthesis of cis-2,5-bis(chloromethyl)-1-(2-methoxyphenyl)-pyrrolidine

cis-2,5-bis(chloromethyl)-1-(2-methoxyphenyl)pyrrolidine hydrochloride (720 mg, 2.32 mmol) was suspended in diethylether and washed with saturated aqueous sodium hydrogen carbonate solution. The aqueous layer was extracted with diethylether again, and then the organic layer was washed with saturated aqueous sodium chloride solution. The organic layer was dried over anhydrous magnesium sulfate and the solvent was evaporated under reduced pressure to give the title compound 614 mg as pale yellow solids.
¹H-NMR (CDCl₃) δ; 1.92 (2H, m), 2.16 (2H, m), 3.27 (2H, dd, J=10.5, 8.8Hz), 3.51 (2H, dd, J=10.5, 3.3Hz), 3.72 (2H, m), 3.85 (3H, s), 6.87 (1H, ddd, J=7.5, 7.5, 1.5Hz), 6.90 (1H, m), 7.00 (1H, dd, J=7.5, 1.5Hz), 7.13 (1H, ddd, J=8.2, 7.5, 1.5Hz).

### e) Synthesis of 8-(2-methoxyphenyl)-3-(3-methoxyphenyl)-8-azabicyclo-[3.2 1]octane-3-carbonitrile

To a suspension of 55%-sodium hydride (541 mg, 0.0124 mol) in N,N-dimethylformamide (16 mL) were added dropwise a solution of cis-2,5-bis(chloromethyl)-1-(2-methoxyphenyl)pyrrolidine (810 mg, 2.95 mmol) and 3-methoxyphenylacetonitrile (870 mg, 5.91 mmol) in N,N-dimethylformamide (14 mL) over 15 min. at 0°C and the contents was rinsed with N,N-dimethylformamide (2.0 mL) thoroughly and stirred at room temperature for 2 hours. After stirring for another 3 hours at 40°C, thereto was added water to quench the reaction and the reactant was extracted with ethyl acetate. The organic layer was dried over anhydrous magnesium sulfate, and filtered and then the solvent was evaporated under reduced pressure. The resulting residue was purified by silica gel column chromatography to give the crude material. This material was washed with hexane/diethylether to give the title compound 472 mg as white solids.
¹H-NMR (CDCl₃) δ; 2.17 (2H, m), 2.32 (2H, m), 2.46-2.57 (4H, m), 3.76 (3H, s), 4.39 (2H, m), 6.80 (1H, m), 6.88 (4H, m), 6.99 (1H, dd, J=2.2, 2.2Hz), 7.08 (1H, m), 7.26 (1H, m).

### f) Synthesis of 8-(2-methoxyphenyl)-3-(3-methoxyphenyl)-8-azabicyclo-[3.2.1]octane-3-carboxamide

To a solution of 8-(2-methoxyphenyl)-3-(3-methoxyphenyl)-8-azabicyclo[3.2.1]octane-3-carbonitrile (285 mg, 0.818 mmol) in dimethyl sulfoxide (4 mL) was added dropwise 6N- aqueous potassium hydroxide solution (4 mL) at room temperature, and the mixture was stirred at 100°C for 10 hours. The reaction solution was allowed to cool to 0°C, thereto was added water (12 mL) and the solution was stirred for 30 min. This mixture was filtered under reduced pressure to give the title compound 295 mg as white solids.
¹H-NMR (DMSO-d₆) δ; 1.73 (2H, m), 1.94 (4H, m), 2.89 (2H, m), 3.69 (3H, s), 3.72 (3H, s), 4.24 (2H, m), 6.67-6.86 (7H, m), 6.92 (1H, brs.), 7.15 (1H, dd, J=8.0, 8.0Hz), 7.34(1H, brs.).

### g) Synthesis of methyl 8-(2-methoxyphenyl)-3-(3-methoxyphenyl)-8-azabicyclo[3.2.1]octane-3-carboxylate

To 8-(2-methoxyphenyl)-3-(3-methoxyphenyl)-8-azabicyclo[3.2.1]-octane-3-carboxamide (345 mg, 0.941 mmol) was added conc. hydrobromic acid (5.0 mL) at room temperature and the mixture was stirred under reflux for 14 hours. The reaction solution was concentrated under reduced pressure and the resulting residue was washed with ethyl acetate to give the hydrobromic acid salt thereof.

To a solution of the hydrobromic acid salt (470 mg) in N,N-dimethylformamide (5.0 mL) were added cesium carbonate (2.19 g, 5.65 mmol) and methyl iodide (279 µL, 3.77 mmol) at room temperature and the mixture was stirred for 4.5 hours. Thereto were added cesium carbonate (1.88 mmol) and methyl iodide (1.88 mmol) and the mixture was stirred for another 14 hours. Thereto was added water to quench the reaction, and the reactant was extracted with ethyl acetate. The organic layer was washed with saturated aqueous sodium chloride solution, and then the organic layer was dried over anhydrous magnesium sulfate and filtered, and the solvent was evaporated under reduced pressure. The resulting residue was purified by silica gel column chromatography to give the title compound 136 mg as white solids.
¹H-NMR (CDCl₃) δ; 1.81 (2H, m), 1.92 (2H, m), 2.34 (2H, m), 2.97 (2H, m), 3.66 (3H, s), 3.77 (3H, s), 3.83 (3H, s), 4.29 (2H, m), 6.74 (1H, m), 6.82 (4H, m), 6.88 (1H, m), 6.92 (1H, d, J=8.0Hz), 7.17 (1H, dd, J=8.0, 8.0Hz).

### h) Synthesis of 8-(2-methoxyphenyl)-3-(3-methoxyphenyl)-8-azabicyclo-[3.2.1]octane-3-carboxylic acid

To a solution of methyl 8-(2-methoxyphenyl)-3-(3-methoxyphenyl)-8-azabicyclo[3.2.1]octane-3-carboxylate (120 mg, 0.315 mmol) in dimethyl sulfoxide (1.2 mL) was added dropwise 6N-aqueous potassium hydroxide solution (1.2 mL) at room temperature, and the mixture was stirred at 70°C for 2 hours. Thereto was added dimethyl sulfoxide (1.2 mL) and the mixture was stirred for another 6 hours. The reaction solution was allowed to cool to room temperature, and then thereto were added water (10 mL) and 1 M-hydrochloric acid to take to pH = 7 to 8. This mixture was filtered under reduced pressure to give the title compound 95 mg as white solids.
¹H-NMR (DMSO-d₆) δ; 1.84 (4H, m), 2.05 (2H, m), 2.81 (2H, m), 3.69 (3H, s), 3.73 (3H, s), 4.28 (2H, m), 6.71-6.88 (7H, m), 7.19 (1H, dd, J=8.2, 8.2Hz), 12.56 (1H, brs.).

### i) Synthesis of 2,6-diisopropylphenyl {[8-(2-methoxyphenyl)-3-(3-methoxyphenyl)-8-azabicyclo[3.2.1]oct-3-yl]carbonyl}sulfamate

To a solution of 8-(2-methoxyphenyl)-3-(3-methoxyphenyl)-8-azabicyclo[3.2.1]octane-3-carboxylic acid (40 mg, 0.109 mmol) in dichloromethane (1 mL) were added N,N-dimethylformamide (2 µL) and oxalyl chloride (20 µL, 0.218 mmol) at 0°C and the mixture was stirred at room temperature for 2 hours. Thereto was added oxalyl chloride (10 µL, 0.109 mmol), and the mixture was stirred for another one hour. The solvent was evaporated under reduced pressure, and thereto was added toluene and the solvent was evaporated azeotropically with toluene several times and then the residue was dried under reduced pressure to give the hydrochloride salt of acid chloride.

Separately, to a solution of 2,6-diisopropylphenyl sulfamate (42 mg, 0.163 mmol) in tetrahydrofuran (1 mL) was added 55%-sodium hydride (7.1 mg, 0.163 mmol) at 0°C to prepare the solution. To this solution was added dropwise the suspension of the above-obtained acid chloride in tetrahydrofuran (1 mL) at 0°C, and then the mixture was stirred overnight. To the reaction solution was added water to quench the reaction and the mixture was extracted with ethyl acetate. This organic layer was dried over anhydrous magnesium sulfate, and filtered, and then the solvent was evaporated under reduced pressure. This residue was purified by silica gel column chromatography and followed by repulping with hexane to give the title compound 6.3 mg as white solids.
High performance liquid chromatography/Mass spectrometry
m/z 607.3 (M+H)
Retention time: 4.20 min.

### Example 57

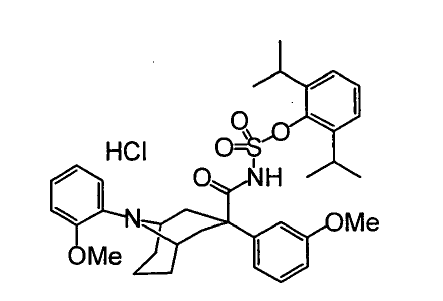

### Synthesis of 2,6-diisopropylphenyl {[9-(2-methoxyphenyl)-3-(3-methoxyphenyl)-9-azabicyclo[3.3.1]nonan-3-yl]carbonyl}sulfamate hydrochloride

### a) Synthesis of dimethyl cis-l-(2-methoxyphenyl)piperidine-2,6-dicarboxylate

To a solution of 2,6-dibromoheptanoic acid dimethylester (10.0 g, 0.0289 mol) in toluene (30 mL) was added dropwise 2-methoxyaniline (5.34 g, 0.0434 mol) at 80°C and the mixture was stirred at 100°C for 19 hours. After allowed to cool to room temperature, the mixture was filtered on celite. The solvent of the filtrate was evaporated under reduced pressure and then this residue was purified by silica gel column chromatography. The resulting crude product was recrystallized from diisopropylether to give the title compound 1.24 g as white solids.
¹H-NMR (CDCl₃) δ; 1.56 (1H, m), 1.92 (5H, m), 3.48 (6H, s), 3.86 (3H, s), 4.21 (2H, m), 6.82 (2H, m), 7.09 (1H, m), 7.15 (1H, dd, J=7.7, 1.8Hz).

### b) Synthesis of [cis-1-(2-methoxyphenyl)piperidin-2,6-diyl]dimethanol

To a suspension of lithium aluminum hydride (222 mg, 5.86 mmol) in tetrahydrofuran (20 mL) was added dropwise a solution of dimethyl cis-1-(2-methoxyphenyl)piperidine-2,6-dicarboxylate (1.20 g, 3.90 mmol) in tetrahydrofuran (8 mL) at 0°C, and the contents was rinsed with tetrahydrofuran (2 mL) thoroughly and stirred at room temperature for 4 hours. To the reaction solution were added water, 2N-aqueous sodium hydroxide solution and water successively to quench the reaction and then the mixture was filtered on celite. The solvent of the filtrate was evaporated under reduced pressure and, the resulting residue was purified by silica gel chromatography to give the title compound 1.01 g as colorless oils.
¹H-NMR (CDCl₃) δ; 1.57 (1H, m), 1.68 (2H, m), 1.95 (3H, m), 2.90 (2H, m), 3.01 (2H, dd, J=10.6, 1.4Hz), 3.11 (2H, m), 3.18 (2H, m), 3.90 (3H, s), 6.98 (1H, dd, J=8.2, 1.3Hz), 7.03 (1H, ddd, J=7.7, 7.7, 1.3Hz), 7.23 (1H, m), 7.33 (1H, dd, J=7.9, 1.6Hz).

### c) Synthesis of cis-2,6-bis(chloromethyl)-1-(2-methoxyphenyl)piperidine

To a solution of [cis-1-(2-methoxyphenyl)piperidin-2,6-diyl]-dimethanol (980 mg, 3.90 mmol) in chloroform (10 mL) was added dropwise thionyl chloride (1.02 g, 8.58 mmol) at 0°C and the mixture was stirred at room temperature for 30 min. After stirring under reflux for another 10 min., the solvent was evaporated under reduced pressure. The concentrated residue was diluted with saturated aqueous sodium hydrogen carbonate solution and the mixture was extracted with diethylether. This organic layer was dried over anhydrous magnesium sulfate, and filtered and then the solvent was evaporated under reduced pressure. This residue was purified by silica gel column chromatography to give the title compound 805 mg as pale yellow solids.
¹H-NMR (CDCl₃) δ; 1.51 (3H, m), 1.91 (1H, m), 2.00 (2H, m), 3.14 (2H, dd, J=11.0, 7.0Hz), 3.19 (2H, d, J=11.0, 2.9Hz), 3.42 (2H, m), 3.82 (3H, s), 6.89 (2H, m), 7.22 (2H, m).

### d) Synthesis of exo-9-(2-methoxyphenyl)-3-(3-methoxyphenyl)-9-azabicyclo[3.3.1]nonane-3-carbonitrile, and endo-9-(2-methoxyphenyl)-3-(3-methoxyphenyl)-9-azabicyclo[3.3. 1]nonane-3-carbonitrile

To a suspension of 55%-sodium hydride (254 mg, 5.83 mmL) in N,N-dimethylformamide (4.0 mL) were added dropwise a solution of cis-2,6-bis(chloromethyl)-1-(2-methoxyphenyl)piperidine (400 mg, 1.39 mmol) and 3-methoxyphenylacetonitrile (409 mg, 2.78 mmol) in N,N-dimethylformamide (3.0 mL) at 0°C and the contents was rinsed with N,N-dimethylformamide (1.0 mL) thoroughly and stirred at room temperature overnight. Thereto was added saturated aqueous ammonium chloride solution to quench the reaction and the mixture was extracted with ethyl acetate. This organic layer was washed with saturated aqueous sodium chloride solution and then dried over anhydrous magnesium sulfate and filtered, and the solvent was evaporated under reduced pressure. This residue was purified by silica gel column chromatography to give the title compound: endo form (218 mg, pale cerise oil) and exo form (150 mg, pale yellow solids).
exo-9-(2-methoxyphenyl)-3-(3-methoxyphenyl)-9-azabicyclo[3.3.1]-nonane-3-carbonitrile; ¹H-NMR (CDCl₃) δ; 1.69 (1H, m), 1.86 (2H, m), 2.06 (2H, m), 2.20 (1H, m), 2.34 (2H, dd, J=14.3, 2.6Hz), 2.67 (2H, dd, J=14.3, 7.5Hz), 3.62 (3H, s), 3.69 (3H, s), 4.14 (2H, m), 6.71-6.89 (5H, m), 7.02 (1H, dd, J=2.1, 2.1Hz), 7.09 (1H, m), 7.22 (1H, dd, J=8.0, 8.0Hz).
endo-9-(2-methoxyphenyl)-3-(3-methoxyphenyl)-9-azabicyclo-[3.3.1]-nonane-3-carbonitrile; ¹H-NMR (CDCl₃) δ; 1.36 (2H, m), 1.68 (1H, m), 1.90-2.13 (5H, m), 2.74 (2H, m), 3.85 (3H, s), 3.88 (3H, s), 4.34 (2H, m), 6.87 (4H, m), 6.96 (1H, m), 7.20 (2H, m), 7.33 (1H, dd, J=7.9, 7.9Hz).

### f) Synthesis of endo-9-(2-methoxyphenyl)-3-(3-methoxyphenyl)-9-azabicyclo[3.3.1]nonane-3-carboxamide

To a solution of endo-9-(2-methoxyphenyl)-3-(3-methoxyphenyl)-9-azabicyclo[3.3.1]nonane-3-carbonitrile (180 mg, 0.497 mmol) in dimethyl sulfoxide (3.6 mL) was added dropwise 6N-aqueous potassium hydroxide solution (1.8 mL) at room temperature and the mixture was stirred at 120°C for 5 hours. The reaction solution was allowed to cool to room temperature and diluted with water. This mixture was filtered under reduced pressure to give the title compound 172 mg as white solids.
¹H-NMR (DMSO-d₆) δ; 1.46 (3H, m), 1.82 (2H, m), 1.95 (1H, m), 2.45 (4H, m), 3.53 (3H, s), 3.63 (3H, s), 3.99 (2H, m), 6.59-6.74 (5H, m), 6.82 (1H, s), 6.89 (1H, m), 6.94 (1H, d, J=7.9Hz), 7.05 (1H, s), 7.10(1H, dd, J=7.9, 7.9Hz).

### g) Synthesis of methyl endo-9-(2-methoxyphenyl)-3-(3-methoxyphenyl)-9-azabicyclo [3.3.1]nonane-3-carboxylate

To endo-9-(2-methoxyphenyl)-3-(3-methoxyphenyl)-9-azabicyclo-[3.3.1]nonane-3-carboxamide (150 mg, 0.394 mmol) was added conc. aqueous hydrobromic acid (3.0mL) and the mixture was refluxed for 3 hours. The reaction solution was concentrated under reduced pressure and the resulting residue was washed with methanol/ethyl acetate/diethylether. This residue was dissolved in N,N-dimethylformamide (3.0 mL) and thereto were added methyl iodide (98 µL, 1.58 mmol) and cesium carbonate (1.03 g, 3.15 mmol) at room temperature and the mixture was stirred for 3 days. Thereto was added saturated aqueous ammonium chloride solution to quench the reaction and the reactant was extracted with ethyl acetate. After washing with saturated aqueous sodium chloride solution, the organic layer was dried over anhydrous magnesium sulfate and filtered, and the solvent was evaporated under reduced pressure. The resulting residue was purified by silica gel column chromatography to give the title compound 166 mg as colorless oils.
¹H-NMR (CDCl₃) δ; 1.63 (4H, m), 2.01 (2H, m), 2.61 (2H, dd, J=14.3, 7.3Hz), 2.71 (2H, dd, J=14.3, 2.7Hz), 3.63 (3H, s), 3.64 (3H, s), 3.70 (3H, s), 4.11 (2H, m), 6.65-6.83 (5H, m), 6.94 (1H, m), 7.00 (1H, m), 7.13 (1H, dd, J=8.0, 8.0Hz).

### h) Synthesis of endo-9-(2-methoxyphenyl)-3-(3-methoxyphenyl)-9-azabicyclo[3.3.1]nonane-3-carboxylic acid

To a solution of methyl endo-9-(2-methoxyphenyl)-3-(3-methoxyphenyl)-9-azabicyclo[3.3.1]nonane-3-carboxylate (150 mg, 0.379 mmol) in dimethyl sulfoxide (2.0mL) was added dropwise 6N-aqueous potassium hydroxide solution (1.0 mL) at room temperature and the mixture was stirred at 100°C for one hour. The reaction solution was allowed to cool under ice-cooling and diluted with water and then taken pH = 5 with conc. hydrochloric acid. This mixture was filtered under reduced pressure and washed with water to give the title compound 114 mg as pale green solids.
¹H-NMR (DMSO-d₆) δ; 1.51 (3H, m), 1.85 (3H, m), 2.41 (2H, dd, J=14.3, 7.3Hz), 2.59 (2H, m), 3.30 (3H, s), 3.65 (3H, s), 3.99 (2H, m), 6.62-6.79 (5H, m), 6.88 (1H, m), 6.97 (1H, m), 7.14 (1H, dd, J=8.0, 8.0Hz), 12.41 (1H, brs.).

### i) Synthesis of 2,6-diisopropylphenyl {[9-(2-methoxyphenyl)-3-(3-methoxyphenyl)-9-azabicyclo[3.3.1]nonan-3-yl]carbonyl}sulfamate hydrochloride

To a solution of endo-9-(2-methoxyphenyl)-3-(3-methoxyphenyl)-9-azabicyclo[3.3.1]nonane-3-carboxylic acid (50 mg, 0.131 mmol) in dichloromethane (1.0 mL) were added N,N-dimethylformamide (2 µL) and oxalyl chloride (33 µL, 0.262 mmol) at 0°C and the mixture was stirred at room temperature for 2 hours. The solvent was evaporated under reduced pressure, and thereto was added toluene and the solvent was evaporated azeotropically with toluene several times to give the hydrochloride of the acid chloride.

Separately, to a solution of 2,6-diisopropylphenyl sulfamate (101 mg, 0.393 mmol) in tetrahydrofuran (1.0 mL) was added 55%-sodium hydride (17.0 mg, 0.393 mmol) at 0°C to prepare the solution. To a suspension of the above-obtained acid chloride in tetrahydrofuran (1.0 mL) was added dropwise the above solution at 0°C and then the mixture was stirred at room temperature for 3 days. Thereto was added saturated aqueous sodium hydrogen carbonate solution to quench the reaction and the mixture was extracted with ethyl acetate. This organic layer was dried over anhydrous magnesium sulfate and filtered, and the solvent was evaporated under reduced pressure. The resulting residue was purified by silica gel column chromatography to give the mixture of title compound 60.0 mg.

To a solution of the mixture (60.0 mg, 0.0967 mmol) in ethyl acetate (1.5 mL) was added 1M-hydrogen chloride/diethylether (97 µL, 0.0967 mmol) at room temperature and the mixture was stirred for one hour. The reaction solution was evaporated under reduced pressure and thereto was added diethylether and then this mixture was filtered under reduced pressure to give the title compound 15.5 mg as white solids.
¹H-NMR (DMSO-d₆) δ; 1.06 (12H, d, J=6.8Hz), 1.47 (3H, m), 1.82 (3H, m), 2.65 (2H, m), 3.26 (2H, m), 3.59 (3H, s), 4.07 (2H, m), 6.70 (5H, m), 6.93 (2H, m), 7.14-7.26 (4H, m).

### Example 58

### Synthesis of 2,6-diisopropylphenyl {(3-methoxyphenyl)[1-(2-methoxyphenyl)piperidin-4-yl]acetyl}sulfamate

### a) Synthesis of (3-methoxyphenyl)(pyridin-4-yl)acetonitrile

To a solution of 3-methoxyphenylacetonitrile (1.03 g, 7.02 mmol) in tetrahydrofuran (20 mL) was added dropwise 1.59M n-butyllithium/hexane solution (4.42 mL, 7.02 mmol) over one hour at - 78°C and the mixture was stirred for 10 min. Thereto was added dropwise a solution of 4-bromopyridine (660 mg, 4.18 mmol) in tetrahydrofuran (10 mL) at -78°C and the contents was rinsed with tetrahydrofuran (2 mL) thoroughly. After one hour, the mixture was warmed gradually and the mixture was stirred at room temperature overnight. Thereto was added saturated aqueous ammonium chloride solution to quench the reaction and the mixture was extracted with ethyl acetate. After washing with saturated aqueous sodium chloride solution, this organic layer was dried over anhydrous magnesium sulfate and filtered, and the solvent was evaporated under reduced pressure. This resulting residue was purified by silica gel column chromatography to give the title compound 441 mg as pale yellow oils.
¹H-NMR (CDCl₃) δ; 3.81 (3H, s), 5.08 (1H, s), 6.84 (1H, m), 6.91 (2H, m), 7.30 (3H, m), 8.62 (2H, m).

### b) Synthesis of methyl (3-methoxyphenyl)(pyridin-4-yl)acetate

To (3-methoxyphenyl)(pyridin-4-yl)acetonitrile (340 mg, 1.52 mmol) was added conc. hydrochloric acid (3.4 mL) and the mixture was heated at 80°C for 2.5 hours with stirring. The reaction solution was concentrated under reduced pressure and the resulting residue was dissolved in methanol (5.5 mL) and thereto was added 4N-hydrogen chloride/dioxane (2.2 mL) and the mixture was stirred at room temperature overnight. The reaction solution was concentrated under reduced pressure and the resulting residue was diluted with water and then neutralized with saturated aqueous sodium hydrogen carbonate solution and the mixture was extracted with ethyl acetate. This organic layer was dried over anhydrous magnesium sulfate, and filtered and then the solvent was evaporated under reduced pressure. The resulting residue was purified by silica gel column chromatography to give the title compound 320 mg as colorless oils.
¹H-NMR (CDCl₃) δ; 3.77 (3H, s), 3.79 (3H, s), 4.95 (1H, s), 6.86 (3H, m), 7.26 (3H, m), 8.54 (2H, m).

### c) Synthesis of methyl (3-methoxyphenyl)(piperidin-4-yl)acetate

To a solution of methyl (3-methoxyphenyl)(pyridin-4-yl)acetate (300 mg, 1.17 mmol) in acetic acid (7 mL) was added platinum oxide (44 mg) and the mixture was subjected to an atmosphere of hydrogen at 4 atmosphere and the mixture was stirred at room temperature for 3 hours. The reaction solution was filtered on celite and the filtrate was concentrated under reduced pressure. The resulting residue was purified by silica gel column chromatography to give the title compound 305 mg as colorless oils.
¹H-NMR (CDCl₃)δ; 0.94 (1H, m), 1.21 (1H, m), 1.26 (1H, m), 1.76 (1H, m), 2.08 (1H, m), 2.49 (1H, m), 2.64 (1H, m), 2.95 (1H, m), 3.07 (1H, m), 3.21 (1H, d, J=8.8Hz), 3.65 (3H, s), 3.80 (3H, s), 6.81 (1H, m), 6.89 (2H, m), 7.20 (1H, dd, J=8.3, 8.3Hz).

### d) Synthesis of methyl (3-methoxyphenyl)[1-(2-methoxyphenyl)-piperidin-4-yl]acetate

To a solution of methyl (3-methoxyphenyl)(piperidin-4-yl)acetate (130 mg, 0.494 mmol) in toluene (6 mL) were added 2-bromoanisole (68 uL, 0.543 mmol), tridibenzylideneacetone dipalladium (13.6 mg, 0.0148 mmol), rac-BINAP (18.4 mg, 0.0296 mmol) and sodium t-butoxide (142 mg, 1.48 mmol) at room temperature and the mixture was stirred at 100°C for one hour. The reaction solution was diluted with ethyl acetate and thereto was added saturated aqueous ammonium chloride solution and the reactant was extracted. The organic layer was dried over anhydrous magnesium sulfate and filtered, and the solvent was evaporated under reduced pressure. The resulting residue was purified by silica gel column chromatography to give the title compound 75 mg as pale yellow oils.
¹H-NMR (CDCl₃) δ; 1.32 (2H, m), 1.64 (1H, m), 1.88 (1H, m), 2.13 (1H, m), 2.44 (1H, m), 2.61 (1H, m), 3.27 (1H, d, J=10.6Hz), 3.35 (1H, m), 3.42 (1H, m), 3.67 (3H, s), 3.81 (3H, s), 3.84 (3H, s), 6.79-7.00 (7H, m), 7.24 (1H, dd, J=8.1, 8.1Hz).

### e) Synthesis of (3-methoxyphenyl)[1-(2-methoxyphenyl)piperidin-4-yl]acetic acid

To a solution of methyl (3-methoxyphenyl)[1-(2-methoxyphenyl)-piperidin-4-yl]acetate (72 mg, 0.195 mmol) in tetrahydrofuran (0.5 mL) and methanol (0.5 mL) was added 2M-aqueous lithium hydroxide solution (487 µL, 0.974 mmol) at room temperature and the mixture was stirred at 50°C for 12 hours. The mixture was taken to pH = 4 with 1M aqueous hydrochloric acid at room temperature and this mixture was diluted with water and then the reactant was extracted with ethyl acetate. The organic layer was dried over anhydrous magnesium sulfate and filtered, and the solvent was evaporated under reduced pressure. The resulting residue was purified by silica gel column chromatography to give the title compound 49 mg as pale yellow amorphous.
¹H-NMR (CDCl₃) δ; 1.39 (2H, m), 1.66 (1H, m), 1.98 (1H, m), 2.14 (1H, m), 2.45 (1H, m), 2.61 (1H, m), 3.28 (1H, d, J=10.7Hz), 3.36 (1H, m), 3.47 (1H, m), 3.81 (3H, s), 3.83 (3H, s), 6.81-7.01 (7H, m), 7.25 (1H, dd, J=7.9, 7.9Hz).

### f) Synthesis of 2,6-diisopropylphenyl {(3-methoxyphenyl)[1-(2-methoxyphenyl)piperidin-4-yl]acetyl}sulfamate

To a solution of (3-methoxyphenyl)[1-(2-methoxyphenyl)piperidin-4-yl]acetic acid (45 mg, 0.127 mmol) in dichloromethane (2 mL) were added N,N-dimethylformamide (3 µL) and oxalyl chloride (23 µL, 0.253 mmol) at 0°C and the mixture was stirred at room temperature for 1.5 hours. The solvent was evaporated under reduced pressure, and thereto was added toluene and the solvent was evaporated azeotropically with toluene several times and dried under reduced pressure. The resulting residue was dissolved in dichloromethane (2 mL) and thereto were added 2,6-diisopropylphenyl sulfamate (39 mg, 0.152 mmol) and triethylamine (71 µL, 0.506 mmol) at 0°C and the mixture was stirred at room temperature for 3 hours. Thereto was added triethylamine (71 µL, 0.506 mmol) and the mixture was stirred overnight. The reaction solution was concentrated under reduced pressure and diluted with saturated aqueous sodium hydrogen carbonate solution and then the mixture was extracted with diethylether. This organic layer was dried over anhydrous magnesium sulfate and then the solvent was evaporated under reduced pressure. This residue was purified by preparative thin layer chromatography and then repulped with hexane to give the title compound 16.0 mg as pale brown solids.
High performance liquid chromatography/ Mass spectrometry
m/z 595.3 (M+H)
Retention time: 3.47 min.

### Example 59

### Synthesis of 2,6-diisopropylphenyl [(3-methoxyphenyl)(1-pyrimidin-2-ylpiperidin-4-yl)acetyl]sulfamate

### a) Synthesis of methyl (3-methoxyphenyl)(1-pyrimidin-2-ylpiperidin-4-yl)acetate

To methyl (3-methoxyphenyl)(piperidin-4-yl)acetate obtained in Example 58c) (130 mg, 0.494 mmol) in N,N-dimethylformamide (2.5 mL) solution were added 2-chloropyrimidine (57 mg, 0.494 mmol) and potassium carbonate (205 mg, 1.48 mmol) at room temperature and the mixture was stirred at 70°C for 3 hours. The reaction was quenched with saturated aqueous ammonium chloride solution and the mixture was extracted with ethyl acetate. This organic layer was dried over anhydrous magnesium sulfate, and filtered and then the solvent was evaporated under reduced pressure. This residue was purified by silica gel column chromatography to give the title compound 164 mg as colorless oils.
¹H-NMR (CDCl₃) δ; 1.05 (1H, ddd, J=16.5, 12.3, 4.4Hz), 1.28 (1H, ddd, J=16.5, 12.3, 4.4Hz), 1.37 (1H, m), 1.86 (1H, m), 2.28(1H, m), 2.77 (1H, ddd, J=13.2. 13.2, 2.8Hz), 2.95 (1H, ddd, J=13.2. 13.2, 2.8Hz), 3.20 (1H, d, J=10.7Hz), 3.67 (3H, s), 3.81 (3H, s), 4.68 (1H, m), 4.76 (1H, m), 6.43 (1H, dd, J=4.7, 4.7Hz), 6.84 (1H, m), 6.89 (2H, m), 7.24 (1H, dd, J=8.1, 8.1Hz), 8.26 (2H, d, J=4.7Hz).

### b) Synthesis of (3-methoxyphenyl)(1-pyrimidin-2-ylpiperidin-4-yl)acetic acid

To a solution of methyl (3-methoxyphenyl)(1-pyrimidin-2-ylpiperidin-4-yl)acetate (150 mg, 0.439 mmol) in dimethyl sulfoxide (2 mL) was added dropwise 6N-aqueous potassium hydroxide solution (1 mL) at room temperature and the mixture was stirred at 40°C for one hour. The reaction solution was diluted with water and then taken to pH = 4 to 5 with conc. hydrochloric acid. This mixture was extracted with ethyl acetate and the organic layer was dried over anhydrous magnesium sulfate, and filtered and then the solvent was evaporated under reduced pressure. This residue was purified by silica gel column chromatography to give the title compound 137 mg as colorless oils.
¹H-NMR (CDCl₃) δ; 1.05 (1H, m), 1.28 (1H, m), 1.36 (1H, m), 1.95 (1H, m), 2.25 (1H, m), 2.78 (1H, m), 2.92 (1H, m), 3.21 (1H, d, J=10.6Hz), 3.80 (3H, s), 4.61 (1H, m), 4.70 (1H, m), 6.44 (1H, dd, J=4.8, 4.8Hz), 6.84 (1H, m), 6.91 (2H, m), 7.24 (1H, dd, J=8.1, 8.1Hz), 8.28 (2H, d, J=4.8Hz).

### c) Synthesis of 2,6-diisopropylphenyl [(3-methoxyphenyl)(1-pyrimidin-2-ylpiperidin-4-yl)acetyl]sulfamate

To a solution of (3-methoxyphenyl)(1-pyrimidin-2-ylpiperidin-4-yl)acetic acid (120 mg, 0.367 mmol) in dichloromethane (3 mL) were added N,N-dimethylformamide (3 µL) and oxalyl chloride (66 µL, 0.733 mmol) at 0°C and the mixture was stirred at room temperature for 2 hours. The solvent was evaporated under reduced pressure and thereto was added toluene and the solvent was evaporated azeotropically with toluene several times and the residue was dried under reduced pressure to give the hydrochloride salt of the acid chloride. This material was dissolved in dichloromethane (3 mL) and thereto were added 2,6-diisopropylphenyl sulfamate (113 mg, 0.440 mmol) and triethylamine (204 µL, 1.47 mmol) at 0°C and the mixture was stirred at room temperature for 1.5 hours. Thereto was added triethylamine (204 µL, 1.47 mmol) and the mixture was stirred overnight. The reaction solution was evaporated under reduced pressure, and to the resulting residue was added saturated aqueous ammonium chloride solution and the mixture was extracted with ethyl acetate. This organic layer washed with saturated aqueous sodium chloride solution, and then dried over anhydrous magnesium sulfate and filtered, and the solvent was evaporated under reduced pressure. This residue was purified by silica gel column chromatography and then repulped with diethylether/hexane to give the title compound 81.1 mg as white solids.
¹H-NMR (CDCl₃) δ; 1.08 (12H, m), 1.28 (3H, m), 2.07 (1H, m), 2.40 (1H, m), 2.75 (1H, m), 2.92 (1H, m), 3.18 (1H, d, J=9.9Hz), 3.22 (2H, m), 3.78 (3H, s), 4.65 (1H, m), 4.76 (1H, m), 6.44 (1H, dd, J=4.8, 4.8Hz), 6.86 (3H, m), 7.11 (2H, m), 7.16 (1H, dd, J=8.6, 6.4Hz), 7.22 (1H, m), 8.26 (2H, d, J=4.8Hz).

### Testing Examples

The effect on an expression of a LDL receptor of the compounds of the present invention was evaluated by the following method.

### 1. Measurement of an expression of a LDL receptor

The activity of potentiating an expression of a LDL receptor by the test compounds were measured using a human hepatic cell line HepG2. Cells were seeded in a 6 well plate at the density of 7.5×10⁵ cells/well (at first day). On third days, the medium was changed to a medium containing lipoprotein-deficient serum and the test compound. On forth days, the cells were scraped and collected by a centrifugation. The recovered cells were dissolved in a cell lysate buffer containing 0.1% TritonX-100 and after centrifugation, the supernatant was used as a solution of cell protein. The amount of the LDL-R protein was evaluated by an immunoblotting method using the above-prepared solution of cell protein. The result thus obtained was showed in the Figure 1.

After treating the HepG2 cell with the compound of Example 1-2, the amount of LDL receptor protein was detected by an immunoblotting method.

The compound of Example 1-2 at 0.1 and 1 µM showed the superior increasing effect on the amount of a LDL receptor protein compared to a control group.

### 2. Effects on an activity of a LDL receptor by the compounds of the present invention in a cultured human hepatic cell line

The 1,1'-dioctadecyl-3,3,3',3'-tetramethylindocarbocyanine perchlorate (DiI)-labeled LDL (DiI-LDL) was prepared as follows. That is, a DiI (Invitrogen Corp., U.S.A) was mixed with a human LDL (Chemicon International, Inc., U.S.A) and the mixture was incubated at 37°C for 8 hours. After adding a density solution (d=1.182) and mixing, the mixture was subjected to a ultracentrifugation (32k rpm, 14 hours; using a Beckman Ti55.2 rotor). After centrifugation, the DiI-labeled LDL fraction was separated and dialyzed with a physiological saline to prepare the above desired solution.

The effects on a LDL receptor activity were determined as the uptake amount of DiI-LDL into HepG2 cell. The cultured human hepatic cell line HepG2 cell was purchased from the Dainippon Pharmaceuticals Co. Ltd. (Osaka, Japan). The HepG2 cells were seeded in a 96 well plate and were incubated in the Dulbecco's modified Eagle/F-12 (DMEM/F-12) medium containing 10% fetal bovine serum and antibiotics in a CO₂ incubator at 37°C for two to three days. After washing the cells, a DMEM/F-12 medium containing 5 -10% lipoprotein-deficient serum, the test substrate and the antibiotics were added thereto and the cells were incubated at 37°C for 19 hours. After adding DiI-LDL, the cells were incubated in a CO₂ incubator at 37°C for another 5 hours. The uptake amount of non-specific DiI-LDL was measured by adding 30 to 50 folds amount of the non-labeled LDL. After washing the cells with Dulbecco's phosphate buffer (Sigma Corp., U.S.A.), the Dulbecco's phosphate buffer was added to each well and then the uptake amount of DiI-LDL into the cell line were measured as the value of fluorescence. After measuring the fluorescence, the Dulbecco's phosphate buffer was removed and thereto was added 1N sodium hydroxide solution to dissolve the cells and the amount of protein was measured by using a part of the resulting solution. The effect on a LDL receptor activity by each compound was determined by calculating the ratio of the amount of fluorescence / the amount of protein, and by subtracting the non-specific uptake amount therefrom and then calculating the control group as 100%.

As showed in table 1 and 2, the compounds of the Examples of the present invention increased the uptake amount of DiI-LDL into HepG2 cell. Thus, the test compounds enhanced the function of a LDL receptor.

**Table 1**

| Example compounds | (Concentration) | Activity of (%) LDL receptor |
|---|---|---|
| 1-2 | (10 µM) | 124 |
| 1-8 | (10 µM) | 134 |
| 1-17 | (10 µM) | 120 |
| 1-19 | (10 µM) | 132 |
| 2-1 | (10µM) | 140 |
| 3-2 | (10 µM) | 154 |
| 10-3 | (10 µM) | 162 |
| 12-2 | (10 µM) | 142 |
| 13-1 | (10 µM) | 129 |
| 15-1 | (10 µM) | 192 |
| 15-2 | (10 µM) | 223 |
| 18-1 | (10 µM) | 230 |
| 18-2 | (10 µM) | 161 |
| 18-3 | (10 µM) | 154 |
| 20 | (1 µM) | 128 |
| 22 | (3 µM) | 156 |

**Table 2**

| Example compounds | (Concentration) | Activity of (%) LDL receptor |
|---|---|---|
| 24 | (1 µM) | 238 |
| 25 | (3 µM) | 153 |
| 27-1 | (10µM) | 108 |
| 28-1 | (10µM) | 186 |
| 28-6 | (10 µM) | 127 |
| 34 | (10 µM) | 139 |
| 36 | (10 µM) | 122 |
| 38 | (10 µM) | 150 |
| 41 | (10 µM) | 173 |
| 42 | (1 µM) | 188 |
| 43 | (3µM) | 146 |
| 48 | (1 µM) | 145 |
| 49 | (1 µM) | 208 |
| 55 | (10 µM) | 140 |
| 57 | (3 µM) | 124 |

### INDUSTRIAL APPLICABILITY

The present compound of formula (1), a prodrug thereof or an acid addition salt of the same can exhibit the high effect of potentiating an expression of a LDL receptor in a human hepatic cell line HepG2, and thereby promote an expression of a LDL receptor protein directly or indirectly. Thus, the compounds of the present invention are useful for prevention or treatment of hyperlipidemia, as well as prevention and/or treatment of arteriosclerosis or various diseases related to arteriosclerosis such as cerebral infarction, cerebral thrombosis, transient cerebral hemorrhage, cardiac angina, myocardial infarction, peripheral thrombus, and obstruction, etc.

## Claims

1. An agent for potentiating an expression of a low density lipoprotein receptor comprising a compound represented by the formula (1): 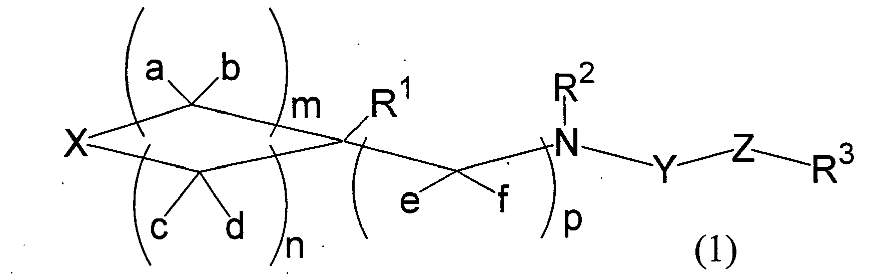 wherein
m, n and p are independently an integer of 0 to 4 with the proviso that 3 ≦ m + n ≦ 8;
X is
(i) an oxygen atom,
(ii) a sulfur atom,
(iii) a group of the formula: NR⁴
(R⁴ is a hydrogen atom; a substituted or unsubstituted alkyl group; a substituted or unsubstituted alkenyl group; a substituted or unsubstituted alkynyl group; a substituted or unsubstituted cycloalkyl group; a substituted or unsubstituted aryl group; a substituted or unsubstituted heteroaryl group; a substituted or unsubstituted arylalkyl group; a substituted or unsubstituted heteroarylalkyl group; a saturated heterocyclic group having 3 to 8 carbon atoms and one - NR¹⁰⁰- or one oxygen atom as ring-forming members (wherein a substituent may optionally present on the carbon atom of said saturated heterocyclic group); a group of the formula: -C(=O)R⁷ (R⁷ is a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, or a substituted or unsubstituted heteroarylalkyl group); a group of the formula: -C(=O)NR⁸R⁹ (R⁸ and R⁹ are independently a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, or a substituted or unsubstituted heteroarylalkyl group); a group of the formula: -C(=O)OR¹⁰ (R¹⁰ is a substituted or unsubstituted alkyl group, a substituted or unsubstituted alkenyl group, a substituted or unsubstituted alkynyl group, a substituted or unsubstituted cycloalkyl group, a substituted or unsubstituted aryl group, or a substituted or unsubstituted heteroaryl group); or a group of the formula: -SO₂R^{10a} (R^{10a} is a substituted or unsubstituted alkyl group, a substituted or unsubstituted alkenyl group, a substituted or unsubstituted alkynyl group, a substituted or unsubstituted cycloalkyl group, a substituted or unsubstituted aryl group, or a substituted or unsubstituted heteroaryl group)),
(iv) a group of the formula: CR⁵R⁶
(R⁵ and R⁶ are independently a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, or a substituted or unsubstituted heteroarylalkyl group, or alternatively R⁵ and R⁶ may combine to form a benzylidene group; or
when R⁵ is a hydrogen atom, R⁶ is a group of the following:
(1) the formula: -NR¹¹R¹² (R¹¹ and R¹² are independently a hydrogen atom; a substituted or unsubstituted alkyl group; a substituted or unsubstituted alkenyl group; a substituted or unsubstituted alkynyl group; a substituted or unsubstituted cycloalkyl group; a substituted or unsubstituted aryl group; a substituted or unsubstituted heteroaryl group; a substituted or unsubstituted arylalkyl group; a substituted or unsubstituted heteroarylalkyl group; a group of the formula: -C(=O)R¹⁰¹; a group of the formula: -SO₂-R¹⁰²; or a saturated heterocyclic group having 3 to 8 carbon atoms and one - NR¹⁰⁰- or one oxygen atom as ring-forming members (wherein a substituent may optionally present on the carbon atom of said saturated heterocyclic group); or alternatively R¹¹ and R¹² may combine together with the adjacent nitrogen atom to form a saturated heterocyclic group having 3 to 8 carbon atoms and optionally further one -NR¹⁰⁰- or one oxygen atom as ring-forming members (wherein a substituent may optionally present on the carbon atom of said saturated heterocyclic group));
(2) a group of the formula: -OR¹³ (R¹³ is a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, or a substituted or unsubstituted heteroarylalkyl group); or
(3) a group of the formula: -SR¹⁴ (R¹⁴ is a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, or a substituted or unsubstituted heteroarylalkyl group);
R¹, R² and R³ are independently a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylcarbonyl group, a substituted or unsubstituted heteroarylcarbonyl group, a substituted or unsubstituted arylsulfonyl group, a substituted or unsubstituted heteroarylsulfonyl group, a substituted or unsubstituted arylalkyl group, or a substituted or unsubstituted heteroarylalkyl group;
Y is a group of the formula: -SO₂-, a group of the formula: - P(O)OR¹⁵- (R¹⁵ is a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, or a substituted or unsubstituted heteroarylalkyl group), or a group of the formula: -C(=O)-;
Z is
(i) an oxygen atom,
(ii) a sulfur atom,
(iii) a group of the formula: -NR¹⁶ (R¹⁶ is a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, a substituted or unsubstituted heteroarylalkyl group, a group of the formula: -C(=O)R¹⁰¹, or a group of the formula: -C(=O)OR¹⁰³), or
(iv) a group of the formula: -(CH₂)_{q}- (q is an integer of 1 to 4),
with the proviso that when Z is a group of the formula: -NR¹⁶, then R³ and R¹⁶ may combine together with the adjacent nitrogen atom to form a saturated heterocyclic group having 2 to 8 carbon atoms and optionally further one oxygen atom as ring-forming members (wherein a substituent may optionally present on the carbon atom of said saturated heterocyclic group);
a, b, c, d, e and f are
(i) the same or different or alternatively independently when two or more exist, a hydrogen atom, a hydroxy group, a substituted or unsubstituted alkyl group, a substituted or unsubstituted alkoxy group, a substituted or unsubstituted alkoxycarbonyl group, a substituted or unsubstituted aryl group, a substituted or unsubstituted heteroaryl group, a substituted or unsubstituted arylcarbonyl group, a substituted or unsubstituted heteroarylcarbonyl group, a substituted or unsubstituted arylalkyl group, a substituted or unsubstituted heteroarylalkyl group, a substituted or unsubstituted arylalkyloxy group, or a substituted or unsubstituted heteroarylalkyloxy group;
(ii) one or more of the combination selected from the group consisting of the combinations of a and b, c and d, and e and f may be independently an oxo group;
(iii) the combination of e and f may be a thioxo group;
(iv) a and c may combine to form an alkylene group;
(v) when any two of a, b, c, d, e or f exist on the adjacent carbon atom, they may form a double bond, or alternatively
when R¹, and any one of a, b, c, d, e or f exist on the adjacent carbon atom, they may form a double bond;
each R¹⁰⁰, when two or more exist, is independently a hydrogen atom, a substituted or unsubstituted alkyl group, a substituted or unsubstituted aryl group, a substituted or unsubstituted heteroaryl group, a substituted or unsubstituted alkoxycarbonyl group, a substituted or unsubstituted arylalkyl group, or a substituted or unsubstituted heteroarylalkyl group;
R¹⁰¹, R¹⁰² and R¹⁰³ may be the same or different or alternatively independently when two or more exist, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, or a substituted or unsubstituted arylalkyl group;
with the proviso that when Y is a group of the formula: -C(=O), then -Z-R³ is not a substituted or unsubstituted alkoxy group]
or a prodrug thereof or a pharmaceutically acceptable salt of the same as an active ingredient.

2. The agent for potentiating an expression of a low density lipoprotein receptor according to claim 1, wherein the agent is an agent for treating hyperlipidemia or arteriosclerosis.

3. A compound represented by the formula (1): 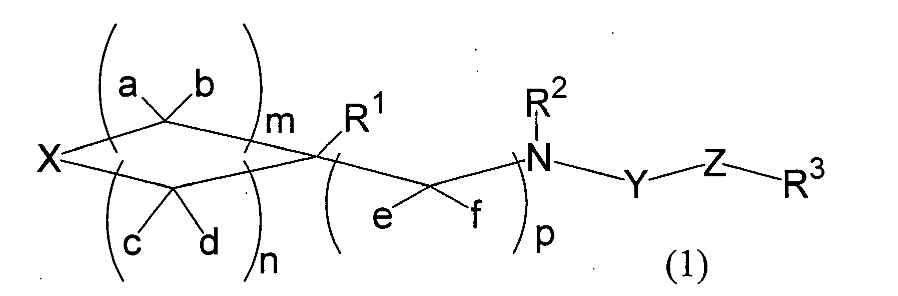 wherein
m, n and p are independently an integer of 0 to 4 with the proviso that 3≦m + n≦8;
X is
(i) an oxygen atom,
(ii) a sulfur atom,
(iii) a group of the formula: NR⁴
(R⁴ is a hydrogen atom; a substituted or unsubstituted alkyl group; a substituted or unsubstituted alkenyl group; a substituted or unsubstituted alkynyl group; a substituted or unsubstituted cycloalkyl group; a substituted or unsubstituted aryl group; a substituted or unsubstituted heteroaryl group; a substituted or unsubstituted arylalkyl group; a substituted or unsubstituted heteroarylalkyl group; a saturated heterocyclic group having 3 to 8 carbon atoms and one - NR¹⁰⁰- or one oxygen atom as ring-forming members (wherein a substituent may optionally present on the carbon atom of said saturated heterocyclic group); a group of the formula: -C(=O)R⁷ (R⁷ is a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, or a substituted or unsubstituted heteroarylalkyl group); a group of the formula: -C(=O)NR⁸R⁹ (R⁸ and R⁹ are independently a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, or a substituted or unsubstituted heteroarylalkyl group); a group of the formula: -C(=O)OR¹⁰ (R¹⁰ is a substituted or unsubstituted alkyl group, a substituted or unsubstituted alkenyl group, a substituted or unsubstituted alkynyl group, a substituted or unsubstituted cycloalkyl group, a substituted or unsubstituted aryl group, or a substituted or unsubstituted heteroaryl group); or a group of the formula: -SO₂R^{10a} (R^{10a} is a substituted or unsubstituted alkyl group, a substituted or unsubstituted alkenyl group, a substituted or unsubstituted alkynyl group, a substituted or unsubstituted cycloalkyl group, a substituted or unsubstituted aryl group, or a substituted or unsubstituted heteroaryl group)),
(iv) a group of the formula: CR⁵R⁶
(R⁵ and R⁶ are independently a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, or a substituted or unsubstituted heteroarylalkyl group, or alternatively R⁵ and R⁶ may combine to form a benzylidene group; or
when R⁵ is a hydrogen atom, R⁶ is a group of the following:
(1) a group of the formula: -NR¹¹R¹² (R¹¹ and R¹² are independently a hydrogen atom; a substituted or unsubstituted alkyl group; a substituted or unsubstituted alkenyl group; a substituted or unsubstituted alkynyl group; a substituted or unsubstituted cycloalkyl group; a substituted or unsubstituted aryl group; a substituted or unsubstituted heteroaryl group; a substituted or unsubstituted arylalkyl group; a substituted or unsubstituted heteroarylalkyl group; a group of the formula: -C(=O)R¹⁰¹; a group of the formula: -SO₂-R¹⁰²; or a saturated heterocyclic group having 3 to 8 carbon atoms and one - NR¹⁰⁰- or one oxygen atom as ring-forming members (wherein a substituent may optionally present on the carbon atom of said saturated heterocyclic group); or alternatively R¹¹ and R¹² may combine together with the adjacent nitrogen atom to form a saturated heterocyclic group having 3 to 8 carbon atoms and optionally further one -NR¹⁰⁰- or one oxygen atom as ring-forming members (wherein a substituent may optionally present on the carbon atom of said saturated heterocyclic group));
(2) a group of the formula: -OR¹³ (R¹³ is a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, or a substituted or unsubstituted heteroarylalkyl group); or
(3) a group of the formula: -SR¹⁴ (R¹⁴ is a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, or a substituted or unsubstituted heteroarylalkyl group);
R¹, R² and R³ are independently a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylcarbonyl group, a substituted or unsubstituted heteroarylcarbonyl group, a substituted or unsubstituted arylsulfonyl group, a substituted or unsubstituted heteroarylsulfonyl group, a substituted or unsubstituted arylalkyl group, or a substituted or unsubstituted heteroarylalkyl group;
Y is a group of the formula: -SO₂-, a group of the formula: - P(O)OR¹⁵- (R¹⁵ is a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, or a substituted or unsubstituted heteroarylalkyl group), or a group of the formula: -C(=O)-;
Z is
(i) an oxygen atom,
(ii) a sulfur atom,
(iii) a group of the formula: -NR¹⁶ (R¹⁶ is a hydrogen atom, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, a substituted or unsubstituted arylalkyl group, a substituted or unsubstituted heteroarylalkyl group, a group of the formula: -C(=O)R¹⁰¹, or a group of the formula: -C(=O)OR¹⁰³), or
(iv) a group of the formula: -(CH₂)_{q}- (q is an integer of 1 to 4),
with the proviso that when Z is a group of the formula: -NR¹⁶, then R³ and R¹⁶ may combine together with the adjacent nitrogen atom to form a saturated heterocyclic group having 2 to 8 carbon atoms and optionally further one oxygen atom as ring-forming members (wherein a substituent may optionally present on the carbon atom of said saturated heterocyclic group);
a, b, c, d, e and f are
(i) the same or different or alternatively independently when two or more exist, a hydrogen atom, a hydroxy group, a substituted or unsubstituted alkyl group, a substituted or unsubstituted alkoxy group, a substituted or unsubstituted alkoxycarbonyl group, a substituted or unsubstituted aryl group, a substituted or unsubstituted heteroaryl group, a substituted or unsubstituted arylcarbonyl group, a substituted or unsubstituted heteroarylcarbonyl group, a substituted or unsubstituted arylalkyl group, a substituted or unsubstituted heteroarylalkyl group, a substituted or unsubstituted arylalkyloxy group, or a substituted or unsubstituted heteroarylalkyloxy group;
(ii) one or more of the combination selected from the group consisting of the combinations of a and b, c and d, and e and f may be independently an oxo group;
(iii) the combination of e and f may be a thioxo group;
(iv) a and c may combine to form an alkylene group;
(v) when any two of a, b, c, d, e or f exist on the adjacent carbon atom, they may form a double bond, or alternatively
when R¹, and any of a, b, c, d, e or f exist on the adjacent carbon atom, they may form a double bond;
each R¹⁰⁰, when two or more exist, is independently a hydrogen atom, a substituted or unsubstituted alkyl group, a substituted or unsubstituted aryl group, a substituted or unsubstituted heteroaryl group, a substituted or unsubstituted alkoxycarbonyl group, a substituted or unsubstituted arylalkyl group, or a substituted or unsubstituted heteroarylalkyl group;
R¹⁰¹, R¹⁰² and R¹⁰³ may be the same or different or alternatively independently when two or more exist, a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, or a substituted or unsubstituted arylalkyl group;
with the proviso that when Y is a group of the formula: -C(=O), then -Z-R³ is not a substituted or unsubstituted alkoxy group] or a prodrug thereof or a pharmaceutically acceptable salt of the same.

4. The compound according to claim 3, wherein R¹ is a substituted or unsubstituted aryl group, a substituted or unsubstituted heteroaryl group, a substituted or unsubstituted arylcarbonyl group, a substituted or unsubstituted heteroarylcarbonyl group, a substituted or unsubstituted arylsulfonyl group, a substituted or unsubstituted heteroarylsulfonyl group, a substituted or unsubstituted arylalkyl group, or a substituted or unsubstituted heteroarylalkyl group, or a prodrug thereof or a pharmaceutically acceptable salt of the same.

5. The compound according to claim 3, wherein R¹ is a substituted or unsubstituted phenyl group, a substituted or unsubstituted pyridyl group, a substituted or unsubstituted benzyl group, a substituted or unsubstituted benzoyl group, a substituted or unsubstituted pyridylmethyl group, a substituted or unsubstituted pyridylcarbonyl group, or a substituted or unsubstituted benzenesulfonyl group, or a prodrug thereof or a pharmaceutically acceptable salt of the same.

6. The compound according to claim 3, wherein R¹ is a substituted or unsubstituted phenyl group or a substituted or unsubstituted benzenesulfonyl group, or a prodrug thereof or a pharmaceutically acceptable salt of the same.

7. The compound according to claim 3, wherein R¹ is a substituted or unsubstituted phenyl group, or a prodrug thereof or a pharmaceutically acceptable salt of the same.

8. The compound according to claim 3, wherein R¹ is a hydrogen atom, and at least one of a, b, c, d, e and f are a substituted or unsubstituted aryl group, a substituted or unsubstituted heteroaryl group, a substituted or unsubstituted arylcarbonyl group, a substituted or unsubstituted heteroarylcarbonyl group, a substituted or unsubstituted arylalkyl group, or a substituted or unsubstituted heteroarylalkyl group, or a prodrug thereof or a pharmaceutically acceptable salt of the same.

9. The compound according to any one of claim 3 to 8, wherein X is a group of the formula: NR⁴, or a prodrug thereof or a pharmaceutically acceptable salt of the same.

10. The compound according to claim 9, wherein R⁴ is a substituted or unsubstituted aryl group or a substituted or unsubstituted heteroaryl group, or a prodrug thereof or a pharmaceutically acceptable salt of the same.

11. The compound according to claim 9, wherein R⁴ is a phenyl group, a pyridyl group, or a pyrimidinyl group, and these group may be optionally substituted, or a prodrug thereof or a pharmaceutically acceptable salt of the same.

12. The compound according to any one of claim 3 to 8, wherein X is a group of the formula: CR⁵R⁶, or a prodrug thereof or a pharmaceutically acceptable salt of the same.

13. The compound according to claim 12, wherein R⁵ is a hydrogen atom and R⁶ is a group of the formula: -NR¹¹R¹², or a prodrug thereof or a pharmaceutically acceptable salt of the same.

14. The compound according to claim 13, wherein R¹¹ and R¹² are independently a hydrogen atom; a substituted or unsubstituted alkyl group; a substituted or unsubstituted aryl group; a substituted or unsubstituted heteroaryl group; a substituted or unsubstituted arylalkyl group; a substituted or unsubstituted heteroarylalkyl group; or a saturated heterocyclic group having 3 to 8 carbon atoms and one - NR¹⁰⁰- as ring-forming members (R¹⁰⁰ is a hydrogen atom, a substituted or unsubstituted alkyl group, a substituted or unsubstituted aryl group, a substituted or unsubstituted heteroaryl group, a substituted or unsubstituted alkoxycarbonyl group, a substituted or unsubstituted arylalkyl group, or a substituted or unsubstituted heteroarylalkyl group) or one oxygen atom (wherein a substituent may optionally present on the carbon atom of said saturated heterocyclic group), or a prodrug thereof or a pharmaceutically acceptable salt of the same.

15. The compound according to claim 14, wherein R¹¹ is a saturated heterocyclic group having as ring-forming members 3 to 8 carbon atoms and one -NR¹⁰⁰- (R¹⁰⁰ is a hydrogen atom, a substituted or unsubstituted alkyl group, a substituted or unsubstituted aryl group, or a substituted or unsubstituted arylalkyl group) or one oxygen atom (wherein a substituent may optionally present on the carbon atom of said saturated heterocyclic group), or a prodrug thereof or a pharmaceutically acceptable salt of the same.

16. The compound according to claim 14, R¹¹ is a substituted or unsubstituted arylalkyl group, or a prodrug thereof or a pharmaceutically acceptable salt of the same.

17. The compound according to any one of claim 3 to 16, wherein m is 2 or 3 and n is 2, or a prodrug thereof or a pharmaceutically acceptable salt of the same.

18. The compound according to claim 17 wherein m is 2, or a prodrug thereof or a pharmaceutically acceptable salt of the same.

19. The compound according to any one of claim 3 to 16, wherein R¹ is a hydrogen atom, and one of a, b, c and d is a substituted or unsubstituted aryl group, a substituted or unsubstituted heteroaryl group, a substituted or unsubstituted arylcarbonyl group, a substituted or unsubstituted heteroarylcarbonyl group, a substituted or unsubstituted arylalkyl group, or a substituted or unsubstituted heteroarylalkyl group, or a prodrug thereof or a pharmaceutically acceptable salt of the same.

20. The compound according to claim 19, wherein m is 3 and n is 1, or a prodrug thereof or a pharmaceutically acceptable salt of the same.

21. The compound according to any one of claim 3 to 16, wherein R¹ is a hydrogen atom, and a partial structural formula (2): 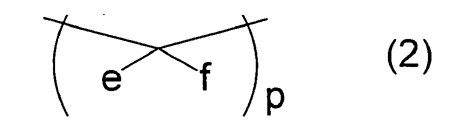 of formula (1) is the group of any the following formula: 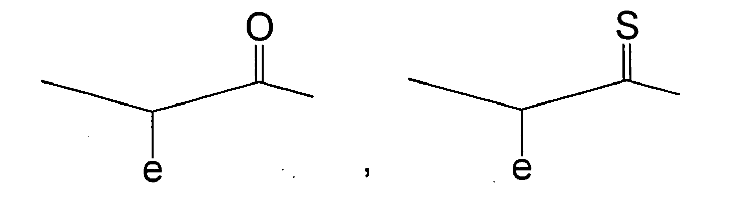 wherein e is a substituted or unsubstituted aryl group, a substituted or unsubstituted heteroaryl group, a substituted or unsubstituted arylcarbonyl group, a substituted or unsubstituted heteroarylcarbonyl group, a substituted or unsubstituted arylalkyl group, or a substituted or unsubstituted heteroarylalkyl group, or a prodrug thereof or a pharmaceutically acceptable salt of the same.

22. The compound according to any one of claim 3 to 16, wherein a and c may combine to form an alkylene group, or a prodrug thereof or a pharmaceutically acceptable salt of the same.

23. The compound according to any one of claim 3 to 16, wherein p is 1, and e and f combine to form a thioxo group, or a prodrug thereof or a pharmaceutically acceptable salt of the same.

24. The compound according to any one of claim 3 to 16, wherein p is 0, 1, or 2, and e and f are independently a hydrogen atom or a substituted or unsubstituted alkyl group, or a prodrug thereof or a pharmaceutically acceptable salt of the same.

25. The compound according to claim 24 wherein p is 1, or a prodrug thereof or a pharmaceutically acceptable salt of the same.

26. The compound according to any one of claim 3 to 25, wherein R² is a hydrogen atom, or a prodrug thereof or a pharmaceutically acceptable salt of the same.

27. The compound according to any one of claim 3 to 26, wherein Y is a group of the formula: -SO₂- group, or a prodrug thereof or a pharmaceutically acceptable salt of the same.

28. The compound according to any one of claim 3 to 26, wherein Y is a group of the formula: -P(O)OR¹⁵-, or a prodrug thereof or a pharmaceutically acceptable salt of the same.

29. The compound according to claim 28, wherein R¹⁵ is an alkyl group, or a prodrug thereof or a pharmaceutically acceptable salt of the same.

30. The compound according to claim 28, wherein R¹⁵ is a substituted or unsubstituted aryl group or a substituted or unsubstituted heteroaryl group, or a prodrug thereof or a pharmaceutically acceptable salt of the same.

31. The compound according to claim 28, wherein R¹⁵ is a substituted or unsubstituted phenyl group, or a prodrug thereof or a pharmaceutically acceptable salt of the same.

32. The compound according to any one of claim 3 to 31, wherein Z is an oxygen atom, or a prodrug thereof or a pharmaceutically acceptable salt of the same.

33. The compound according to any one of claim 3 to 31, wherein Z is a group of the formula: -NR¹⁶, or a prodrug thereof or a pharmaceutically acceptable salt of the same.

34. The compound according to claim 33, wherein R¹⁶ is a hydrogen atom, a substituted or unsubstituted alkyl group, a substituted or unsubstituted cycloalkyl group, a substituted or unsubstituted aryl group, a substituted or unsubstituted heteroaryl group, a substituted or unsubstituted arylalkyl group, a substituted or unsubstituted heteroarylalkyl group, a group of the formula: -C(=O)R¹⁰¹ or a group of the formula: -C(=O)OR¹⁰² (R¹⁰¹ and R¹⁰² are independently a substituted or unsubstituted alkyl group, a substituted or unsubstituted alkenyl group, a substituted or unsubstituted alkynyl group, a substituted or unsubstituted cycloalkyl group, a substituted or unsubstituted aryl group, a substituted or unsubstituted heteroaryl group, or a substituted or unsubstituted arylalkyl group); or alternatively R³ and R¹⁶ combine together with the adjacent nitrogen atom to form a saturated heterocyclic group having 2 to 8 carbon atoms and optionally one oxygen atom as ring-forming members (wherein a substituent may optionally present on the carbon atom of said saturated heterocyclic group), or a prodrug thereof or a pharmaceutically acceptable salt of the same.

35. The compound according to claim 34, wherein R¹⁶ is a group of the formula: -C(=O)R¹⁰¹ and R¹⁰¹ is a substituted or unsubstituted alkyl group, a substituted or unsubstituted cycloalkyl group, a substituted or unsubstituted aryl group, a substituted or unsubstituted heteroaryl group, or a substituted or unsubstituted arylalkyl group, or a prodrug thereof or a pharmaceutically acceptable salt of the same.

36. The compound according to claim 34, wherein R¹⁶ is a group of the formula: -C(=O)OR¹⁰² and the R¹⁰² is a substituted or unsubstituted alkyl group, a substituted or unsubstituted cycloalkyl group, a substituted or unsubstituted aryl group, a substituted or unsubstituted heteroaryl group, or a substituted or unsubstituted arylalkyl group, or a prodrug thereof or a pharmaceutically acceptable salt of the same.

37. The compound according to any one of claim 3 to 36, wherein Z is a group of the formula: -(CH₂)_{q}-, or a prodrug thereof or a pharmaceutically acceptable salt of the same.

38. The compound according to claim 37 wherein q is 0 or 1, or a prodrug thereof or a pharmaceutically acceptable salt of the same.

39. The compound according to any one of claim 3 to 38, wherein R³ is a substituted or unsubstituted alkyl group, a substituted or unsubstituted aryl group, a substituted or unsubstituted heteroaryl group, a substituted or unsubstituted arylalkyl group, or a substituted or unsubstituted heteroarylalkyl group, or a prodrug thereof or a pharmaceutically acceptable salt of the same.

40. The compound according to any one of claim 3 to 38, wherein R³ is a hydrogen atom, a substituted or unsubstituted aryl group, or a substituted or unsubstituted heteroaryl group, or a prodrug thereof or a pharmaceutically acceptable salt of the same.

41. The compound according to any one of claim 3 to 38, wherein R³ is a hydrogen atom or a substituted or unsubstituted phenyl group, or a prodrug thereof or a pharmaceutically acceptable salt of the same.

42. A pharmaceutical composition comprising a compound according to any one of 3 to 41 or a prodrug thereof or a pharmaceutically acceptable salt of the same as an active ingredient.

43. A method for treating hyperlipidemia or arteriosclerosis, which comprises administering a therapeutically effective amount of a compound according to any one of claim 3 to 41, or a prodrug thereof or a pharmaceutically acceptable salt of the same to a patient in need thereof.

44. A use of a compound according to any one of claim 3 to 41, or a prodrug thereof or a pharmaceutically acceptable salt of the same in preparation of an agent for treating hyperlipidemia or arteriosclerosis.
